(19) Europäisches Patentamt
European Patent Office
Office européen des brevets

(11) EP 1 501 929 B1

(12) **EUROPÄISCHE PATENTSCHRIFT**

(45) Veröffentlichungstag und Bekanntmachung des Hinweises auf die Patenterteilung:
**27.03.2013 Patentblatt 2013/13**

(21) Anmeldenummer: **03732319.3**

(22) Anmeldetag: **06.05.2003**

(51) Int Cl.:
***C12N 15/11*** *(2006.01)*

(86) Internationale Anmeldenummer:
**PCT/EP2003/004746**

(87) Internationale Veröffentlichungsnummer:
**WO 2003/093472 (13.11.2003 Gazette 2003/46)**

(54) **CGRP BINDENDE NUKLEINSÄUREN**

CGRP BINDING NUCLEIC ACIDS

ACIDES NUCLEIQUES LIANT LE CGRP

(84) Benannte Vertragsstaaten:
**AT BE BG CH CY CZ DE DK EE ES FI FR GB GR HU IE IT LI LU MC NL PT RO SE SI SK TR**

(30) Priorität: **06.05.2002 DE 10220188**
**04.11.2002 DE 10251246**

(43) Veröffentlichungstag der Anmeldung:
**02.02.2005 Patentblatt 2005/05**

(73) Patentinhaber: **Noxxon Pharma AG**
**10589 Berlin (DE)**

(72) Erfinder:
- **VATER, Axel**
**13467 Berlin (DE)**
- **MAASCH, Christian**
**13509 Berlin (DE)**
- **JAROSCH, Florian**
**13469 Berlin (DE)**
- **BELL, Mathias**
**10437 Berlin (DE)**
- **HELMLING, Steffen**
**Narbeth, PA 19072 (US)**
- **ESCHGFÄLLER, Bernd**
**90491 Nürnberg (DE)**
- **MOYROUD, Elisabeth**
**4056 Basel (CH)**
- **STARK, Sandra**
**10587 Berlin (DE)**
- **KLUSSMANN, Sven**
**10709 Berlin (DE)**
- **RUPPERT, Thorsten**
**13509 Berlin (DE)**
- **SCHIENE, Klaus**
**41363 Jüchen (DE)**
- **BAHRENBERG, Gregor**
**52156 Montschau (DE)**
- **GILLEN, Clemens**
**4730 Raeren (BE)**

(74) Vertreter: **Bohmann, Armin K.**
**bohmann**
**Anwaltssozietät**
**Nymphenburger Strasse 1**
**80335 München (DE)**

(56) Entgegenhaltungen:
**WO-A-92/14843**

- **EDVINSSON L ET AL: "Characterisation of the effects of a non-peptide CGRP receptor antagonist in SK-N-MC cells and isoalted human cerebral arteries" EUROPEAN JOURNAL OF PHARMACOLOGY, AMSTERDAM, NL, Bd. 415, 9. März 2001 (2001-03-09), Seiten 39-44, XP002210408 ISSN: 0014-2999**
- **RUSCONI C P ET AL: "RNA aptamers as reversible antagonsits of coagulation factor IXa" NATURE, MACMILLAN JOURNALS LTD. LONDON, GB, Bd. 419, 5. September 2002 (2002-09-05), Seiten 90-94, XP002956811 ISSN: 0028-0836**
- **BRAIN S.D. ET AL.,: "CGRP receptors:a headache to study,but will antagonists prove therapeutic in migraine" TRENDS IN PHARMACOLOGICAL SCIENCES, Bd. 23, Nr. 2, Februar 2002 (2002-02), Seite 51-53, XP002254636**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- FAMULOK M ET AL: "APTAMERS AS TOOLS IN MOLECULE BIOLOGY AND IMMUNOLOGY" CURRENT TOPICS IN MICROBIOLOGY AND IMMUNOLOGY, SPRINGER, BERLIN,, DE, Bd. 243, 1999, Seiten 123-136, XP002952168 ISSN: 0070-217X
- WILLIAMS KELLY P ET AL: "Bioactive and nuclease-resistant L-DNA ligand of vasopressin" PROCEEDINGS OF THE NATIONAL ACADEMY OF SCIENCES OF USA, NATIONAL ACADEMY OF SCIENCE. WASHINGTON, US, Bd. 94, Nr. 21, 1997, Seiten 11285-11290, XP002185340 ISSN: 0027-8424

## Beschreibung

Schon Hippokrates beschrieb die visuellen Symptome von Migräne, und auch der ägyptischen Medizin waren diese Kopfschmerzen, wie Papyrusfunde bezeugen, nicht unbekannt. Während des 17. Jahrhunderts wurde erkannt, daß die Gefäße des Körpers eine bedeutende Rolle beim Migränekopfschmerz spielen. Es war schon damals bekannt, daß Migräne, neben der Schwere der Attacken, benigne und erblich ist, und von den Jahreszeiten, vom Luftdruck und vom Verzehr bestimmter Nahrungsmittel abhängt (Willis, T., Cerebri anatome, Martin Allestry, London, 1664). Nach Willis (Willis, T., Cerebri anatome, Martin Allestry, London, 1664) wird Kopfschmelz durch einen sich langsam entwickelnden Vasospasmus, der in der Peripherie des Gefäßsystems beginnt, ausgelöst. Ein weiterer wichtiger Aspekt der Migräne-Pathophysiologie tauchte im 19.Jahrhundert auf. Liveing (Liveing, E., Churchill, London, 1873, 1-512) ordnete Migräne einer Dysfunktion des Gehirns zu, die durch "*nerve storms*" innerhalb des Gehirns entsteht. Er glaubte, daß eine Beziehung zwischen Migräne und Epilepsie bestehe, da beide durch Entladungen des Zentralen Nervensystems bedingt sind. Die Studien von Ray und Wolff (Ray, B.S. et al., 1940, Arch. Surg., 41, 813-856) demonstrieren, daß nur die großen cerebralen Arterien der Schädelbasis und die meningealen (duralen) Arterien und Venen sensitiv für schädliche Stimuli sind. Das lenkte das Interesse zu den intercranialen Gefäßwänden als putative Schmerzquelle bei Kopfschmerzen.

Nach der IHS-Klassifikation von 1998 gibt es hauptsächlich zwei Gruppen von Migräne: Migräne mit und Migräne ohne Aura. Die frühere Einteilung in einfache, klassische und komplizierte Migräne wurde aufgegeben.

Die vorliegende Erfindung betrifft eine CGRP bindende L-Nukleinsäure, die Verwendung einer L-Nukleinsäure als ein Antagonist von CGRP und/oder des CGRP Rezeptorsystems, die Verwendung einer L-Nukleinsäure für die Herstellung eines Medikaments, eine Zusammensetzung umfassend eine L-Nukleinsäure, einen Komplex umfassend CGRP und eine L-Nukleinsäure, die Verwendung einer L-Nukleinsäure zum in vitro-Nachweis von CGRP, ein Verfahren zum Screenen von CGRP-Antagonisten, ein Verfahren zum Screenen von CGRP-Agonisten, ein Kit für den Nachweis von CGRP und eine L-Nukleinsäure zur Verwendung in einem Verfahren zur Behandlung einer Erkrankung.

Migräne wird heute verstanden als eine neurovaskuläre Funktionsstörung, von der etwa 10 % der erwachsenen Bevölkerung betroffen sind. Charakterisiert ist diese Erkrankung durch Attacken intensiver wiederkehrender Kopfschmerzen (Doods, H., 2001, Current opinion in investigational Drugs 2(9), 1261-1268), Übelkeit und übertriebene Sensitivität gegenüber externen Stimuli wie Licht und Geräuschen. Die oftmals halbseitigen Kopfschmerzen (Hemikranie) sind pulsierend und pochend und treten in der Regel einseitig auf. Häufig wechselt die Seite der Kopfschmerzen von einem Migräneanfall zum anderen oder sogar während einer Attacke. Die Attacke wird oft von weiteren Symptomen begleitet. Die Betroffenen klagen über Appetitlosigkeit, Übelkeit und Erbrechen. Sie sind besonders licht- und lärmempfindlich und reagieren extrem sensibel auf Gerüche und zeigen Nerven- und Sehstörungen. Zwischen den Migräneattacken verschwinden die Kopfschmerzen. Vor und nach den Attacken können sich Stimmung und Appetit, der Flüssigkeitshaushalt und die Darmfunktion verändern.

Die Pathophysiologie der Migräne steht immer noch unter Diskussion. Die vorherrschende derzeitige Meinung ist, daß Migräne eine neurovaskuläre Erkrankung ist, deren Auslösemechanismus möglicherweise im ZNS lokalisiert ist. Diese Auslösung führt letztendlich zur Vasodilatation mit einer nachfolgenden Aktivierung trigeminaler afferenter senorischer Neurone und zentraler 'nociceptiver' Neurone in höheren Schmerzzentren (Hargreaves, R.J. et al., 1999, Can. J. Neurol. Sci. 26, 512-519). Es gibt einige Belege und Hinweise, die die Beteiligung von CGRP bei Migräne bestätigen. CGRP ist abundant in trigeminalen sensorischen Nerven und einer der potentesten bekannten Vasodilatatoren. Des weiteren ist der CGRP1 Rezeptor auf endothelialen Zellen der meningealen Arterien exprimiert. Es wird angenommen, daß CGRP eine Schlüsselrolle als Mediator der meningealen Vasodilatation einnimmt. Neben diesen physiologischen Betrachtungen gibt es einige Tierstudien, die eine Beteiligung des CGRP1-Rezeptors in der Migräne stützen. Die Stimulation des trigeminalen Ganglions in anästhesierten Ratten führt zur meningealen Vasodilatation, welche durch den $\alpha$CGRP-Antagonisten CGRP8-37 inhibiert werden kann (Kurosawa, M. et al., 1995, Br. J. Pharmacol. 114, 1397-1402). Dieses Experiment impliziert einen erhöhten CGRP-Level nach trigeminaler Stimulation, was bereits 1993 von Goadsby und Edvinsson beschrieben wurde (Goadsby, P. et al., 1993, Ann. Neurol., 33, 48-56).

Die Analyse von CGRP-Plasmakonzentrationen in humanen Probanden ergab, daß die Konzentration von CGRP im Plasma ein wichtiger Parameter in der Migränediagnostik ist. Es wurden erhöhte Plasmakonzentrationen in Patienten mit akuter Migräne, in Patienten mit Cluster-Kopfschmerz (engl. *cluster headache*) und in Menschen nach trigeminaler Stimulation (Edvinsson, L. et al., 1994, Cephalalgia 14, 320-327) gefunden. In Übereinstimmung mit den gerade beschriebenen Tierstudien können die erhöhten CGRP-Konzentrationen, die in Migränepatienten beschrieben werden, durch Sumatriptan oder Dihydroergotamin reduziert werden (Goadsby, P. et al., .1993, Ann. Neurol. 33, 48-56).

Im Rahmen der Therapie von Migräne, insbesondere akuter Migräne und vor Ausbildung des Vollbilds des Migräne-Kopfschmerzes, gilt als Mittel der Wahl immer noch die Einnahme von 1 -1,5 g Acetylsalicylsäure (z. B. Aspirin). Zusätzlich können zu Acetylsalicylsäure noch 1 bis 1,5 g Paracetamol gegeben werden. Alternativen zu Acetylsalicylsäure stellen NSAIDs, d. h. nicht-steroidale anti-inflammatorische Wirkstoffe, und nicht-steroidale Antirheumatika, wie z. B. Naproxen, Diclofenac oder Ibuprofen dar.

Als besonders wirksam haben sich bei der Behandlung von Migräne die sog. Triptane erwiesen, die derzeit die wohl potentesten Therapeutika zur Akutbehandlung mittelschwerer bis schwerer Migräneattacken darstellen (Tepper, S.J. et al., 1999, CNS Drugs 12, 403-417; Doods, H., 2001, Current opinion in investigational Drugs 2(9), 1261-1268).

Aufgrund ihrer hohen Spezifität für $5HT_{1B/D}$-Rezeptoren ermöglichen sie auch bei Langzeitanwendung eine sehr effektive und unter Beachtung der Kontraindikationen insgesamt auch nebenwirkungsarme, sichere Therapie akuter Migräneattacken. Betrachtet man die Risiken und sekundären Kosten durch die z. T. gravierenden Ergotamin-Nebenwirkungen bzw. insuffizient behandelten Migräneattacken, erscheint der Einsatz dieser zur Zeit zwar teuersten, aber auch potentesten Migräneakuttherapeutika in vielen Fällen gerechtfertigt. Im für Migräne so bedeutsamen trigeminovaskulären System hemmen die Triptane auch die Freisetzung der vasoaktiven und algogenen Neuropeptide (CGRP, Neurokinine) aus nociceptiven trigeminalen Nervenendigungen und verhindern dadurch die Initiierung bzw. Aufrechterhaltung der neurogenen Entzündung perivaskulär, im besonderen der Dura-Arterien, dem vermutlichen Entstehungsort des Migräne-Kopfschmerzes. Nach der erfolgreichen Einführung von Sumatriptan wurden einige neue Triptane entwickelt und am Markt zugelassen. Diese unterscheiden sich hauptsächlich im Beginn der Wirkung und der oralen Bioverfügbarkeit. Obwohl die Triptane wirksam sind und gut toleriert werden, gibt es Limitationen für diese Substanzklasse. Das Auftreten von Brustdruck, Enge/Anspannung und Angst deuten oft auf Angina pectoris in bis zu 15% der Patienten hin (Doods, H., 2001, Current opinion in investigational Drugs 2(9), 1261-1268; Brown, E.G. et al. Eur. Neurol., 31, 339-344). Der Gebrauch von Sumatriptan wird auch mit Myokardinfarkt (Ottervanger, J.P. et al., 1993, Lancet, 341, 861-862) und Herzstillstand in cardiovaskulären Risikopatienten (Kelly, K.M., 1995, Neurology, 45, 1211-1213) in Verbindung gebracht. Außerdem wurde beobachtet, daß die Gabe von Sumatriptan, Rizatriptan (Merck & Co. Inc.) und Zolmitriptan (Glaxo Wellcome plc / AstraZeneca plc) eine geringfügige Blutdruckerhöhung induziert (De Hoon, J.N.J.M. et al., 2000, Clin. Pharmacol. Ther., 68, 418-426). Die vasokonstriktorische Wirksamkeit der Triptane auf das Koronarsystem konnte *in vitro* und *in vivo* deutlich gezeigt werden, obwohl es den Anschein hat, daß die Triptane selektiv auf cerebrale Gefäße wirken (Doods, H., 2001, Current opinion in investigational Drugs 2(9), 1261-1268).

Eine weitere Gruppe von grundsätzlich zu verwendenden Wirkstoffen sind die Ergotamine.

Die Freisetzung von CGRP bei primären Kopfschmerzen, die Pharmakologie des trigeminovaskularen Systems, das Konzept der neurogenen Entzündung (Moskowitz, M.A et al., 1993, Brain Metab. Rev. 5, 159-177) und die Antwort auf Triptane (Humphrey, P.P.A et al., 1991, Trends Pharmacol. Sci., 12, 444-446) sind Schlüsselelemente in der Pathologie der Migräne (Edvinsson, L., 2001, Pharmacology & Toxicology 89, 65-73). Entsprechend hat sich die neueste pharmakologische Forschung auf dieses Neuropeptid konzentriert.

Das 37 Aminosäuren lange Neuropeptid αCGRP, *calcitonin gene-related peptide,* wurde 1982 als extrem potenter Vasodilator identifiziert (Amara et al., 1982, Nature 298, 240-244). CGRP entsteht durch alternatives splicing des CGRP-Gens. Neben dem αCGRP gibt es ein zweites CGRP, βCGRP, das eine hohe Sequenzhomologie zum erst genannten aufweist, jedoch von einem anderen Gen transkribiert wird. Beide Peptide zeigen ähnliche biologische Effekte wie Vasodilatation, erhöhter Blutdruck, Hypotonie und Tachycardie. αCGRP und Calcitonin entstehen durch alternatives Splicing des Calcitonin-Genes (Amara, G.S. et al., 1982, Nature 298, 240-244). Die Struktur für hCGRP wurde zum Teil durch $^1$H-NMR bestimmt. Das Peptid besteht aus einer definierten N-terminalen Schleife, die aus den Aminosäuren 2 bis 7 durch Verknüpfung von zwei Cysteinen über eine Disulfidbrücke gebildet wird, an der sich etwa drei Windungen einer α-Helix anschließen. Richtung C-Terminus schließt sich ein schlecht definierter Knick an, der wiederum in eine instabile Struktur am C-Terminus selbst mündet (Breeze, A.L. et al., 1991, Biochemistry, 30, 575-582). Des weiteren liegt das C-terminale Phenylalanin in amidierter Form vor.

Ergotaminpräparate sind klassische Pharmaka zur Kupierung eines Migräneanfalls, die wegen der möglichen Nebenwirkungen jedoch nicht ganz unproblematisch sind. Die Gefahr einer Gewöhnung und Auslösung eines zusätzlichen Dauerkopfschmerzes steigt mit zunehmender Einnahmehäufigkeit. Aus diesem Grunde sollten pro Woche nicht mehr als 6 mg Ergotamintartrat und pro Migräneattacke nicht mehr als 4 mg eingenommen werden. Grundsätzlich gilt auch hier, daß beim Migränekopfschmerz die Verwendung von Mischpräparaten (z. B. Ergotamintartrat mit Koffein oder Prophyphenazon, Codein, Paracetamol usw.) strikt vermieden werden soll. Auf dieser Therapiestufe kann auch 1-1,5 mg Dihydroergotamin (Hydergin®) i.m. oder ganz langsam i.v. versucht werden. Besonders bei ausgeprägten vegetativen Migräne-Begleiterscheinungen hat sich die zusätzliche Gabe von 1-2 mg Flunitrazepam (Rohypnol®, ein Schlafmittel) sehr bewährt. Vor allem auch unter dem Aspekt, Schmerzmittel einzusparen, zumal die Patienten in dieser Situation ohnehin das Bedürfnis haben, sich hinzulegen. Werden die Migränekopfschmerzen von Übelkeit und Erbrechen begleitet (evtl. auch schon vor dem erwarteten Auftreten dieser Symptome), ist die Verabreichung von Metoclopramid (Paspertin®) sehr wirksam. Es ist vorteilhaft, diese Substanz vor einem Analgetikum einzunehmen, weil Metoclopramid die Darmtätigkeit steigert und somit die Resorption weiterer verabreichter Substanzen fördert. Alternativ kann auch der Dopamin-Antagonist Domperidon (Motilium®) verwendet werden.

Im Stand der Technik sind eine Reihe von CGRP-Antagonisten sowie davon abgeleitete Derivaten bekannt. So ist beispielsweise ein Quinine-Analogon als CGRP-Antagonist in der internationalen Patentanmeldung mit der Veröffentlichungsnummer WO 97/09046 beschrieben. Diese Verbindungen zeigen jedoch nur eine schwache Affinität für den humanen CGRP-Rezeptor im mikromolaren Bereich und sind deshalb nicht von großer Wichtigkeit.

Ein erster potenter nicht-peptidischer CGRP-Rezeptorantagonist ist die Verbindung BIBN-4096BS, wie beschrieben in DE 19911039. Diese Substanz ist ein Lys-Tyr-Dipeptidderivat und weist eine hohe Affinität für den humanen CGRP1-Rezeptor auf ($K_i$=14,4 pM). In Untersuchungen an cerebralen Gefäßen konnte BIBN-4096BS die CGRP-vermittelte Vasodilatation umkehren (Doods, H. et al., 2000, Br. J. Pharmacol., 129, 420-423). Obwohl hohe Dosen von CGRP cardioprotektiv sind, hatte der $\alpha$-CGRP-Antagonist BIBN-4096BS keinen negativen Effekt auf Myokardinfarkte oder die Freisetzung von Creatinphosphatkinase.

Basierend auf der Struktur von BIBN-4096BS wurde ein Cyclopropyl-Derivat entwickelt, bei welchem der Dipeptidkem durch einen Cyclopropylring ersetzt wurde. Diese Verbindung ist Gegenstand der internationalen Patentanmeldung mit der Veröffentlichungsnummer WO 01/32648. Weitere CGRP-Rezeptor-Antagonisten sind Gegenstand der internationalen Patentanmeldung mit der Veröffentlichungsnummer WO 01/32649, die Naphthalene, Piperidine, Imidazole und Quinazoline als CGRP-Rezeptor-Antagonisten beschreiben.

Weitere Strukturklassen, die Gegenstand von Untersuchungen sind, sie als CGRP-Rezeptor-Antagonisten einzusetzen, sind 3,4-Dinitrobenzamine, wie beispielsweise in der internationalen Patentanmeldung mit der Veröffentlichungsnummer WO 98/09630 beschrieben, sowie 4-Sulfinylbenzanilide, wie in der internationalen Patentanmeldung mit der Veröffentlichungsnummer WO 98/56779 beschrieben. Ausführliche Bindungsanalysen zeigen jedoch, daß diese Substanzen irreversible Bindungseigenschaften besitzen. Darüber hinaus weisen sie, insbesondere einige Vertreter der 4-Sulfinylbenzanilide, Einschränkungen dergestalt auf, daß sie eine vergleichsweise geringe Löslichkeit und orale Verfügbarkeit sowie eine kurze Halbwertszeit von ca. 10 Minuten haben, was eine intensive in vivo-Charakterisierung ausschließt.

Der vorliegenden Erfindung lag somit die Aufgabe zugrunde, ein Mittel bereitzustellen, welches zur Behandlung von Migräne und den damit im Zusammenhang stehenden Formenkreis weiterer Erkrankungen geeignet ist. Eine weitere der vorliegenden Erfindung zugrundeliegende Aufgabe ist die Bereitstellung eines Antagonisten für CGRP sowie das CGRP-Rezeptorsystem. Des weiteren ist es eine Aufgabe der vorliegenden Erfindung, ein Verfahren bereitzustellen, mit dem CGRP-Antagonisten, insbesondere im Rahmen eines Screening-Verfahrens, bereitgestellt werden können. Schließlich ist es eine Aufgabe der vorliegenden Erfindung, weitere Verwendungen der Antagonisten bereitzustellen.

Diese und andere der vorliegenden Erfindung zugrunde liegende Aufgaben werden durch den Gegenstand der beigefügten unabhängigen Ansprüche gelöst. Bevorzugte Ausführungsformen ergeben sich aus den beigefügten abhängigen Ansprüchen.

Genauer wird die der vorliegenden Erfindung zugrunde liegende Aufgabe in einem ersten Aspekt, der auch die erste Ausführungsform des ersten Aspektes ist, gelöst durch eine CGRP bindende L-Nukleinsäure umfassend eine Sequenz gemäß SEQ ID No. 246 oder eine dazu homologe CGRP bindende L-Nukleinsäure, wobei die Homologie wenigstens 75 % beträgt, wobei das CGRP $\alpha$-CGRP oder $\beta$-CGRP ist, und wobei die L-Nukleinsäure ein Antagonist von $\alpha$-CGRP oder $\beta$-CGRP ist.

In einer zweiten Ausführungsform des ersten Aspektes, die auch eine Ausführungsform der ersten Ausführungsform des ersten Aspektes ist, beträgt die Homologie zu der Sequenz gemäß SEQ ID No. 246 wenigstens 85 %.

In einer dritten Ausführungsform des ersten Aspektes, die auch eine Ausführungsform der ersten und der zweiten Ausführungsform des ersten Aspektes ist, ist die L-Nukleinsäure ein Antagonist des CGRP-Rezeptors und bindet an einen Liganden des Rezeptors, wobei der Ligand $\alpha$-CGRP oder $\beta$-CGRP ist.

In einer vierten Ausführungsform des ersten Aspektes, die auch eine Ausführungsform der ersten, der zweiten und der dritten Ausführungsform des ersten Aspektes ist, ist die L-Nukleinsäure eine L-Nukleinsäure mit einer Sequenz, wobei die Sequenz ausgewählt ist aus der Gruppe umfassend die Sequenzen gemäß SEQ ID No. 191, 192, 196, 199, 200, 245, 251, 252, 253, 254, 255, 256, 257, 258, 259, 260, 261, 262 und 263.

In einer fünften Ausführungsform des ersten Aspektes, die auch eine Ausführungsform der ersten, der zweiten, der dritten und der vierten Ausführungsform des ersten Aspektes ist, ist die L-Nukleinsäure ausgewählt aus der Gruppe umfassend DNA und RNA.

In einer sechsten Ausführungsform des ersten Aspektes, die auch eine Ausführungsform der ersten, der zweiten, der dritten, der vierten und der fünften Ausführungsform des ersten Aspektes ist, weist die L-Nukleinsäure eine Länge auf, wobei die Länge ausgewählt ist aus der Gruppe, die Längen von 15 bis 150 Nukleotiden, 20 bis 100 Nukleotiden, 20 bis 80 Nukleotiden, 20 bis 60 Nukleotiden, 20 bis 50 Nukleotiden oder 30 bis 50 Nukleotiden umfasst und die Länge am meisten bevorzugt 25 bis 45 Nukleotide umfasst.

Die der vorliegenden Erfindung zugrunde liegende Aufgabe wird in einem zweiten Aspekt, der auch die erste Ausführungsform des zweiten Aspektes ist, gelöst durch die Verwendung einer L-Nukleinsäure nach einer der ersten, der zweiten, der dritten, der vierten, der fünften und der sechsten Ausführungsformen des ersten Aspektes als ein Antagonist von CGRP und/oder des CGRP-Rezeptorsystems.

Die der vorliegenden Erfindung zugrunde liegende Aufgabe wird in einem dritten Aspekt, der auch die erste Ausführungsform des dritten Aspektes ist, gelöst durch die Verwendung einer L-Nukleinsäure nach einer der ersten, der zweiten, der dritten, der vierten, der fünften und der sechsten Ausführungsformen des ersten Aspektes für die Herstellung eines Medikaments.

In einer zweiten Ausführungsform des dritten Aspektes, die auch eine Ausführungsform der ersten Ausführungsform des dritten Aspektes ist, ist das Medikament für die Behandlung und/oder die Prävention einer Erkrankung, die aus der Gruppe ausgewählt ist, die Migräne, Cluster-Kopfschmerzen, Appetitlosigkeit, Übelkeit, Erbrechen, neurogene Entzündung, insbesondere neurogene Entzündung, die mit anderen Neuropeptiden vermittelt wird, Vasodilatation, erhöhter Blutdruck, Hypotonie, Tachikadie, Erkrankungen, die auf eine Aktivierung trigeminaler afferenter sensorischer Neurone und zentraler "nociceptiver" Neuronen, insbesondere höheren Schmerzzentren, zurückgehen, und chronisch entzündliche Schmerzen, umfasst und/oder zur Behandlung von Schmerzen, insbesondere chronische Schmerzen, akute Schmerzen, entzündliche Schmerzen, visceraler Schmerzen und neuropathischer Schmerzen.

Die der vorliegenden Erfindung zugrunde liegende Aufgabe wird in einem vierten Aspekt, der auch die erste Ausführungsform des vierten Aspektes ist, gelöst durch eine Zusammensetzung umfassend eine L-Nukleinsäure nach einer der ersten, der zweiten, der dritten, der vierten, der fünften und der sechsten Ausführungsform des ersten Aspektes und bevorzugterweise einen pharmazeutisch akzeptablen Träger.

Die der vorliegenden Erfindung zugrunde liegende Aufgabe wird in einem fünften Aspekt, der auch die erste Ausführungsform des fünften Aspektes ist, gelöst durch einen Komplex umfassend CGRP und eine L-Nukleinsäure nach einer der ersten, der zweiten, der dritten, der vierten, der fünften und der sechsten Ausführungsformen des ersten Aspektes.

Die der vorliegenden Erfindung zugrunde liegende Aufgabe wird in einem sechsten Aspekt, der auch die erste Ausführungsform des sechsten Aspektes ist, gelöst durch die Verwendung einer L-Nukleinsäure nach einer der ersten, der zweiten, der dritten, der vierten, der fünften und der sechsten Ausführungsformen des ersten Aspektes zum in vitro-Nachweis von CGRP, bevorzugterweise α-CGRP oder β-CGRP und am bevorzugtesten menschliches α-CGRP oder β-CGRP.

Die der vorliegenden Erfindung zugrunde liegende Aufgabe wird in einem sechsten Aspekt, der auch die erste Ausführungsform des sechsten Aspektes ist, gelöst durch ein Verfahren zum Screenen von CGRP-Antagonisten umfassend die folgenden Schritte:

- Bereitstellen eines Kandidaten-CGRP-Antagonisten,
- Bereitstellen einer L-Nukleinsäuren nach einer der ersten, der zweiten, der dritten, der vierten, der fünften und der sechsten Ausführungsformen des ersten Aspektes,
- Vorsehen eines Testsystems, welches ein Signal in Gegenwart eines CGRP-Antagonisten ergibt, und
- Bestimmen, ob der Kandidaten-CGRP-Antagonist ein CGRP-Antagonist ist.

In einer zweiten Ausführungsform des sechsten Aspektes, die auch eine Ausführungsform der ersten Ausführungsform des sechsten Aspektes ist, ist das CGRP α-CGRP und/oder β-CGRP, bevorzugterweise menschliches α-CGRP und/oder β-CGRP.

Die der vorliegenden Erfindung zugrunde liegende Aufgabe wird in einem siebten Aspekt, der auch die erste Ausführungsform des siebten Aspektes ist, gelöst durch ein Verfahren zum Screenen von CGRP-Agonisten umfassend die folgenden Schritte:

- Bereitstellen von CGRP,
- Bereitstellen einer L-Nukleinsäuren nach einer der ersten, der zweiten, der dritten, der vierten, der fünften und der sechsten Ausführungsformen des ersten Aspektes, bevorzugterweise einer markierten L-Nukleinsäure nach einer der ersten, der zweiten, der dritten, der vierten, der fünften und der sechsten Ausführungsformen des ersten Aspektes,
- Zugabe eines Kandidaten-CGRP-Agonisten, und
- Bestimmen, ob der Kandidaten-CGRP-Agonist ein CGRP-Agonist ist.

In einer zweiten Ausführungsform des siebten Aspektes, die auch eine Ausführungsform der ersten Ausführungsform des siebten Aspektes ist, erfolgt das Bestimmen dadurch, dass festgestellt wird, ob die L-Nukleinsäure durch den Kandidaten-CGRP-Agonisten verdrängt wird.

Die der vorliegenden Erfindung zugrunde liegende Aufgabe wird in einem achten Aspekt, der auch die erste Ausführungsform des achten Aspektes ist, gelöst durch ein Kit für den Nachweis von CGRP, bevorzugterweise α-CGRP oder β-CGRP, umfassend eine L-Nukleinsäure ach einer der ersten, der zweiten, der dritten, der vierten, der fünften und der sechsten Ausführungsform des ersten Aspektes.

Die der vorliegenden Erfindung zugrunde liegende Aufgabe wird in einem neunten Aspekt, der auch die erste Ausführungsform des neunten Aspektes ist, gelöst durch eine L-Nukleinsäure nach einer der ersten, der zweiten, der dritten, der vierten, der fünften und der sechsten Ausführungsformen des ersten Aspektes, zur Verwendung in einem Verfahren zur Behandlung einer Erkrankung, bevorzugterweise einer Erkrankung, die ausgewählt ist aus der Gruppe, die Migräne, Cluster-Kopfschmerzen, Appetitlosigkeit, Übelkeit, Erbrechen, neurogene Entzündung, insbesondere neurogene Entzündung, die mit anderen Neuropeptiden vermittelt wird, Vasodilatation, erhöhter Blutdruck, Hypotonie, Tachikadie, Erkrankungen, die auf eine Aktivierung trigeminaler afferenter sensorischer Neurone und zentraler "nociceptiver" Neuronen, insbesondere höheren Schmerzzentren, zurückgehen, Schmerzen, chronisch entzündliche Schmerzen, chronische Schmerzen, akute Schmerzen, entzündliche Schmerzen, visceraler Schmerzen und neuropathische Schmerzen.

In einer Ausführungsform der verschiedenen Aspekte der vorliegenden Erfindung ist vorgesehen, dass die erfindungsgemäßen Nukleinsäuren, unabhängig von möglichen weiteren Merkmalen und Eigenschaften, einen IC50-Wert aufweisen, wobei der IC50-Wert bevorzugterweise 0,5 - 30 nM, bevorzugtererweise 0,5 - 10 nM, noch bevorzugtererweise 0,5 - 3 nM und am bevorzugtesten 1 - 2 nM beträgt.

Der vorliegenden Erfindung liegt die überraschende Erkenntnis zugrunde, dass es möglich ist, spezifisch an CGRP bindende Nukleinsäuren zu erzeugen. Nachdem umfangreiche Belege dafür vorliegen, dass CGRP im Schmerzgeschehen und an der Ausbildung von Migräne in ihren verschiedenen Ausbildungsformen beteiligt ist, ergibt sich somit, dass derartige Nukleinsäuren als Antagonisten für CGRP bzw. das CGRP-Rezeptorsystem verwendet werden können und insoweit auch als pharmazeutische Wirkstoffe bei der Behandlung von Erkrankungen des Formenkreises der Migräne. Dabei ist besonders bemerkenswert, dass es sich bei CGRP um ein vergleichsweise kleines Peptid handelt, gegen welches bindende Nukleinsäuren nur schwer erzeugt werden können. Die Aminosäuresequenz von humanem αCGRP und βCGRP unterscheiden sich in drei Aminosäuren, die Aminosäuresequenz von αCGRP und βCGRP und der Ratte unterscheiden sich in einer Aminosäure. Die verschiedenen Sequenzen sind beschrieben in Hakala und Vihinen (Hakala J.M.L. und Vihinen M., 1994, Protein Engineering 7 (9), 1069-1075. (Accession numbers: human αCGRP P06881, human βCGRP P10092, rat αCGRP P01256, rat βCGRP P10093.

Diese Anwendung stützt sich dabei im wesentlichen auf die Beobachtung, daß CGRP in neuralem Gewebe und insbesondere in somatischen Sinneszellen reichlich vorhanden ist und häufig mit anderen Neuropeptiden, wie beispielsweise Substanz P co-exprimiert wird, und die weiteren im folgenden geschilderten Ergebnisse der Schmerz- und Migräneforschung.

Immunocytochemische Studien zeigen, daß Substanz P fast immer mit CGRP in kleinen DRG-Neuronen (*dorsal root ganglion*) assoziiert ist, wohingegen CGRP auch ohne Substanz P beobachtet wird (Wiesenfeld-Hallin, Z. et al., 1984, Neurosci. Lett. 52, 199-203). Die Freisetzung von CGRP in der Peripherie führt zu einer Vasodilatation und zusammen mit anderen Neuropeptiden, wie Substanz P, zur neurogenen Entzündung. CGRP wird auch im Dorsalhorn des Rückenmarks als Antwort auf schädliche Stimulationen in der Peripherie freigesetzt und stellt somit einen Applikations- und Untersuchungsort zur Anwendung der erfindungsgemäßen Nukleinsäuren, Antagonisten und Mittel dar.

Der CGRP1 Antagonist CGRP8-37 wurde in unterschiedlichen Schmerzmodellen getestet. Um endogenes CGRP zu antagonisieren, wurde ein anti-CGRP-Antiserum in verschiedenen Schmerzmodellen nach intrathekaler Gabe getestet. Die Erzeugung von αCGRP-defizienten Mäusen und Verhaltensexperimente dazu schließen das Bild. Der anti-nociceptive Effekt von CGRP8-37 konnte in verschiedenen Schmerzmodellen wie Phenylquinone-induziertes (PQ) *Writhing* (Saxen, M.A. et al., 1994, Life Sciences 55, 1665-1674), Essigsäure-induziertes *Writhing* (Friese, N. et al., 1997, Regulatory Peptides 70, 1-7), viszeraler Schmerz (colorectales Distensions-Modell, Plourde, V. et al., 1997, Am. J. Physiol, 36, G191-G196), Brandschmerz (Hitzeinduzierte Hyperalgesie, Lofgren, O. et al., 1997, Neuropeptides 31, 601-607), und neuropathischen Schmerz (spinale Hemisektion, Bennett, A.D. et al., 2000, Pain 86, 163-175). Im Maus *tail-flick,* einem Modell für den Akutschmerz, konnte kein Effekt beobachtet werden.

Um den Effekt des spinal freigesetzten CGRP's zu blockieren wurde ein Antiserum in verschiedenen Modellen für chronischen Schmerz getestet. In chronisch-entzündlichen Schmerzmodellen wie beispielsweise Adjuvant-induzierter Arthritis (Kuraishi, Y. et al., 1988, Neurosci. Lett. 92, 325-329) oder Carrageenin-induzierter Hyperalgesie (Kawamura, M. et al., 1989, Brain Res. 497, 199-203) zeigte das anti-CGRP-Antiserum anti-nociceptive Wirkung. Dieses Antiserum kann auch in Ratten eine wiederholte Stress-induzierte Hyperalgesie verhindern (Satoh, M. et al., 1992, Pain 49, 273-278).

Die anti-nociceptiven Effekte, die sowohl mit dem trunkierten Peptid CGRP8-37 und als auch mit dem Antiserum beobachtet werden, passen gut mit der in αCGRP-defizienten Knockout-Mäusen beobachteten Hyperalgesie zusammen (Salmon, A.-M. et al., 1999, Neuroreport 10, 849-954). $CGRP^{-/-}$ Mäuse zeigen im Vergleich zu den $CGRP^{+/+}$-Mäusen reduzierte Hyperalgesie bei chronisch entzündlichem Schmerz der durch Formalin- oder Capsaicin-Injektionen in die Hinterpfote ausgelöst wurde (Salmon, A.-M. et al., 2001, Nature 56, 357-358). Eine zweite αCGRP-defiziente Maus wurde erzeugt, um die Rolle des Calcitonins zu untersuchen. Die $CGRP^{-/-}$Mäuse werden normal geboren, sind fertil und leben ein normales Leben. Diese Mäuse entwickeln in einem chronischen Arthritis-Modell (Kaolin-Carragenin-Mix wurde ins Kniegelenk injiziert) im Vergleich zum Wildtyp, keine sekundäre Hyperalgesie (Zhang, L. et al., 2001, Pain 89, 265-273).

Infolge dieser Ergebnisse können α-CGRP-Antagonisten wie die hierin offenbarte CGRP-Antagonisten bzw. die erfindungsgemäßen Nukleinsäuren auch effektiv für die Behandlung von chronisch entzündlichem und viszeralem Schmerz eingesetzt werden.

Wie hierin verwendet bezeichnet der Begriff des CGRP-Rezeptors grundsätzlich einen jeden CGRP-Rezeptor.

Bevorzugte CGRP-Rezeptoren sind dabei die sogenannten CGRP1-Rezeptoren und CGRP2-Rezeptoren, an den die erfindungsgemäßen Nukleinsäuren binden bzw. für die die hierin offenbarten Nukleinsäuren Antagonisten darstellen. Dabei ist der Rezeptor CGRP1 ein solcher für alpha-CGRP und CGRP2 ein solcher für beta-CGRP.

Eine weitere der vorliegenden Erfindung zugrundeliegende Erkenntnis ist die, dass auch Antagonisten auf der Grundlage von Nukleinsäuren gegen Amylin hergestellt werden können, bzw. die an CGRP bindenden Nukleinsäuren, die eine antagonistische Wirkung zu CGRP bzw. zu dem CGRP-Rezeptor aufweisen, mit Amylin wechselwirken und insoweit eine antagonistische Aktivität aufweisen.

Amylin ist ein 37 Aminosäuren langes Peptidhormon. Es wird sowohl in diabetischen als auch in gesunden Individuen zusammen mit Insulin aus den β-Zellen der Langerhansschen Inseln sekretiert. Amylin wurde erst im Jahre 1987 entdeckt und gilt zur Zeit als drittes aktives Hormon der Bauchspeicheldrüse, welches zur Kontrolle des Blutzuckerspiegels beiträgt. Amylin verhindert eine zu schnelle Entleerung des Magens und verlangsamt auf diese Weise die Glukose-Aufnahme nach den Mahlzeiten. Darüber hinaus konnte gezeigt werden, dass Amylin sowohl die Glucagon- als auch die Somatostatinsekretion hemmt. Amylin bindet ebenso wie andere Mitglieder der Calcitionin-ähnlichen Peptide an einen G Protein gekoppelten Rezeptor. Ebenso wie die anderen Mitglieder der G Protein gekoppelten Receptoren, die Calcitionin-ähnlichen Peptide binden, ist der Amylinrezeptor aus verschiedenen Untereinheiten aufgebaut, die neben dem Calcitonin Rezeptor Genprodukt (CTR) entweder das Protein RAMP1 (receptor activity modifying protein 1) oder das Protein RAMP3 (receptor activity modifying protein 3) enthält. Beide Isoformen bilden den Amylinrezeptor; es gibt aber auch Berichte, daß auch RAMP 2 am Aufbau des Rezeptors beteiligt sein kann (G. Christopoulos et al. Mol Pharmacol. 1999 Jul;56(1):235-42 und N. Tilakaratne J Pharmacol Exp Ther 2000 Jul;294(1):61-72)

Es ruft außerdem, offensichtlich durch eine Interaktion mit dem zentralen Nervensystem, ein Gefühl der Sattheit und des Durstes hervor. Bindungsstellen für Amylin wurden in der Nierenrinde (insbesondere im Bereich des juxtaglomerulären Apparates) gefunden (Hayden, M. R. (2002). "Islet amyloid, metabolic syndrome, and the natural progressive history of type 2 diabetes mellitus." Jop 3(5): 126-38). Eine Aktivierung des Renin-Angiotensin-Aldosteron-Systems, welches unter anderem den Blutdruck und die Ausscheidung von Natrium reguliert, wurde ebenfalls beschrieben (Cooper, M. E., P. G. McNally, et al. (1995). "Amylin stimulates plasma renin concentration in humans." Hypertension 26(3): 460-4). Gerade in Patienten, die an Diabetes mellitus erkrankt sind, wurde eine erhöhte Sensitivität gegenüber den Komponenten des systemischen Renin-Angiotensin-Systems festgestellt (Carlsson, P. O. (2001). "The renin-angiotensin system in the endocrine pancreas." Jop 2(1): 26-32 2001).

In Patienten mit Typ 2-Diabetes mellitus, übergewichtigen Patienten mit Insulinresistenz und in Patienten mit eingeschränkter Glukose-Toleranz sind die Amylin-Spiegel im Blut erhöht.

Eine erhöhte Amylinproduktion, die stets eine Begleiterscheinung einer erhöhten Insulinproduktion darstellt, führt häufig zur Bildung von amyloiden Plaques in der Bauchspeicheldrüse. Diese Plaques breiten sich ausgehend von den β-Zellen enthaltenen Langerhansschen Inseln über die gesamte Bauchspeicheldrüse aus, wie in transgenen Mäusen gezeigt werden konnte, die menschliches Amylin exprimieren und typische amyloide Ablagerungen bilden (Wang, F., R. L. Hull, et al. (2001). "Islet amyloid develops diffusely throughout the pancreas before becoming severe and replacing endocrine cells." Diabetes 50(11): 2514-20). Es ist vielfach in der Literatur beschrieben, dass die amyloiden Plaques vor allem die β-Zellen der Pankreas absterben lassen oder zummindest in ihrer Funktion als Insulinproduzent beeinträchtigen (Jaikaran, E. T. and A. Clark (2001). "Islet amyloid and type 2 diabetes: from molecular misfolding to islet pathophysiology." Biochim Biophys Acta 1537(3): 179-203). Daraus ergibt sich für Jaikaran und Clark (2001) (Jaikaran, E. T. and A. Clark (2001). "Islet amyloid and type 2 diabetes: from molecular misfolding to islet pathophysiology." Biochim Biophys Acta 1537(3): 179-203) die Schlussfolgerung, dass die Plaque-Bildung ein Vorgang ist, den es durch neue therapeutische Interventionen zu stoppen gilt (Jaikaran, E. T. and A. Clark (2001). "Islet amyloid and type 2 diabetes: from molecular misfolding to islet pathophysiology." Biochim Biophys Acta 1537(3): 179-203). Endgültig geklärt ist allerdings noch nicht, ob die Ablagerungen des für β-Zellen toxischen amyloiden Polypeptides nur ein gleichzeitig auftretendes Phänomen, eine Verstärkung oder gar einen Auslöser für den Verlauf von Typ2 Diabetes mellitus darstellt. Klar ist, dass die amyloiden Plaques in über 70 % der Patienten beobachtet werden (Hayden, M. R. (2002). "Islet amyloid, metabolic syndrome, and the natural progressive history of type 2 diabetes mellitus." Jop 3(5): 126-38). Andere Quellen sprechen von über 90 % (Scrocchi, L. A., Y. Chen, et al. (2002). "Design of peptide-based inhibitors of human islet amyloid polypeptide fibrillogenesis." J Mol Biol 318(3): 697-706) bis über 95 % (Kapurniotu, A., A. Schmauder, et al. (2002). "Structure-based design and study of non-amyloidogenic, double N- methylated IAPP amyloid core sequences as inhibitors of IAPP amyloid formation and cytotoxicity." J Mol Biol 315(3): 339-50).

Auch Falle von Patienten mit Nierenversagen, die an Dialyseprogrammen teilnehmen, wurden erhöhte Konzentrationen von amyloiden Polypeptiden im Blut nachgewiesen (de Koning, E. J., K. A. Fleming, et al. (1995). "High prevalence of pancreatic islet amyloid in patients with endstage renal failure on dialysis treatment." J Pathol 175(2): 253-8).

Auch andere Gruppen haben bereits versucht die Bildung von Fibrillen aus Amylin zu verhindern. So berichten Scrocchi et al. und Kapurniotu et al. (2002) von einem peptidischen Antagonisten der Fibrillenbildung (Scrocchi, L. A., Y. Chen, et al. (2002). "Design of peptide-based inhibitors of human islet amyloid polypeptide fibrillogenesis." J Mol Biol 318(3): 697-706) (Kapurniotu, A., A. Schmauder, et al. (2002). "Structure-based design and study of non-amyloidogenic, double N- methylated IAPP amyloid core sequences as inhibitors of IAPP amyloid formation and cytotoxicity." J Mol Biol 315(3): 339-50).

Infolge der vorstehend beschriebenen Beteiligung von Amylin an einer Reihe von biologischen Prozessen können sich daraus entsprechende Anwendungen zur Behandlung und/oder Prävention von Organismen, insbesondere Säugetieren, und ganz besonders bevorzugt des Menschen, ergeben. Die entsprechenden Erkrankungen sind insbesondere Bluthochdruck sowie Diabetes, insbesondere Diabetes mellitus.

Wie im Falle der CGRP-Rezeptoren, hierin auch als CGRP-Rezeptorsystem bezeichnet, sind die Nukleinsäuren bzw. die Antagonisten von Amylin auch solche des Amylinrezeptors, der hierin auch als Amylin-Rezeptorsystem bezeichnet wird. Bei dem Amylin und/oder dem Amylinrezeptor handelt es sich bevorzugterweise um Amylin bzw. Amylinrezeptor vom Menschen oder der Ratte. Amylin-Rezeptoren sind als soches beispielsweise beschrieben in Christopoulos G, Perry KJ, Morfis M, Tilakaratne N, Gao Y, Fraser NJ, Main MJ, Foord SM, Sexton PM. Multiple amylin receptors arise from receptor activity-modifying protein interaction with the calcitonin receptor gene product. Mol Pharmacol. 1999 Jul; 56(1):235-42; Tilakaratne N, Christopoulos G, Zumpe ET, Foord SM, Sexton PM. Amylin receptor phenotypes derived from human calcitonin receptor/RAMP coexpression exhibit pharmacological differences dependent on receptor isoform and host cell environment; Tilakaratne N, Christopoulos G, Zumpe ET, Foord SM, Sexton PM. J Pharmacol Exp Ther 2000 Jul;294(1):61-72.

Die erfindungsgemäßen Nukleinsäuren bzw. Antagonisten können somit zur Inhibierung der physiologischen Wirkung von Amylin verwendet werden, insbesondere der Inhibierung der amyloidabhängigen Stimulierung des Renin-Angiotensin-Aldosteron-Systems oder der Wirkung auf das zentrale Nervensystem. Weiterhin können derartige Nukleinsäuren bzw. Antagonisten zur Vermeidung von amyloiden Plaques, beispielsweise in der Bauchspeicheldrüse, eingesetzt werden. Es ist auch im Rahmen der vorliegenden Erfindung, dass die amyloiden Plaques unter Anwendung der erfindungsgemäßen Antagonisten bzw. Nukleinsäuren aufgelöst werden. Zu der Gruppe der erfindungsgemäßen Nukleinsäuren, die an Amlyin binden bzw. einen Amylinrezeptor-Antagonisten darstellen, gehören insbesondere jene Nukleinsäuren mit der SEQ ID No. 191 bis SEQ ID No. 196.

Die Nukleinsäuren gemäß der vorliegenden Erfindung sollen auch solche Nukleinsäuren umfassen, die im wesentlichen homolog zu den spezifisch hierin offenbarten Sequenzen sind. Der Begriff im wesentlichen homolog soll hierin so verstanden werden, daß die Homologie wenigstens 75 %, bevorzugterweise 85 %, bevorzugtererweise 90 % und am bevorzugtesten mehr als 95, 96, 97, 98 oder 99 % beträgt.

Der Begriff erfindungsgemäße Nukleinsäure oder Nukleinsäure gemäß der vorliegenden Erfindung soll auch jene Nukleinsäuren umfassen, die einen Teil der hierin offenbarten Nukleinsäuresequenzen bzw. Nukleinsäuren umfassen, bevorzugterweise in dem Umfang umfassen, daß die besagten Teile bei der Bindung von CGRP beteiligt sind. Diese Art von Nukleinsäuren können von den hierin offenbarten abgeleitet werden, beispielsweise durch Verkürzung oder Trunkierung. Die Verkürzung soll sich entweder auf eines oder beide Enden der hierin offenbarten Nukleinsäuren beziehen. Die Verkürzung kann sich auch auf eine Nukleotidsequenz innerhalb der jeweiligen Nukleinsäure bzw. Nukleinsäuresequenz beziehen, d. h. sie kann sich beziehen auf ein oder mehrere Nukleotide zwischen dem 5'- bzw. dem 3'-terminalen Nukleotid. Im Zusammenhang damit soll Verkürzung die Deletion von so wenig wie einem einzelnen Nukleotid von der Sequenz der hierin offenbarten Nukleinsäuren umfassen. Verkürzung kann auch mehr als einen Bereich der erfindungsgemäßen Nukleinsäure(n) betreffen. Beispiele für die Verkürzung der Nukleinsäuren werden in dem Beispielsteil der vorliegenden Beschreibung gelehrt.

Die Nukleinsäuren gemäß der vorliegenden Erfindung sind L-Nukleinsäuren. 1) , daß ein oder mehrere Teile der Nukleinsäure als D-Nukleinsäure(n) ausgebildet sind, oder wenigstens ein oder mehrere Teile der Nukleinsäure als L-Nukleinsäure ausgebildet sind. Der Begriff "Teil" der Nukleinsäure soll so wenig wie ein Nukleotid bezeichnen. Derartige Nukleinsäuren werden im allgemeinen hierin als D- bzw. L-Nukleinsäure bezeichnet.

Es ist auch im Rahmen der vorliegenden Erfindung, daß die erfindungsgemäßen Nukleinsäuren Teil einer längeren Nukleinsäure sind, wobei diese längere Nukleinsäure mehrere Teile umfaßt, wobei wenigstens ein Teil eine Nukleinsäure gemäß der vorliegenden Erfindung oder ein Teil davon ist. Der andere Teil oder die anderen Teile dieser längeren Nukleinsäuren können entweder eine D-Nukleinsäure oder eine L-Nukleinsäure sein. Eine jegliche Kombination kann in Verbindung mit der vorliegenden Erfindung verwendet werden. Dieser andere Teil bzw. diese anderen Teile der längeren Nukleinsäure können eine Funktion aufweisen, die vom Binden und insbesondere vom Binden an CGRP, verschieden ist. Eine mögliche Funktion besteht darin, eine Wechselwirkung mit anderen Molekülen, z. B. zu Zwecken der Immobilisierung, Kreuzvernetzung, Nachweis oder Amplifikation zu erlauben.

Die L-Nukleinsäuren sind somit Nukleinsäuren die aus L-Nukleotiden bestehen, bevorzugterweise vollständig aus L-Nukleotiden bestehen.

D-Nukleinsäuren sind somit solche Nukleinsäuren, die aus D-Nukleotiden bestehen, bevorzugterweise vollständig aus D-Nukleotiden bestehen.

Unabhängig davon, ob die erfindungsgemäße Nukleinsäure aus D-Nukleotiden, L-Nukleotiden oder einer Kombination beider besteht, wobei die Kombination z. B. eine zufällige Kombination oder eine definierte Sequenz von Abfolgen von Nukleotiden ist, die aus wenigstens einem L-Nukleotid bzw. einem D-Nukleotid besteht, kann die Nukleinsäure aus einem oder mehreren Desoxyribonukleotid(en), Ribonukleotid(en) oder Kombinationen davon bestehen.

Die Ausbildung der erfindungsgemäßen Nukleinsäuren als Nukleinsäure ist aus einer Reihe von Gründen mit Vorteilen verbunden. Die L-Nukleinsäuren sind Enantiomere der natürlicherweise auftretenden Nukleinsäuren. Die D-Nukleinsäuren sind jedoch in wäßrigen Lösungen und insbesondere in biologischen Systemen oder biologischen Proben nicht sehr stabil infolge der weiten Verbreitung von Nukleasen. Natürlicherweise auftretende Nukleasen, insbesondere Nukleasen von tierischen Zellen oder Geweben oder Zell- oder Körperflüssigkeiten, sind nicht in der Lage, L-Nukleinsäuren abzubauen. Infolge dessen ist die biologische Halbwertszeit der L-Nukleinsäure in derartigen Systemen, einschließlich des menschlichen und tierischen Körpers, wesentlich verlängert. Bedingt durch das Fehlen einer Abbaubarkeit von L-Nukleinsäuren werden keine Nuklease-Abbauprodukte erzeugt und es werden insoweit keine darauf zurückgehende Nebenwirkungen beobachtet. Dieser Aspekt unterscheidet die L-Nukleinsäure von all jenen anderen Verbindungen, die bei der Therapie von Erkrankungen, die auf die Anwesenheit von CGRP abstellen, verwendet werden.

Es ist auch im Rahmen der vorliegenden Erfindung, daß die erfindungsgemäßen Nukleinsäuren, unabhängig davon, ob sie als D-Nukleinsäuren, L-Nukleinsäuren oder D,L-Nukleinsäuren vorliegen, oder ob sie als DNA oder RNA vorliegen, als einzelsträngige oder doppelsträngige Nukleinsäuren vorhanden sein können. Typischerweise sind die erfindungsgemäßen Nukleinsäuren einzelsträngige Nukleinsäuren, die eine definierte Sekundärstruktur infolge der Primärsequenz aufweisen und auch Tertiärstrukturen ausbilden können. Die erfindungsgemäßen Nukleinsäuren können jedoch auch als doppelsträngig in dem Sinne vorliegen, daß zwei zueinander komplementäre Stränge infolge Hybridisierung miteinander gepaart vorliegen. Dies verleiht der Nukleinsäure eine Stabilität, die von Vorteil ist, wenn die Nukleinsäure in der natürlicherweise vorkommenden D-Form anstelle der L-Form vorliegt.

Die erfindungsgemäßen Nukleinsäuren können auch modifiziert sein. Eine besonders vorteilhafte Modifikation im Zusammenhang mit der vorliegenden Erfindung stellt dabei die Ausgestaltung der erfindungsgemäßen Nukleinsäure(n) dar, bei der mindestens ein, bevorzugterweise mehrere und am bevorzugtesten alle die Nukleinsäure ausbildenden Pyrimidinnukleotide an der 2'-Position des Riboseteils der jeweiligen Nukleotide eine 2'-Fluorogruppe aufweisen. Andere Modifikationen stellen beispielsweise solche mit PEG dar.

Weitere Beispiele für eine Modifizierung der erfindungsgemäßen Nukleinsäuren können solche Modifikationen sein, die ein einzelnes Nukleotid der Nukleinsäure betreffen und im Stand der Technik sehr gut bekannt sind. Entsprechende Beispiele sind, unter anderem, beschrieben in Kusser, W. (2000) J Biotechnol 74, 27-38; Aurup, H. et al., 1994, Nucleic Acids Res 22, 20-4; Cummins, L.L. et al, 1995, Nucleic Acids Res 23, 2019-24; Eaton, B.E. et al., 1995, Chem Biol 2, 633-8; Green, L.S. et al., 1995, Chem Biol 2, 683-95; Kawasaki, A.M. et al., 1993, J Med Chem 36, 831-41; Lesnik, E.A. et al., 1993, Biochemistry 32, 7832-8; Miller, L.E. et al., 1993, J Physiol 469, 213-43.

Die Nukleinsäuren gemäß der vorliegenden Erfindung können als einteilige, zwei-, drei oder mehrteilige Form ausgebildet sein. Von den mehrteiligen Form ist besonders die zweiteilige oder bipartite Form bevorzugt. Eine mehrteilige Form einer erfindungsgemäßen Nukleinsäure ist hierin insbesondere eine solche, die aus wenigstens zwei Nukleinsäuresträngen besteht. Diese beiden Nukleinsäurestränge bilden eine funktionale Einheit, wobei die funktionale Einheit ein Ligand oder bindendes Molekül für ein Zielmolekül ist. Die wenigstens zwei Stränge können von einer der erfindungsgemäßen Nukleinsäuren abgeleitet sein oder werden durch entweder Spaltung einer an das Zielmolekül bindenden erfindungsgemäßen Nukleinsäure, um zwei oder mehrere Stränge zu bilden, oder durch Synthese einer Nukleinsäure, die einem ersten Teil der erfindungsgemäßen vollständigen Nukleinsäure entspricht und einer weiteren Nukleinsäure, die einem zweiten Teil der erfindungsgemäßen vollständigen Nukleinsäure entspricht. Es ist anzuerkennen, daß sowohl die Spaltung als auch die Synthese angewandt werden kann, um eine mehrteilige Nukleinsäure herzustellen, die aus mehr als den beiden vorstehend beispielhaft beschriebenen Strängen besteht. Im übrigen ist betreffend die mehrteiligen Formen der erfindungsgemäßen Nukleinsäure festzuhalten, daß wenigstens zwei Nukleinsäurestränge typischerweise verschieden sind von zwei Strängen, die zueinander komplementär sind und miteinander hybridisieren, obgleich ein gewisser Grad an Komplementarität zwischen den verschiedenen Nukleinsäure(teilen) existieren kann.

Die Nukleinsäuren gemäß der vorliegenden Erfindung weisen typischerweise eine hohe Affinität zum Zielmolekül auf. Eine Möglichkeit, die Affinität, ausgedrückt als Bindungskonstante, der erfindungsgemäßen Nukleinsäuren zu bestimmen, ist die Verwendung der sog. Biacore-Vorrichtung, die den Fachleuten auf diesem Gebiet bekannt und beispielsweise beschrieben ist in Jönsson, U. et al., 1991, Biotechniques, 11 (5), 620. Die Affinitäten wurden auch gemessen durch isothermale Titrationskalorimetrie (ITC), wie in den Beispielen und in Haq, I. & Ladburg, J., 2000, J. Mol. Recognit. 13 (4): 188 beschrieben. Insoweit sind die hierin beschriebenen Affinitätswerte als durch isothermale Titrationskalorimetrie gemessen zu verstehen, wobei die Temperatur für die einzelne Messung 25°C betrug, sofern keine gegenteiligen Angaben gemacht werden. Eine weitere verwendete Methode wird manuell angewendet; es handelt sich um einen sogenannten Bead-Assay. Hierbei werden konstante Konzentrationen an radioaktiv markierter Nukleinsäure mit verschiedenen Konzentration an biotinyliertem Zielmolekül wie beispielsweise einem Target-Peptid zusammengegeben. Nach definierter Zeit werden die gebildeten Komplexe über die Zugabe von mit Streptavidin-beladenen Beads aus der Lösung entfernt und die jeweilige Menge an Radioaktivität wird bestimmt. Aus den erhaltenen Werten lassen sich über entsprechende Auftragung in Graphen die Bindungskonstanten bestimmen. Dieser Bead-Assay ist ausführlicher u. a. in Beispiel 3 beschrieben. Sofern hierin auf eine erfindungsgemäße Nukleinsäure Bezug genommen wird, sollen damit grundsätzlich alle erfindungsgemäßen Nukleinsäuren bzw. alle hierin offenbarten Nukleinsäuren verstanden werden, sofern keine gegenteiligen Angaben gemacht werden.

Es ist auch im Rahmen der vorliegenden Erfindung, daß die erfindungsgemäßen Sequenzen entweder vollständig oder teilweise aus dem randomisierten Teil der Mitglieder einer Nukleinsäurebibliothek entstammen, die als Ausgangsmaterial für den Selektionsprozeß verwendet werden. Es ist jedoch auch im Rahmen der vorliegenden Erfindung, daß die erfindungsgemäßen Sequenzen entweder vollständig oder teilweise aus dem nicht-randomisierten Teil der Mitglieder der Nukleinsäurebibliothek, die als Ausgangsmaterial für den Selektionsprozeß dient, entstammen. Ein derartiger nicht-randomisierter Teil ist beispielsweise der Teil, der als Bindungsstelle für den Amplifikations-Primer verwendet wird.

Die erfindungsgemäßen Nukleinsäuren können verwendet werden für die Erzeugung oder Herstellung eines Medikaments. Ein derartiges Medikament enthält wenigstens eine der erfindungsgemäßen Nukleinsäuren, optional zusammen mit weiteren pharmazeutisch aktiven Verbindungen, wobei die erfindungsgemäße(n) Nukleinsäure(n) bevorzugterweise selbst als pharmazeutisch aktive Verbindung fungiert. Beispielsweise könnte eine der erfindungsgemäßen Nukleinsäuren mit einem weiteren Wirkstoff kombiniert werden, der die Konzentration von CGRP bzw. Amylin beeinflusst. Generell scheint die Kombination der erfindungsgemäßen Nukleinsäuren mit dem weiteren Wirkstoff besonders dann von Vorteil zu sein, wenn beide Komponenten an CGRP bzw. Amylin und dessen Freisetzung über unterschiedliche Wirkmechanismen angreifen. Eine in diesem Sinne vorteilhafte Kombination könnte beispielsweise aus einer der erfindungsgemäßen Nukleinsäuren sowie einem Triptan, wie beispielsweise Sumatriptan bestehen, da beide Substanzen unterschiedliche Wirkmechanismen ansprechen. Bei einer Migräneattacke könnte man sich ein Kombi-Präparat und dessen Wirkung wie folgt vorstellen: im Plasma vorkommendes CGRP wird durch eine erfindungsgemäße Nukleinsäure weggefangen und gleichzeitig werden durch Triptane die CGRP-Freisetzung reduziert/verhindert. Derartige Medikamente umfassen in bevorzugten Ausführungsformen wenigstens einen pharmazeutisch akzeptablen Träger. Derartige Träger können z. B. Wasser, Puffer, Stärke, Zucker, Gelatine und dergleichen sein. Derartige Träger sind den Fachleuten auf dem Gebiet bekannt.

Die Erkrankungen, für die die erfindungsgemäßen Nukleinsäuren und die unter ihrer Verwendung identifizierten CGRP-Antagonisten verwendet werden können, sind im wesentlichen solche, die zum Formenkreis der Migräne gehören insbesondere Migräne mit und ohne Aura, einfache Migräne, klassische Migräne und komplizierte Migräne. Dazu gehören unter anderem Kopfschmerzen, insbesondere wiederkehrende Kopfschmerzen, Übelkeit, Erbrechen, übertriebene Sensitivität gegenüber externen Stimuli wie Licht und Geräuschen, Appetitlosigkeit und Störungen des Flüssigkeitshaushaltes. Der Kopfschmerz ist dabei besonders ein solcher, der bei den Patienten als pulsierend und pochend auftritt. Typischerweise treten diese Kopfschmerzen einseitig auf. Weitere Erkrankungen, die unter Verwendung der erfindungsgemäßen Nukleinsäuren und der auf der Grundlage deren Verwendung identifizierten Kandidaten-CGRP-Antagonisten identifiziert werden können, sind Vasodilatation, erhöhter Blutdruck, Hypotonie und Tachykardie, insbesondere jene Formen der vorstehend genannten Erkrankungen, die im Zusammenhang mit Migräne, insbesondere einem Migräneanfall, zu beobachten sind. Weitere Erkrankungen, die unter Verwendung der erfindungsgemäßen Nukleinsäure bzw. der unter Anwendung des erfindungsgemäßen Verfahrens identifizierten Kandidaten-CGRP-Antagonisten bereitgestellt werden können, sind solche Erkrankungen, die mit einer Aktivierung trigeminaler afferenter sensorischer Neurone, mit der Aktivierung zentraler nociceptiver Neuronen und Kombinationen der Aktivierung beider Neuronenklassen einhergehen oder verbunden sind. Insbesondere handelt es sich bei den Neuronen um solche, die höheren Schmerzzentren zugeordnet sind. Weitere Erkrankungen im vorstehend genannten Sinne sind neben Akutschmerz solche Erkrankungen, die mit chronisch entzündlichen Schmerzen verbunden sind. Zu derartigen chronisch entzündlichen Schmerzen gehören insbesondere jene, die von CGRP zusammen mit anderen Neuropeptiden verursacht werden, wie beispielsweise Substanz P.

Die erfindungsgemäßen Nukleinsäuren können weiterhin als Ausgangsmaterial für das Design von pharmazeutischen Wirkstoffen (engl. *drug design*) verwendet werden. Grundsätzlich gibt es hierzu zwei mögliche Ansätze. Ein Ansatz besteht in dem Screening von Bibliotheken von Verbindungen, wobei derartige Bibliotheken von Verbindungen bevorzugterweise Bibliotheken von niedermolekularen Verbindungen (engl. *low* oder *small molecules)* sind. Derartige Bibliotheken sind den Fachleuten auf diesem Gebiet bekannt. Alternativ können gemäß der vorliegenden Erfindung die Nukleinsäuren für das rationale Design von Wirkstoffen verwendet werden.

Das rationale Design von Wirkstoffen kann dabei seinen Ausgangspunkt von irgendeiner der Nukleinsäuren gemäß der vorliegenden Erfindung nehmen und umfaßt eine Struktur, insbesondere eine dreidimensionale Struktur, die ähnlich der Struktur der erfindungsgemäßen Nukleinsäure(n) ist oder identisch ist zu dem Teil der Struktur der erfindungsgemäßen Nukleinsäure(n), die die Bindung an CGRP bzw. Amylin vermittelt. In einem jeden Fall zeigt eine derartige Struktur noch das gleiche oder ein zumindest ähnliches Bindungsverhalten, wie die erfindungsgemäße Nukleinsäure (n). In entweder einem weiteren Schritt oder als ein alternativer Schritt werden bei dem rationalen Design von Wirkstoffen die bevorzugterweise dreidimensionale Struktur jener Teile der an CGRP bzw. Amylin bindenden Nukleinsäuren durch chemische Gruppen nachgeahmt, die bevorzugterweise verschieden sind von Nukleotiden und Nukleinsäuren. Durch dieses Nachahmen, auch als Mimikri bezeichnet, kann eine Verbindung konstruiert werden, die von der Nukleinsäure bzw. den Nukleinsäuren verschieden ist, die als Ausgangsmaterialien für das rationale Design des Wirkstoffes verwendet wurde. Eine derartige Verbindung oder Wirkstoff ist bevorzugterweise ein kleines Molekül (engl. *small molecule*) oder ein Peptid.

Im Falle des Screenings von Verbindungsbibliotheken, wie bei Verwendung eines kompetitiven Tests, die den Fachleuten auf dem Gebiet bekannt sind, können geeignete CGRP-Analoga, CGRP-Agonisten, CGRP-Antagonisten, Amylin-Analoga, Amylin-Agonisten oder Amylin-Antagonisten gefunden werden. Derartige kompetitive Assays können wie folgt aufgebaut sein. Die erfindungsgemäße Nukleinsäure, bevorzugterweise ein Spiegelmer, das als Zielmolekül bindende L-Nukleinsäure ausgebildet ist, wird an eine feste Phase gekoppelt. Um CGRP-Analoga zu identifizieren, werden mit einer Markierung versehene Neuropeptide zu dem Testsystem hinzugegeben. Ein potentielles Analogon würde mit den CGRP-Molekülen, die an das Spiegelmer binden, kompetitieren, was mit einer Abnahme des Signals, das von der entsprechenden Markierung erhalten wird, einhergehen würde. Das Screenen auf Agonisten oder Antagonisten kann die Verwendung eines Zellkultur-Testsystems umfassen, das den Fachleuten auf dem Gebiet bekannt ist. Im Prinzip die gleichen Ansätze können unter Verwendung von Amylin verwendet werden.

Auch offenbart ist, dass die erfindungsgemäßen Nukleinsäuren für die Targetvalidierung verwendet werden 1) infolge ihres charakteristischen Bindungsverhaltens an CGRP bzw. Amylin. Die erfindungsgemäßen Nukleinsäuren können in einem *ex vivo*-Organmodell verwendet werden, um die Funktion von CGRP bzw. Amylin zu studieren. Grundsätzlich existieren zwei ex vivo Modelle, in denen CGRP Agonisten/Antagonisten getestet werden können. Im Meerschweinchen-Atrium können Antagonisten für den CGRP2-Rezeptor getestet werden, im Ratten Vas deferens-Modell können Antagonisten hinsichtlich ihrer Spezifität für den CGRP-Rezeptor überprüft werden.

In einem weiteren Aspekt der Erfindung betrifft diese einen Komplex umfassend CGRP. Amylin und wenigstens eine der erfindungsgemäßen Nukleinsäuren. Es ist überraschenderweise festgestellt worden, daß die erfindungsgemäßen Nukleinsäuren relativ starr sind und eine genau definierte Struktur einnehmen und insoweit auch dem Zielmolekül, d. h. dem CGRP selbst, eine vergleichsweise starre Struktur aufprägen, wobei das CGRP infolge seiner Länge allgemein als vergleichsweise flexibel verstanden wird.

Der Kit gemäß der vorliegenden Erfindung kann wenigstens eine oder mehrere der erfindungsgemäßen Nukleinsäuren umfassen. Zusätzlich kann der Kit wenigstens eine oder mehrere Positiv- oder Negativkontrollen umfassen. Als Positivkontrollen kann z. B. CGRP verwendet werden, gegen das die erfindungsgemäße Nukleinsäure selektiert wurde, oder an die diese bindet, bevorzugterweise in flüssiger Form. Als Negativkontrolle kann unter anderem ein Peptid verwendet werden, welches sich hinsichtlich seiner biophysikalischen Eigenschaften ähnlich wie CGRP verhält, welches jedoch nicht durch die erfindungsgemäßen Nukleinsäuren erkannt wird oder ein Peptid mit gleicher Aminosäurezusammensetzung aber von CGRP verschiedener Sequenz.

Weiterhin kann der Kit einen oder mehrere Puffer umfassen. Die verschiedenen Bestandteile können in dem Kit in trockener oder lyophilisierter Form, oder gelöst in einer Flüssigkeit vorliegen. Der Kit kann eine oder mehrere Behältnisse umfassen, die wiederum ein oder mehrere der Bestandteile des Kits enthalten können. Bevorzugterweise enthalten die Gefäße Reaktionsansätze, wie sie für eine einmalige Durchführung eines Experimentes unter Verwendung einer oder mehrere Bestandteile des Kits erforderlich sind.

Es ist weiter im Rahmen der vorliegenden Erfindung, dass die erfindungsgemäßen Nukleinsäuren zum Nachweis des Zielmoleküls wie CGRP bzw. Amylin verwendet werden können bzw. den daraus hergestellten Strukturen wie beispielsweise amyloide Plaques oder Fibrillen. Hierzu können die Nukleinsäuren direkt oder indirekt markiert werden. Bevorzugterweise ist die Markierung ausgewählt aus der Gruppe, die radioaktive Markierungen, Fluoreszenzmarkierungen oder für die Magnetresonanz-Spektroskopie geeignete Markierungen, wie beispielsweise Europium, umfasst.

Die erfindungsgemäßen Nukleinsäuren, Antagonisten oder die diese enthaltenden Medikamente können sowohl systemisch als auch lokal appliziert werden. Im Falle der Verwendung von Amylin-Antagonisten bzw. entsprechenden Nukleinsäuren ist dabei beispielsweise eine lokale Verabreichung im Sinne einer Injektion in die Bauchspeicheldrüse grundsätzlich denkbar. Es ist auch im Rahmen der vorliegenden Erfindung die Antagonisten bzw. Nukleinsäuren in biokompatible Gele als Depots aufzunehmen, die dann im Bauchraum oder im Pankreas freigesetzt werden.

Die vorliegende Erfindung wird weiter anhand der folgenden Figuren und Beispiele erläutert, aus denen sich weitere Merkmale, Ausführungsformen und Vorteile ergeben. Dabei zeigt

| | |
|---|---|
| Fig. 1 | das Prinzip der einer RNA-Selektion von an CGRP bindenden Nukleinsäuren umfassend einen Anbindungsschritt mit den Teilschritten Denaturierung und Faltung sowie Anbindung-Waschen-Elution und den Amplifizierungsschritt umfassend den Schritt der reversen Transkription-PCR sowie T7-Transkription und Aufreinigung; |
| Fig. 2 | den Verlauf einer Selektion von 2'-Fluoro-modifizierten, an CGRP bindenden Nukleinsäuren, wobei insbesondere |
| Fig. 2A | die in einer jeden Selektionsrunde verwendete CGRP-Konzentration und die dazugehörige |

| | |
|---|---|
| | prozentuale Anbindung des F-RNA-Pools und |
| Fig. 2B | eine graphische Darstellung der in Fig. 2A angegebenen Werte; |
| Fig. 3 | das Ergebnis einer Sequenzanalyse der im Rahmen von Beispiel 1 erhaltenen CGRP bindenden Nukleinsäuren; |
| Figs. 4-11 | die Sekundärstruktur von verschiedenen an CGRP bindenden Nukleinsäuren, wie sie im Rahmen von Beispiel 2 erzeugt wurden; |
| Fig. 12 | den Verlauf der Vorsäulen während der Selektion von an CGRP bindenden Nukleinsäuren; |
| Fig. 13 | den Verlauf der CGRP bindenden 2'-F-RNA und der Peptidkonzentration des Selektionsansatzes NA unter Verwendung von Neutravidin als Selektionsmatrix; |
| Fig. 14 | den Verlauf der an CGRP bindenden 2'-F-RNA und der Peptidkonzentration des Selektionsansatzes SA-1 und SA-0,1 unter Verwendung Streptavidin derivatisierten magnetischen Beads als Selektionsmatrix; |
| Fig. 15 | das Reaktionsschema zur Synthese von 5'-DMT-2'-fluoro-L-Uridin-Phosphoramidit; |
| Fig. 16 | die Darstellung von 2'-Fluoro-L-Cytidin-Phosphoramidit ausgehend von 2'-Fluoro-L-Uridin; |
| Fig. 17 | die Synthese von 2'-Fluoro-L-Cytidin ausgehend von 2'-Fluoro-L-Uridin; |
| Fig. 18 | die Sequenzen der Ausgangssequenzen und der verkürzten Sequenzen der CGRP bindenden Nukleinsäuren von Beispiel 2; |
| Fig. 19 | die prozentuale Bindung verschiedener CGRP erkennender Nukleinsäuren, wie in Beispiel 3 näher beschrieben; NA-A bedeutet hier NeutrAvidin-Agarose mit Affinitätselution, NA-D bedeutet NeutrAvidin-Agarose mit denaturierender Elution, SA-1 bedeutet Streptavidin Beads mit 1 nM Peptid- und SA 0,1 nM Peptidkonzentration |
| Fig. 20 | das Ergebnis einer Kompetitionsanalyse verschiedener gemäß Beispiel 3 selektierter CGRP bindender Nukleinsäuren; |
| Fig. 21 | eine Darstellung von Versuchen zur Bestimmung der Dissoziationskonstanten verschiedener CGRP bindender Nukleinsäuren; |
| Fig. 22 | eine weitere Darstellung von Versuchen zur Bestimmung der Dissoziationskonstanten verschiedener CGRP bindender Nukleinsäuren; |
| Fig. 23 | die Hemmung der cAMP-Produktion durch humanes CGRP bindende Spiegelmere; |
| Fig. 24 | die Hemmung der cAMP-Produktion durch Ratten-CGRP bindende Spiegelmere, |
| Fig. 25 | Bindungsstudien mit den verschieden gereinigten Spiegelmeren 732_045 (55mer) und 732_096 (59mer) im [$^{35}$S]GTP$\gamma$S-Assay, |
| Figs. 26-27 | die Sekundärstruktur von verschiedenen an CGRP bindenden Nukleinsäuren, wie sie im Rahmen von Beispiel 2 erzeugt wurden und die aus mehr als einem Nukleinsäurestrang zusammengesetzt sind, |
| Fig. 28, 30, 32, 34, 36 | graphisch den Verlauf der Bedingungen verschiedener Selektionsrunden, |
| Fig. 29, 31, 33, 35, 37 | tabellarisch den Verlauf der Bedingungen verschiedener Selektionsrunden, |
| Fig. 38 | die Dosis-Wirkungskurve für CGRP, |

Fig. 39 bis 42 den Verlauf der kalorimetrischen Bestimmung der Bindungskonstanten verschiedener CGRP bindender Nukleinsäuren;

Fig. 43 bis 47 die Sequenzen verschiedener, im Rahmen von Beispiel 4 erhaltenen CGRP bindenden Nukleinsäuren, und

Fig. 48 und 49 die Sequenzen verschiedener, im Rahmen von Beispiel 3 erhaltenen CGRP bindenden Nukleinsäuren.

Fig. 50 die Hemmung der cAMP-Produktion durch Spiegelmer STAR-R02-15xx-F12 (NOX-504) mit 1 nM Ratten-α-CGRP bei 37°C;

Fig. 51 ein Enthalpiediagramm für die Bindung von Spiegelmer STAR-R02-15xx-F12 (NOX-504) an Ratten-α-CGRP;

Fig. 52 einen Strukturvorschlag für Spiegelmer STAR-R02-15xx-F12 (NOX-504) und die Verkürzungsstelle (im Kasten);

Fig. 53 die Dosis-Wirkungs-Kurve von Spiegelmer NOX-504-014 mit 1 nM Ratten-α-CGRP;

Fig. 54 die Dosis-Wirkungs-Kurve von Spiegelmer NOX-504-014 mit 1 nM humanem α-CGRP;

Fig. 55 die Dosis-Wirkungs-Kurve von Spiegelmer NOX-504-014 mit 1 nM humanem β-CGRP;

Fig. 56 die Dosis-Wirkungs-Kurve von Spiegelmer NOX-504-014 bei 30 nM Ratten-Adrenomedullin;

Fig. 57 die Dosis-Wirkungs-Kurve von Spiegelmer NOX-504-014 mit humanem Adrenomedullin;

Fig. 58 die cAMP-Bildung durch Kompetition mit Ratten-Amylin bei 1 nM Ratten α-CGRP und 100 nM Spiegelmer NOX-504-014;

Fig. 59 die cAMP-Bildung durch Kompetition mit humanem Amylin bei 1 nM humanem α-CGRP und 100 nM Spiegelmer NOX-504-014;

Fig. 60 die cAMP-Bildung durch Kompetition mit Ratten-Calcitonin bei 1 nM Ratten- α-CGRP und 100 nM Spiegelmer NOX-504-014;

Fig. 61 die cAMP-Bildung durch Kompetition mit humanem Calcitonin bei 1 nM humanem α-CGRP und 100 nM Spiegelmer NOX-504-014;

Fig. 62 die Dosis-Wirkungs-Kurve von Spiegelmer NOX-504-089 mit 1 nM Ratten-a-CGRP;

Fig. 63 die Sequenzen der CGRP-bindenden RNA-Klone, Mutationen der reselektierten Sequenz (Grt2-STAR-504-5-B0.1-C10) im Vergleich zur Ausgangssequenz des 504-ad3-18%-Pools (NOX-504-ad3) sind unterstrichen;

Fig. 64 den Verlauf der Reselektion, mit der Peptid-Konzentration gekennzeichnet als Linie (rechte Größenachse) und dem Signal/Hintergrund-Verhältnis als Balken (linke Größenachse);

Fig. 65 die Sequenzen der RNA-Spiegelmere für die Zellkultur-Versuche, wobei Mutationen durch Unterstreichungen hervorgehoben sind;

Fig. 66A die Dosis-Wirkungskurven von L097 mit hCGRP, wobei der Pfeil die Konzentration einer 50%-igen Hemmung der hCGRP-Wirkung ($IC_{50}$) kennzeichnet;

Fig. 66B die Dosis-Wirkungskurven von L097 mit rCGRP, wobei der Pfeil die Konzentration einer 50%-igen Hemmung der rCGRP-Wirkung ($IC_{50}$) kennzeichnet;

Fig. 67 die Mutationsanalyse von L097, dargestellt ist die Hemmung der cAMP-Bildung durch verschiedene Spiegelmere in den Konzentrationen 10 nM und 100 nM;

Fig. 68 die Dosis-Wirkungskurven von L108 und L109 mit hCGRP, dargestellt ist die Hemmung der cAMP-Bildung in Abhängigkeit der Spiegelmer-Konzentration, wobei der Pfeil die Konzentration einer 50%-igen Hemmung der hCGRP-Wirkung ($IC_{50}$) kennzeichnet;

Fig. 69 die Dosis-Wirkungskurven von NOX-504-095 und NOX-504-096 mit Ratten α-CGRP; dargestellt ist die Hemmung der cAMP-Bildung in Abhängigkeit der Spiegelmer-Konzentration, wobei der Pfeil die Konzentration einer 50%-igen Hemmung der α-CGRP-Wirkung ($IC_{50}$) kennzeichnet;

Fig. 70 die Dosis-Wirkungskurve für Ratten-Amylin;

Fig. 71 die Dosis-Wirkungskurve für humanes Amylin;

Fig. 72 die Inhibitionskurve für die drei Spiegelmere NOX-504-014, NOX-504-089, NOX-504-097 und Ratten-Amylin;

Fig. 73 die Inhibitionskurve für die drei Spiegelmere NOX-504-014, NOX-504-089, NOX-504-097 und humanes Amylin; und

Fig. 74 Verlauf der kalorimetrischen Bedstimmung der Bindungskonstanten von Spiegelmer NOX-504-L097 bzgl. hCGRP bei 37°C.

**Beispiel 1: Herstellung von 2'-Fluoro-modirizierten Spiegelmeren**

Ausgehend von dem Verfahren zur Identifizierung von Nukleinsäuren, insbesondere D-Nukleinsäuren, die an ein Zielmolekül binden, wie beispielsweise dargestellt in Fig. 1, wurden 2'-Fluoro-modifizierte Spiegelmere unter Verwendung von 2'-fluorierten RNA-Molekülen verwendet.

Die in dem Selektionsprozeß benutzten Fluoro-modifizierten Nukleinsäuren (F-RNA) unterscheiden sich von biologisch vorkommender RNA durch das Vorhandensein einer FluoroGruppe am 2'-Kohlenstoff der Ribose. Fluor ersetzt hier die natürliche Hydroxylgruppe der Ribose. Diese Modifikation wurde hier auf Pyrimidinnukleotide begrenzt. Die Erstellung solcher künstlicher Nukleinsäuren erfolgt enzymatisch mit einer mutierten T7-Polymerase (Epicentre, Madison, WI, USA), die toleranter gegen modifizierte Nukleotide ist, unter Verwendung von 2'-Fluoro-UDP und 2'-Fluoro-CTP. Die Mutation der T7-Polymerase ermöglicht den effizienten Einbau von Fluoro-modifizierten Nukleosidtriphosphaten.

**A. Materialien**

GTP, ATP, und dNTPs wurden von Larova, Berlin; Fluoro-dUTP und Fluoro-dCTP von TriLink, San Diego, USA bezogen. Die T7 DNA/RNA Polymerase für die Herstellung fluorierter RNA wurde von Epicentre, USA bezogen. Die Enzyme Taq DNA Polymerase und Superscript II Reverse Transkriptase stammten von Life Technologies, der Kit für die Reverse Transkriptase/Taq Polymerase war von Qiagen. Radioaktives $^{32}$[P]-α-GTP zur Markierung der F-RNA wurde von Hartmann, Braunschweig, bezogen.

D-CGRP wurde zunächst von JERINI AG, Berlin, und später von Bachem, Heidelberg synthetisiert. Das zur Selektion verwendete Peptid trägt eine Biotingruppe am Carboxylterminus, um die Trennung von ungebundenen Nukleinsäuren mittels der Biotin-Streptavidin oder Biotin-NeutrAvidin Wechselwirkung zu ermöglichen. Hierfür wurden NeutrAvidin-Agarose von Pierce und *Streptavidin Paramagnetic Particles* von Roche verwendet. Unbiotinyliertes Peptid (ebenfalls von JERINI, Berlin, und Bachem, Bubendorf, Schweiz) wurde in den ersten sieben Runden zur Affinitätselution benutzt.

DNA-Pool

Die DNA für den Startpool wurde im Hause synthetisiert und basiert auf dem beschriebenem Pool PB40 mit der Sequenz 5'-GGA GCT CAG CCT TCA CTG C-$N_{40}$-GGC ACC ACG GTC GGA TCC AC-3' (Burgstaller & Famulok, 1994, Angew. Chem. Int. Ed. 33,1084).

Die 5'- und 3'-Primer hatten die Sequenz

PB40-R-For: 5'-TCT AAT ACG ACT CAC TAT AGG AGC TCA GCC TTC ACT GC-3' und
PB40-R-Rev: 5-GTA GAT CCG ACC GTG GTG CC-3'.

Die einzelsträngige DNA wurde auf ihre Amplifizierbarkeit mit radioaktiven Primem überprüf; daraus wurde eine Komplexität von 1,41 x$10^{14}$ Moleküle/nmol DNA abgeleitet. Mittels PCR wurde die einzelsträngige DNA in einem Gesamtvolumen von 8 ml zu doppelsträngiger DNA aufgearbeitet.

**B. Selektionsschritte**

**Denaturierung und Faltung der fluorierten RNA**

Alle nicht-enzymatischen Schritte der Selektion mit Ausnahme der Denaturierung wurden in Selektionspuffer (HEPES-KOH, pH7,5; 150 mM NaCl, 1 mM $MgCl_2$, 1 mM $CaCl_2$ und 0,1 % Tween 20) durchgeführt. Die Denaturierung erfolgte für 3 Minuten bei 94 °C in Selektionspuffer ohne Tween 20, $MgCl_2$ und $CaCl_2$. Nach der Denaturierung wurde die enzymatisch hergestellte F-RNA in Selektionspuffer zunächst für 30 Minuten bei 37 °C inkubiert, $MgCl_2$ und $CaCl_2$ wurden zugesetzt und die Faltung nochmals bei 37 °C für 30 Minuten fortgesetzt.

**Anbindung**

Im Anschluß an die Faltung wurde die F-RNA zunächst bei 37 °C für 15 Minuten ohne Peptid mit der Matrix (NeutrAvidin-Agarose oder Streptavidin-paramagnetische Partikel) inkubiert. Diese sogenannte Prä-Selektion diente der Vorisolierung von potentiellen Matrixbindern. Nach diesem Inkubationsschritt wurde die F-RNA von der Matrix getrennt, mit den aus Fig. 2A ersichtlichen Konzentrationen von biotinyliertem CGRP versetzt und für mindestens 3 Stunden bei 37 °C belassen. Daraufhin wurde dem Bindungsansatz die Biotin-bindende Matrix zugesetzt und nochmals 5-10 Minuten bei 37 °C inkubiert. Die Matrix wurde von der Lösung getrennt und mit Selektionspuffer gewaschen. Das hierbei verwendete Waschvolumen lag in den ersten Runden bei der 5-10-fachen Menge der Matrix, in späteren Runden wurden bis zu 60-fache Waschvolumina benutzt. Die nach dem Waschen an der Matrix verbleibende Menge an F-RNA wurde durch Szintillationszählung detektiert und quantifiziert. Der Anbindungswert wurde als Prozentsatz der eingesetzten F-RNA ausgedrückt.

Elution

In den Runden 1-9 wurde die bindende F-RNA in drei Schritten mit nicht-biotinyliertem Peptid eluiert. Hierbei wurde in der Regel ein 10-facher Überschuß des nicht-biotinyliertem gegenüber des biotinylierten CGRP eingesetzt und erst eine, dann drei Stunden und letztlich über Nacht bei 37°C eluiert. Die eluierte F-RNA wurde mit Phenol-Chloroform-Isoamylalkohol extrahiert, mit Ethanol gefällt und in Wasser resuspendiert. Die nach diesen Affinitätselutionsschritten auf der Matrix verbleibende RNA wurde nicht eluiert sondern direkt auf der Matrix amplifiziert (siehe unten).
Ab Runde 10 wurde nur noch in einem Schritt unter denaturierenden Bedingungen eluiert. Hierzu wurde die nach dem Waschen auf der Matrix verbliebene F-RNA in zwei Schritten mit jeweils 100 μl 8 M Harnstoff für 5 Minuten bei 65°C inkubiert. Die eluierte F-RNA wurde mit Phenol-Chloroform-Isoamylalkohol extrahiert, mit Ethanol gefällt und in Wasser aufgenommen.

**C. Amplifikation -Enzymatische Reaktionen**

**Transkription - Erstellung fluorierter RNA für Verwendung in der Selektion**

Transkriptionen wurden mit 150 U T7 RNA/DNA Polymerase und dem vom Hersteller mitgelieferten Puffer (40 mM Tris-HCl pH 7,5; 10 mM NaCl; 6 mM $MgCl_2$; 2 mM Spermidin; 10 mM DTT) in einem Gesamtvolumen von 100 μl durchgeführt. Den Reaktionen wurde generell $MgCl_2$ auf eine Endkonzentration von 11 mM zugesetzt. Die Endkonzentrationen von ATP und GTP betrugen jeweils 1 mM während der ganzen Selektion; 2'-Fluoro-UTP und 2'-Fluoro-CTP wurden bis in die 9. Runde mit einer Konzentration von jeweils 3 mM verwendet, ab der 9. Runde nur noch mit 1,5 mM. Pro Reaktion von 100 μl wurden zwischen 20 und 50 pmol Template eingesetzt, d. h. doppelsträngige DNA Template in der ersten Runde und in allen Folgerunden auf doppelsträngige DNA, welche durch enzymatische Aufarbeitung der im vorausgehenden Zyklus selektierten F-RNA erstellt. Für die radioaktive Markierung der fluorierten RNA wurde $^{32}$[P]-α-GTP benutzt. Die Reaktionen wurden über Nacht bei 37° C inkubiert und mit DNase I versetzt, um das Template zu verdauen. Die erzeugte F-RNA wurde daraufhin unter denaturierenden Bedingungen über ein 8 % Polyacrylamidgel mit 8 M Harnstoff von nicht eingebauten NTPs getrennt. Die transkribierte F-RNA wurde aus dem Gel eluiert, mit Ethanol gefällt, getrocknet und in reinem Wasser aufgenommen.

**Reverse Transkription - Erstellung von cDNA von selektierten F-RNAs**

Die reverse Transkription eluierter Fluoro-RNA wurde mit Superscript II und den Pufferbedingungen des Herstellers in einem Volumen von 20 μl mit 200 units Enzym und einer dNTP Konzentration von jeweils 1 mM durchgeführt. In der Regel wurden bis zu 8 pmol der eluierten F-RNA in 10 μl Wasser gelöst und mit 2 μl einer 100 μM Lösung des reversen Primers vermischt. Dieses Template-Primer-Gemisch wurde für 2 Minuten bei 94 °C denaturiert, auf Eis überführt und dann im Thermocycler auf eine Temperatur von 50 °C eingestellt. Nach 2 Minuten bei 50 ° C wurden 8 μl eines Gemischs aus Puffer, dNTPs und Enzym zugesetzt und die Probe für 15 Minuten bei 50 °C, weitere 15 Minuten bei 55 °C und letztlich 10 Minuten bei 68 °C inkubiert.

In den Runden 1-9 wurde die nach der Affinitätselution auf der Matrix verbliebene F-RNA auch direkt auf dem Träger revers transkribiert und mit Hilfe der PCR amplifiziert. Hierfür wurde der RT-PCR Kit von Qiagen benutzt.

**PCR- Erstellung des doppelsträngigen DNA Templates**

Die in der reversen Transkription erzeugte cDNA wurde direkt in die PCR eingesetzt. Dabei dienten 6,66 μl des 20 μl RT-Ansatzes als Template für eine 100 μl Reaktion mit 5 units Taq DNA Polymerase. Die Konzentration der Primer belief sich auf 2,5 μm. Die DNA wurde zunächst für 2 Minuten bei 94 °C denaturiert und dann mit einem PCR-Profil von 30 Sekunden bei 94 °C, 30 Sekunden bei 55 °C und 30 Sekunden bei 72 °C amplifiziert. Die Anzahl der Zyklen wurde generell so gering wie möglich gehalten und belief sich in der Regel auf etwa 5-10 Zyklen.

Resultate

Der Verlauf der Selektion ist in Fig. 2 dargestellt. Die in Fig. 2A angegebenen Werte stellen die in der jeweiligen Runde verwendeten CGRP-Konzentrationen und die dazugehörige prozentuale Anbindung des F-RNA Pools dar.

Abgebildet ist dabei nur der Strang, der letztendlich auch die nachstehenden Sequenzen hervorbrachte. In den meisten Runden wurde die Anbindung mit unterschiedlichen Peptidkonzentrationen sowie einer Kontrollselektion ohne Peptid durchgeführt (nicht dargestellt). Generell wurde die selektierte F-RNA des stringentesten Stranges, d. h. der geringsten Peptidkonzentration, welche noch ein signifikantes Signal über der Nullkontrolle ergab, als Template für die nächste Runde aufgearbeitet. Fig. 2B ist eine graphische Darstellung dieser Daten.

Die erste Selektionsrunde wurde mit 2,5 nmol F-RNA-Molekülen durchgeführt, die Komplexität hatte eine Größenordnung von etwa 2,82 x $10^{14}$ unterschiedlichen Sequenzen. Da unter diesen Bedingungen jede Sequenz nur etwa fünf mal vertreten war, wurde in der ersten Runde ein relative hoher Prozentsatz selektierter Sequenzen in die 2. Selektionsrunde überführt. Von der 2. bis zur 5. Runde wurden zwischen 0,36 % und 2,65 % der eingesetzten F-RNA in die jeweilige Folgerunde eingesetzt. Während diesen fünf Runden wurde die Stringenz durch eine Verringerung des zur Bindung angebotenen biotinyliertes D-CGRP von 33 μM auf 3,33 μM angezogen. In der 6. Runde war ein leichter Anstieg in der Anbindung zu verzeichnen, der sich mit einem Wert von 12.8 % der eingesetzten F-RNA in der 7. Runde fortsetzte.

Durch kontinuierliche Verringerung der CGRP-Konzentration in den Folgerunden wurde die Stringenz zunehmend verschärft, was zu einem Einbruch der Anbindung der eingesetzten F-RNA an das Peptid führte. Eine kontinuierliche Zunahme der Anbindungswerte in Runde 8 und 9 bis auf 5,14 % in Runde 10 korrelierte mit einem Anstieg in der Prä-Selektion (nicht dargestellt) und war somit nicht auf peptidvermittelte Bindung zurückzuführen. Aus diesem Grund wurde in Runde 11 die Matrix von NeutrAvidin-Agarose auf Streptavidin-paramagnetische Partikel gewechselt. Dies spiegelte sich in einem Rückgang der prozentualen Anbindung auf nunmehr 1,23 % wider. Ein peptidvermittelter Anstieg auf 5,74 % war jedoch in Runde 13 zu verzeichnen. Über die nächsten drei Runden stellte sich ein Wert von etwa 3,5 % Anbindung bei einer CGRP Konzentration von 125 nM ein. Da dieser Wert trotz gleichbleibender Stringenz in den Runden 15, 16 und 17 (Runde 17 nicht abgebildet) nicht weiter anstieg, wurden die Runden 17 und 18 als sogenannte Doppelrunde durchgeführt. Die Überlegung für diesen methodischen Ansatz beruht auf der Annahme, daß eine Stagnation der prozentualen Anbindung bei gleichbleibender Peptidkonzentration die Folge einer Art Gleichgewicht zwischen der eigentlichen Anbindung und der darauffolgenden Amplifizierung ist. Um dieses Gleichgewicht zu Gunsten der Bindung zu verschieben, wurde die F-RNA einem zweifachen Bindungsprozeß unterworfen: Die F-RNA wurde zunächst bei der weniger stringenten CGRP-Konzentration von 1,25 μM angebunden, daraufhin eluiert und gereinigt. Die auf diese Weise gesammelte F-RNA wurde nun nicht wie üblicherweise durch enzymatische Amplifizierung aufgearbeitet, sondern neu gefaltet und direkt in eine weitere Selektionsrunde unter stringenteren Bedingungen eingesetzt. Wie aus Fig. 2A und B ersichtlich, konnten durch diese Methode in Runde 18 bei Peptidkonzentrationen von 1 und 10 nM noch jeweils 4,89 % und 31,51 % Anbindung der eingesetzten F-RNA (wie aus Fig. 2A ersichtlich) erzielt werden. Diese F-RNA wurde revers transkribiert und durch PCR amplifiziert. Die DNA wurde kloniert, und es wurden insgesamt 192 Klone sequenziert.

**Sequenzen**

Das Ergebnis der Sequenzanalyse ist in Fig. 3 dargestellt. Dabei ist die kursiv dargestellte Sequenz (dritte Sequenz von oben) diejenige des Klons 732. Mutationen sind in Fettdruck dargestellt, der Beitrag der Primerbindungsstelle ist durch Unterstreichung markiert. Für die verschiedenen Klone wurde die folgende Reihenfolge festgestellt:

| Position in Fig. 3 | Klon-Bezeichnung | Häufigkeit | SEQ.ID.No. |
|---|---|---|---|
| 1 | 666 | 168 x | 1 |
| 2 | 711 | 1 x | 2 |
| 3 | 732 | 2 x | 3 |
| 4 | 669 | 5 x | 4 |
| 5 | 670 | 1 x | 5 |
| 6 | 781 | 1 x | 6 |
| 7 | 836 | 1 x | 7 |
| 8 | 748 | 1 x | 8 |

Von den 192 Klonen konnten in 180 Klonen beide Primer gefunden werden. Von diesen 180 Sequenzen war eine Sequenz mit 168 Exemplaren deutlich am häufigsten vertreten. Die übrigen zwölf Sequenzen unterschieden sich von der Hauptsequenz nur durch Punktmutationen und Basendeletionen: Eine Punktmutation kam dabei fünfmal, fünf weitere je einmal vor. Eine Deletion von zwei Nukleotiden kam zweimal vor.

Ein Vergleich der Bindungsstärke dieser Sequenzen wurde unter Verwendung der Biacore-Vorrichtung angestellt und ergab, daß sieben von den acht Sequenzen dieselbe Affinität für das Targetmolekül zeigen. Der $K_D$ des um zwei Nukleotide verkürzten Klons wurde mit ca. 10 nM vermessen. Die Bindungskonstante lag bei 37 °C um den Faktor zwei höher. Bindungskonstanten wurden sowohl mit der Biacore-Vorrichtung, als auch mit Isothermaler Kalorimetrie (ITC) bestimmt. Die Kalorimetrie-Experimente wurden bei 37 °C in entgastem Selektionspuffer mit einer Rührgeschwindigkeit von 300 U/min durchgeführt. Die Messzelle wurde mit einer 10 µM Lösung des jeweiligen 2'F-Spiegelmers gefüllt. Die Injektionsspritze enthielt eine 25 µM Lösung CGRP, die nach einer initialen 5 µl Injektion in 7,5 µl Fraktionen in die Messzelle eingespritzt wurde. Der Injektionsvorgang dauerte jeweils 10 s, das Zeitintervall zwischen zwei Injektionen betrug 300s.

Die Biacore-Experimente wurden bei 37°C in entgastem Selektionspuffer durchgeführt. Auf dem Streptavidin-Chip war biotinyliertes CGRP immobilisiert (Blank - 140 RU - 640 RU - 900 RU). Spiegelmer-Lösung in einer Konzentration von 500 ergab ein typisches Bindungssignal, das mit Standard-Biacore-Software (1:1 Bindungsmodell) ausgewertet wurde.

**<u>Beispiel 2</u>: Verkürzung von 2'-F-RNA-Aptameren, die freies D-CGRP binden**

Ausgehend von den in Beispiel 1 beschriebenen D-CGRP-bindenden 2'-F-RNA-Aptameren und den hierzu korrespondierenden 2'-F-RNA-Spiegelmeren, die freies L-CGRP erkennen können, wurde versucht, die Länge der jeweiligen Aptamere bzw. Spiegelmere zu verkürzen. Von den insgesamt acht Klonen, wie in Beispiel 1 dargestellt, zeichneten sich sieben Klone durch ähnliche Bindungskonstanten von 10 bis 50 nM aus. Der Klon 670 erkannte D-CGRP deutlich schlechter. Wie erwartet, erkannten die selektierten Aptamere, die D-Oligonukleotide, die von entsprechend aus der Klonierung und Sequenzierung erhaltenen Plasmiden über einen zwischengeschalteten PCR-Schritt exprimiert wurden, natürliches L-CGRP nicht, wie Messungen am Biacore ergeben haben.

Die Sekundärstruktur einiger der in Beispiel 1 beschriebenen Aptamere und Spiegelmere wurde mit dem Programm *rnafold* (I.L. Hofacker, et al., 1994, Monatsh. Chem 125, 167-188) bestimmt. Im besonderen werden die Sekundärstrukturmodelle der Aptamers 666 und 732 sowie der Spiegelmere 732-029, 732-026, 732-100 und 732-108 gezeigt. Die Ergebnisse sind in Figs. 4 und 5 sowie für die Spiegelmere in den Figs. 6, 7, 8 und 9 dargestellt. Die Sekundärstrukturmodelle stimmten gut mit den Ergebnissen von enzymatischen *Probing*-Experimenten überein (Ehresmann, C., et al., 1987, Nucleic Acids Res 15(22): p. 9109-28).

Die Sekundärstrukturen bestehen jeweils aus einem Stamm, zwei Haarnadelschleifen und einer 15- oder 16-nt langen Auswölbung und bilden damit eine Kleeblatt-ähnliche Struktur. Die Auswölbungen im Strukturkern, geformt von C(10)-C(11)-U(12) und C(54)-U(55)-C(56), und die Haarnadelschleife U(13) bis A(26) scheinen essentiell für die Erkennung des Zielmoleküls CGRP zu sein. Für CGRP bindende Aptamere bzw. L-CGRP-bindende Spiegelmere kann somit ein minimales Bindungsmotiv festgehalten werden, welches eine Kleeblatt-ähnliche Struktur aufweist. Ein alternatives minimales Bindungsmotiv ist die Ausbildung eines Stammes mit zwei Haarnadelschleifen und einer 15 oder 16 Nukleotide umfassenden Auswölbung. Bevorzugterweise wird die Auswölbung im Strukturkern gebildet durch die Nukleotidabfolge C(10)-C(11)-U(12) und C(54)-U(55)-C(56)und die Haarnadelschleife durch die Nukleotidabfolge U(13) bis A(26), wie in den Figs. 4 bis 9 dargestellt.

Aufgrund der gemessen Bindungsaffinität zum Zielmolekül CGRP mit einem $K_d$ von 10 nM wurde Klon 666 als Ausgangsklon für die Verkürzung und Optimierung von 2'-F-RNA Aptameren und Spiegelmeren ausgewählt. Wie in Beispiel 1 beschrieben, wurde Klon 666 aus einem N40 Pool, d. h. einem Pool dessen randomisierte Sequenz eine Länge von 40 Nukleotiden aufweist, isoliert und bestand aus einer 79-nt langen Sequenz. Die Sequenz hat einen besonders auffälligen U-reichen Bereich zwischen den Nukleotidposition 41-50, in dem 7 von 10 Nukleobasen Uracile sind. Die Sekundärstruktur des Klons 666 wurde mit dem Programm mafold I.L. Hofacker, et al., 1994, Monatsh. Chem. 125, 167-188 vorhergesagt und ist in Fig. 4 dargestellt. Die Sekundärstruktvorhersage stimmte auch in diesem Fall gut mit den Ergebnissen enzymatischer Probing-Experimente überein (Ehresmann, C. et al., 1987, Nucleic Acids Res. 15 (22), 9109-28). Das Aptamer bildet eine charakteristische Kleeblatt-ähnliche Struktur, bestehend aus einem Stamm mit mehreren kleinen Auswölbungen, zwei Haarnadelschleifen (I und III) und einer scheinbar unstrukturierten 16-nt Auswölbung (II), die die beiden Haarnadelschleifen verbindet. Alle vier Strukturelemente haben ihren Ursprung in zwei gegenüberliegenden Auswölbungen bestehend aus jeweils drei Basen. Die Primerbindungsstellen, vor allem die 5'-Primerbindungsstelle, sind im Stamm integriert.

Für die Synthese von 2'-F-RNA Spiegelmeren war es notwendig, die Sequenz von Klon 666 soweit zu verkürzen und zu optimieren, daß das resultierende minimale Bindungsmotiv eine chemische Synthese mit vertretbarem Aufwand erlaubt bei gleichzeitigem Erhalt der hohen Affinität zum Zielmolekül.

Ein minimales Bindungsmotiv wurde unter Verwendung von direkten Nukleotidpunktmutationen und -deletionen identifiziert. Eine Serie von Deletionen mit Tetranukleotidblöcken ergab eine erste Übersicht über Regionen, die für die Bindung des Zielmoleküls essentiell sind. Deletionen in allen drei Doppelhelixregionen waren möglich und führten nicht zu einer signifikanten Verringerung der Bindungsaffinität, allerdings nur solange, wie eine Stammbildung noch möglich war. Sobald zu viele Deletionen zum Verlust der Stammbildung führten, war keine Bindung an das Zielmolekül CGRP mehr zu beobachten. Dies war zu beobachten, wenn der schließende Stamm des Nukleinsäurebinders entweder auf weniger als fünf Basenpaare oder die Haarnadelschleifen I und III auf weniger als 3 Basenpaare verkürzt wurden.

Deletionen in den drei Schleifen führte zu unterschiedlichen Ergebnissen.

Eine jeder der im Folgenden beschriebenen Ausführungsformen der folgenden Sequenzen stellt eine erfindungsgemäße Nukleinsäure dar. Das Ausschneiden der Nukleotide G(20)-A(21) in Haarnadelschleife I (in Analogie zu Klon 732) führte zu einem Nukleinsäurebinder der weiterhin hochaffin war ($K_d$ = 20 nM), jede weitere getestete Deletion oder Mutation in Schleife 1 führte jedoch zu einem kompletten Bindungsverlust. In Schleife III konnten die Nukleotide A(46) und U(47) ohne Bindungsverlust herausgenommen werden. Wie zu erwarten, führte die Deletion einer der Basen U(48), U(49) oder U(50) in Kombination mit A(46) zum gleichen Ergebnis. Verschiedene Verkürzungen waren ebenfalls in Schleife II möglich, im speziellen wurde das Herausnehmen der Basen G(28), U(29), A(30), U(31), A(33) und A(35) toleriert, allerdings führte es zu verschieden starken Veränderungen in der Bindungsaffinität. Eine Kombination dieser Deletionen war ausschließlich im Fall der Co-Deletion von U(29)-A(30) möglich. Der Stamm der Haarnadelschleife I erlaubte eine weitere Verkürzung der Stammlänge auf drei Basenpaare bei der Deletion des Basenpaares A(15) und U(24) (die Stammlänge wird bereits bei der Deletion der Nukleotide G(20)-A(21) in der Schleife I von 5 Basenpaare auf 4 Basenpaare verkürzt (Klon 732, Fig. 5)). Die Auswölbung C(10) bis U(12) und C(54) bis C(56) scheinen essentiell für die Erkennung des Zielmoleküls bzw. für die hierzu benötigte Tertiärstruktur zu sein, da bereits die Deletion eines dieser Nukleotide zum vollständigen Verlust der Erkennung des Zielmoleküls CGRP führt.

Zusammenfassend läßt sich somit feststellen, daß insbesondere die Schleife der Haarnadelschleife I und die zentrale Auswölbung der Kleeblattstruktur essentiell für die Bindung des Zielmoleküls sind. Die Haarnadelschleife III, die kleineren Auswölbungen im Zentrum der Kleeblattstruktur und der daran anschließende Stamm dienen wahrscheinlich der Stabilisierung einer speziellen Tertiärstruktur.

Weitere Verkürzungen konnten mit dem fast vollständigen Herausnehmen der 3'-Primerbindungsstelle C(64) bis C(79) und dem Herausnehmen des Dinukleotides A(3)-G(4), einem kleinen Teil der 5'-Primerbindungsstelle aus dem abschließenden Stamm des Nukleinsäurebinders, erzielt werden. Eine derartige Verkürzung stellt der Klon 732_029 dar. Diese Verkürzung ging einher mit einem nur geringfügigen Affinitätsverlust ($K_d$ = 30 nM). Der abschließende Stamm konnte weiter durch die Deletionen der Basenpaare C(5):G(61) und U(6):G(60) auf fünf Basenpaare verkürzt werden ($K_d$ = 30 nM, wie dies auch in Fig. 7 beschrieben ist).

Das Austesten verschiedener Kombinationen dieser unterschiedlichen Deletionen führte zu Klon 732_100 (Länge 51 nt, $K_d$ = 75 nM, Fig. 8). Hierbei wurden die Deletionen A(3)-G(4), C(5):G(61), U(6):G(60), G(20)-A(21), A(15): U(24), A(46)-U(47) und C(64) bis C(79) miteinander kombiniert. Dieses 51mer bindet mit einem $K_d$ von 75 nM an das Zielmolekül CGRP. Eine Analyse der mittels des Programms *rnafold* vorhergesagten Struktur zeigt allerdings eine potentielle Alternativfaltung, die durch eine Mutation des Basenpaares C(14):G(25) zu G(14):C(25) (Klon 732_103) bzw. des Basenpaares C(44):G(52) nach G(44):C(52) (Klon 732_104) verhindert werden kann. In der Tat zeigen diese beiden Klone auch eine erhöhte Affinität zu CGRP mit einem $K_d$ von 20 bzw. 40 nM. Klon 732_104 läßt sich weiter mit der Deletion G(32) kombinieren, wodurch man Klon 732_108 erhält, ein 50mer mit einem $K_d$ von 35 nM (Fig. 9).

Die Sequenzen 732_026 und 732_029 wurden für die Herstellung von Spiegelmeren zur Bestimmung ihrer biologischen Aktivitäten ausgewählt. Da kein Festphasenmaterial mit L-2'-F-modifiziertem C zur Verfügung stand, wurde zunächst getestet, ob eine Mutation des jeweils endständigen GC-Basenpaares in ein CG-Basenpaar entsprechend den Klonen 732_045 bzw. 732_096 (Fig. 10 und Fig. 11) ohne meßbaren Verlust der Bindungsaffinität möglich war. Da nahezu identische $K_d$-Werte für 732_045 bzw. 732_096 im Vergleich zu 732_026 bzw. 732_029 erhalten wurden, wurden diese für die Produktion von Spiegelmeren zur Bestimmung ihrer biologischen Aktivitäten ausgewählt.

Eine weitere Methode, um ein minimales Bindungsmotiv zu finden, wurde verfolgt, als Klon 666 oder verkürzte Formen davon aus zwei unabhängig voneinander synthetisierten Fragmenten kombiniert wurden. In zwei Vorversuchen wurde eine Fragmentierung in jeweils einer der beiden Haarnadelschleifen I und III getestet. Zum einen G(1) bis C(19) und A(22) bis C(79) (dargestellt mit Klon 732_070a und 732_070b, Fig. 26) und zum anderen G(1) bis A(46) + AG und C+U(49) bis C(79) (dargestellt mit Klon 732_071a und 732_071b, Fig. 27). Das zusammengesetzte System 732_070ab zeigte keine meßbare Bindungsaffinität zu CGRP. Dieses Ergebnis verstärkt die Vermutung, daß die Haarnadelschleife I in den Bindungsprozeß mit CGRP involviert ist, wie auch Daten des enzymatischen *Probing*-Experimenten nahelegen. Die Fragmente 732_071a + 732_071b dagegen bilden einen Nukleinsäurebinder, der mit einem $K_d$ von 100 nM an das Zielmolekül CGRP bindet. Weitere Experimente zeigten, daß der Stamm der ehemaligen Haarnadelschleife m mindestens fünf Basenpaare lang sein muß, um ein meßbares Bindungsereignis zu erhalten. Das Zusammensetzen eines Nukleinsäurebinders aus zwei Fragmenten ist eine effiziente Alternative, um ein minimales Bindungsmotiv zu erhalten. Es gestattet die Synthese und Produktion von relativ langen Nukleinsäurebindern in einem großen Syntheseumfang unter vertretbarem wissenschaftlichen und auch wirtschaftlichen Aufwand. Für die Generierung von zusammengesetzten CGRP-bindenden Nukleinsäurestrukturen gelten die gleichen Grundlagen wie für einteilige Nukleinsäurebinder. Die Affinität und Bioaktivität des resultierenden erfindungsgemäßen Nukleinsäurebinders 732_071ab war vergleichbar mit denen der einteiligen Nukleinsäurebindern 732_108, 732_096 und 732_045 in den gemessenen Bereichen in den verwendeten Assays (732_071ab, $K_d$ = 100 nM), 732_108 $K_d$ = 35 nM, 732_096 $K_d$ = 30 nM und 732_045 $K_d$ = 30 nM).

Die Spiegelmer/L-CGRP- und Aptamer/D-CGRP-Komplexe ergaben innerhalb der experimentellen Fehlerabweichung übereinstimmende Gleichgewichtsbindungskonstanten.

Die Nukleinsäurebinder 732_045, 732_096 und 732_071ab wurden ausgewählt für weiterfiihrende biologische Studien und in den entsprechenden enantiomeren Formen synthetisiert.

Die verschiedenen, im Rahmen dieses Beispiels erzeugten und verwendeten Sequenzen sind in Fig. 18 dargestellt. Den in Fig. 18 dargestellten Sequenzen kann dabei die jeweils folgende SEQ.ID.No. zugeordnet werden:

| Sequenz | SEQ. ID. No. |
|---|---|
| 666 | 9 |
| 732 | 10 |
| 732_026 | 11 |
| 732_029 | 12 |
| 732_045 | 13 |
| 732_096 | 14 |
| 732_100 | 15 |
| 732_103 | 16 |
| 732_104 | 17 |
| 732_108 | 18 |
| 732_070a | 19 |
| 732_070b | 20 |
| 732_071a | 21 |
| 732_071b | 22 |

**Beispiel 3: Selektion von 2'-F-RNA-Aptameren**

Es wurde eine weitere Selektion durchgeführt mit dem Ziel, 2'-F-RNA-Aptamere gegen D-CGRP zu identifizieren. Die dabei angewandten Reaktionsbedingungen sind die folgenden:

Bei dem verwendeten Zielmolekül handelt es sich um CGRP aus Ratten. Sequenz und Bereitstellung erfolgte analog zu der in Beispiel 1 beschriebenen Vorgehensweise.

*Selektionspool, Erstellung des Startpools*

Der Selektionspool SK60 besteht aus einem randomisierten Bereich von 60 Nukleotiden, welcher von dem T7-Primer (37 Nukleotide) vom 5'-Ende und vom reverse-Primer (20 Nukleotide) am 3'-Ende flankiert wird. Der T7 Primer beinhaltet einen Transkriptions- initiierenden und einen Forward-Primerbereich. Der Forward-Primer fängt zur Verbesserung der Transkriptionseffizienz mit einen Guanosintriplett an.

| | |
|---|---|
| SK60 Pool: | 5'-GGG AAT TCG AGC TCG GTA CC -$N_{60}$-CTG CAG GCA TGC AAG CTT GG-3' |
| SK.60T7: | 5'-**TAA TAC GAC TCA CTA TA**G GGA ATT CGA GCT CGG TAC C-3' |
| SK.60F: | 5'-GGG AAT TCG AGC TCG GTA CC-3' |
| SK.60R: | 5'-CCA AGC TTG CAT GCC TGC AG-3' |

Die Annealing-Temperatur für die Primer wurde theoretisch berechnet und im berechneten Rahmen anschließend experimentell optimiert.

| | $T_m$ (°C) | $T_p$ (°C) | Verwendet (°C) | |
|---|---|---|---|---|
| For | 56.1 | 68.72 | RNA | 2'-F-RNA |
| Rev | 56.9 | 68.72 | 68-72 | 70-72 |
| $T_m$ = Schmelztemperatur, $T_p = 22 + 1.46 \times [2 \times (\#GC) + (\#AT)]$ = optimale Annealing-Temperatur mit optimaler Amplifikation, nach Wu *et al.,* 1991, *DNA and Cell Biology* **10**, 233; ohne T7-Promotor-Region. | | | | |

Der Pool wurde synthetisch hergestellt, die Basenzusammensetzung bestimmt und eine Komplexität von $1x10^{15}$ Molekülen entsprechend 1,78 nmol ssDNA über Polymerase-Kettenreaktion (engl. *polymerase chain reaction* [PCR]) amplifiziert. 1,78 nmol der doppelsträngigen DNA wurden in der *in vitro* Transkription mit fluorierten Pyrimidin-Nukleotiden (TriLink BioTechnologies, San Diego, CA92121) in 2'-fluoroierte RNA nach Protokoll 1 umgeschrieben. Der so hergestellte 2'-F-SK60 RNA Startpool wurde in die erste Selektionsrunde eingesetzt.

| **Komponente** | **Stock-Konzentration** | **100 µl Ansatz** |
|---|---|---|
| T7 Buffer (Epicentre) | 5 x | 20 |
| DTT | 100 mM | 5 |
| $Mn^{2+}$ | 25 mM | 10 |
| 2'-F-CTP | 100 mM | 3 |
| 2'-F-UTP | 100 mM | 3 |
| rATP | 100 mM | 1 |
| rGTP | 100 mM | 1 |
| PCR-Template | ca.100 pmol/µl | 0,5 |
| T7-Polymerase | 5 U/µl | 2 |
| ddH2O | - | 54,5 |

Protokoll 1. Ansatz der *in vitro* Transkription für die 1. Runde

*Selektionspuffer*

Der Selektionspuffer (20 mM HEPES, 150 mM NaCl, 5 mM KCl, 1 mM $MgCl_2$ und 1 mM $CaCl_2$) ist an physiologische Bedingungen des menschlichen Bluts angelehnt und während der ganzen Selektion verwendet. Der pH Wert von 7,4 wurde bei 37 °C eingestellt.

*Selektionsstränge und Stringenzen*

Es wurden 2 verschiedene Selektionsansätze zur Identifizierung einer CGRP bindenden 2'-Fluoro-RNA durchgeführt, deren Charakteristika in der folgenden Tabelle zusammengefaßt sind.

**Tab.: Charakteristika der Selektionsansätze gegen rCGRP-1**

| | **Ansatz 1- NA-A/D** | **Ansatz 2 - SA** |
|---|---|---|
| **Immobilisierungsmatrix** | Neutravidin-Agarose | Streptavidin magnetische beads |
| **Elution** | in zwei Schritten<br>1. Affinitätselution<br>2. denaturierende Elution | denaturierende Elution |
| **Amplifikation** | getrennte Amplifikation der Eluate | gemeinsame Amplifikation der Eluate |

Der erste Ansatz wurde unter Verwendung von Neutravidin-Agarose (Pierce) als Matrix zur Immobilisierung des 2'-F-RNA/ Peptidkomplexes durchgeführt. Die gebundene 2'-Fluoro-RNA wurde in 2 Schritten von der Matrix eluiert. Zunächst wurden die Binder über Affinitätselution durch Kompetition mit einem Überschuß an nicht-biotinyliertem CGRP eluiert. Anschließend erfolgte eine Elution mit 8 M Harnstoff durch Denaturierung des 2'-Fluoro-RNA-Peptidkomplexes. Die Eluate wurden getrennt in der Amplifikation aufgearbeitet, um einen Selektionsdruck seitens der enzymatischen Reaktionen zu limitieren.

Der zweite Selektionsansatz wurde auf Steptavidin-derivatisierten magnetischen Partikeln als Matrix zur Immobilisierung des 2'-Fluoro-RNA-Peptidkomplexes durchgeführt. Die Elution erfolgte ebenfalls mit 8 M Harnstoff durch Denaturierung des RNA/ Peptidkomplexes.

*Faltung der 2' Fluoro-RNA*

Um eine $Mg^{2+}$- und $Ca^{2+}$-Ionen unabhängige Faltung der 2'-Fluoro RNA zu erhalten, wurde die Denaturierung und Renaturierung (Faltung) in Selektionspuffer ohne $Ca^{2+}$- und $Mg^{2+}$-Ionen durchgeführt. Die 2'-Fluoro-RNA wurde in Selektionspuffer ohne $Mg^{2+}/Ca^{2+}$ für 5 Minuten bei 95 °C denaturiert und anschließend für 30 Minuten auf 37 °C gefaltet. Danach erfolgte die Zugabe der Ionen und eine weitere Faltung für 10 Minuten bei 37 °C. Der Ansatz wurde direkt auf eine Vorsäule gegeben.

*Vorsäulen*

Die Vorsäule wurde zur Gegenselektion (counter-Selektion) von potentiell Matrix-bindenden 2'F-RNA Molekülen durchgeführt und dient somit generell der Abreicherung Matrix-bindender 2'-F-RNA innerhalb des Selektionsprozesses. Unter einer Vorsäule versteht man eine unbeladene Säule, welcher der eigentlichen Peptidsäule innerhalb einer Selektionsrunde vorgeschaltet ist. Die Vorsäule wurde gleich der Hauptsäule dimensioniert. Die 2'-F-RNA wurde für 30 Minuten bei 37 °C mit der Matrix inkubiert, und anschließend wurde die 2'-F-RNA mit einem Säulenvolumen Selektionspuffer von der Matrix gewaschen. Die an der Matrix verbleibende markierte RNA wurde bestimmt und als Vorsäule dokumentiert. Die eluierte 2'-F-RNA wurde für den Selektionsprozeß weiter verwendet.

Der Verlauf der Vorsäulen während der Selektion, d. h. der an der Matrix verbleibende Anteil markierter RNA, ist in Fig. 12 dargestellt. Dabei ist beachtlich, daß ab der Runde 13 die Selektion SA in zwei Selektionsstränge aufgeteilt wurde, nämlich Selektionsstrang SA-1 und SA-0,1, die sich in der Peptidkonzentration unterschieden. Im Falle von SA-1 betrug die Peptidkonzentration in Runde 13 10 nM und in den Runden 14 bis 16 1 nM, wohingegen im Selektionsansatz SA-0,1 die Konzentration an Peptid in der 13. Runde 10 nM und anschließend in den Runden 14 bis 16 nur 0,1 nM betrug.

*Anbindung und Immobilisierung*

Zu der 2'F-RNA in Lösung wurde direkt das biotinylierte D-CGRP zugegeben. Die Konzentration des biotinylierten Peptids im Bindungsansatz wurde im Laufe des Selektionsprozesses, wie in der nachfolgenden Tabelle dargestellt, sukzessive verringert.

**Tab.: Verringerung der Peptidkonzentration**

| | NA-A/D | SA-1 | SA-0,1 |
|---|---|---|---|
| **1.Runde** | 15 μM | 3,4 μM | 3,4 μM |
| **2.Runde** | 3 μM | 1 μM | 1 μM |
| **3.Runde** | 3 μM | 1 μM | 1 μM |
| **4.Runde** | 1,5 μM | 500 nM | 500 nM |
| **5.Runde** | 1,5 μM | 500 nM | 500 nM |
| **6.Runde** | 1,5 μM | 500 nM | 500 nM |
| **7.Runde** | 500 nM | 500 nM | 500 nM |
| **8.Runde** | 500 nM | 500 nM | 500 nM |
| **9.Runde** | 100 nM | 100 nM | 100 nM |
| **10.Runde** | 100 nM | 100 nM | 100 nM |
| **11.Runde** | 10 nM | 100 nM | 100 nM |
| **12.Runde** | 10 nM | 50 nM | 50 nM |
| **13.Runde** | 10 nM | 10 nM | 10 nM |
| **14.Runde** | 10 nM | 1 nM | 0,1 nM |
| **15.Runde** | 10 nM | 1 nM | 0,1 nM |
| **16.Runde** | 10 nM | 1 nM | 0,1 nM |

Die Anbindung des biotinylierten rCGRP-2'-F-RNA Ansatzes an die Neutravidin- bzw. Steptavidin-derivatisierte Matrix erfolgte durch direkte Zugabe des Bindungsansatzes auf die Matrix. Die Bindung erfolgte im Thermoschüttler (Eppendorf) für 10 Minuten bei 37 °C mit einer Schüttelgeschwindigkeit von 800 UpM.

*Waschen*

Der an der Matrix immobilisierte bio-CGRP-2'F-RNA Komplex wurde mit Selektionspuffer gewaschen, um nicht gebundene 2'F-RNA in einzelnen Waschschritten von der Säule zu entfernen. CGRP-1 bindende 2'F-RNA verbleibt dabei auf der Matrix. Ein Waschvolumen betrug 100 μl Selektionspuffer. Während der Selektion wurde die Stringenz der Selektion über die Erhöhung der Waschschritte angezogen, wie dies auch aus der nachfolgenden Tabelle ersichtlich ist.

**Tab.: Verlauf der Waschschritte**

| | NA-A/D | SA-1 | SA-0,1 |
|---|---|---|---|
| **1. Runde** | 400 μl | 400 μl | 400 μl |
| **2. Runde** | 400 μl | 400 μl | 400 μl |
| **3. Runde** | 600 μl | 600 μl | 600 μl |
| **4. Runde** | 600 μl | 600 μl | 600 μl |
| **5. Runde** | 600 μl | 1000 μl | 1000 μl |
| **6. Runde** | 1000 μl | 1000 μl | 1000 μl |
| **7. Runde** | 1000 μl | 1200 μl | 1200 μl |
| **8. Runde** | 1000 μl | 1200 μl | 1200 μl |
| **9. Runde** | 1000 μl | 1200 μl | 1200 μl |
| **10. Runde** | 1000 μl | 1200 μl | 1200 μl |
| **11. Runde** | 1000 μl | 1200 μl | 1200 μl |

(fortgesetzt)

| | NA-A/D | SA-1 | SA-0,1 |
|---|---|---|---|
| **12. Runde** | 1000 µl | 1200 µl | 1200 µl |
| **13. Runde** | 1000 µl | 1200 µl | 1200 µl |
| **14. Runde** | 2000 µl | 1200 µl | 1200 µl |
| **15. Runde** | 2000 µl | 5000 µl | 5000 µl |
| **16. Runde** | 2000 µl | 5000 µl | 5000 µl |

*Elution der gebundenen 2'-Fluoro-RNA*

Selektionsansatz NA-A: Die Elution der an der Matrix über das Peptid gebundenen 2'-F-RNA erfolgte zunächst über Kompetition mit nicht-biotinyliertem CGRP in einem 10-fachen Überschuß über dem immobilisiertem bio-CGRP. Die Inkubation erfolgte für 12 Stunden bei 37 °C im Überkopfschüttler. Anschließend erfolgte eine Elution der verbleibenden 2-F-RNA durch Denaturierung des 2'-F-RNA / Peptidkomplexes durch Zugabe von 8 M Harnstoff. Die Inkubation erfolgte bei 65 °C für 20 Minuten im Thermoschüttler bei einer Schüttelgeschwindigkeit von 1200 UpM.

Selektionsansatz SA-1/0,1: Die Elution erfolgte durch Denaturierung des 2'-F-RNA/ Peptidkomplexes durch Zugabe von 8 M Harnstoff. Die Inkubation erfolgte bei 65 °C für 20 Minuten in einem Thermoschüttler bei einer Schüttelgeschwindigkeit von 1200 UpM.

Der Verlauf der eluierten 2'-F-RNA und der Peptidkonzentration des Selektionsansatzes NA-A ist in Fig. 13 dargestellt. Es konnte eine Anreicherung bindender 2-F-RNA in der sechsten Selektionsrunde bei 1 µM Peptidkonzentration gezeigt werden. Die Peptidkonzentration wurde kontinuierlich bis auf 10 nM reduziert. Eine Reduktion der Peptidkonzentration erfolgte jeweils nur nach erneuter Anreicherung bindender 2'-F-RNA bei gegebener Peptidkonzentration, wie dies in den Runden 6, 8, 10 und 13 dargestellt ist. Die Selektion wurde ab Runde 13 bis 16 bei 10 nM Peptid in Plateau geführt, und die angereicherten Nukleinsäuren wurden kloniert und sequenziert.

Der Verlauf der eluierten 2'-F-RNA und der Peptidkonzentration der Selektionsstränge SA-1 und SA-0,1 ist in Abb. 14 A/B dargestellt. Die Selektionen SA-1 und SA-0,1 wurden bis zur 13. Runde gemeinsam als ein Selektionsstrang geführt und dann in die unterschiedlichen Selektionsstränge SA-1 und SA-0,1 mit unterschiedlicher Fortführung der Peptidkonzentration bis zur Runde 16 geführt, kloniert und sequenziert. Eine Reduktion der Peptidkonzentration erfolgte dabei jeweils nur nach erneuter Anreicherung bindender 2'-F-RNA bei gegebener Peptidkonzentration, wie dies in den Runden 8, 11 und 13 dargestellt ist.

***Amplifikation***

*Extraktion und Fällung*

Die eluierte 2'-F-RNA wurde mittels Phenol-Chloroform-Extraktion gereinigt und anschließend mit Glycogen als Carrier, 0,3 M Natriumacetat pH 5,5 und 3 Volumina 50:50 (v/v) Ethanol-Isopropanol für 30 Minuten bei -80°C gefällt.

*Reverse Transkription (RT)*

Die gefällte 2'-F-RNA wurde mittels reverser Transkription in einzelsträngige DNA (ssDNA) umgeschrieben. Es werden < 5pmol 2'-F-RNA-Templat pro 40 µl Ansatz eingesetzt. Zunächst wurden der 10 µM SK60- reverse Primer nach Denaturierung (5 Minuten 95 °C) des 2'-F-RNA Templates in der Anwesenheit von 0,8 M Betain durch direktes Abkühlen der Probe auf Eis (sogenanntes. *snap cooling*) annealed. Anschließend wird der *first strand* Puffer (250 mM Tris/HCl, pH 8,3, 375 mM KCl, 15 mM $MgCl_2$), 10 mM DTT und Desoxyribonukleinsäure zugegeben. Die Pufferbedingungen entsprechen denen für das Enzym Superscript II (Standardbedingungen des Herstellers). Der Ansatz wird für 2 Minuten auf 48 °C erwärmt, und 5 Units der Reversen Transkriptase werden hinzugegeben. Die Inkubation erfolgte über einen Temperaturstufengradienten (48 °C 30 Minuten, 50 °C 20 Minuten, 55 °C 10 Minuten, 70 °C 15 Minuten) in einem PCR Thermocycler.

*Polymerase-Kettenreaktion (Polymerase-chain-reaction)*

Jeweils 10 µl des RT-Ansatzes wurden als Template für die PCR eingesetzt, was einer Template-Menge von 1-5 pmol/100 µl PCR Ansatz entspricht. Die Nukleotidsequenzen der Primer sind:

SK.60T7: 5'-**TAA TAC GAC TCA CTA TA**G GGA ATT CGA GCT CGG TAC C-3'
SK.60R: 5'-CCA AGC TTG CAT GCC TGC AG-3'

Der PCR-Ansatz wurde wie folgt zusammen gegeben und bis zu einer Ausbeute an dsDNA von ca. 1 pmol/µl Ansatz bei 1 Minute 94 °C Denaturierung, 1 Minute 72 °C Annealing und 1 Minute 72 °C Elongation gefahren. Die Reaktion wurde auf einem denaturierenden 8 %-igen Polyacrylamidgel analysiert.

| **Komponente** | **Stock-Konzentration** | **100µl Ansatz** |
|---|---|---|
| PCR-Puffer (Gibco) | 10x | 10 |
| $MgCl_2$ | 50 mM | 5 |
| dNTP-Mix (A, T, G, C) | 10 mM je NTP | 2,5 |
| T7-Primer | 100 mM | 3 |
| reverse-Primer | 100 mM | 3 |
| Template (aus RT) | 1-5 pmol/100 µl | 10 |
| Taq-Polymerase (Gibco) | | 1 |
| ddH20 | - | 65,5 |

Protokoll 2 : PCR-Reaktion während der Selektion

Die PCR Ansätze wurden mit Ethanol gefällt, in Wasser resuspendiert, und 50-100 pmol Material wurden für die *in vitro* Transkription eingesetzt.

*2'-Fluoro Transkription*

50 pmol dsDNA wurden als Template für die Transkription mit Fluoro-Pyrimidinnukleosidtriphosphaten eingesetzt. Das Verhältnis der 2'-Fluroro-Pyrimidinnucleosidtriphosphaten zu den nicht-modifizierten Purinnukleosidtriphosphaten ist 3:1. Die Inkubation wurde über Nacht bei 37 °C in einem Thermoblock durchgeführt.

*4. Ergebnisse*

*Verlauf der Selektionen*

Die Selektionen wurden in der ersten Runde als Sammelrunde angelegt, um möglichst keine bindenden F-RNA-Moleküle zu verlieren. In den folgenden Runden wurde über die Intensivierung der Waschschritte, die Verringerung der Peptidkonzentration, sowie die Erhöhung des RNA:Peptid-Verhältnisses die Stringenz hinsichtlich der Selektion hochaffiner bindender 2'-F-RNA kontinuierlich angezogen.
Es konnte für den Selektionsstrang NA eine erste Anreicherung an CGRP-bindender 2-F-RNA in den Runden 6 und 8 gezeigt werden. Nach Erhöhung der Stringenzfaktoren und anschließendem Einbruch des Signals konnte jeweils ein erneuter Anstieg an bindender 2'-F-RNA beobachtet werden. Die Selektion wurde schließlich bei einer Peptidkonzentration von 10 nM hinsichtlich des Signals in ein Plateau geführt, und die Eluate (Affinitätselution und denaturierende Elution) wurden getrennt amplifiziert, kloniert und sequenziert. NA-A bezeichnet die Sequenzen der Eluate der Affinitätselution und NA-D die Eluate der anschließenden denaturierenden Elution.
Der Selektionsstrang SA zeigte in Runde 8 eine Anreicherung an CGRP bindender 2'-F-RNA. Eine Reduktion der Peptidkonzentration führte immer zunächst zu einem Einbruch des Signals mit anschließender erneuter Anreicherung (Runden 11 und 13). Nach der Runde 13 wurde der Selektionsstrang in die beiden Stränge SA-1 und SA-0,1 gespalten. SA-1 wurde bei einer Peptidkonzentration von 1 nM, SA-0,1 bei einer Peptidkonzentration von 100 pM in ein Signalplateau gebracht; beide Strängen wurden dann getrennt amplifiziert, kloniert und sequenziert.

*Sequenzen:*

Im Rahmen der Selektion wurden die folgenden Sequenzen erhalten:

**Familien**

Tab x.:

| # | Sequenz (von 5' nach 3' dargestellt) |
|---|---|
| 1 | *GGGAAUUCGAGCUCGGU-*<br>*ACC* **UUAACCCGUAUGGGGUCACUGUUUCGAUUUCAUUCGCCUUAUCGAGCCGAUUCACUU**<br>**GCG** *CUGCAGGCAUGAAGCUUGG* |
| 2 | *GGGAAUUCGAGCUCGGU-*<br>*ACC* **UUGUUACCCACUGUUUAGUAUCUCGCGAUACUCAUUACCGAGACACAGUCCCAUUAC**<br>**UGC** *CUGCAGGCAUGAAGCUUGG* |
| 3 | *GGGAAUUCGAGCUCGGU-*<br>*ACC* **GCACUUUCGUUACAUACGAUAUACUGGGCUAUAGUCUAUCCUUGUGCCUACAGGUAC**<br>**UG** *CUGCAGGCAUGAAGCUUGG* |
| 4 | *GGGAAUUCGAGCUCGGU-*<br>*ACC* **UACUGCUCGACUAAUUGUCUAGUACAAUAUGCUUACCACAUUAUCUGUUAGUGAGCU**<br>**CC** *CUGCAGGCAUGAAGCUUGG* |
| 5 | *GGGAAUUCGAGCUCGGU-*<br>*ACC* **UUGUUCUGACUCUGUUUAUGCGUUUUCCGCGUCUUUACCGGACUCCUUCUUCCCCAG**<br>**UGC** *CUGCAGGCAUGAAGCUUGG* |
| 6 | *GGGAAUUCGAGCUCGGU-*<br>*ACC* **UAUCGUCGAACAUUCGAUCUGUUUUUACGUAAGUAACUUUACCGUCCUCGUUUUUCC**<br>**CGC** *CUGCAGGCAUGAAGCUUGG* |
| 7 | *GGGAAUUCGAGCUCGGU-*<br>*ACC* **AAACAUCACUUACAUGUGCUCUGCGUUUUUUGCAUAGUUUUUUGGUCGAGCGCUUCC**<br>**UCC** *CUGCAGGCAUGAAGCUUGG* |
| 8 | *GGGAAUUCGAGCUCGGU-*<br>*ACC* **GCGGAGUCUGUCACAAGAUCUCGUCCUUAUCGUUGAUGUAUCGUACAAGUCUUUGCC**<br>*CUGCAGGCAUGAAGCUUGG* |

(fortgesetzt)

| # | Sequenz (von 5' nach 3' dargestellt) |
|---|---|
| 9 | *GGGAAUUCGAGCUCGGU-ACC* **UAAUACGACUNACUAUAGGGAAUUCGAGCUNGGUACCUUAACCCGUAUGGGGUUACUG** *CUGCAGGCAUGAAGCUUGG* |
| 10 | *GGGAAUUCGAGCUCGGU-ACC* **AAUGCCUGCUUUGUUUUGAGUUUUCCUUCACACUAGGGAUGGAUAAUACAGUCCUUACC** *CUGCAGGCAUGAAGCUUGG* |

| Sequenz | Ursprung aus Selektion | Anzahl der Sequenzen | insgesamt sequenziert | Anzahl der mutierten Position |
|---|---|---|---|---|
| **1** | SA-1<br>SA-0,1<br>NA-A<br>NA-D | 18<br>14<br>13<br>13 | 33<br>33<br>45<br>26 | 7 |
| **2** | SA-1 | 2 | 33 | 0 |
| **3** | SA-1<br>SA-0, 1<br>NA-A<br>NA-D | 2<br>2<br>7<br>3 | 33<br>33<br>45<br>26 | 9 |
| **4** | SA-1 | 2 | 33 | 0 |
| **5** | SA-0,1 | 6 | 33 | 3 |
| **6** | SA-0,1<br>NA-D | 3<br>9 | 33<br>26 | 3 |
| **7** | SA-0,1 | 2 | 33 | 1 |
| **8** | SA-0,1 | 2 | 33 | 0 |
| **9** | NA-A | 5 | 45 | 6 |
| **10** | NA-D | 5 | 26 | 4 |

Die vorstehend genannten Sequenzen entsprechen dabei den folgenden SEQ.ID.No.:

| Sequenz | SEQ.ID.No. |
|---|---|
| 1 | 23 |
| 2 | 24 |
| 3 | 25 |
| 4 | 26 |
| 5 | 27 |
| 6 | 28 |
| 7 | 29 |
| 8 | 30 |
| 9 | 31 |
| 10 | 32 |

*Ranking*

Ein Ranking der Klone wurde über Bindungsversuche gegen 1 μM D-CGRP in Lösung ermittelt. Die gefaltete, radioaktiv markierte RNA wurde 2 Stunden bei 37 °C mit dem Peptid inkubiert, über Neutravidin-Agarose immobilisiert, gewaschen, und die prozentuale Anbindung der 2'-F-RNA an CGRP wurde ermittelt. Die Klone D7, E1, G10 und E3 zeigten die höchste prozentuale Anbindung. Das Ergebnis ist auch in Fig. 19 dargestellt. Die Zuordnung der Herkunft der Klone aus den jeweiligen Selektionssträngen NA-A (Affinitätselution), NA-D (denaturierende Elution), SA-1 und SA-0,1 ist unter der x-Achse dargestellt.

**Kompetition**

Zur weiteren Charakterisierung der Klone C5, E1, F1 D7, B3 und F10 wurden Kompetitionstests durchgeführt. Das Ergebnis ist in Fig. 20 dargestellt. Die Kompetition durch nicht-biotinyliertes D-CGRP wurde durch Inkubation des biotinylierten D-CGRP (1 μM) und des freien CGRP (10 μM) bei 37 °C mit radioaktiv markierter 2-F-RNA für 2 Stunden bestimmt. Es konnte gezeigt werden, daß die Klone C5, E1 und D7 durch freies CGRP kompetierbar sind, wohin gegen die Klone F1, B3 und F10 das nicht-biotinylierte CGRP nicht erkennen.

**Bead-Assay zur Bestimmung der Dissoziationskonstanten**

Jeweils 50 pmol der markierten 2'-F-RNA der Klone E1, D7 und 732 (Referenz, Ursprung der 2'-F-RNA-Selektion mit Pool PB40) wurden gefaltet und für 3 Stunden mit unterschiedlichen bio-CGRP-Konzentrationen in Lösung bei 37 °C im Thermoschüttler inkubiert. Anschließend wurde eine konstante Menge magnetischer Steptavidin-Beads (Roche) als Matrix zugegeben und der 2'-F-RNA/ Peptid-Komplex für 10 Minuten bei 37 °C und einer Schüttelgeschwindigkeit von 800 UpM immobilisiert. Die magnetische Partikelmatrix wurde mittels eines Magnetseparators getrennt, der Überstand abgenommen und die Differenz der gebundenen/ungebundenen 2'-F-RNA ermittelt. Von den ermittelten Werten wurde die Kontrolle (0 μM bio-CGRP) als Background abgezogen. Die ermittelten Werte wurden in das Software-Programm GraphFit (Erithacus Software Ltd.) eingegeben und die Dissoziationskonstante bestimmt.

Die abgeschätzte Dissoziationskonstante ist danach 196 nM für E1 und 42 nM für D7. Der Referenzklon 732 (siehe Anwendungsbeispiel 1) aus der Selektion mit dem Startpool PB40 als interner Kontrolle für den hier verwendeten Bead-Assay ergab eine Dissoziationskonstante von 23 nM. Das Ergebnis ist in den Figs. 21 und 22 dargestellt. Weitere erhaltene Sequenzen sind in den Figs. 48 und 49 dargestellt.

**<u>Beispiel 4:</u> Verwendung von Nukleinsäuren ohne Primer-Bindungsstellen für die Selektion von D-CGRP bindenden Nukleinsäuren**

Für die hierin beschriebenen Untersuchungen wurden die folgenden Materialien verwendet, wobei die Angaben zu den Herstellern der folgend aufgeführten Substanzen, Lösungen und Enzyme bei den entsprechenden Textpassagen aufgeführt wurden. In allen Fällen wurde LiChromosolv Wasser von Merck verwendet.

Ratten α-D-CGRP wurde von Bachem, Heidelberg synthetisiert. Das zur Selektion verwendete Peptid trägt eine Biotingruppe am Carboxylterminus um die Trennung von ungebundenen Nukleinsäuren mittels der Biotin-Streptavidin oder Biotin-Neutravidin Anbindung zu ermöglichen. Hierfür wurde als Matrix Neutravidin-Agarose und Ultralink Plus Immobilized Streptavidin Gel (beides von Pierce) verwendet.

Die verwendeten Oligonukleotide, d. h. RNA- und DNA-Nukleotide, wie Primer, Startpool, Ligationskonstrukte und dergleichen wurden alle bei der NOXXON Pharma AG mit Standard-Phosphoramiditchemie synthetisiert. Die Sequenzen finden sich in der folgenden Übersicht.

**Verwendete und getestete Oligonukleotide**

**STAR-1 Oligonukleotide**

Für die hierin offenbarten Sequenzen gilt hinsichtlich der Schreibweise folgendes:

Angaben in *kursiv Schrift* bezeichnen Nukleotide der Ligationsmatrizen, die mit der Bibliothek hybridisieren.
Der <u>unterstrichene</u> Bereich einer Sequenz bedeutet, dass dieser Bereich dem *T7* RNA-Polymerase-Promotor entspricht.
$N_{OH}$ bezeichnet allgemein ein Ribo-Nukleotid.
$C_{Ome}$ bezeichnet ein 2'-OMethyl-modifiziertes Cytosin.
P bezeichnet ein 5'-Phosphat

Übersicht der verschiedenen Sequenzen:

**STAR-1-Pool während der Selektion:**
*pGGAC- $(N)_{40}$-GACAGG*

**STAR-1-Pool während der Ligation:**

```
GCG ACU ACU AAT ACG ACU CAC UAUA'GGAC-(N)40-GACAGG'ACG CTG AGC TGA ACT CG C
                            TGC TGA GTG ATAT.CCTG        CTGTCC.UCG GAC UCG ACT TGA GC G
```

*<u>Bibliothek</u>*

**STAR-1 initialer Pool, Reverse Strang:**
5' $C_{OMe}C_{OMe}$T GTC-$(dN)_{40}$-GTC CTA TAG TGA GTC GTA TTA GTA GTG CGA AG 78 nt

*Forward*

**STAR-1 Forward Primer:**
5' GCG ACT ACT AAT ACG ACT CAC TAT A GGAC 29nt

**STAR-1 Forward Ligat (RNA):**
5' GCG ACU ACU AAU ACG ACU CAC UAU A 25nt

**STAR-1 Forward Matrix:**
5' *GTCC* TA TAG TGA GTC G 3'dT 17 nt

*Reverse*

**STAR-1 Reverse Primer Ribo U:**
5' GCG AGT TCA GC $U_{OH}$ CAG CG $U_{OH}$ *CCT GTC* 24 nt

**STAR-1 Reverse Primer:** nur für die Amplifikation vor der Sequenzierung
5' GCG AGT TCA GCT CAG CGT *CCT GTC* 24 nt

**STAR-1 Reverse Matrix entspricht STAR-1 Reverse Primer**

*Reverse Ligate:*

**STAR-1 Reverse Ligat:**
pACG CTG AGC TGA ACT CG 3' dC 18 nt

**STAR-1 N+1-Reverse Ligat**
pCG CTG AGC TGA ACT CG 3'dC 17 nt

**STAR-1 Reverse Matrix = STAR-1 Rev Primer Ribo U:**
5' GCG AGT TCA GC $U_{OH}$ CAG CG $U_{OH}$ CCT GTC 24 nt

**STAR-1 N+1-Reverse Matrix Ribo I:**
5' GCG AGT TCA GCT CAG CG $I_{OH}$ CCT GTC 24 nt

Herstellung des Startpools

Für eine Komplexität von $1x10^{15}$ Molekülen wurden 1,67 nmol Startpool in die Selektion eingebracht. Entsprechend wurde 33,4 Transkriptionen mit 50 pmol *"STAR-1 initialer Pool, Reverse Strang*" (5' $C_{OMe}C_{OMe}$T GTC-$(dN)_{40}$-GTC CTA TAG TGA GTC GTA TTA GTA GTG CGA AG) als Templat einer 100 µl Reaktion verwendet. Zur Bewerkstelligung eines DNA-Doppelstranges im Bereich des T7 Promotors wurde *"STAR-1 Forward Primer"* im 1,5 fachen Überschuß zu *"STAR-1 initialer Pool, Reverse Strang"* dem Reaktionsansatz zugefügt. Die RNA wurde im Anschluß gelgereinigt und gefällt (Transkription und Gelreinigung siehe unten).

**Selektionsschritte**

***Denaturierung und Faltung der RNA***

Alle nicht-enzymatischen Schritte der Selektion - mit Ausnahme der Denaturierung - wurden in Selektionspuffer (10 mM Hepes-KOH pH 7,5 (Biochrom AG); 150 mM NaCl; 4 mM KCl; 1 mM $MgCl_2$; 1 mM $CaCl_2$ (alle Ambion) und 0,05 % Tween-20 (Sigma)) durchgeführt. Die Denaturierung erfolgte für 5 Minuten bei 95°C in Selektionspuffer ohne Tween-20, $MgCl_2$ und $CaCl_2$. Nach der Denaturierung wurde die RNA zunächst für 15 Minuten bei 37°C (oder Raumtemperatur) auf 37°C (oder Raumtemperatur) abgekühlt. Tween-20, $MgCl_2$ und $CaCl_2$ wurden zugesetzt und die Faltung nochmals bei 37°C (oder Raumtemperatur) für 15 Minuten fortgesetzt.

***Anbindung***

Im Anschluss an die Faltung wurde die RNA zunächst bei 37°C (oder Raumtemperatur) für 30 Minuten ohne Peptid entweder mit der Matrix Neutravidin-Agarose oder mit Ultralink Plus Immobilized Streptavidin Gel inkubiert. Diese sogenannte Vorselektion diente der Abtrennung von potentiellen Matrixbindern. Nach diesem Inkubationsschritt wurde die ungebundene RNA von der Matrix mittels Filtration durch MobiTec-Säulchen getrennt, mit verschiedenen Konzentrationen von biotinyliertem CGRP versetzt und für mindestens 2 Stunden - bei niedrigen Peptidkonzentrationen gegebenenfalls auch über Nacht - bei 37°C (oder Raumtemperatur) belassen. Daraufhin wurde dem Bindungsansatz die biotinbindende Matrix zugesetzt. Die Matrix mit der daran gebundenen RNA wurde nach 30 min von der Lösung durch Zentrifugation getrennt und mit Selektionspuffer gewaschen. Das hierbei verwendete Waschvolumen lag in den ersten Runden bei der 5-10fachen Menge der Matrix, in späteren Selektionsrunden bei 25fachem Waschvolumen.

Elution der bindenden RNA-Moleküle

Hierzu wurde die nach dem Waschen auf der Matrix verbliebene RNA zweimal mit jeweils 400µl 8 M Harnstoff mit 10 mM EDTA (beide von Ambion) für 15 Minuten bei 65° C vom Matrixmaterial eluiert. Die eluierte RNA wurde mit 400 µl Phenol/(Chloroform/Isoamylalkohol)-Gemisch (1:(1:1/24)) (Applichem) versetzt, 5 min bei 13000 rpm bei Raumtemperatur zentrifugiert, die wässrige Phase (Überstand) abgenommen, die phenolische Phase einmal mit 100 µl Wasser re-extrahiert, die wässrigen Phasen vereinigt und mit 500µl Chloroform-Isoamylyalkohol-Gemisch (24:1) (Applichem) geschüttelt, 5 min bei 13000 rpm auf Raumtemperatur zentrifugiert und die obere wässrige Phase abgenommen.

Die wässrige Phase wurde daraufhin mit 2,5 fachen Volumen 100% Ethanol (Fluka), 0,3 M Natriumacetat (Ambion) und 1 µl Glycogen (Roche) für 30 min bei -80°C gefällt und für 30 min bei 14.000 rpm (4°C) zentrifugiert. Das Pellet wurde einmal mit eiskaltem 70 %igem Ethanol (Fluka) gewaschen.

**Ligation und Amplifikation**

Vorbereitung für die Ligation

Entscheidend für eine erfolgreiche Ligation war die Ermittlung der zu ligierenden RNA-Menge. Die eluierte RNA-Menge wurde über die Radioaktivität und - wenn möglich - über die OD bestimmt. Die OD-Bestimmung hat den Nachteil, dass ein Minimalvolumen von 50 µl nötig ist. Für den Ligationsansatz durfte die RNA aber nicht in zu großem Volumen aufgenommen werden.

Deshalb wurde zu Beginn einer jeden Selektionsrunde die spezifische Aktivität [cpm/pmol] bestimmt. Damit ließ sich die Menge der eluierten RNA gut abschätzen.

***Abschätzung der $H_2O$-Menge zur Lösung des Pellets:***

Da die Ligationsansätze für Templat-Mengen von bis 5 pmol Templat beziehungsweise ab 5 pmol Templat optimiert wurden, wurde für die folgende Berechnung der $H_2O$-Menge das entsprechendene Protokoll gewählt. Mit Hilfe des Ligationsprotokolls wurde die maximale Menge $H_2O$ bestimmt und das Pellet entsprechend gelöst.

Ligation

Für die Ligation wurden in Vorversuchen 2 Ansätze mit unterschiedlichen (Templatmenge)/(Reaktionsvolumen + Enzymmenge)-Verhältnissen bestimmt. Die Ansätze variieren zudem, wenn statt der hier aufgeführten "Simultan-Ligation" die "Parallele-2-Schritt-Ligation" durchgeführt wurde (siehe unten).

**Ansätze bis 5 pmol: "Simultan Ligation"**

Volumen des Ansatzes: 14 µl pro 1 pmol RNA-Templat

| [Stammlsg.] | Komponente | [final] |
|---|---|---|
| | eluierte RNA, in $H_2O$ gelöst | alles |
| 10x | Ligation Buffer (MBI Fermentas) | 1x |
| 50% | PEG 4000 (MBI Fermenats) | 5% |
| 10 μM | ds F-Adapter (F Ligat RNA/F Matrix) | 20x [eluierte RNA] |
| 10 μM | ds R-Adapter 1 (Rev Ligat/ Rev Primer Ribo U) | 1 0 x [eluierte RNA] |
| 10 μM | ds R-Adapter 2 (Rev Ligat/ N+1 Matrix dI) | 1 0 x [eluierte RNA] |
| 5 min auf 50°C + 5 min auf 25°C | | |
| **[Stammlsg.]** | **Komponente** | **[final]** |
| | RNAse out (Invitrogen) | 0,5 μl |
| 30 U/μl | T4 DNA-Ligase (MBI Fermentas) | 36 U/pmol RNA (1,2 μl) |
| 16 h auf 25°C + 15 min bei 65°C | | |

**"Parallele-2-Schritt-Ligation"**: In diesem Fall wurde die gelöste RNA in zwei Ansätze geteilt. Der erste Ansatz wurde zunächst am 5'-Ende ligiert, während der zweite Ansatz zunächst am 3'-Ende ligiert wurde. Dementsprechend wurden zunächst jeweils nur die entsprechende Menge *"ds F- oder R-Adapter"* in den Ansatz gegeben. Nach dem ersten Ligationschritt wurden entsprechend dann die jeweils noch fehlenden *"ds F- oder R-Adapter"* zugesetzt, 5 min. auf 50°C erhitzt und danach erneut RNAse-out und Ligase dazugegeben.

**<u>Ansätze ab 5 pmol: "Simultan Lieation"</u>**

Volumen des Ansatzes: 14 μl pro 5 pmol RNA-Template

| [stock] | component | [final] |
|---|---|---|
| | eluierte RNA, in $H_2O$ gelöst | Alles |
| 10x | Ligation Buffer (MBI Fermentas) | 1x |
| 50 % | PEG 4000 (MBI Fermentas) | 5% |
| 100 μM | ds F-Adapter (F Ligat RNA/F Matrix) | 20x eluierte RNA |
| 100 μM | ds R-Adapter (Rev Ligat/ Rev Primer Ribo U) | 10 x eluierte RNA |
| 100 μM | ds R-Adapter n+1 (Rev Ligat/ N+1 Matrix dI) | 10 x eluierte RNA |
| 5 min auf 50°C + 5 min bei 25°C | | |
| **[stock]** | **Component** | **[final]** |
| | RNAse out (Invitrogen) | 0,5 μl |
| 30 U/μl | T4 DNA-Ligase (MBI Fermentas) | 7,2 U/pmol RNA (0,24 μl) |
| 16 h auf 25°C +15 min bei 65°C | | |

**"Parallele-2-Schritt-Ligation"**: (wie oben beschrieben)

**Ligation nach der ersten Runde**

Im Gegensatz zu dem oben beschriebenen Protokoll, ist nach der 1. Runde die Zugabe von *ds R-Adapter 2 (für n+1Transkripte)* nicht notwendig, da die Transkription vor der 1. Runde durch Einsatz von zwei terminalen 2'-O-Methyl-Nukleotiden sauber terminiert wurde. Deshalb wurd im Anschluß an die erste Runde nur ds R-Adapter 1 (Rev Ligat/ Rev Primer) im 20x Überschuß zur eluierten RNA eingesetzt.

Reverse Transkription

Die Reverse Transkription fand - direkt im Anschluss an die Ligation - mit bis zu 15 pmol ligierter RNA in einem Volumen von bis zu 100 µl statt. Bei größeren Stoffmengen müssen mehrere Parallelansätze gemacht werden. Die Größe des rT-Ansatzes ist durch die unterschiedlich konzentrierten Ligationsansätze bestimmt (Simultan-Ligation/Parallele-2-Schritt Ligation bis bzw. ab 5 pmol).

Eckpunkte für die RT waren:

| [stock] | component | [final] |
|---|---|---|
| 71,4 nM | RNA (bei Simultan-Ligation bis 5 pmol) | 30 nM |
| 357 nM | RNA (bei Simultan-Ligation ab 5 pmol) | 150 nM |
| 60 nM | RNA (bei Parallele-2-Schritt-Ligation bis 5 pmol) | 25 nM |
| 300 nM | RNA (bei Parallele-2-Schritt -Ligation ab 5 pmol) | 125 nM |
| | | |
| 5x | 1st strand buffer (Invitrogen) | 1x |
| 5x | Q solution (Qiagen) | 1x |
| 100 mM | DTT (Invitrogen) | 10 mM |
| 25 mM | dNTPs (Larova) | 0,5 mM |
| 200 U/µl | Superscript (Invitrogen) | 200 U |

Die Temperaturbedingungen waren: 20 min auf 51°C + 10 min auf 54°C; dann 4°C

PCR

PCR wurde mit maximal 1 pmol Template pro 100-µl-PCR durchgeführt. Wenn eine größere Stoffinenge RNA eluiert wurde, mussten mehrere PCRs parallel angesetzt werden. Wenn man bedenkt, dass vor Beginn eines Anstieges häufig nur 1 pmol eluiert werden und somit die Kompexität nicht größer als die Anzahl der Moleküle in einem pmol sein kann, kann man ohne Zweifel auch nur einen Teil (> 1 pmol) der cDNA PCR-amplifizieren (man muss nicht die gesamte cDNA in PCR einsetzen). Dies wurde ab Runde 5 so gehandhabt.

Grund für den Einsatz von maximal 1 pmol Ligationstemplat in die PCR ist die Ligationsmatrix für die n+1-Transkripte (3'-Mikroheterbgenitäten in der Transkription). Die genutzte Ligationsmatrix mit der "universal"-Base (2'-Desoxyinosin) wird in die PCR verschleppt und kann dort auch zwangsläufig als Reverse Primer primen. So entsteht ein nicht spaltbares PCR-Produkt. Je mehr Templat in die PCR gebracht wird, desto mehr Ligationsadapter werden verschleppt und desto mehr unspaltbares PCR-Produkt entsteht. Deshalb sollte nie mehr als 1 pmol Templat in die PCR eingesetzt werden.

Wird dagegen Inosin als "universal base" für die Ligationsmatrix verwendet, kann bis zu 5 pmol (oder mehr, nicht getestet) Templat in die PCR eingesetzt werden. In diesem Fall sind auch die durch die Ligationsmatrize geprimten PCR-Produkte alkalisch verdaubar (Wurde in dieser Selektion nicht verwendet). Wie bei der Ligation und reversen Transkription gab es zwei verschiedene PCR-Protokolle für Simultan-Ligation/Parallele-2-Schritt Ligation bis bzw. ab 5 pmol.

Eckpunkte für die PCR waren:

| [Stammlösung] | Komponente | [Endkonzentration] |
|---|---|---|
| 30 nM | cDNA (bei Simultan-Ligation bis 5 pmol) | 1-10 nM |
| 150 nM | cDNA (bei Simultan-Ligation ab 5 pmol) | 1-10 nM |
| 25 nM | cDNA (bei Parallele-2-Schritt-Ligation bis 5 pmol) | 1-10 nM |
| 125 nM | cDNA (bei Parallele-2-Schritt-Ligation bis 5 pmol) | 1-10 nM |

| | | |
|---|---|---|
| 10x | PCR-Puffer Roche) | 1x |
| 100 μM | Forward-Primer | 5 *μM* |
| 100 μM | Reverse-Primer (RiboT) | 5 μM |
| 10 mM | dNTPs (Larova) | 0,2 mM |
| 5 U/μl | Taq DNA-Polymerase (Roche) | 5 U |

| **Temperaturbedingungen für den STAR-1-Pool**: | | | |
|---|---|---|---|
| 15 Zyklen | 1. | 95 °C | 0 min |
| | 2. | 95°C | 4 min |
| | 3. | 95°C | 1 min |
| | 4. | 68°C | 1 min |
| | 5. | 72°C | 1 min |
| | 6. | Goto 3 14x | |
| | 7. | 72°C | 6 min |
| | 8. | 4°C | unendl. |

In der Regel wurden 13-15 PCR-Zyklen gefahren.

Alkalische Spaltung des Reverse-Stranges und Testgel

Zunächst wurden 10 μl PCR-Produkt testweise alkalisch gespalten. Das Standardrezept für die analytische alkalische Spaltung des Reverse-Stranges sah wie folgt aus:

| **[Stammlsg.]** | **Komponente** | **Final** | **V [μl]** |
|---|---|---|---|
| | PCR-Produkt | | 10 |
| 2,5 M | NaOH (Merck) | 1/7 vol. des PCR-Prod. = 310 mM | 1,43 |
| 10 min auf 95°C im PCR-Block, auf 4°C abgekühlt, auf Eis weiterarbeitet | | | |
| **[Stammlsg.]** | **Komponente** | **Final** | **V [μl]** |
| 3 M | NaOAc (Ambion) | 1/25 vol. des PCR-Prod. (ca. 0,1 nM) | 0,4 |
| 2,5 mm | HCl (Merck) | 1/7 vol. des PCR-Prod. = V(NaOH) | 1,43 |

Im Anschluss wurde der pH-Wert mittels Indikatorpapier überprüft. Der pH-Wert sollte in etwa bei pH 5-6 liegen.

Das gespaltene PCR Produkt wurde mittels eines ssDNA-Standards (Länge 78 nt, zur Probe wurde 3,55 μmol NaCl (Ambion) zugegeben, um Salzeffekt auszugleichen) auf einem 10 % denaturierenden PAGE quantifiziert, um zu bestimmen, welches Volumen alkalisch gespaltenes PCR-Produkt in die folgende *in vitro*-Transkription (Ziel-Menge: 50 pmol PCR-Produkt pro 100-μl-Ansatz) eingesetzt werden muss. In der Regel wurden 100 pmol PCR-Produkt alkalisch verdaut (für zwei 100 μl *in vitro*-Transkriptionen).

Ethanolfällung mit Natriumacetat

Die Ethanolfällung mit Natriumacetat (Ambion) diente an dieser Stelle hauptsächlich zum Entsalzen der Proben.

Es wurde 1/10 Volumen der PCR an 3 M NaOAc (pH 5,3), 2,5fache PCR-Volumen an 100 % Ethanol und 1-2 μl Glycogen (20μg/ml) hinzugefügt. Der Ansatz wurde 30 min bei -80°C gefällt, 30 min bei 13.000 - 14.000 rpm bei 4°C zentrifugiert, das Pellet einmal mit 500 μl 70 %igem Ethanol gewaschen und 5 min bei 13.000 - 14.000 rpm abzentrifugiert. Das Pellet wurde in Wasser (1/10 des ursprünglichen PCR-Volumens) resuspendiert oder der T7-Transkriptionsansatz direkt auf das Pellet pipettiert (und dann resuspendiert).

T7-Transkription

Während der T7-Transkription wurde radioaktives alpha-$^{32}$P-GTP (Hartmann) eingebaut. In die *in vitro* Transkription wurden 50 pmol Templat (pro 100 μl Ansatz) eingebracht. Als Besonderheit gegenüber anderen Transkriptionen wurde ein 8-facher Überschuss an Guanosin-5'-Monohosphat (8 x GMP) über GTP zugegeben, so dass die meisten Transkripte ein 5'-Monophosphat getragen haben dürften.

Eckpunkte für die *in vitro* Transkription waren:

| [Stammlsg.] | Komponente | final |
|---|---|---|
| | alkalisch verdautes PCR-Produkt | 50 pmol |
| 10x | Txn-Puffer (SK) | 1x |
| 100 mM | DTT (Invitrogen) | 10 mM |
| 10 mg/ml | BSA (Invitrogen) | 0,12 mg/ml |
| 25 mM | NTPs (Larova) | 4 mM |
| 250 mM | 5'-GMP (Sigma) | 32 mM |
| | RNAse out (Invitrogen) | 1 μl |
| 5 U/μl | T7-RNA Polymerase (Invitrogen) | 2 μl |

Der 10x Txn-Puffer (SK) enthält 800 mM Hepes/KOH (Biochrom), 220 mM MgCl2 (Ambion), 10 mM Spermidin (Fluka), pH 7,5.

Die *in vitro*-Transkription wurde für 8-16 Stunden bei 37°C durchgeführt. Im Anschluß wurde die verbliebene DNA mit ca. 10 units DNAse I (Sigma) verdaut (20 min bei 37°C). Der Verdau wurde durch Zugabe von EDTA (Ambion) gestoppt (Endkonzentration 25 mM).

Gelreinigung der transkribierten RNA

Den 100-μl-Transkriptionsansätzen wurde je 50μl konzentrierte Harnstoff-Lösung (Roth) mit Blaumarker (7,2 M urea, 10% Bromphemolblau (Merck)) zugesetzt, 5 min bei 95°C denaturiert und auf ein präparatives denaturierendes 10 % PAA-Gel gegeben (Laufzeit etwa 1 Stunde bei 50 Watt). Als Größenstandards wurden zusätzlich ein RNA-(50mer) und DNA-(78mer)-Größenstandard (je 250 pmol) aufgetragen. Die Banden wurden mittels "UV-Shadowing" (bei 256 nm) sichtbar gemacht.

Die ausgeschnittene Gel-Bande wurde mittels "Crush & Soak" eluiert. Dazu wurden die Gelstücke in 360 μl H2O und 140 μ 5 M Ammoniumacetat (Endkonzentration ca. 2 M) aufgenommen. Die Crush-and-soak-Elution lief 2 x 1,5 Stunden bei 68°C auf dem Thermoschüttler (1000 rpm). Die Überstände wurden durch Ultrafree MC-Säulchen (Millipore) in der Tischzentrifuge in 2 ml Eppis gefiltert. Dem Crush-and-Soak Filtrat (500 μl) wurden 1-2 μl Glycogen (20 μg/ml) und 1250 μl 100% Ethanol zugegben, die RNA 30 min bei Raumtemperatur gefällt, für 30 min bei 13.000-14.000 rpm abzentrifugiert, das Pellet einmal mit 400 μl eiskaltem 70 %igem Ethanol gewaschen und letztendlich für 5 min bei 13.000-14.000 rpm abzentrifugiert. Das getrocknete Pellet wurde in 100 μl Wasser aufgenommen und die RNA-Konzentration bei 260 nm photometrisch bestimmt.

**Ergebnisse:**

**Simultane 5'- und 3'-Ligation: STAR-R02**

Selektion STAR-R02 wurde in Runde 6 in zwei Stränge geteilt. Der erste Strang wurde mit einer Peptidkonzentration von 10 μM und einem 2-fachem Peptid-Überschuss ins Plateau geführt. Runde 10 wurde kloniert und sequenziert. Das Ergebnis ist graphisch in Fig. 28 und tabellarisch in Fig. 29 dargestellt.

*Sequenzierung von Selektionsrunde STAR-R02-10*

Es wurden von 82 auswertbaren Sequenzen 62 verschiedene Sequenzen erhalten. Davon tragen 19 das charakteristische Kem-Motiv 1 (CATACGGTGAAAGAAACGAT), jedoch mit mit leichten Abwandlungen als in späteren Selektionsrunden. Sieben Klone zeigen ein über den gesamten randomisierten Bereich gespanntes Motiv 1/1, das nur an drei aufeinanderfolgenden Positionen beliebige Variabilität zulässt. Es enthält im Zentrum das Motiv 1. (ggacGACATGTTC NNN GAACATACGGTGAAAGAAACGATTGTCGgacagg). Darüber hinaus tauchen zwei Klone in der Sequenzierung von STAR-R02-12MW wieder auf. Ein weiterer findet sich in STAR-R02-15d.

Selektion mit höherer Stringenz

Selektionsrunde 6 wurde wiederholt, wobei mehrere Parallelansätze selektiert wurden. Diese unterschieden sich lediglich in einer fallenden Peptidkonzentration. In den folgenden Runden wurde die Peptidkonzentration immer weiter verringert. Die Wahl der Peptidkonzentrationen geschah wie folgt: Die höchste Peptidkonzentration entsprach der Konzentration bei der in der vorangegangenen Runde noch erfolgreich hatte selektiert werden können (Signal > 1 %, mindestens 3-fach über Leerselektion). Zusätzlich wurden um den Faktor 3,16 und den Faktor 10 verringerte Peptidkonzentrationen eingesetzt. Als Kontrolle diente eine Leerselektion ganz ohne Peptid. Die Runden 11-14 wurden mit einem verringerten RNA:Peptid-Verhältnis von 5:1 selektiert, Runde 15 als Doppelrunde (2x Selektion mit Verzicht auf Amplifikation) Das Ergebnis ist graphisch in Fig. 30 und tabellarisch in Fig. 31 dargestellt.

*Sequenzierung von Selektionsrunde STAR-R02-15d*

Es wurden von 41 auswertbaren Sequenzen 22 verschiedene Sequenzen erhalten, die alle das charakteristisches Motiv 1 (CATACGGTGAAAGAAACGAT) aufwiesen. 14 davon zeigten das erweiterte Motiv 1/1. 20 der 41 Klone treten auch in Selektion STAR-RNA-03-11 mit paralleler 2-Schritt-Ligation auf. 17 zum Teil überschneidende finden sich in STAR-R02-12MW wieder, 3 in der 37°C-Selektion STAR-R02-15xx. Eine einzige Sequenz findet sich in Selektion STAR-R02-10.

Matrixwechsel

Ab Runde 8 wurde ausgehend von Runde 7s (1μM) ein weiterer, paralleler Seletionsstrang eröffnet. Dieser zeichnet sich gegenüber den vorher genannten Seletionssträngen durch den wiederholten Wechsel der Selektionsmatrix (Festphase) aus. Es wurde zwischen *Streptavidin ultralink Plus* (Pierce/Perbio) und *Neutravidin-Agarose* (Pierce/Perbio) gewechselt. Das Ergebnis ist graphisch in Fig. 32 und tabellarisch in Fig. 33 dargestellt.

*Sequenzierung von Selektionsrunde STAR-R02-12MW*

Es wurden von 35 auswertbaren Sequenzen 22 verschiedene Sequenzen erhalten (1 davon hat ein N). 20 der 22 (19 ohne N der 22) Sequenzen tragen das charakteristische Motiv 1, das auch schon in Selektion STAR-R02-15d beobachtet wurde (CATACGGTGAAAGAAACGAT). 11 Klone bestehen aus dem erweiterten Motiv 1/1. 2 der 35 Klone sind aus Selektion STAR-R02-10 bekannt, 21 aus STAR-R02-15d. 2 treten auch in STAR-R02-15xx und 15 Klone in STAR-R03-11 auf.

Selektion bei 37°C

Ab Runde 10 wurde aus der Matrixwechsel-Selektion (RNA 02MW) ein weiterer Strang abgezweigt und bei 37°C weiterselektiert. Runden 13xx, 14xx, 15xx wurden als Doppelrunden selektiert. Das Ergebnis ist graphisch in Fig. 34 und tabellarisch in Fig. 35 dargestellt.

*Sequenzierung von Selektionsrunde STAR-R02-15xx*

Klonierung und Sequenzierung lieferten 40 auswertbare Klone mit 17 verschiedenen Sequenzen (3 davon tragen ein N). 6 Sequenzen (5 ohne eine Seq. mit N) tragen das typische Motiv 1 (CATACGGTGAAAGAAACGAT). 4 (3 ohne N) Sequenzen tragen ein Teilmotiv von dem oben genannten: (GAAAG) und sind beinahe identisch. Ein Klon zeigt das charakteristische Motiv 1/1. 7 Klone (davon 6 identisch) tragen das Motiv 1/1 jedoch mit vier aufeinander folgenden variablen Positionen (ggacGACATGTTC NNNN GAACATACGGTGAAAGAAACGATTGTCGgacagg).

Aus diesem Selektionsstrang ging Klon R02-15xx-A11 hervor, der in den **Beispielen 9 und 11** in der Zellkultur und in **Beispiel 12** im Micro-Kalorimeter charakterisiert wurde.

6 (5 ohne, eine mit N) Sequenzen tragen mit leichten Variationen ein neu identifiziertes Motiv 2 (GATGGCGCGGTCTNAAAAAACGCCGNNNGGGNGAGGG). Eine weitere ein sehr ähnliches mit einer Abwandlung (6 nt) im Zentrum des Motivs. Von den 40 Klonen waren bereits drei identische in Selektion STAR-R02-10 zu finden. Zwei traten in STAR-R02-15d auf Einer davon auch in STAR-R02-12MW. 7 Klone waren in Selektion R03-11 zu finden.

**Parallele 2-Schritt-Ligation: Selektion STAR-R03**

Die Selektion, deren Eluate zunächst geteilt und dann erst 5' und dann 3' bzw. erst 3' und dann 5' ligiert wurden, wurde mit steigender Stringenz bis in Runde 11 geführt. Das Ergebnis ist graphisch in Fig. 36 und tabellarisch in Fig. 37 dargestellt.

*Sequenzierung von Selektionsrunde STAR-R03-11*

Die Klonierung und Sequenzierung lieferte von 47 Klonen 29 verschiedene Sequenzen. 26 davon enthielten das charakteristische Motiv (CATACGGTGAAAGAAACGAT). Einer Sequenz fehlt davon das 5'C (--> A). Eine Sequenz zeigt das verkürzte Motiv (GAAAG, siehe STAR-R02-15xx). 14 der 26 zeigten das erweiterte Motiv 1/1. Zwei weitere das Motiv 1/1 mit vier aufeinanderfolgenden variablen Nukleotiden, das auch in Selektion STAR-R02-15xx auftrat. Eine Sequenz ist offensichtlich ein Orphan.

Es gibt keine Sequenzüberschneidungen mit STAR-R02-10. 20 Klone fanden sich auch in Selektion STAR-R02-15d, 19 Klone in Selektion STAR-R02-12MW und 7 Klone in STAR-R02-15xx.

Insgesamt ist also auf Sequenzebene aufgrund der großen Überschneidung kein Unterschied zwischen gleichzeitiger und paralleler 2-Schritt-Ligation festzustellen. Von zwei Klonen wurden bereits abgewandelte Moleküle synthetisiert (verkürzt und terminaler Stem stabilisiert). Dies sind: STAR-R03-11-F10-45-001 und STAR-R03-11-C12-48-001.

Aus dieser Selektion ging der Klon R03-11-F10 hervor, der in **Beispiel 12** im Mikrokalorimeter näher charakterisiert wurde.

Die verschiedenen erhaltenen Sequenzen sind in Figs. 43 - 47 dargestellt.

<u>Beispiel 5:</u> **Selektion eines an biotinyliertes CGRP bindenden Aptamers unter Anwendung nicht-modifizierter RNA**

In diesem Beispiel wird die Selektion von Aptameren beschrieben, die gegen CGRP, genauer biotinyliertes CGRP von der Ratte binden. Das CGRP wurde erworben von Jerini AG und weist 37 Aminosäuren bei einem pI gemäß SWISS PROT-Datenbankeintrag von 7,8 auf. Als Matrix wurde Neutravidin-Agarose verwendet, nach Runde 9 wurde auf magnetische Streptavidin-Beads entsprechend den in Beispiel 3 verwendeten Materialien gewechselt. Die Immobilisierung für die Selektion mit prä-immobilisiertem CGRP erfolgte auf NeutrAvidin-Agarose unter Verwendung des folgenden Puffers, der auch als Selektionspuffer verwendet wurde: 10 mM HEPES (pH 7,4), 1 mM $CaCl_2$, 1 mM $MgCl_2$, 4 mM KCl, 150 mM NaCl, 0,1 % Tween 20.

Die Komplexbildung erfolgte auf der Matrix, nach Runde 9 in Lösung. Die Elution erfolgte durch Affinitätselution für das prä-immobilisierte Target, mit 8 M Harnstoff für die Streptavidin-Beads. Die Selektionstemperatur war 37°C und als Pool wurde der RNA-Pool verwendet, wie er im Zusammenhang mit Beispiel 1 verwendet wurde, mit der Ausnahme, daß hier keine 2'-Fluormodifizierten Pyrimidinnukleotide verwendet wurden.

<u>Charakteristika der Selektion:</u>

Die Selektionen wurden bei einer konstanten Konzentration des Zielmoleküls von 50 μM bis zur Runde 9 durchgeführt. Ab Runde 9 wurde diese Konzentration schrittweise auf 10 nM abgesenkt, um die Stringenz der Selektion langsam zu erhöhen. Im Verlauf der Selektion wurden ausgehend von einen 2,5-fachen Waschvolumen der Waschanteil auf ein 5-faches (Runden 3-6) bzw. 10-faches (ab Runde 7) Waschvolumen erhöht. Das Zielmolekül war in den Runden 1-8 prä-immobilisiert auf NeutrAvidin - Agarose. In den folgenden Runden (ab Runde 9) erfolgte die Komplexbildung in Lösung an magnetischen Streptavidin Beads. Als Elutionsmethode wurde jeweils 5 Minuten mit 7 M Harnstoff bei 65 °C eluiert.

<u>Verlauf der Selektion:</u>

Im Verlauf der Selektion konnte ein erster Anstieg in der Runde 4 beobachtet werden. Dieser Anstieg konnte sich jedoch erst in Runde 9 mit 17 % Anbindung durchsetzten. Da in dieser Runde auch ein Anstieg auf der Vorsäule zu beobachten war, erfolgte in dieser Runde der oben erwähnte Umstieg auf die magnetischen Streptavidin Beads, wobei noch ca. 10 % der RNA eine Anbindung zeigten. Durch die Steigerung der Stringenz der Selektion wurde versucht, die Qualität der bindenden Moleküle hinsichtlich einer höheren Bindungskonstante zu verbessern. In Runde 15 wurden bei einer Konzentration von 500 nM des Zielmoleküls eine Anbindungsrate von 16 % erreicht und der Pool wurde kloniert und sequenziert. Die Sequenzierung ergab 4 Sequenzfamilien, die mit 14 bis 32 Klone stark vertreten waren, weitere 13 Sequenzen waren 2 bis 7 mal im Pool vorhanden. Weiterhin wurden 36 Einzelsequenzen gefunden. Die Bestimmung von Bindungskonstanten unter Verwendung der Biacore-Vorrichtung ergaben Affinitäten, die von der Zielaffinität von

$K_D$ < 30 nM noch zu weit entfernt waren. Die beste Bindungskonstante wurde für die Einzelsequenz G12 ermittelt. Aufgrund dieses Ergebnisses wurde die Selektion bei weiterer Erhöhung der Stringenz bis in Runde 19 (Konzentration an Zielmolekül 10 nM) weitergeführt. In Runde 18 erfolgte als Kontrollexperiment eine Anbindung bei einer Konzentration des Zielmoleküls von 5 μM und einem Verhältnis RNA zu Zielmolekül von 1:1, dabei wurde eine 50%ige Anbindung erreicht. Nach Abschluß der Runde 19 bei 10 nM und einer Anbindungsrate von 8,5 % wurde der erhaltene Pool sequenziert. Aus dem Ergebnis der Sequenzierung konnte eine Sequenzfamilie identifiziert werden, die über 60 % des Pools ausmacht. Dieses Sequenzfamilie entsprach dem Klon G 12, dem besten Binder aus der Sequenzierung nach Runde 15 (wobei G12 dort eine Einzelsequenz war). Für den Klon G12 wurde eine $K_D$ von 100 - 200 nM bei 25°C bestimmt.

Die Ergebnisse der Biacore-Versuche legen ein bivalentes Bindeverhalten des Klons nahe. Dies konnte in Versuchen mit nicht-biotinyliertem CGRP gezeigt werden. Auch zeigt die Wiederholung der Runde 19 mit Affinitätselution, bei der die Elution der an biotinyliertem CGRP gebundenen RNA mit freiem, nicht-biotinyliertem CGRP erfolgt, nur einen geringen Anteil an eluierbarer RNA. Diese Runde 19 mit Affinitätselution wurde nicht sequenziert. Weiterhin wurde in der Sequenzierung der Runde 19 mit denaturierender Elution neben dem dominanten G12 Klon eine weitere Motivfamilie mit 14 Vertretern, sowie 30 neue Einzelsequenzen gefunden. Die Bestimmung der Dissoziationskonstanten unter Verwendung der Biacore-Vorrichtung ergab, daß alle diese neuen Sequenzen eine geringere Affinität und damit eine schlechtere Dissoziationskonstante als der Klon G12 aufwiesen.

Der Verlauf der Selektion ist in der nachfolgenden Tabelle wiedergegeben.

| Runde | Temp. Selektion | Prä-[Selection%] | Nucl. acid [pmol] | Peptid [pmol] | Nucleic acid:pepti d | Volumen | | | Σ Elution trolle [%] | Kon- [%] | Kommentar |
|---|---|---|---|---|---|---|---|---|---|---|---|
| | | | | | | Reaktion | Waschen | Elution | | | |
| 1 | 37 | | 5000 | 10000 | 1:5 | 250 | 5 x 100 | 2 x 100 + Säule | 0,10 | - | 200 µl Matrix |
| 2 | 37 | 100 µl; 1,83 | 1000 | 10000 | 1:10 | 200 | 5x100 | 2 x 100 + Säule | 0,17 | - | 200 µl Matrix |
| 3 | 37 | 100 µl; 1,42 | 500 | 5000 | 1:10 | 100 | 5x100 | 2 x 100 + Säule | 0,17 | | 100 µl Matrix ab hier weitergehend |
| 4 | 37 | 100 µl; 0,15 | 500 | 5000 | 1:10 | 100 | 5x100 | 2 x 100 + Säule | 2,45 | - | |
| 5 | 37 | 100 µl; 1,23 | 500 | 5000 | 1:10 | 100 | 5x100 | 2 x 100 + Säule | 1,16 | 0,05 | |
| 6 | 37 | 100 µl; 0,51 | 500 | 5000 | 1:10 | 100 | 5x100 | 2 x 100 + Säule | 2,55 | 0,11 | |
| 7 | 37 | 100 µl; 3,97 | 500 | 5000 | 1:10 | 100 | 10x100 | 2 x 100 + Säule | 3,66 | 0,16 | |
| 8 | 37 | 100 µl; 1,77 | 500 | 5000 | 1:10 | 100 | 10x100 | 2 x 100 + Säule | 6,25 | 0,26 | |
| 3a | 37 | 100 µl; 18,36 | 500 | 5000 | 1:10 | 100 | 10x100 | 2 x 100 + Säule | 17,16 | 0,07 | |
| 9b | 37 | 100 µl; 0,08 | 500 | 5000 | 1:10 | 100 | 10x100 | 2 x 100 | 10,75 | | Umstellung auf Anbindung in Lösung |
| 10 | 37 | 100 µl; 0,14 | 500 | 1000 | 1:2 | 100 | 10x100 | 2x100 | 4,89 | 2,15 | |
| 11 | 37 | 100 µl; 0,08 | 500 | 1000 | 1:2 | 100 | 10 x 100 | 2 x 100 | 2,15 | 0,25 | |
| 12 | 37 | 100 µl; 0,11 | 500 | 500 | 1:1 | 100 | 10 x 100 | 2 x 100 | 12,22 | 0,20 | |
| 13 | 37 | 100 µl; 0,15 | 500 | 500 | 1:1 | 100 | 10 x 100 | 2 x 100 | 14,15 | 0,15 | |
| 14a | 37 | 100 µl; 0,17 | 500 | 500 | 1:1 | 100 | 10 x 100 | 2 x 100 + Säule | 30,30 | - | |
| 14b | 37 | 100 µl; 0,17 | 500 | 100 | 5:1 | 100 | 10 x 100 | 2x100 | 12,24 | 0,92 | |

(fortgesetzt)

| Runde | Temp. Selektion | Prä-[Selection%] | Nucl. acid [pmol] | Peptid [pmol] | Nucleic acid:pepti d | Volumen | | | Σ Elution trolle [%] | Kon- [%] | Kommentar |
|---|---|---|---|---|---|---|---|---|---|---|---|
| | | | | | | Reaktion | Waschen | Elution | | | |
| 15a | 37 | 100 µl; 0,29 | 500 | 100 | 5:1 | 100 | 10 x 100 | 2 x 100 | 16,26 | 1,54 | Pool wurde sequenziert, Auftreten von G12 als Einzelsequenz |
| 15b | 37 | 100 µl; 0,31 | 500 | 50 | 10:1 | 100 | 10 x 100 | 2x100 | 8,74 | 1,54 | Pool wurde sequenziert. |
| 16 | 37 | 100 µl; 0,29 | 500 | 10 | 50:1 | 100 | 10 x 100 | 2x100 | 2,55 | 0,84 | |
| 17a | 37 | 100 µl; 0,32 | 500 | 10 | 50:1 | 100 | 10 x 100 | 2x100 | 4,52 | 0,68 | |
| 17b | 37 | 100 µl; 0,27 | 500 | 5 | 100:1 | 100 | 10x100 | 2 x 100 | 1,07 | 0,68 | |
| 17c | 37 | 100 µl; 0,30 | 500 | 3 | 166:1 | 100 | 10 x 100 | 2 x 100 | 0,77 | 0,68 | |
| 17d | 37 | 100 µl; 0,28 | 500 | 1 | 500:1 | 100 | 10 x 100 | 2 x 100 | 1,12 | 0,68 | |
| 18 | 37 | 100 µl; 0,38 | 500 | 1 | 500:1 | 100 | 10 x 100 | 2 x 100 | 7,00 | 0,72 | Selektion Runde |
| 18c | 37 | 100 µl; 0,38 | 500 | 500 | 1:1 | 100 | 10 x 100 | 2 x 100 | 50,08 | 0,72 | Kontrollselektion |
| 19 | 37 | 100 µl; 0,38 | 500 | 1 | 500:1 | 100 | 10 x 100 | 12 x 100 | 8,53 | 1,96 | Pool wurde sequenziert, Auftreten von G12 als dominierende Sequenz |
| 19 verd | 37 | 100 µl; 0,38 | 500 | 1 | 500:1 | 500 | 10x100 ; | 2 x 100 | 1,40 | 0,56 | Niedrige Konzentrationen |
| 19 Aff | 37 | 100 µl; 0,38 | 500 | 1 | 500:1 | 100 | 10x100 | 2 x 100 | 1,39 | 0,43 | Affinitätselution als Test für Affinität gegen freies Target |

Die bevorzugten Einzelsequenzen können wie folgt dargestellt werden.
GGAGCUCAGCCUUCACUGC-N40- GGCACCACGGUCGGAUCCAG

G12 - Gruppe 12:

- GGAGCUCAGCCUUCACUGCAUCGAGGCGAUCCAAGUUGUAGGAAUGGGGUGGCUUGGAG GGCACCACGGUCGGAUCCAG (G 12) (SEQ. ID NO. 234)

+ Mutationen:

- GGAGCUCAGCCUUCACUGCAUCGAGGCAUCCAAGUUAUAGGAAUAGGGUGGCUUGAAG GGCACCACGGUCGGAUCCAG (G 12a) (SEQ. ID NO. 235)

- GGAGCUCAGCCUUCACUGCAUCGAGGCAUCCAAGUUAUAGGAAUAGGGUGGCUUGAAG GGCACCACGGUCGGAUCCAG (G 12b) (SEQ. ID NO. 236)

- GGAGCUCAGCCUUCACUGCAUCGAGGCGAUCCAAGUUGAGGAAUGGGGUGGCUUGGAG GGCACCACGGUCGGAUCCAG (G 12c) (SEQ. ID NO. 237)

GGAGCUCAGCCUUCACUGCAUCGAAGGCGAUCCAAGUUGUAAGAAUGGGGGUGGCUUGGAG GGCACCACGGUCGGAUCCAG (G 12d) (SEQ. ID NO. 238)

- GGAGCUCAGCCUUCACUGCAUCGAGGCGAUCCAAGUUGUAGGAAUUGGAUGACUUGGAG GGCACCACGGUCGGAUCCAG (G 12e) (SEQ. ID NO. 239)

- GGAGCUCAGCCUUCACUGCAUCGAGGGCGAUCCAAGUUGUAGGAAUGGGGUGGCUUGGAG GGCACCACGGUCGGAUCCAG (G 12f) (SEQ. ID NO. 240)

- GGAGCUCAGCCUUCACUGCAUCGAAGCGAUCCAAGUUGUAGGAAUGGGGUAGCUUGGAG GGCACCACGGUCGGAUCCAG (G 12g) (SEQ. ID NO. 241)

wobei die $K_D$-Werte nur in einem sehr schmalem Band variierten.

H08 - Gruppe 6:

- GGAGCUCAGCCUUCACUGCGGGUGGGAGGGUGGAUGGUGAAGAACGAGCGCUGACCGC GGCACCACGGUCGGAUCCAG (H 08a) (SEQ. ID NO. 242)

- GGAGCUCAGCCUUCACUGCGGGUGGGAGGGUGGAUGGUGGAGAACGAGCACUGACCUC GGCACCACGGUCGGAUCCAG (H 08b) (SEQ. ID NO. 243)

Die bindenden Nukleinsäuren dieser Gruppe zeigen $K_D$-Werte schlechter als die Nukleinsäure gemäß Klon G12.

H03 - Gruppe 6:

- GGAGCUCAGCCUUCACUGCCAUUGAGGAUAUGCCGCGGUCCGCAUGGAGUGGGUGUUG GGCACCACGGUCGGAUCCAG (H 03a) (SEQ. ID NO. 244)

Die bindende Nukleinsäuren dieser Gruppe zeigt einen $K_D$-Wert schlechter als die Nukleinsäure gemäß Klon G 12.

**Beispiel 6: Synthese von L-2'-Fluoro-phosphoramiditen**

Die Synthese von 5'-DMT-2'-fluoro-*L*-uridin und 5'-DMT-2'-Fluoro-N(Ac)-*L*-cytidin phosphoramidites (3 and 5) ist in Figs. 15 bis 17 veranschaulicht:

2'-Fluoro-*L*-uridine 2 wurde gemäß Codington, J. F., Doerr, I. L., & Fox, J. J. (1964) J. Org. Chem. 29, 558, aus *L*-Arabinose 1 synthetisiert. Anschließend wurde sie zu 5'-DMT-2'-fluoro-*L*-uridine konvertiert und phosphityliert (Fig. 15), um das Phosphoramidit zu erhalten (Fig. 17).

Die Bezüge gemäß Fig. 15 bedeuten dabei:

a) Cyanamid, MeOH, $NH_4OH$; b) Methylpropiolat, EtOHaq; c) HF, Dioxan, Autoklav 120°C; d) DMTCl, DMAP, Pyr; e) Cl-P($OCH_2CH_2CN$)N(iPr)$_2$, DIPEA, DMAP, THF.

Für die Konversion von D-Uridin-Derivaten in *D*-Cytidin-Derivate sind in der Literatur (Sung W. L. (1982) J. Org. Chem. 47, 3623; Reese C.B., Ubasawa A. (1980) Tetrahedron Lett., 2265; Sung W. L. (1981) J. Chem. Soc. B, 1089; Krug A., Schmidt S., Lekschas J., Lemke K., Cech D. (1989) Journal f. prakt. Chemie, 331(5), 835) viele Synthesewege beschrieben. Diese Synthesen nutzen als Ausgangsgruppe 1,2,4-Triazol (Sung W. L. (1982) J. Org. Chem. 47, 3623) oder 1-Tetrazol (Reese C.B., Ubasawa A. (1980) Tetrahedron Lett., 2265; Sung W. L. (1981) J. Chem. Soc. B, 1089; Krug A., Schmidt S., Lekschas J., Lemke K., Cech D. (1989) Journal f. prakt. Chemie, 331(5), 835). Die Synthese von 2'-Fluoro-*L*-cytidin 4 wurde mit der 1,2,4-Triazol-Chemie (Krug A., Schmidt S., Lekschas J., Lemke K., Cech D. (1989) Journal f. prakt. Chemie, 331(5), 835) durchgeführt. Das hierdurch gewonnene 2'-Fluoro-*L*-cytidin 4 wurde anschließend aminoacetyliert, trityliert und phosphoryliert (Fig. 16) um 5 zu erzielen.

Die Bezüge gemäß Fig. 16 bedeuten dabei:

a) BzCl, Pyr; b) 1,2,4-Triazol, 4-Chlorophenylphosphordichloridate, Pyr; c) $NH_3$ (28% in Wasser) /Dioxan; d) $Ac_2O$, DMF; e) DMTCl, DMAP, Pyr; f) Cl-P($OCH_2CH_2CN$)N(iPr)$_2$, DIPEA, DMAP, THF.

Die Details der Konversion von 2'-Fluoro-*L*-uridin 2 in 2'-Fluoro-*L*-Cytidin 4 werden in der folgenden Abbildung beschrieben (Fig. 17):

Die Bezüge gemäß Fig. 17 bedeuten dabei:

a) BzCl, Pyr; b) 1,2,4-Triazol, 4-Chlorophenylphosphordichloridat, Pyr; c) $NH_3$ (28% in Wasser)/Dioxan; oder $NH_3$ (28% in Wasser)/Dioxan und MeO/$NH_3$ sat.

2'-Fluoro-*L*-uridin 2 wurde zuerst benzoyliert um 6 zu erzielen. Die Substanz 6 wurde ohne Aufreinigung direkt weiter mit 1,2,4-Triazol und 4-Chlorophenylphosphordichloridat umgesetzt um 7 zu ergeben. Nach der Behandlung mit Ammoniak/Dioxan kann 2'-Fluoro-L-cytidin direkt aus der ungereinigten Substanz als beige-braunes Kristallisationsprodukt erhalten werden.

Von besonderem Interesse bei dieser Syntheseart ist, daß keine Aufreinigung der Zwischensubstanzen notwendig ist. Die Endsubstanz kann von der Rohsubstanz mit hoher Ausbeute von etwa 70 % erhalten werden.

**Versuchsaufbau**

2'-Fluoro-*L*-uridine 2 (22,35 g, 57 % Reinheit, 90 mmol) wurde dreimal mit trockenem Pyridin (3 x 40 ml) coevaporiert. Die Rohsubstanz wurde in trockenes Pyridin eingebracht (150 ml, mit Molekularsieb) und auf 0 °C gekühlt. Benzoylchlorid wurde tropfenweise hinzugegeben, und die Reaktion wurde 1 h bei 0 °C umgerührt und über Nacht bei Zimmertemperatur gelagert (TLC (Hex/EE 1/2): Rf 0,5). Die Reaktion wurde mit Methanol gestoppt (5 ml) und bis zur Trockene eingeengt. Die Restsubstanz wurde in DCM (250 ml) aufgelöst und mit $NaHCO_3$ (50 %, 150 ml, dreimal) verdünnt. Die organische Phase wurde über $Na_2SO_4$ getrocknet und eingeengt.

Die Rohsubstanz wurde in trockenem Pyridin (300ml) aufgelöst. Die Mischung wurde mit Molekularsieb bei 0°C unter $N_2$ 30 Minuten lang umgerührt. 1,2,4-Triazol (4,0 eq, 360 mmol, 29,9 g) wurde dann hinzugefügt. Diese Mischung wurde bei Raumtemperatur 10 Minuten lang umgerührt. Danach wurde langsam 4-Chlorophenyl-phosphodichloridat (2,0 eq, 180 mmol, 29,3 ml) der Mischung zugeführt. Nach 30 Minuten wurde die Reaktion exotherm.

Die Mischung wurde 2 h bei 0 °C umgerührt und über Nacht bei Raumtemperatur aufbewahrt. Das ungereinigte Produkt lief in auf der Dünnschichtplatte in einem Fleck (TLC, Hexan / Essigsäureethylester 1/2): Rf: 0,34). Die Restsubstanz wurde in DCM (250 ml) aufgelöst und die organische Phase mit verdünntem $NaHCO_3$ (50 %, 300 ml) (exotherm) gewaschen. Die organischen Phasen wurden über $Na_2SO_4$ getrocknet und evaporiert.

Die Rohsubstanz wurde in Dioxan (600 ml) und $NH_3$ (600 ml, 28 % in Wasser) aufgelöst, dann 72 h bei Raumtemperatur umgerührt. Nach der TLC Kontrolle (DCM/MeOH 9/1, Rf: 0,34) wurde die Mischung bis zur Trockene eingeengt (bräunliches Öl) und mit Methanol (zweimal 50 ml) coevaporiert. Die bräunliche Restsubstanz wurde in einer Mischung aus Wasser (200 ml) und DCM (200 ml) aufgelöst. Die wäßrige Phase wurde anschließend dreimal mit DCM (150 ml) gewaschen und soweit konzentriert, bis ein minimales Volumen (50 ml) erreicht wurde. Nach einer Nacht im Kühlschrank wurde die Substanz filtriert, mit DCM (zweimal 25 ml) sowie $Et_2O$ (2 x 25 ml) (beige-braune Kristalle, 6,1 g (50 % Ausbeute) gewaschen. Ein zweiter Ertrag (3,0 g, 23 % Ausbeute) wurde nach einer kurzen Chromatographie von der Originalsubstanz (Lagerung auf Silica Gel, Verlauf von MeOH in DCM (5-30 %)) und einer zweiten Kristallisierung aus Wasser (Totalausbeute 73 %) erzielt.

Eine alternative Methode könnte sich aus der Suspension der Rohsubstanz in Dioxan (300 ml) und $NH_3$ (300 ml, 28% in Wasser) ergeben. Die Mischung wurde 2 h bei Raumtemperatur umgerührt. Nach der TLC Kontrolle (DCM/MeOH 9/1) wurde die Mischung zur Trockne (bräunliches Öl) evaporiert und mit Methanol (zweimal 50 ml) coevaporiert. Die Rohsubstanz wurde in Methanol (ca. 5 ml) aufgelöst, und gesättigter methanolischer Ammoniak (200 ml) wurde zugegeben. Die Mischung wurde über Nacht umgerührt.

**Beispiel 7: Bestimmung der Zytotoxizität von an CGRP bindenden selektierten L-Nukleinsäuren**

Auf der Grundlage der in Beispiel 2 selektierten L-Nukleinsäuren wurde eine der Verbindungen, nämlich 732_096, verwendet, um die Toxizität der L-Nukleinsäure zu bestimmen. Als zelluläres Testsystem wurde dabei die Neuroblastom-Zellinie SK-N-MC mit der Accession Nummer ACC 203 (DSMZ, Deutsche Sammlung von Mikroorganismen und Zellkulturen GmbH, Braunschweig) verwendet

**AlamarBlue Test**

Der AlamarBlue Test ist ein Assay zu Erfassung von Zellwachstum und Zytotoxizität. AlamarBlue ist ein Indikatorfarbstoff zur quantitativen Messung der Proliferation von eukaryotischen und prokaryotischen Zellen. AlamarBlue ist ein ungiftiger, wasserlöslicher Oxidations-Reduktions-Indikator, der bei erfolgter chemischer Reduktion sowohl seine Fluoreszenz- als auch seine colorimetrischen Eigenschaften ändert. Chemische Reduktion des Indikators findet in lebenden Zellen proportional zum zellulären Metabolismus und zur intrazellulären Enzymaktivität statt und ist daher ein Maß für die Zytotoxizität getesteter Substanzen (Ahmend et al., 1994, J. Immunol. Methods 170, 211-224, Page et al., 1993, Int. J. Oncology 3, 473-476,. Die Absorption kann mit einem Mikrotiterplattenphotometer bei 570 nm (Excitation) (600 nm Emission; Referenz) gemessen werden.

Zur Durchführung wird AlamarBlue in Kulturmedium zu 5 % verdünnt, mit 100 μl/Napf eine Mikrotiterplatte (96-er Format) auf die vorher mit den Testsubstanzen (Spiegelmere) 30 Minuten inkubierten Zellen gegeben und für eine bestimmte Zeit im Brutschrank inkubiert. Die Inkubationszeit richtet sich nach Zellart und Zellzahl. Als Richtwert gelten für die verwendeten SK-N-MC-Zellen (Neuroblastom Zellinie) 2 h. Nach Ende der Inkubationszeit erfolgt die photometrische Messung.

Als Maß für die Zytotoxizität wird die prozentuale Veränderung der behandelten Näpfe gegen die jeweiligen Kontrollen ermittelt. Hemmungen größer 20 % werden als cytotoxische Effekte betrachtet.

Das Ergebnis ist tabellarisch in der nachfolgenden Tabelle angegeben.

| Spiegelmer-Konzentration [M] | Hemmung [%] |
|---|---|
| $1 \times 10^{-5}$ | 0,51 |
| $5 \times 10^{-6}$ | 0 |
| $1 \times 10^{-6}$ | 1,93 |

(fortgesetzt)

| Spiegelmer-Konzentration [M] | Hemmung [%] |
|---|---|
| 5 x $10^{-7}$ | 0,82 |
| 1 x $10^{-7}$ | 0 |
| 5 x $10^{-8}$ | 1,83 |
| 1 x $10^{-8}$ | 2,91 |
| 5 x $10^{-9}$ | 8,22 |
| 1 x $10^{-9}$ | 0 |
| 1 x $10^{-10}$ | 0 |
| 1 x $10^{-11}$ | 0 |
| 1 x $10^{-12}$ | 0,001 |
| 0 (Kontrolle) | 0 |
| | |
| 1 μM CGRP8-37 (Antagonist Kontrolle) | 4,60 |
| IBMX (Kontrolle) | 2,96 |

**Beispiel 8: Hemmung der cAMP-Produktion durch humanes CGRP bindende Spiegelmere**

Die Analyse der biologischen Wirksamkeit von CGRP-bindenden L-Nukleinsäuren, d. h. von Spiegelmeren wurde wie folgt vorgenommen.

Zellen der humanen Neuroblastoma-Linie SK-N-MC mit der Accession Nummer ACC 203 (DSMZ, Deutsche Sammlung von Mikroorganismen und Zellkulturen GmbH, Braunschweig) werden in einer Zahl von 4 x $10^4$ pro Napf einer 96 Napf-Mikrotiterplatte ausgesät und bei 37°C und 5% $CO_2$ in DMEM (1,000mg/L Glucose), das zusätzlich 10 % hitzeinaktiviertes fötales Kälberserum (FCS), 4 mM L-Alanyl-L-Glutamin (GLUTAMAX ), 50 units/ml Penicillin, 50 μg/ml Streptomycin enthält, kultiviert. 48 h nach Aussaat sind die Zellen zu 80-90 % konfluent und werden für die Versuche eingesetzt.

Die Spiegelmere werden zusammen mit 1 nM humanem oder Ratten-CGRP (Bachem) in Hank's balanced salt solution (HBSS) + 1 mg/ml BSA für 15-60 min bei RT oder 37 °C in einer 0,2 ml *"low profile 96-tube"-Platte* inkubiert. Kurz vor Zugabe zu den Zellen werden 2 μl einer 50 mM IBMX-Lösung zugegeben. Die Zellen werden 20 min vor Zugabe der CGRP/Spiegelmer-Ansätze mit 1 mM IBMX vorbehandelt.

Zur Stimulation wird das Medium von den Zellen abgesaugt, und die vorinkubierten Ansätze werden zugegeben. Nach Inkubation für 30 min bei 37 °C werden die Zellüberstände abgesaugt und die Zellen mit 50 μl/Napf Lysis-Puffer für 30 min bei 37 °C lysiert. Der Lysis-Puffer ist Bestandteil des "cAMP-Screen™ System"-kits (Applied Biosystems), mit dem der cAMP-Gehalt der Extrakte bestimmt wird. Jeweils 10 μl der Extrakte werden in den Test eingesetzt. Die Durchführung des Tests erfolgt wie vom Hersteller beschrieben:

> In einer Assay-Platte (beschichtet mit Ziegen anti-Kaninchen-IgG) werden zu 50 μl des Lysis-Puffers 10 μl/Napf des Lysats gegeben und mit 30 μl/Napf des nach Herstellerangaben verdünnten cAMP-Alkalische Phosphatase-Konjugats vermischt. Danach werden 60 μl/Napf des im Kit mitgelieferten cAMP-Antikörpers hinzugefügt. Es folgt eine Inkubationszeit von 1 h unter Schütteln bei Raumtemperatur. Danach werden die Lösungen aus den Näpfen entfernt und diese sechsmal mit dem mitgelieferten Waschpuffer gewaschen. Zur Detektion werden 100 μl/Napf CSPD/Sapphire-II RTU Substrat zugegeben, 30 min bei RT inkubiert und die Lumineszenz in einem POLARstar Galaxy Multidetektions-Plattenlesegerät (BMG) gemessen.

In diesem Testsystem wurde zwei im Rahmen von Beispiel 2 hierin beschriebenen L-Nukleinsäuren getestet. Dabei handelt es sich um die Sequenzen 732_096 und 732_045 entsprechend den SEQ.ID.No 14 und 13.

Die Spiegelmere 732_045 und 732_096 wurden vor Zugabe zu den Zellen zusammen mit 1 nM humanem CGRP in Medium (M199) ohne Serum für 15 min vorinkubiert. Nach 30 min bei 37 °C erfolgte die Lyse der Zellen. Die Extrakte aus jeweils zwei gleich behandelten "Näpfen" wurden vereinigt und die Mengen an cAMP wie beschrieben bestimmt.

Das Ergebnis ist in Fig. 23 dargestellt. Aufgetragen wurden die gebildeten Mengen an cAMP als Prozentsatz der Kontrolle. Die graphische Auswertung ergibt als Konzentration an Spiegelmer, bei der nur noch 50 % der in der Kontrolle vorhandenen cAMP-Menge gebildet wird (IC50), einen Wert von ca. 60 nM für 732_096 (59mer) und von ca. 22 nM für 732_045 (55mer). Diese sehr guten IC50 Werte, die auch mit den Dissoziationskonstanten korrelieren, zeigen deutlich, daß die identifizierten Fluoro-Spiegelmere extrem potent sind, potenter als die mit gleichen Methoden bisher selektierten unmodifizierten RNA-Moleküle.

**Beispiel 9: Hemmung der cAMP-Produktion durch Ratten-CGRP bindende Spiegelmere**

Der Versuch wurde wie im Zusammenhang mit Beispiel 8 beschrieben durchgeführt. Das Spiegelmer L 501L, das im Rahmen von Beispiel 4 selektiert worden war, wurde vor Zugabe zu den Zellen zusammen mit 1 nM Ratten-CGRP in Hank's balanced salt solution (HBSS) + 1 mg/ml BSA für 60 min bei 37 °C vorinkubiert. Nach 30 min Stimulationsdauer erfolgte die Lyse der Zellen und die Bestimmung der cAMP-Mengen in den Lysaten wie beschrieben. Es wurden Dreifachbestimmungen durchgeführt und die gebildeten Mengen an cAMP als Prozentsatz der Kontrolle (kein Spiegelmer im Vorinkubationsansatz) ± Standardabweichung aufgetragen in Fig. 24. Die graphische Auswertung ergibt als Konzentration an Spiegelmer, bei der nur noch 50 % der in der Kontrolle vorhandenen cAMP-Menge gebildet wird, einen Wert von ca. 60 nM.

Mit einer halbmaximalen stimulierenden Konzentration durch CGRP von ca. 1 nM, wie in Beispiel 1 bestimmt, ergibt sich daraus ein Ki-Wert von ca. 30 nM.

**Beispiel 10: Bindungsstudien an zellulärem CGRP-Rezeptor**

Membranen von CHO-K1 Zellen, die mit humanem *calcitonin-receptor related receptor* (CRLR, Genbank: U17473) und humanem *receptor-associated modifying protein type 1* (RAMP1, Genbank: AJ001014) transfiziert sind (Fa. Euroscreen, Brussels, Belgium), werden zügig aufgetaut, in 20 mM HEPES, 100 mM NaCl 10 mM $MgCl_2$, 1 mM EDTA pH 7.4 verdünnt und durch Homogenisieren resuspendiert. Für jeden Testansatz werden 2 μg Membranprotein in 20 mM HEPES pH 7.4, 10 mM $MgCl_2$, 100 mM NaCl, 1 mM EDTA, 1 μM GDP und den Spiegelmeren in einem Volumen von 150 μl inkubiert. Nach 30-minütiger Inkubation bei Raumtemperatur werden 50 μl 2 nM [$^{35}$S]GTPγS (Fa. Amersham) hinzugefügt und für weitere 30 Minuten bei Raumtemperatur inkubiert. Danach werden 25 μg WGA-PVT-Kügelchen (Weizenkeimagglutinin-beladene Polyethyleneimine-behandelt) (Amersham) in 50 μl hinzupipettiert, für 30 Minuten bei Raumtemperatur inkubiert und die Assay-Platte für 10 min bei 3200 rpm zentrifugiert. Die gebundene Aktivität wurde mit einem Wallac1450 MicroBeta™ scintillation counter (Wallac) bestimmt.

Auswertung. Die Daten werden durch nicht-lineare Regression analysiert (GraphPad Prism, Vers. 3.02, Graphpad Software Inc., San Diego, CA, USA).

Die Bindungsstudien wurden mit den beiden Spiegelmeren 732_045 und 732_096 durchgeführt, die im Rahmen von Beispiels 2 selektiert wurden. Das Ergebnis ist in Fig. 25 dargestellt.

Beide Spiegelmere hemmen effizient die Bindung des Liganden an den Rezeptor. Das bedeutet, daß das Spiegelmer spezifisch das CGRP erkennt, wegfängt und deshalb keine Rezeptorbindung und Rezeptoraktivierung erfolgt. Folglich kann auch kein [$^{35}$S]GTPγS eingebaut werden.

732_045 und 732_096 wurden jeweils mittels HPLC oder Gel gereinigt. Fig. 25 zeigt, daß die Reinigungsmethode im Rahmen des Fehlers nur einen unwesentlichen Einfluß auf die biologische Aktivität der Fluoro-modifzierten RNA-Spiegelmere hat.

**Beispiel 11: Zellkulturexperimente - Dosis-Wirkungskurve für CGRP**

SK-N-MC- Zellen wurden wie in Beispiel 8 beschrieben in 96-well-Platten kultiviert. 20 min vor Stimulation mit Ratten α-CGRP wurde das Medium durch jeweils 100 μl Hank's balanced salt solution (HBSS) + 1 mg/ml BSA ersetzt und mit 1 mM 3-Isobutyl-1-methylxanthin (IBMX) versetzt. Die Stimulation mit CGRP erfolgte durch Zugabe aus 100-bzw 30 fach konzentrierten Stammlösungen von in PBS gelöstem Ratten- α-L-CGRP (Bachem). Nach 30 min bei 37°C wurde der Überstand abgesaugt und die Zellen mit 50 μl Lysis-Puffer lysiert. Der cAMP-Gehalt der Extrakte wurde bestimmt wie **in Beispiel 8** beschrieben. Die Auftragung der gebildeten cAMP-Mengen gegen die zur Stimulation verwendete CGRP-Konzentration, wie dargestellt in Fig. 38 ergab eine halbmaximale Aktivierung von ca. 1 nM.

**Beispiel 12: Kalorimetrische Bestimmung der Bindungskonstanten und der Aktivität**

Durchführung

Die Bindung von Spiegelmeren an Ratten alpha-CGRP wurde auch über das Verfahren der Isothermen Titrations-Kalorimetrie (ITC) mit dem *VP ITC (Microcal)* bestimmt.

Dazu wurden 1,465 ml einer 10 μM Lösung der Spiegelmere in die adiabate Messzelle des Gerätes vorgelegt. Die bei Zugabe von 7,5 μl einer 70 μM Ratten α-L-CGRP-Lösung frei werdende Bindungsenthalpie wird von dem Gerät registriert. Durch wiederholte Zugabe des Peptides und Messung der jeweils frei werdenden Bindungsenthalpien können Bindungskonstanten und Aktivitäten der Moleküle bestimmt werden.

Das Spiegelmer STAR-R02-15xx-A11 wurde bei 37°C vermessen. Das Ergebnis ist in den Figs. 39 und 40 dargestellt.

Das Spiegelmer STAR-R03-F10 wurde bei 25°C gemessen. Das Ergebnis ist in den Figs. 41 und 42 dargestellt.

<u>Ergebnisse der ITC-Messung</u>

Die Bindungskonstante oder Dissoziationskonstante $K_D$ ist der Kehrwert der Assoziationskonstante $K_A$ (K im Enthalpiediagramm) und ergab sich für das Spiegelmer STAR-R02-15xx-A11 zu 65 nM. Die Aktivität war 42 %. Dies ist etwas schlechter als die Daten der Zellkultur, aber in der gleichen Größenordnung.

Spiegelmer STAR-R03-11-F10 zeigte bei 25°C eine Bindungskonstante von 28 nM und eine Aktivität von 83 %.

**<u>Beispiel 13</u>: Charakterisierung von Spiegelmer STAR-R02-15xx-F12 (NOX-504)**

Der Versuch wurde, wie im Zusammenhang mit Beispiel 8 beschrieben, durchgeführt. Das Spiegelmer STAR-R02-15xx-F12 (auch als NOX-504 bezeichnet) wurde vor Zugabe zu den Zellen zusammen mit 1 nM Ratten-αCGRP in Hank's balanced salt solution (HBSS) + 1 mg/ml BSA 60 min bei 37°C vorinkubiert. Nach 30 min Stimulationsdauer erfolgte die Lyse der Zellen und die Bestimmung der cAMP-Mengen in den Lysaten wie beschrieben. Es wurden Doppelbestimmungen durchgeführt und die gebildeten Mengen an cAMP ± Standardabweichung in Fig. 50 aufgetragen. Die graphische Auswertung ergab als Konzentration an Spiegelmer, bei der nur noch 50% der in der Kontrolle (Inkubation der Zellen mit CGRP aber ohne Spiegelmer) vorhandenen cAMP-Menge gebildet wird, einen Wert von ca. 7 nM. Mit einer halbmaximal stimulierenden Konzentration durch CGRP von ca. 1 nM, wie in Beispiel 11 bestimmt, ergibt sich daraus ein $K_i$-Wert von ca. 3,5 nM.

Auch mit der Methode der Microkalorimetrie wurde dieses Spiegelmer charakterisiert, wie in Beispiel 12 für andere Spiegelmere beschrieben. Die Bindungskonstante oder Dissoziationskonstante $K_D$ ist der Kehrwert der Assoziationskonstanten $K_A$ (K im Enthalpiediagramm, Fig. 51) und ergab sich für das Spiegelmer STAR-R02-15xx-F12 zu 44 nM. Die Aktivität war 47 %.

**<u>Beispiel 14</u>: Identifikation verkürzter Spiegelmere**

Ausgehend von Spiegelmer STAR-R02-15xx-F12 (48 Nukleotide), welches in Zellkultur die beste CGRP-Bindung gezeigt hatte (siehe Beispiel 13), wurden verkürzte Varianten hergestellt, da die Synthese kürzerer Moleküle kostengünstiger ist, was bei Großmaßstabsynthesen über den kommerziellen Erfolg eines Produkts mitentscheidend sein kann. Wichtig für den Erfolg einer Verkürzung ist dabei jedoch, dass die Bindungsfähigkeit an CGRP erhalten bleibt. Fig. 52 zeigt einen Strukturvorschlag für das Spiegelmer. Aufgrund dieses Strukturvorschlages lassen sich keine rationalen Verkürzungsvorschläge erarbeiten. Eine Verkürzung vom 3'-Ende her lieferte dann aber überraschenderweise gleichbleibend gute Bindungseigenschaften bei Steigerung des Anteils aktiver (bindender) Moleküle, was möglicherweise auf eine Begünstigung der bindenden Konformation (Faltung) durch eine Verringerung störender Einflüsse der 3'-terminalen Nukleotide zurückzuführen sein könnte. Die um 6 Nukleotide verkürzte Sequenz lautet wie folgt:

STAR-R02-15xx-014 (auch als NOX-504-014 bezeichnet):

GGA CUG AUG GCG CGG UCC UAU UAC GCC GAA AGG GAG AGG GGA (SEQ ID No. 245).

Eine weitere Ausbeutesteigerung in der Spiegelmersynthese ist durch Auflösung des 3'-terminalen Purin-Bereiches ab G28 zu erwarten. Daher wurden dort gezielt Punktmutationen eingeführt. Die Sequenz, welche trotz zweier A-U-Mutationen (A30U und A35U) überraschenderweise noch eine gleichbleibend hohe Bindung an CGRP zeigte, lautet wie folgt:

NOX-504-089:

GGA CUG AUG GCG CGG UCC UAU UAC GCC GAU AGG GUG AGG GGA (SEQ ID No. 246).

**<u>Beispiel 15</u>: Charakterisierung der verkürzten Varianten**

Die verkürzten Varianten des Spiegelmers STAR-R02-15xx-F12 wurden wie in Beispiel 13 beschrieben getestet.

Darüber hinaus wurde die Spezifität des 42 Nukleotide langen Spiegelmers NOX-504-014 (verkürztes STAR-R02-15xx-F12) bei 37°C gegen Calcitonin (CT), Calcitonin gene related peptide I (α-CGRP), Calcitonin gene related peptide II (β-CGRP), Adrenomedullin (AMD) und Amylin getestet.

Zunächst wurden die Dosis-Wirkungs-Kurven der Peptide ohne Zugabe von Spiegelmeren bestimmt. Für diejenigen Peptide, die eine gute Rezeptorantwort zeigten (gemessen über erhöhte intrazelluläre cAMP-Levels), wurden direkte Versuche mit zunehmenden Spiegelmerkonzentrationen durchgeführt und die $IC_{50}$ Werte bestimmt. Die folgenden Peptide wurden in direkten Versuchen gemessen:

- α-CGRP aus der Ratte und humanes α-CGRP ($EC_{50}$ bei 1 nM), dargestellt in Fig. 53 und Fig. 54,
- humanes β-CGRP ($EC_{50}$ bei 1 nM), dargestellt in Fig. 55 und
- Adrenomedullin aus der Ratte und humanes Adrenomedullin ($EC_{50}$ bei 30 nM), dargestellt in Fig. 56 und Fig. 57.

Das Spiegelmer NOX-504-014 bindet stark an Ratten α-CGRP und humanes α-CGRP. Die $IC_{50}$-Werte waren jeweils 2,8 nM und 25 nM (Fig. 53 und 54).

Wie den Figuren 53-55 zu entnehmen ist, bindet das Spiegelmer NOX-504-014 humanes β-CGRP ähnlich gut wie Ratten α-CGRP und daher besser als humanes α-CGRP. Der $IC_{50}$ liegt bei etwa 1,5 nM. Die Versuche wurden mit jeweils 1 nM CGRP durchgeführt, was dem $EC_{50}$ entspricht. Daher ergeben sich folgende $K_I$s für NOX-504-014:

α-CGRP der Ratte: 1,4 nM, humanes α-CGRP: 12,5 nM und β-CGRP: 750 pM.

Das Spiegelmer NOX-504-014 hemmt nur schwach die Wechselwirkung zwischen Ratten bzw. humanem Adrenomedullin mit dem Calcitonin-Rezeptor-ähnlichen Rezeptor (CRLR). Der $IC_{50}$ konnte nicht bestimmt werden. In diesen Versuchen wurde 30 nM Adrenomedullin eingesetzt, was dem $EC_{50}$ von Adrenomedullin am CRLR entspricht. Sogar mit 100-fachem Überschuss an Spiegelmer (3 μM) wurde keine eindeutige Reduktion in der cAMP-Produktion beobachtet.

Die übrigen Peptide wurden in Kompetitionsversuchen mit α-CGRP getestet. In diesen Versuchen wurde die Konzentration von α-CGRP und Spiegelmer konstant gehalten, während die Konzentration der Peptide erhöht wurde. Im Falle einer Kreuz-Reaktivität werden Kompetitor-Spiegelmerkomplexe gebildet. Dadurch erniedrigt sich die Konzentration an freiem Spiegelmer.

Dann werden Spiegelmere aus den α-CGRP-Spiegelmer-Komplexen freigesetzt. Das somit freigesetzte α-CGRP führt folglich zur Rezeptor-Stimulierung. Peptide die in Kompetitionsversuchen getestet wurden, waren:

- Amylin aus der Ratte und humanes Amylin (EC50 jeweils bei 1 μM + 3 μM), dargestellt in Fig. 58 und Fig. 59 und
- Calcitonin aus der Ratte und humanes Calcitonin ($EC_{50}$ nicht bestimmbar), dargestellt in Fig. 60 und Fig. 61.

Amyline stimulieren den Calcitonin-Rezeptor. Diese Stimulierung ist jedoch mit einem $EC_{50}$ von 1 μM und 3 μM für jeweils Ratten- und humanes Amylin recht schwach. Um direkte Versuche durchzuführen, müssten sehr hohe Spiegelmer-Konzentrationen benutzt werden. Dies könnte zu ungewünschten unspezifischen Effekten führen. Daher wurde ein indirekter Ansatz gewählt. Wir stellten uns dazu die Frage, wie viel Amylin notwendig sei, um die Konzentration von freien Spiegelmeren durch Bildung von Amylin-Spiegelmer-Komplexen zu reduzieren, so dass CGRP aus CGRP-Spiegelmer-Komplexen freigesetzt werden würde. Das freigesetzte freie CGRP würde dann durch Rezeptor-Stimulation und eine starke cAMP-Antwort (CGRP hat eine $EC_{50}$ von 1 nM) nachgewiesen werden. Die Graphen für Ratten-Amylin (Fig. 58) sowie humanes Amylin (Fig. 59) zeigen, dass Amylin bei einer Konzentration von 100 nM offensichtlich fähig ist, das Gleichgewicht in Richtung weniger freies Spiegelmer zu verschieben, und daher in Richtung von mehr freiem α-CGRP. Bei 100 nM Amylin ist die Stöchiometrie zwischen Amylin und Spiegelmeren 1:1.

Im Ratten-Amylin Versuch bei 100 nM ist der Beitrag von CGRP zum Gesamtsignal 56 pmol cAMP hoch. Dies ist zirka 50 % des Gesamtsignals. Wenn man die Resultate des Ratten α-CGRP-Graphen ($IC_{50}$ von 2 nM Spiegelmer bei 1 nM Ratten α-CGRP) hinzuzieht, könnte man schließen, dass bei der Konzentration von 100 nM Amylin nur 2 nM freies Spiegelmer zur Bindung an CGRP zur Verfügung stehen. Die verbleibenden Spiegelmere müssten daher im Komplex mit Ratten-Amylin vorliegen. Da überdies die Stöchiometrie von Amylin und Spiegelmer 1:1 ist, deutet dies auf eine niedrige Bindungskonstante des Spiegelmers für Amylin hin. Vermutlich liegt sie im nanomolaren Bereich.

Im gleichen Versuch mit humanen Peptiden ist die Höhe der ausschließlich durch humanes α-CGRP-induzierten cAMP-Produktion bei 26 % des Maximums, wenn 100 nM humanes Amylin eingesetzt wird. Dies stimmt, wenn man die Dosis-Wirkungs-Kurve von humanem α-CGRP hinzuzieht (Fig. 54), mit einer Spiegelmer Konzentration von fast 100 nM überein. Bei 300 nM h-Amylin werden bereits 50 % der maximalen cAMP-Produktion durch die CGRP-Rezeptor-Interaktion erreicht. Hier muss die Konzentration freier Spiegelmere vergleichbar der $IC_{50}$ für humanes CGRP (d.h. 25 nM) sein. 75 nM (75 %) der Spiegelmere müssen daher im Komplex mit den anwesenden 300 nM humanem Amylin sein. Die Interaktion ist demzufolge etwas schwächer, vermutlich aber immer noch im nanomolaren Bereich.

Weder Ratten- noch humanes Calcitonin stimulieren die intrazelluläre cAMP-Produktion der SK-N-MC Zellen. Daher wurde ein Kompetitionsexperiment mit 1 nM α-CGRP und 100 μM Spiegelmer durchgeführt, um ein cAMP-Signal zu erhalten. In diesem Fall ist die Menge des cAMP, welches durch die direkte Wirkung von Calcitonin produziert wird, unbedeutend. Auch bei steigender Menge Kompetitor (Calcitonin) wurde kein zusätzliches cAMP gebildet. Calcitonin ist daher offensichtlich nicht in der Lage, Spiegelmer zu binden und so durch eine Verschiebung des Gleichgewichtes CGRP aus CGRP-Spiegelmer-Komplexen zu befreien, was in einer cAMP-Antwort resultiert hätte. Wie aus Figuren 60 und 61 ersichtlich, zeigten die Experimente, dass eine keine Kreuz-Reaktivität der Spiegelmere mit Calcitonin nicht vorliegt.

In einem weiteren Versuch wurde eine Dosis-Wirkungskurve für das Spiegelmer NOX-504-089 mit den A-U-Mutationen in Zellkultur aufgenommen. Die Sequenz des Spiegelmers NOX-504-089 ist in Beispiel 14 aufgezeigt. Wie sich aus der Figur 62 ergibt, konnten die Mutationen überraschenderweise ohne Verlust der Bindungsgüte eingebaut werden.

**<u>Beispiel 16</u>: Reselektion von STAR-R02-15xx-F12 (NOX-504)**

Die in der Selektion mit biotinyliertem Ratten-CGRP erhaltene RNA-Sequenz NOX-504 (SEQ ID No. 504) (Fig. 63) diente als Ausgangspunkt für eine Reselektion mit humanem biotinyliertem D-CGRP (D-hCGRP).

Um eine wechselseitige Kontamination von RNA-Pools bei der Reselektion zu vermeiden, wurden die sechs 3'-terminalen Nukleotide (Positionen 43-48) der Ausgangssequenz modifiziert (Grt2-STAR-504-ad3, auch genannt NOX-504-ad3; siehe Fig. 63). Die Modifikation besteht dabei in einer Änderung der Sequenz am 3'-Ende, genauer der letzten sechs Nukleotide, die von GACACG zu GCACGG geändert wurden, um eine Kontamination des Reaktionsansatzes zu vermeiden.

Es wurde eine 18%-Mutations-Bibliothek von Molekülen basierend auf NOX-504-ad3 generiert. Dafür wurden die vier 5'-terminalen und sechs 3'-terminalen Positionen der Sequenz konstant gehalten, während in den Positionen 5 bis 42 die in der Sequenz vorgegebenen Basen nur zu 82%, die anderen Basen zu jeweils 6 % eingebaut werden; die Bibliothek repräsentiert also die Sequenz NOX-504-ad3 mit eine Mutationsrate von 0,18 in den Positionen 5 bis 42.

Die Reselektion sollte Hinweise auf die Variabilität der Sequenz und darüber auf die Struktur des RNA-Moleküls liefern. Aus diesem Grund wurden zum einen eine vergleichsweise geringe Mutationsrate des RNA-Pools und zum anderen eine niedrige Rundenzahl für die Selektion gewählt.

Die Durchführung der Selektion folgt der Beschreibung im Beispiel 4, wobei der RNA-Pool und seine Herstellung, einige Oligonukleotide, sowie die Elution und die Ligation der eluierten RNA-Moleküle wie folgt verändert wurden:

**<u>Verwendung eines Mutations-Pools</u>**

Der Mutationspool mit vorgeschaltetem T7-Promotor wird als reverser ssDNA-Strang synthetisiert, enzymatisch in dsDNA umgewandelt und anschließend in eine RNA-Bibliothek transkribiert:

504-ad3-18%-Bibliothek (reverser ssDNA-Strang)

5'- $C_{OMe}C_{OMe}$G TGC ***TCC CCT CTC CCT TTC GGC GTA ATA GGA CCG CGC CAT CA***G TCC TAT AGT GAG TCG TAT TAG TAG TCG C -3'

(in kursiv bezeichnete Positionen sind zu je 6% die von der Sequenz abweichenden Desoxynukleotide (A,T,G,C) eingebaut).

504-ad3-18%-Bibliothek (RNA-Sequenz nach Transkription)

**5'- GGA C*UG AUG GCG CGG UCC UAU UAC GCC GAA AGG GAG AGG GGA* GCA CGG -3'**

(in kursiv bezeichnete Positionen sind zu je 6% die von der Sequenz abweichenden Ribonukleotide (A,T,G,C) eingebaut).

**Oligonukleotide für die Ligation der Primersequenzen "primer ligation"**

*STAR-1 Reverse Primer Ribo U (= STAR-1 Reverse Matrix)*
5'- GCG AGT TCA $GCU_{OH}$ CAG $CGU_{OH}$ CCG TGC - 3' 24 nt
*STAR-1 N+1-Reverse Matrix Ribo I*
5'- GCG AGT TCA $GCU_{OH}$ CAG $CGI_{OH}$ CCG TGC - 3' 24 nt

**Herstellung der initialen RNA-Bibliothek**

Der reverse ssDNA-Strang der 504-ad3-18%-Bibliothek wurde mit einem dreifachen molaren Überschuss STAR-1 Forward Primer (s. Beispiel 4) gemischt und enzymatisch durch AmpliTaq DNA Polymerase Stoffel Fragment (Applied Biosystems) in doppelsträngige DNA überführt. Diese wurde als Template in die T7 Transkription (s. Beispiel 4) eingesetzt.

**Elution der bindenden RNA-Moleküle**

Die nach dem Waschen auf der Matrix verbliebene RNA wird zweimal mit jeweils 100 µl Guanidiniumisothiocyanat (Roti-Quick 1; Fa. Roth) in 15 min bei 37 °C eluiert.

**Ligation**

Die Ligate werden nach dem Protokoll der Simultan-Ligation mit der eluierten RNA verknüpft.

Es wurden 5 Runden (R1 bis R5) Reselektion durchgeführt. Die Stringenz wurde dabei erhöht, indem die Peptid-Konzentration von 1 µM (R1) über 100 nM (R2) und 1 nM (R3, R4) auf 0,1 nM (R5) gesenkt wurde. Gleichzeitig wurde das RNA:Peptid-Verhältnis von 1:2 (R1, R2, R3) über 1:1 (R4) auf 10:1 (R5) erhöht. Nach anfänglichem Anstieg des Signals in den Runden R1 und R2 auf ein Signal/Hintergrund-Verhältnis von 4,5 reduzierte sich der Quotient mit steigender Stringenz bis auf einen Wert von ca. 1,3. Der Selektionsverlauf ist in der Fig. 64 dargestellt.

Die CGRP-bindende RNA der Runde 5 wurde amplifiziert, kloniert und sequenziert. Dabei wurden 22 Sequenzen erhalten: die Ausgangssequenz der Reselektion sowie weitere 16 Sequenzen mit unterschiedlichen Mutationen. Unter diesen befindet sich die Sequenz Grt2-STAR-504-5-B0.1-C10 (SEQ ID No. 252), welche durch die Substitution A37U, sowie zwei Insertionen von Guanosin zwischen den Positionen 29/30 und 41/42 charakterisiert ist (ins[29G30]; A37U; ins[41G42]; s. Figur 63).

**<u>Beispiel 17</u>: Biologische Wirksamkeit von Grt2-STAR-504-5-B0.1-C10**

Grt2-STAR-504-5-B0.1-C10 wurde als D-RNA mittels biotinyliertem D-hCGRP selektiert. Um die biologische Aktivität des entsprechenden Spiegelmers (L-RNA) mit L-hCGRP zu analysieren, wurde auf das in Beispiel 8 beschriebene Zellkultur-Modell zurückgegriffen.

Die Ausgangssequenz der Reselektion (NOX-504-ad3) mit den modifizierten 3'-terminalen Nukleotiden ist im Vergleich zu NOX-504 hinsichtlich der Bindung von CGRP kaum verändert. Deswegen wurde vermutet, dass der variierende Sequenz-Bereich für die Peptid-Bindung nicht von Bedeutung ist. Für die Zellkultur-Studien wurde entsprechend das um sechs Nukleotide verkürzte Spiegelmer L097 (Fig. 65) verwendet.

Im Zellkultur-Experiment wurden die Dosis-Wirkungskurven von L097 (SEQ ID No. 253) mit hCGRP und rCGRP bestimmt. Dabei wurde ein $IC_{50}$ von [L097] = 6 nM für die Inhibition von hCGRP und [L097] = 1,5 nM für die Inhibition von rCGRP ermittelt (Figs. 66A und 66B).

**<u>Beispiel 18</u>: Mutationsanalyse von L097 im Zellkultur-Modell**

Die drei Mutationen, welche L097 von der Ausgangssequenz der Reselektion unterscheiden, führen zu einer etwa fünffachen Verbesserung der Bindung an hCGRP. Um zu analysieren, welche dieser drei Mutationen für die Bindungsverbesserung notwendig sind, wurden Spiegelmere mit den Einzelmutationen (ins[29G30]; A37U; ins[41G42]), sowie deren Kombinationen synthetisiert und deren biologische Aktivität im Zellkultur-Modell untersucht. Die Durchführung der Experimente folgt der Beschreibung im Beispiel 8. Die Sequenzen der L-RNA sind in Fig. 65 aufgeführt, wobei L097 SEQ ID No. 253, L102 SEQ ID No. 254, L103 SEQ ID No. 255, L104 SEQ ID No. 256, L105 SEQ ID No. 257, L106 SEQ ID No. 258, L107 SEQ ID No. 259, L108 SEQ ID No. 260, L109 SEQ ID No. 261 entspricht.

Jedes der Spiegelmere wurde sowohl in einer Konzentration von 10 nM, als auch 100 nM eingesetzt und in seiner biologischen Wirkung auf hCGRP vermessen (Fig. 67). Dabei zeigte sich, dass keine der Einzelmutationen (L102, L103, L104) die CGRP-Wirkung derart stark hemmt wie L097 (vgl. L097, L102, L103, L104). Das gleiche gilt für die Kombination von Mutationen (vgl. L097, L105, L106) mit Ausnahme des Klones L107: die Insertion der zwei Nukleotide zwischen die Positionen 29/30 und 41/42 ist ausreichend für die verbesserte Bindung des RNA-Spiegelmers an hCGRP.

Der 3'-Terminus von L097 ist stark Purin-reich. Zur Vereinfachung der chemischen Synthese und Erhöhung der Ausbeute des Spiegelmers sollte untersucht werden, ob weitere AU→U-Subsitutionen eingeführt werden können, ohne dass die biologische Aktivität beeinflusst wird.

Hierzu wurden in die Sequenz von L097 die zusätzlichen Mutationen A30U (=L108) oder A35U (=L109) eingeführt (Sequenzen vgl. Fig. 65) und im Zellkultur-Modell eine Dosis-Wirkungskurve der entsprechenden Spiegelmere erstellt (Fig. 68).

Dabei wurden $IC_{50}$-Werte von [L108]=5,5 nM und [L109]=9 nM für hCGRP ermittelt. Im Vergleich zum Spiegelmer L097 wird die biologische Wirkung durch die Einführung der zusätzlichen Mutationen nicht wesentlich verschlechtert.

## Beispiel 19: Charakterisierung von Reselektionsvarianten

In der Reselektion von NOX-504 (s. Beispiel 16) wurden in den 22 Sequenzen als häufigste Mutationen die Substitutionen A30U (4 von 22), A30G (5 von 22) und A37U (5 von 22) beobachtet. Die tolerierte Mutation A30U war bereits bekannt (vgl. NOX-504-089, Beispiel 14). Zur Charakterisierung der beiden anderen Varianten wurden folgende Sequenzen generiert:

NOX-504-095:

**GGA CUG AUG GCG CGG UCC UAU UAC GCC GAG AGG GAG UGG GGA (SEQ. ID.** No. 262)

Diese Sequenz unterscheidet sich von NOX-504-014 durch die Substitutionen A30G und A37U.

NOX-504-096:
**GGA CUG AUG GCG CGG UCC UAU UAC GCC GAU AGG GUG UGG GGA** (SEQ.ID.No. 263)

Hier ist die Sequenz NOX-504-089 durch die Substitution A37U erweitert.

Es wurden Spiegelmere (L-RNA) der beiden Sequenzen synthetisiert und deren biologische Aktivität im Zellkultur-Modell untersucht. Dazu wurden Dosis-Wirkungskurven der Spiegelmere gegen Ratten-α-CGRP (Methode s. Beispiel 9) erstellt, und die ermittelten $IC_{50}$-Werte mit denen von NOX-504-014 und NOX-504-089 verglichen. Die Dosis-Wirkungskurve ist in Figur 69 dargestellt.

Es wurden $IC_{50}$-Werte von [NOX-504-095] = 1-2 nM und [NOX-504-096] = 10 nM für CGRP der Ratte ermittelt. NOX-504-095 bindet damit etwa gleich gut wie NOX-504-014 und NOX-504-089 (vgl. Beispiel 15), wohingegen der $IC_{50}$-Wert von NOX-504-096 etwa um den Faktor 5 schlechter ist. Folglich ist eine Vereinfachung der Synthese des Spiegelmers NOX-504-089 durch Einführung einer weiteren A→U-Substitution nicht möglich (vgl. Beispiel 14).

## Beispiel 20: Bindung der Spiegelmere NOX-504-014, NOX-504-089, NOX-504-097 an Ratten- und humanes Amylin und Wirkung als Amylin-Antagonisten in Zellkultur

Wie in Beispiel 15 bereits beschrieben, ist eine Bindung des Spiegelmers 504-014 an Amylin mit einer $IC_{50}$ im nanomolaren Bereich wahrscheinlich. Zur genaueren Klärung wurden direkte Versuche mit MCF-7 Zellen (DSZM-Deutsche Sammlung von Mikroorganismen und Zellkulturen GmbH, Braunschweig, Zugangsnummer 115) durchgeführt.

MCF-7- Zellen wurden in folgendem Medium kultiviert: Ham's F12 nutrient mix und DMEM (1:1) und Zusatz von 10% FBS und 50 Einheiten/ml Penicillin und 50 μg/ml Streptomycin. Alle weiteren Parameter der Kultivierung, der Aussaat und des Ablaufs des Assays zur Stimulation der cAMP-Produktion und der cAMP-Messung sind mit den in Beispiel 8 beschriebenen identisch.

Zur Stimulierung der intrazellulären cAMP-Produktion diente in den hier beschriebenen Experimenten allerdings nicht CGRP, sondern Amylin der Ratten bzw. Amylin vom Menschen (beides von BACHEM, Bubendorf, Schweiz)

Zunächst wurden Dosis-Wirkungs-Kurven der beiden Amyline aufgenommen, um den sensitiven und den linearen Bereich des Assays bestimmen zu können.

Wie den Figs. 70 und 71 zu entnehmen ist, liegen die $EC_{50}$-Werte bei 3,1 nM für Ratten-Amylin und 22 nM für humanes Amylin.

Die Amylin-Konzentrationen in den Experimenten zur Bestimmung der biologischen Wirksamkeit der Spiegelmere wurden auf 3 nM Ratten-Amylin und 30 nM humanes Amylin festgesetzt. Die Ergebnisse sind in Fig. 72 und Fig. 73 dargestellt.
Folgende Spiegelmer-Konzentrationen führten zu einer halbmaximalen Inhibition der cAMP-Produktion:

| Spiegelmer \ Peptid | $IC_{50}$ bei 3 nM Amylin (Ratte) | $IC_{50}$ bei 30 nM Amylin (human) |
|---|---|---|
| **NOX-504-014** | 19 nM | 15 nM |
| **NOX-504-089** | 5 nM | 16 nM |
| **NOX-504-097** | 10 nM | 21 nM |

Da hier die $IC_{50}$ im Bereich der Konzentration des stimulierenden Peptides liegt, ist folglich der Anteil des Spiegelmers, der überhaupt als freies Spiegelmer vorliegt, am $IC_{50}$ bereits deutlich unterhalb der eingesetzten Konzentration. Zur Berechnung der Dissoziationskonstanten $K_D$ muss allerdings der Anteil an freiem Spiegelmer herangezogen werden.
Die Dissoziationskonstante errechnet sich aus dem $IC_{50}$ nach folgender Formel:

$$K_D = IC_{50} - 0,5 * [\text{Amylin}]$$

Daraus ergeben sich folgende Dissoziationskonstanten:

| Spiegelmer \ Peptid | $K_D$ | $K_D$ |
|---|---|---|
| **NOX-504-014** | 17,5 nM | < 1 nM |
| **NOX-504-089** | 3,5 nM | 1 nM |
| **NOX-504-097** | 8,5 nM | 6 nM |

**<u>Beispiel 21</u>: Kalorimetrische Bestimmung der Bindungskonstanten und der Aktivität von NOX-504-L097**

<u>Durchführung</u>

Die Bindung von Spiegelmeren an humanes alpha-CGRP wurde auch über das Verfahren der Isothermen Titrations-Kalorimetrie (ITC) mit dem *VP ITC (Microcal)* bestimmt.
Dazu wurden 1,465 ml einer 10 μM Lösung der Spiegelmere in die adiabate Messzelle des Gerätes vorgelegt. Die bei Zugabe von 7,5 μl einer 70 μM humanen α-L-CGRP-Lösung frei werdende Bindungsenthalpie wird von dem Gerät registriert. Durch wiederholte Zugabe des Peptides und Messung der jeweils frei werdenden Bindungsenthalpien können Bindungskonstanten und Aktivitäten der Moleküle bestimmt werden.
Das Spiegelmer NOX-504-L097 wurde bei 37°C vermessen. Das Ergebnis ist in Fig. 74 dargestellt.

Ergebnisse der ITC-Messung

Die Bindungskonstante oder Dissoziationskonstante $K_D$ ist der Kehrwert der Assoziationskonstante $K_A$ (K im Enthalpiediagramm) und ergab sich für die Bindung des Spiegelmers NOX-504-L097 an humanes alpha-CGRP zu 52 nM. Die Aktivität war 80 % (Fig. 74).

**Patentansprüche**

1. CGRP bindende L-Nukleinsäure umfassend eine Sequenz gemäß SEQ ID No. 246 oder eine dazu homologe CGRP bindende L-Nukleinsäure, wobei die Homologie wenigstens 75 % beträgt, wobei das CGRP α-CGRP oder β-CGRP ist, und wobei die L-Nukleinsäure ein Antagonist von α-CGRP oder β-CGRP ist.

2. L-Nukleinsäure nach Anspruch 1, wobei die Homologie zu der Sequenz gemäß SEQ ID No. 246 wenigstens 85 % beträgt.

3. L-Nukleinsäure nach Anspruch 1 und 2, wobei die L-Nukleinsäure ein Antagonist des CGRP-Rezeptors ist und an einen Liganden des Rezeptors bindet und wobei der Ligand α-CGRP oder β-CGRP ist.

4. L-Nukleinsäure nach einem der Ansprüche 1 bis 3 mit einer Sequenz, wobei die Sequenz ausgewählt ist aus der Gruppe umfassend die Sequenzen gemäß SEQ ID No. 191, 192, 196, 199, 200, 245, 251, 252, 253, 254, 255, 256, 257, 258, 259, 260, 261, 262 und 263.

5. L-Nukleinsäure nach einem der Ansprüche 1 bis 4, **dadurch gekennzeichnet, dass** die L-Nukleinsäure ausgewählt ist aus der Gruppe umfassend DNA und RNA.

6. L-Nukleinsäure nach einem der Ansprüche 1 bis 5, **dadurch gekennzeichnet, dass** sie eine Länge aufweist, wobei die Länge ausgewählt ist aus der Gruppe, die Längen von 15 bis 150 Nukleotiden, 20 bis 100 Nukleotiden, 20 bis 80 Nukleotiden, 20 bis 60 Nukleotiden, 20 bis 50 Nukleotiden oder 30 bis 50 Nukleotiden umfasst und die Länge am meisten bevorzugt 25 bis 45 Nukleotide umfasst.

7. Verwendung einer L-Nukleinsäure nach einem der Ansprüche 1 bis 6 als ein Antagonist von CGRP und/oder des CGRP-Rezeptorsystems.

8. Verwendung einer L-Nukleinsäure nach einem der Ansprüche 1 bis 6 für die Herstellung eines Medikaments.

9. Verwendung nach Anspruch 8, **dadurch gekennzeichnet, dass** das Medikament für die Behandlung und/oder die Prävention einer Erkrankung ist, die aus der Gruppe ausgewählt ist, die Migräne, Cluster-Kopfschmerzen, Appetitlosigkeit, Übelkeit, Erbrechen, neurogene Entzündung, insbesondere neurogene Entzündung, die mit anderen Neuropeptiden vermittelt wird, Vasodilatation, erhöhter Blutdruck, Hypotonie, Tachikadie, Erkrankungen, die auf eine Aktivierung trigeminaler afferenter sensorischer Neurone und zentraler "nociceptiver" Neuronen, insbesondere höheren Schmerzzentren, zurückgehen, und chronisch entzündliche Schmerzen, umfasst und/oder zur Behandlung von Schmerzen, insbesondere chronische Schmerzen, akute Schmerzen, entzündliche Schmerzen, visceraler Schmerzen und neuropathischer Schmerzen.

10. Zusammensetzung umfassend eine L-Nukleinsäure nach einem der Ansprüche 1 bis 6 und bevorzugterweise einen pharmazeutisch akzeptablen Träger.

11. Komplex umfassend CGRP und eine L-Nukleinsäure nach einem der Ansprüche 1 bis 6.

12. Verwendung einer L-Nukleinsäure nach einem der Ansprüche 1 bis 6 zum *in vitro*-Nachweis von CGRP, bevorzugterweise α-CGRP oder β-CGRP und am bevorzugtesten menschliches α-CGRP oder β-CGRP.

13. Verfahren zum Screenen von CGRP-Antagonisten umfassend die folgenden Schritte:

- Bereitstellen eines Kandidaten-CGRP-Antagonisten,
- Bereitstellen einer L-Nukleinsäuren nach einem der Ansprüche 1 bis 6,
- Vorsehen eines Testsystems, welches ein Signal in Gegenwart eines CGRP-Antagonisten ergibt, und
- Bestimmen, ob der Kandidaten-CGRP -Antagonist ein CGRP-Antagonist ist.

14. Verfahren nach Anspruch 13, wobei das CGRP α-CGRP und/oder β-CGRP ist, bevorzugterweise menschliches α-CGRP und/oder β-CGRP.

**15.** Verfahren zum Screenen von CGRP-Agonisten umfassend die folgenden Schritte:

- Bereitstellen von CGRP,
- Bereitstellen einer L-Nukleinsäuren nach einem der Ansprüche 1 bis 6, bevorzugterweise einer markierten L-Nukleinsäure nach einem der Ansprüche 1 bis 6,
- Zugabe eines Kandidaten-CGRP-Agonisten, und
- Bestimmen, ob der Kandidaten-CGRP -Agonist ein CGRP-Agonist ist.

**16.** Verfahren nach Anspruch 15, **dadurch gekennzeichnet, dass** das Bestimmen **dadurch** erfolgt, dass festgestellt wird, ob die L-Nukleinsäure durch den Kandidaten-CGRP-Agonisten verdrängt wird.

**17.** Kit für den Nachweis von CGRP, bevorzugterweise α-CGRP oder β-CGRP, umfassend eine L-Nukleinsäure nach einem der Ansprüche 1 bis 6.

**18.** L-Nukleinsäure nach einem der Ansprüche 1 bis 6, zur Verwendung in einem Verfahren zur Behandlung einer Erkrankung, bevorzugterweise einer Erkrankung, die ausgewählt ist aus der Gruppe, die Migräne, Cluster-Kopfschmerzen, Appetitlosigkeit, Übelkeit, Erbrechen, neurogene Entzündung, insbesondere neurogene Entzündung, die mit anderen Neuropeptiden vermittelt wird, Vasodilatation, erhöhter Blutdruck, Hypotonie, Tachikadie, Erkrankungen, die auf eine Aktivierung trigeminaler afferenter sensorischer Neurone und zentraler "nociceptiver" Neuronen, insbesondere höheren Schmerzzentren, zurückgehen, Schmerzen, chronisch entzündliche Schmerzen, chronische Schmerzen, akute Schmerzen, entzündliche Schmerzen, visceraler Schmerzen und neuropathische Schmerzen.

## Claims

**1.** A CGRP binding L-nucleic acid comprising a sequence according to SEQ ID No. 246 or a CGRP binding L-nucleic acid homologous thereto, wherein the homology is at least 75 %, whereby the CGRP is α-CGRP or β-CGRP and wherein the L-nucleic acid is an antagonist of α-CGRP or β-CGRP.

**2.** The L-nucleic acid according to claim 1, wherein the homology to the sequence according to SEQ ID No. 246 is at least 85 %.

**3.** The L-nucleic acid according to claim 1 and 2, wherein the L-nucleic acid is an antagonist of the CGRP-receptor and binds to a ligand of the receptor, and wherein the ligand is α-CGRP or β-CGRP.

**4.** The L-nucleic acid according to any one of claims 1 to 3 with a sequence, wherein the sequence is selected from the group comprising the sequences according to SEQ ID No. 191, 192, 196, 199, 200, 245, 251, 252, 253, 254, 255, 256, 257, 258, 259, 260, 261, 262 and 263.

**5.** The L-nucleic acid according to any one of claims 1 to 4, **characterized in that** the L-nucleic acid is selected from the group comprising DNA and RNA.

**6.** The L-nucleic acid according to any one of claims 1 to 5, **characterized in that** it is of a length, wherein the length is selected from the group comprising lengths of 15 to 150 nucleotides, 20 to 100 nucleotides, 20 to 80 nucleotides, 20 to 60 nucleotides, 20 to 50 nucleotides or 30 to 50 nucleotides, wherein the most preferred length comprises 25 to 45 nucleotides.

**7.** Use of an L-nucleic acid according to any one of claims 1 to 6, as an antagonist of CGRP and/or the CGRP receptor system.

**8.** Use of an L-nucleic acid according to any one of claims 1 to 6 for the manufacture of a medicament.

**9.** Use according to claim 8, wherein the medicament is for the treatment and/or prevention of a disease selected from the group comprising migraine, cluster headache, lack of appetite, nausea, vomiting, neurogenic inflammation, in particular neurogenic inflammation imparted by other neuropeptides, vasodilation, increased blood pressure, hypotension, tachycardia, illnesses attributed to activation of trigeminal afferent sensory neurons and central "nociceptive" neurones, in particular higher pain centres, and chronic inflammatory pain, and/or for treating pain, in particular chronic pain, acute pain, inflammatory pain, visceral pain and neuropathic pain.

**10.** A composition comprising an L-nucleic acid according to any one of claims 1 to 6 and preferably a pharmaceutical acceptable carrier.

**11.** A complex comprising CGRP and an L-nucleic according to any one of claims 1 to 6.

**12.** Use of an L-nucleic acid according to any one of claims 1 to 6 for *in vitro* detection of CGRP, preferably α-CGRP or β-CGRP and most preferably human α-CGRP or β-CGRP.

**13.** A method for the screening of CGRP antagonists comprising the following steps:

- providing a candidate CGRP antagonist,
- providing an L-nucleic acid according to any one of claims 1 to 6,
- providing a test system providing a signal in the presence of a CGRP antagonist, and
- determining whether the candidate CGRP antagonist is a CGRP antagonist.

**14.** The method according to claim 13, wherein the CGRP is α-CGRP and/or β-CGRP, preferably human α-CGRP and/or β-CGRP.

**15.** A method for the screening of CGRP agonists comprising the following steps:

- providing CGRP,
- providing an L-nucleic acid according to any one of claims 1 to 6, preferably a labeled L-nucleic acid according to any one of claims 1 to 6,
- adding a candidate CGRP agonist, and
- determining whether the candidate CGRP agonist is a CGRP agonist.

**16.** The method according to claim 15, **characterized in that** the determining is made by assessing whether the L-nucleic acid is displaced by the candidate CGRP agonist.

**17.** A kit for the detection of CGRP, preferably α-CGRP or β-CGRP, comprising an L-nucleic acid according to any one of claims 1 to 6.

**18.** The L-nucleic acid according to any one of claims 1 to 6, for use in a method for the treatment of a disease, preferably a disease selected from the group comprising migraine, cluster headache, lack of appetite, nausea, vomiting, neurogenic inflammation, in particular neurogenic inflammation imparted by other neuropeptides, vasodilation, increased blood pressure, hypotension, tachycardia, illnesses attributed to activation of trigeminal afferent sensory neurons and central "nociceptive" neurones, in particular higher pain centres, pain, chronic inflammatory pain, chronic pain, acute pain, inflammatory pain, visceral pain and neuropathic pain.

## Revendications

**1.** Acide L-nucléique liant le CGRP, comprenant une séquence selon SEQ ID N° 246, ou acide L-nucléique liant le CGRP, qui en est homologue, l'homologie étant d'au moins 75 %, le CGRP étant l'α-CGRP ou le β-CGRP, et l'acide L-nucléique étant un antagoniste de l'α-CGRP ou du β-CGRP.

**2.** Acide L-nucléique selon la revendication 1, dans lequel l'homologie avec la séquence selon SEQ ID N° 246 est d'au moins 85 %.

**3.** Acide L-nucléique selon les revendications 1 et 2, l'acide L-nucléique étant un antagoniste du récepteur du CGRP et se liant à un ligand du récepteur, le ligand étant l'α-CGRP ou le β-CGRP.

**4.** Acide L-nucléique selon l'une des revendications 1 à 3, ayant une séquence, la séquence étant choisie dans le groupe comprenant les séquences selon SEQ ID N° 191, 192, 196, 199, 200, 245, 251, 252, 253, 254, 255, 256, 257, 258, 259, 260, 261, 262 et 263.

**5.** Acide L-nucléique selon l'une des revendications 1 à 4, **caractérisé en ce que** l'acide L-nucléique est choisi dans le groupe comprenant un ADN et un ARN.

6. Acide L-nucléique selon l'une des revendications 1 à 5, **caractérisé en ce qu'**il a une longueur, la longueur étant choisie dans le groupe qui comprend les longueurs de 15 à 150 nucléotides, de 20 à 100 nucléotides, de 20 à 80 nucléotides, de 20 à 60 nucléotides, de 20 à 50 nucléotides ou de 30 à 50 nucléotides, et la longueur comprenant de la manière la plus préférée 25 à 45 nucléotides.

7. Utilisation d'un acide L-nucléique selon l'une des revendications 1 à 6 en tant qu'antagoniste du CGRP et/ou du système récepteur du CGRP.

8. Utilisation d'un acide L-nucléique selon l'une des revendications 1 à 6 pour la fabrication d'un médicament.

9. Utilisation selon la revendication 8, **caractérisée en ce que** le médicament est destiné au traitement et/ou à la prévention d'une maladie qui est choisie dans le groupe qui comprend la migraine, l'algie vasculaire de la face, la perte d'appétit, les nausées, les vomissements, l'inflammation neurogène, en particulier l'inflammation neurogène qui est médiée par d'autres neuropeptides, la vasodilatation, l'hypertension, l'hypotonie, la tachycardie, les maladies qui sont dues à une activation des neurones sensoriels afférents trigéminaux et des neurones "nociceptifs" centraux, en particulier des centres supérieurs de la douleur, et les douleurs inflammatoires chroniques, et/ou au traitement de douleurs, en particulier de douleurs chroniques, de douleurs aiguës, de douleurs inflammatoires, de douleurs viscérales et de douleurs neuropathiques.

10. Composition comprenant un acide L-nucléique selon l'une des revendications 1 à 6 et de préférence un support pharmaceutiquement acceptable.

11. Complexe comprenant du CGRP et un acide L-nucléique selon l'une des revendications 1 à 6.

12. Utilisation d'un acide L-nucléique selon l'une des revendications 1 à 6 pour la détection *in vitro* du CGRP, de préférence de l'$\alpha$-CGRP ou du $\beta$-CGRP, et de la manière la plus préférée de l'$\alpha$-CGRP ou du $\beta$-CGRP humain.

13. Procédé pour le criblage d'antagonistes du CGRP, comprenant les étapes suivantes :

   - mise à disposition d'un antagoniste du CGRP candidat,
   - mise à disposition d'un acide L-nucléique selon l'une des revendications 1 à 6,
   - fourniture d'un système de test, qui donne un signal en présence d'un antagoniste du CGRP, et
   - détermination si l'antagoniste CGRP candidat est, ou non, un antagoniste du CGRP.

14. Procédé selon la revendication 13, dans lequel le CGRP est l'$\alpha$-CGRP et/ou le $\beta$-CGRP, de préférence l'$\alpha$-CGRP et/ou le $\beta$-CGRP humain.

15. Procédé pour le criblage d'agonistes du CGRP, comprenant les étapes suivantes :

   - mise à disposition de CGRP,
   - mise à disposition d'un acide L-nucléique selon l'une des revendications 1 à 6, de préférence d'un acide L-nucléique marqué selon l'une des revendications 1 à 6,
   - addition d'un agoniste du CGRP candidat, et
   - détermination si l'agoniste du CGRP candidat est, ou non, un agoniste du CGRP.

16. Procédé selon la revendication 15, **caractérisé en ce que** la détermination est effectuée par le fait que l'on constate si l'acide L-nucléique est, ou non, déplacé par l'agoniste du CGRP candidat.

17. Kit pour la détection du CGRP, de préférence l'$\alpha$-CGRP ou le $\beta$-CGRP, comprenant un acide L-nucléique selon l'une des revendications 1 à 6.

18. Acide L-nucléique selon l'une des revendication 1 à 6, pour utilisation dans un procédé destiné au traitement d'une maladie, de préférence une maladie qui est choisie dans le groupe qui comprend la migraine, l'algie vasculaire de la face, la perte d'appétit, les nausées, les vomissements, l'inflammation neurogène, en particulier l'inflammation neurogène qui est médiée par d'autres neuropeptides, la vasodilatation, l'hypertension, l'hypotonie, la tachycardie, les maladies qui sont dues à une activation des neurones sensoriels afférents trigéminaux et des neurones "nociceptifs" centraux, en particulier des centres supérieurs de la douleur, et les douleurs inflammatoires chroniques, et/ou au traitement de douleurs, en particulier de douleurs chroniques, de douleurs aiguës, de douleurs inflammatoires, de douleurs viscérales et de douleurs neuropathiques.

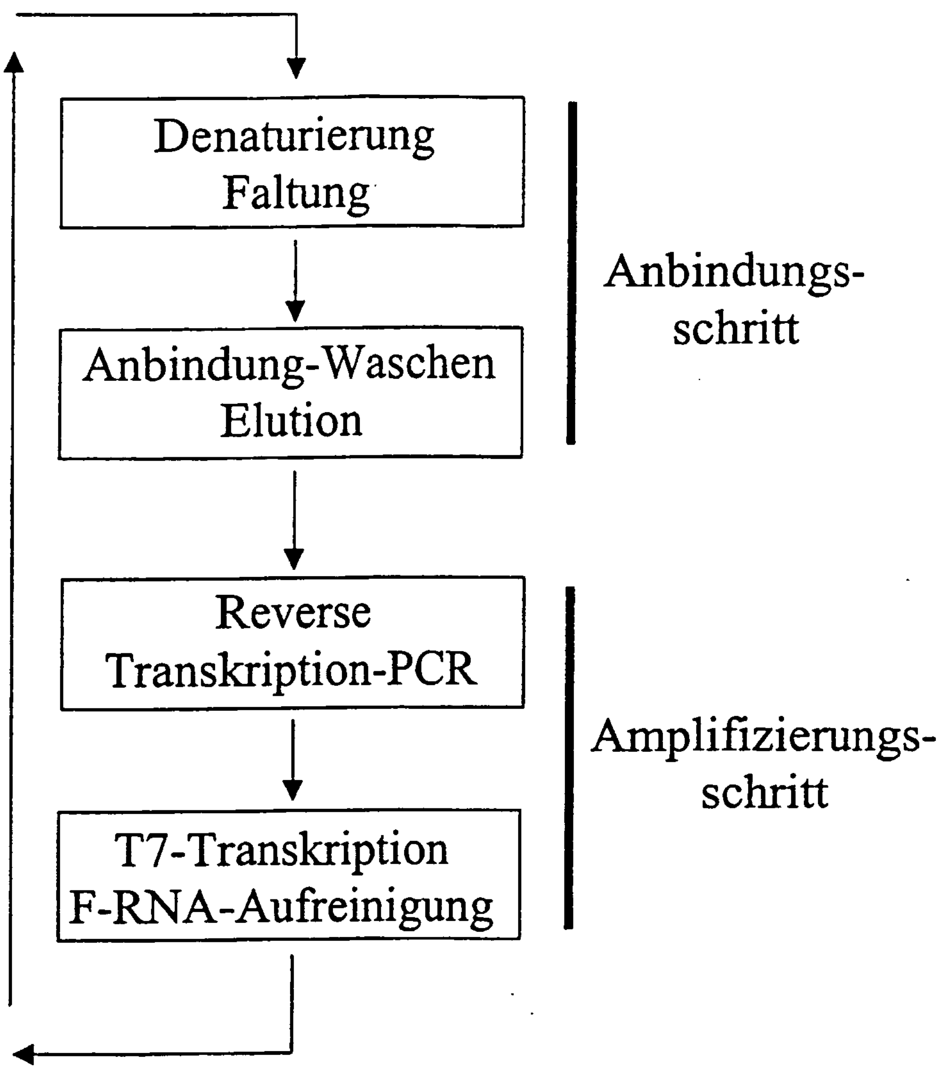
Denaturierung
Faltung
Anbindung-Waschen
Elution
Anbindungs-
schritt
Reverse
Transkription-PCR
T7-Transkription
F-RNA-Aufreinigung
Amplifizierungs-
schritt

**Fig. 1**

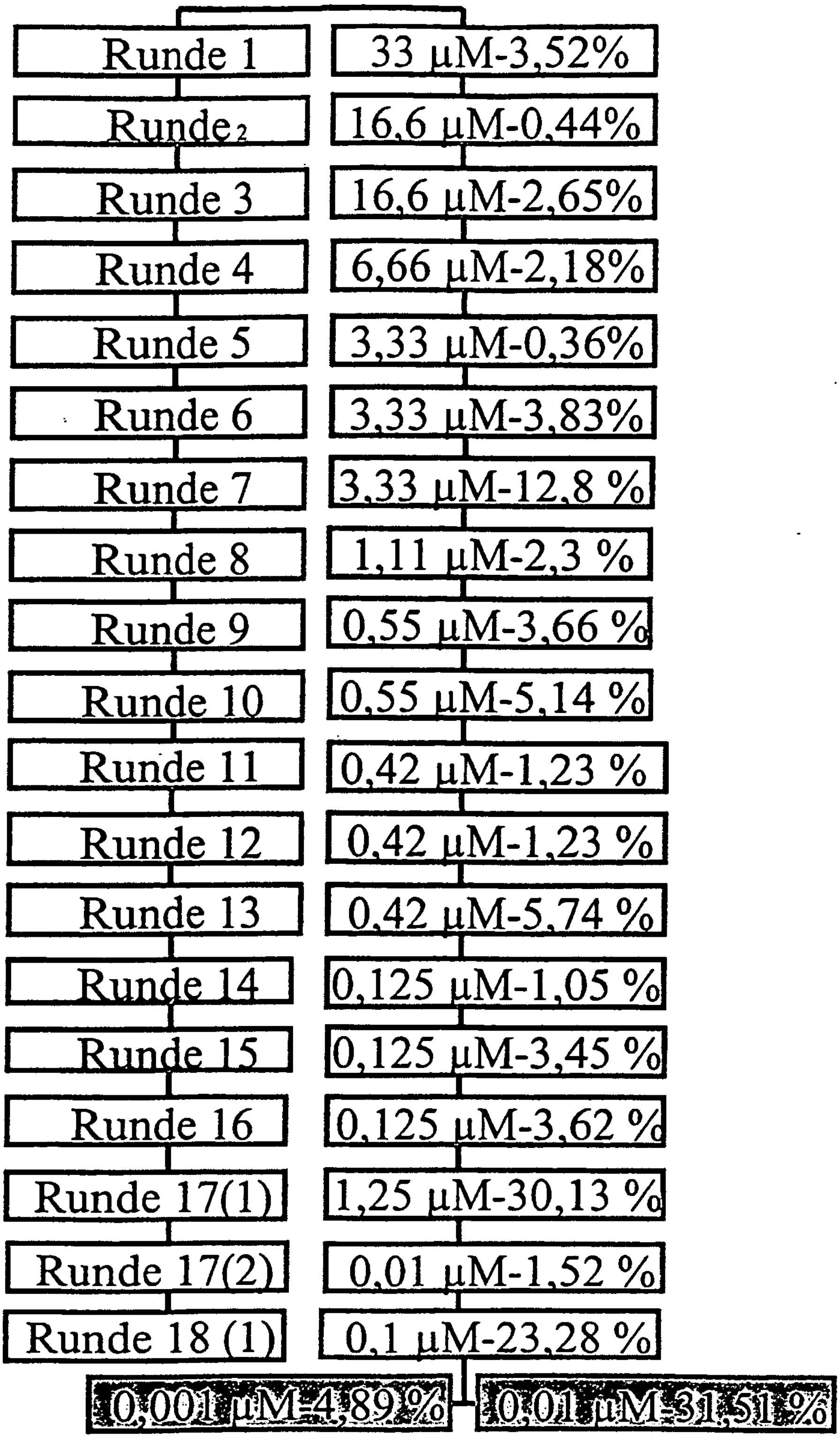
Runde 1
33 µM-3,52%
Runde 2
16,6 µM-0,44%
Runde 3
16,6 µM-2,65%
Runde 4
6,66 µM-2,18%
Runde 5
3,33 µM-0,36%
Runde 6
3,33 µM-3,83%
Runde 7
3,33 µM-12,8 %
Runde 8
1,11 µM-2,3 %
Runde 9
0,55 µM-3,66 %
Runde 10
0,55 µM-5,14 %
Runde 11
0,42 µM-1,23 %
Runde 12
0,42 µM-1,23 %
Runde 13
0,42 µM-5,74 %
Runde 14
0,125 µM-1,05 %
Runde 15
0,125 µM-3,45 %
Runde 16
0,125 µM-3,62 %
Runde 17(1)
1,25 µM-30,13 %
Runde 17(2)
0,01 µM-1,52 %
Runde 18 (1)
0,1 µM-23,28 %
0,001 µM-4,89%
0,01 µM-31,51 %

**Fig. 2A**

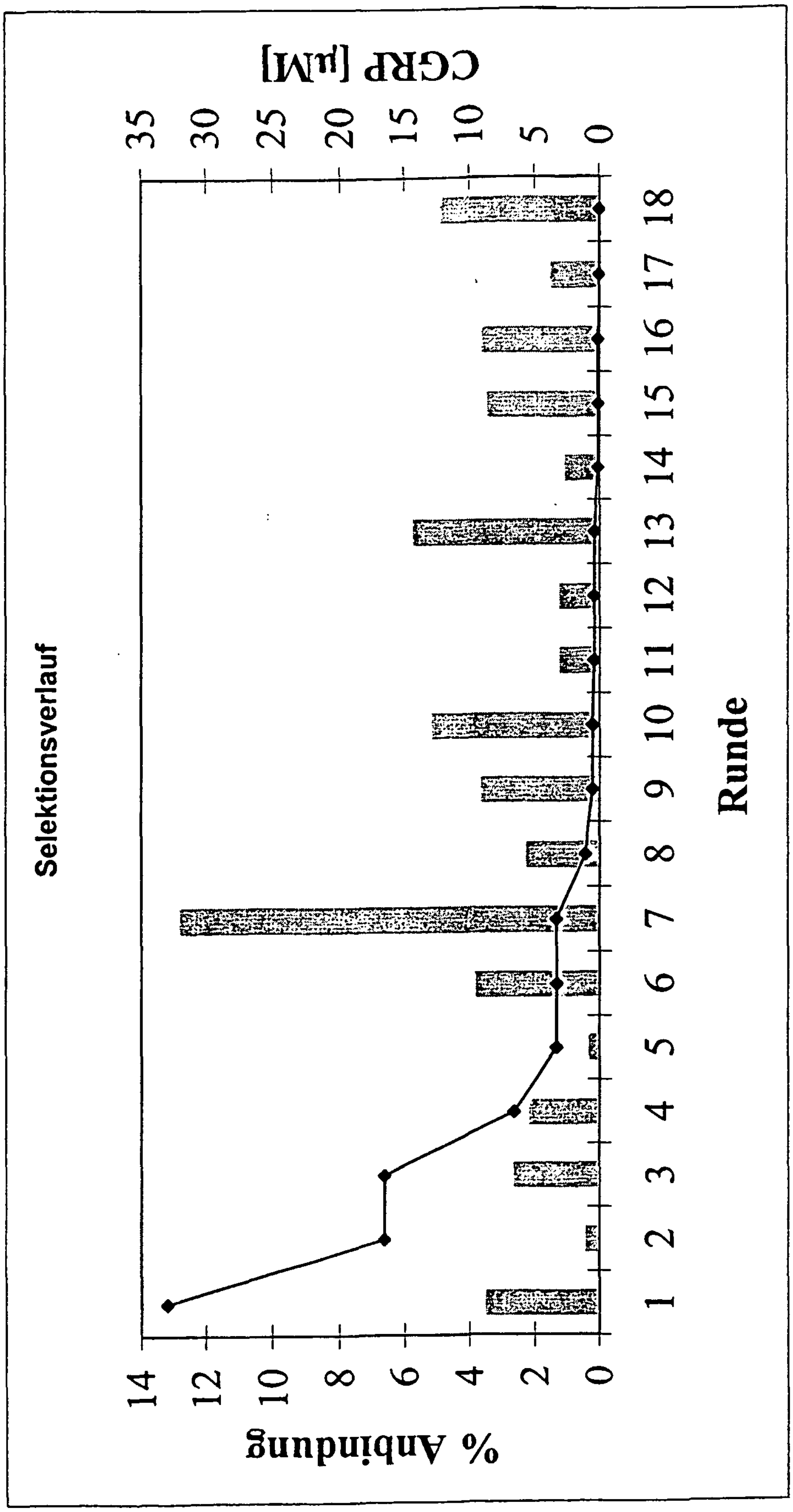
Selektionsverlauf
% Anbindung
CGRP [µM]
Runde
0 2 4 6 8 10 12 14
0 5 10 15 20 25 30 35
1 2 3 4 5 6 7 8 9 10 11 12 13 14 15 16 17 18

Fig. 2B

## Aligned sequences (+)strand

```
5'GGAGCTCAGCCTTCACTGCGAAGTGACGCACGTATGATAGTTTCCATTTTGGACTCCTGGGCACCACGGTCGGATCCAC 168x
5'GGAGCTCAGCCTTCACTGCGAAGTGATGCACGTATGATAGTTTCCATTTTGGACTCCTGGGCACCACGGTCGGATCCAC 1x
5'GGAGCTCAGCCTTCACTGC--AGTGACGCACGTATGATAGTTTCCATTTTGGACTCCTGGGCACCACGGTCGGATCCAC 2x
5'GGAGCTCAGCCTTCACTGCAAAGTGACGCACGTATGATAGTTTCCATTTTGGACTCCTGGGCACCACGGTCGGATCCAC 5x
5'GGAGCTCAGCCTTCACTGCGAAGTGACGCACGTATTATAGTTTCCATTTTGGACTCCTGGGCACCACGGTCGGATCCAC 1x
5'GGAGCTCAGCCTTCACTGCTAAGTGACGCACGTATGATAGTTTCCATTTTGGACTCCTGGGCACCACGGTCGGATCCAC 1x
5'GGAGCTCAGCCTTCACTGCGAAGTGACGCACGTATGATAGTTTCCGTTTTGGACTCCTGGGCACCACGGTCGGATCCAC 1x
5'GGAGCTCAGCCTTCACTGCGAAGTGACGTACGTATGATAGTTTCCATTTTGGACTCCTGGGCACCACGGTCGGATCCAC 1x
   FF primer (19)                                                  REV primer (20)
```

**Die Reihenfolge der 8 Klone ist: 666**
**711**
**732**
**669**
**670**
**781**
**836**
**748**

Fig. 3

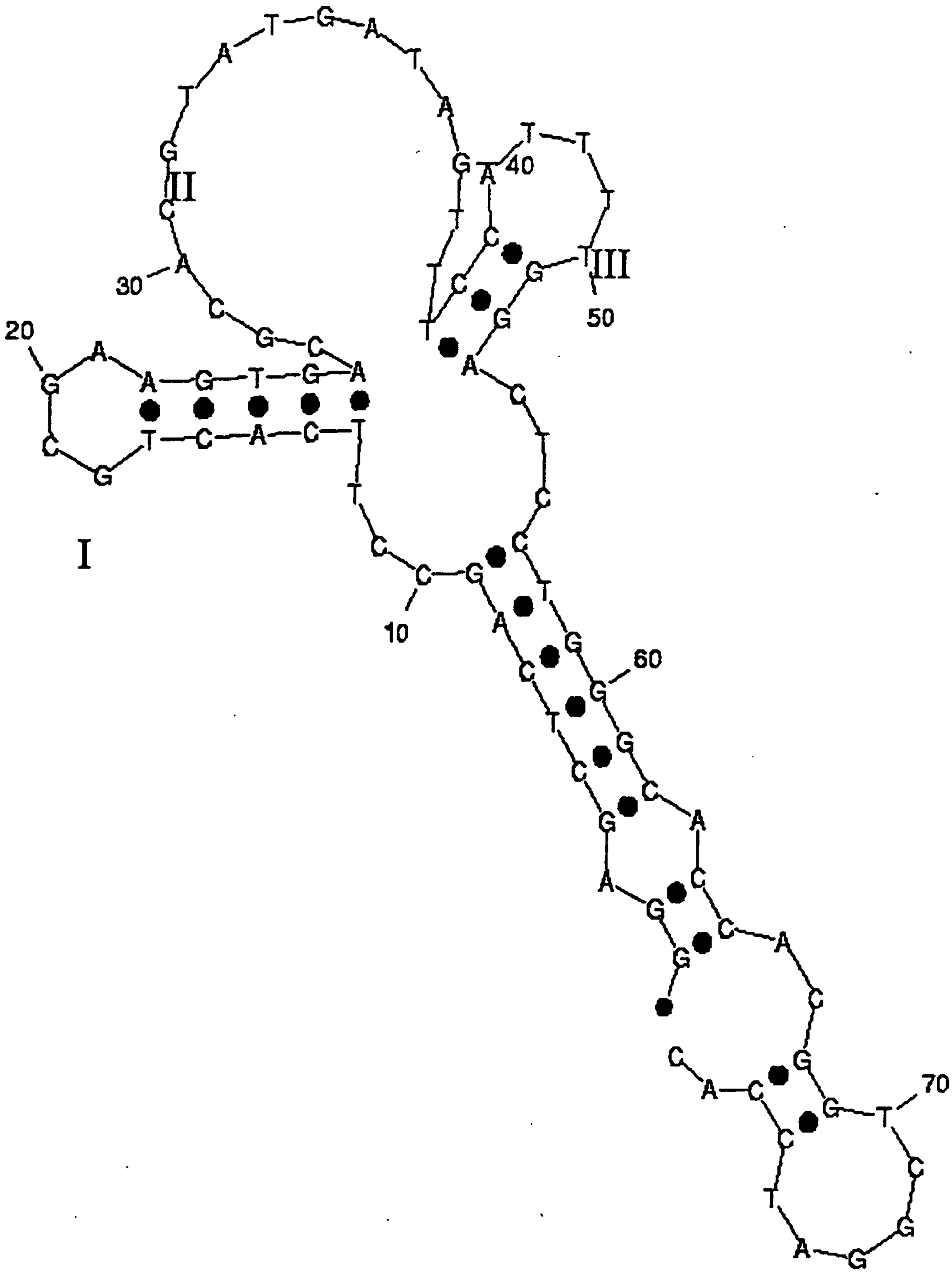


**Fig. 4**

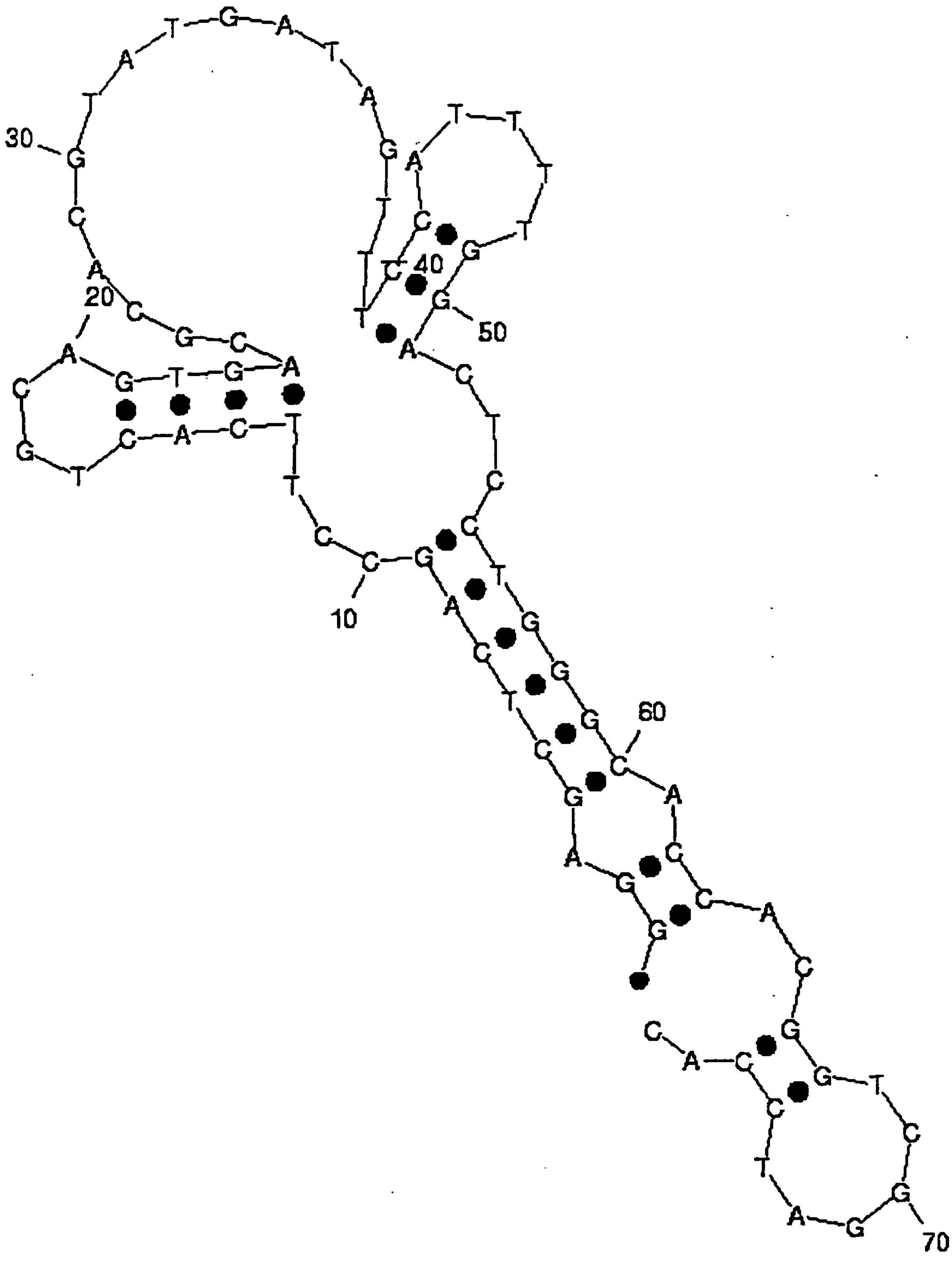


**Fig. 5**

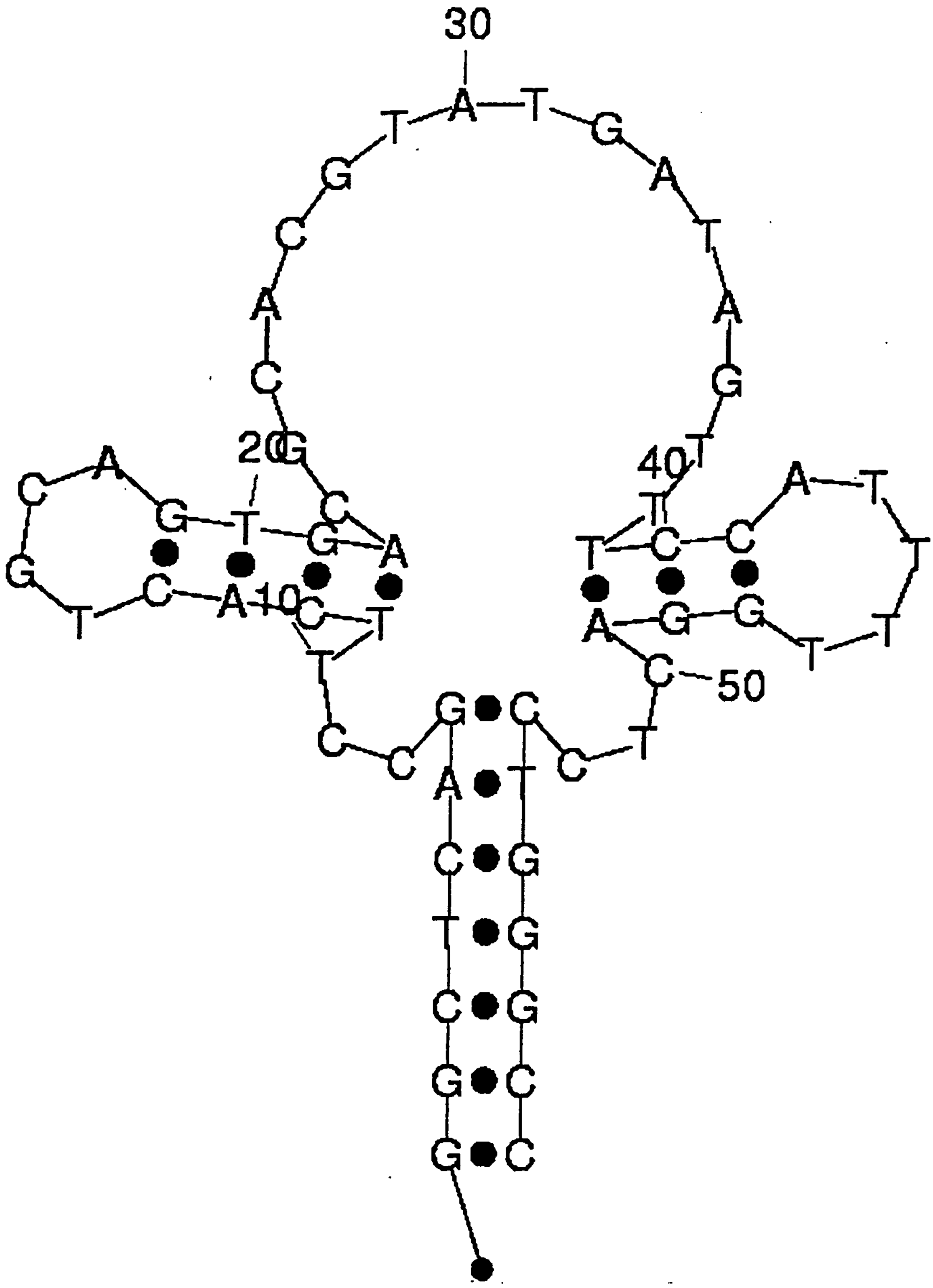


**Fig. 6**

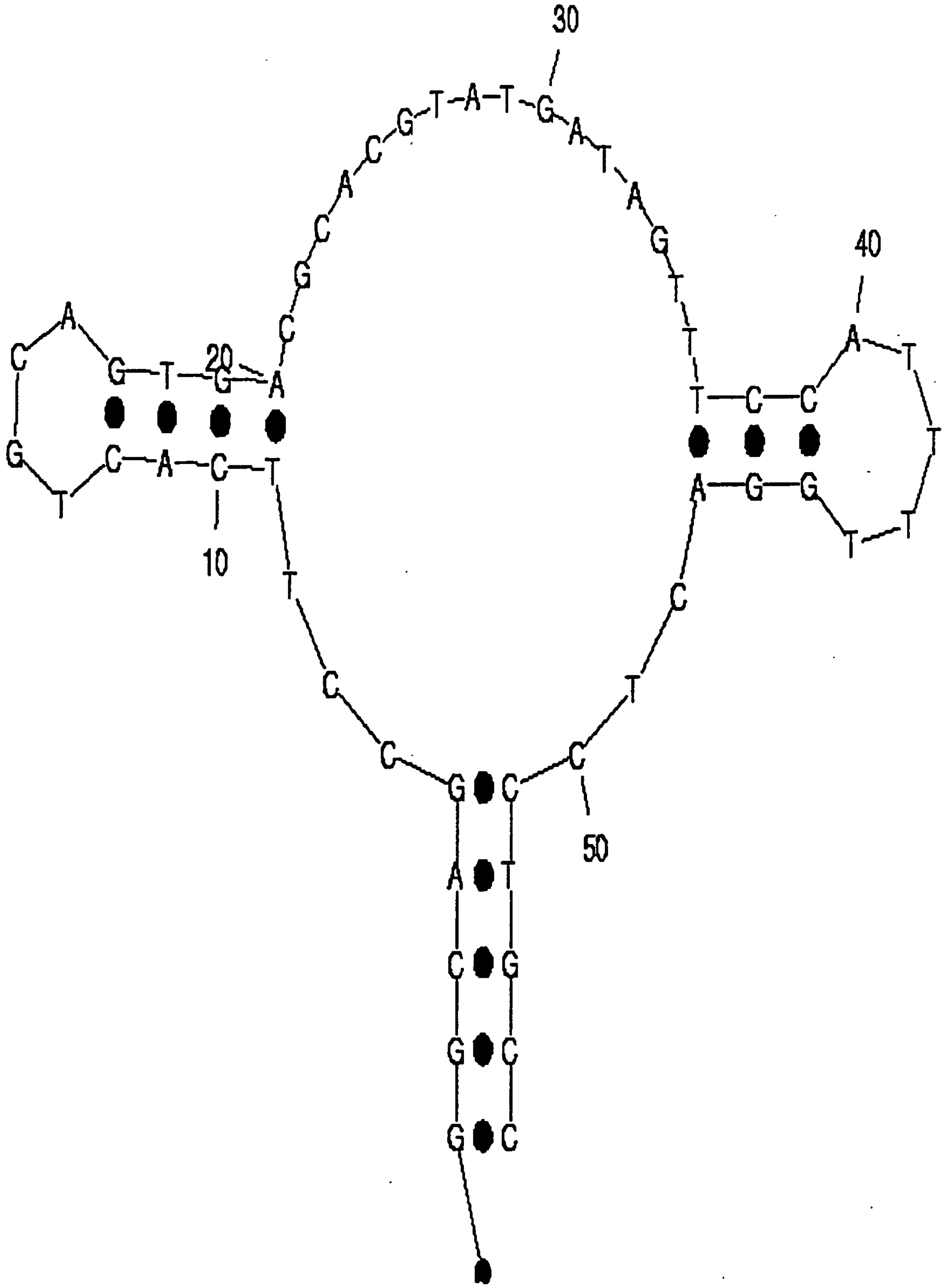


**Fig. 7**

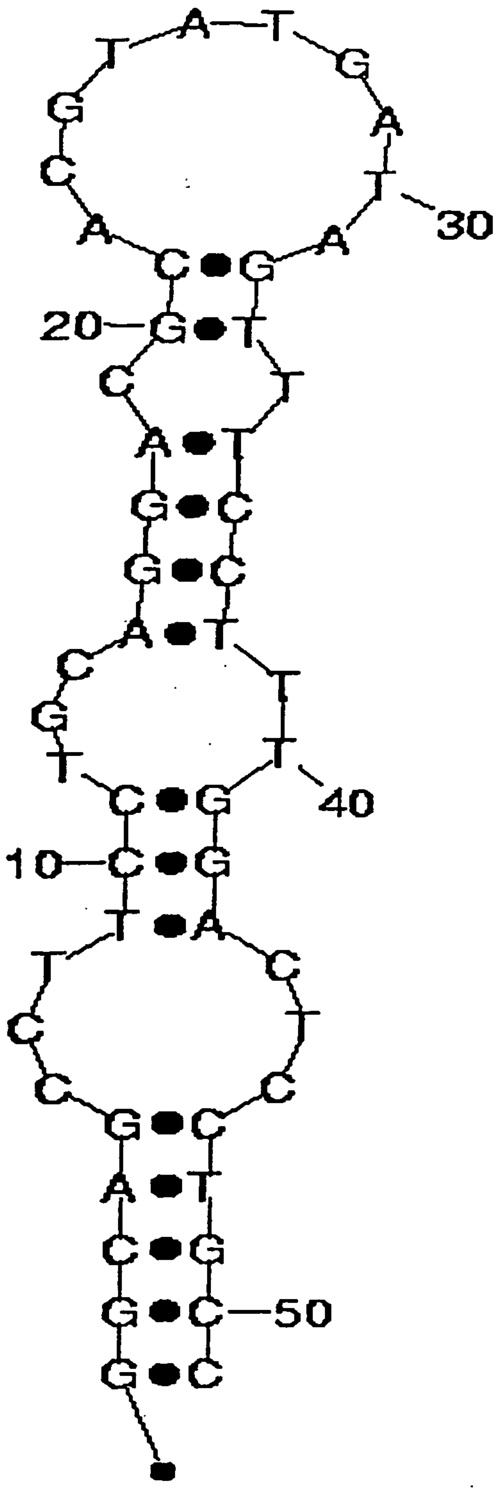


Fig. 8

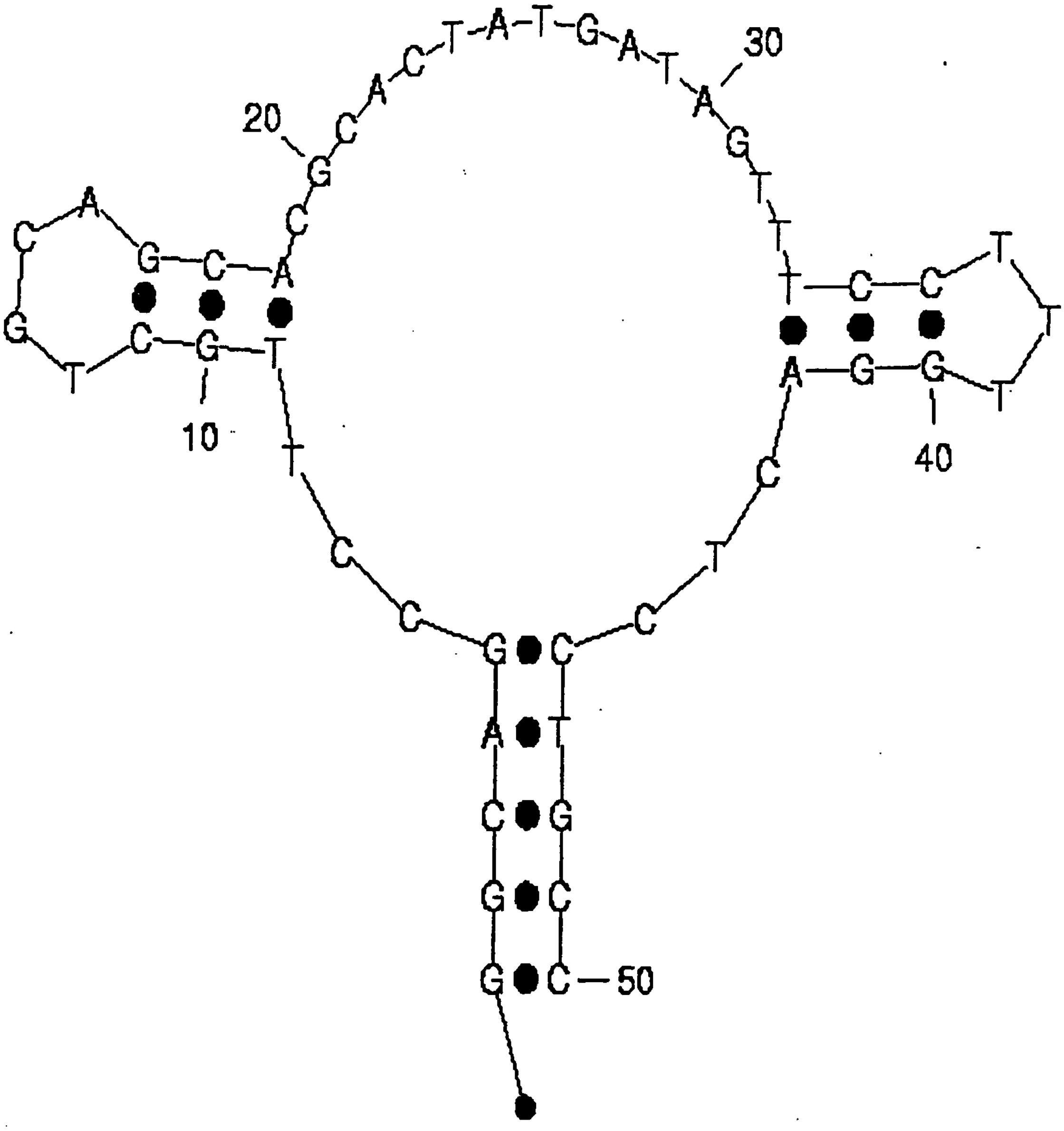


Fig. 9

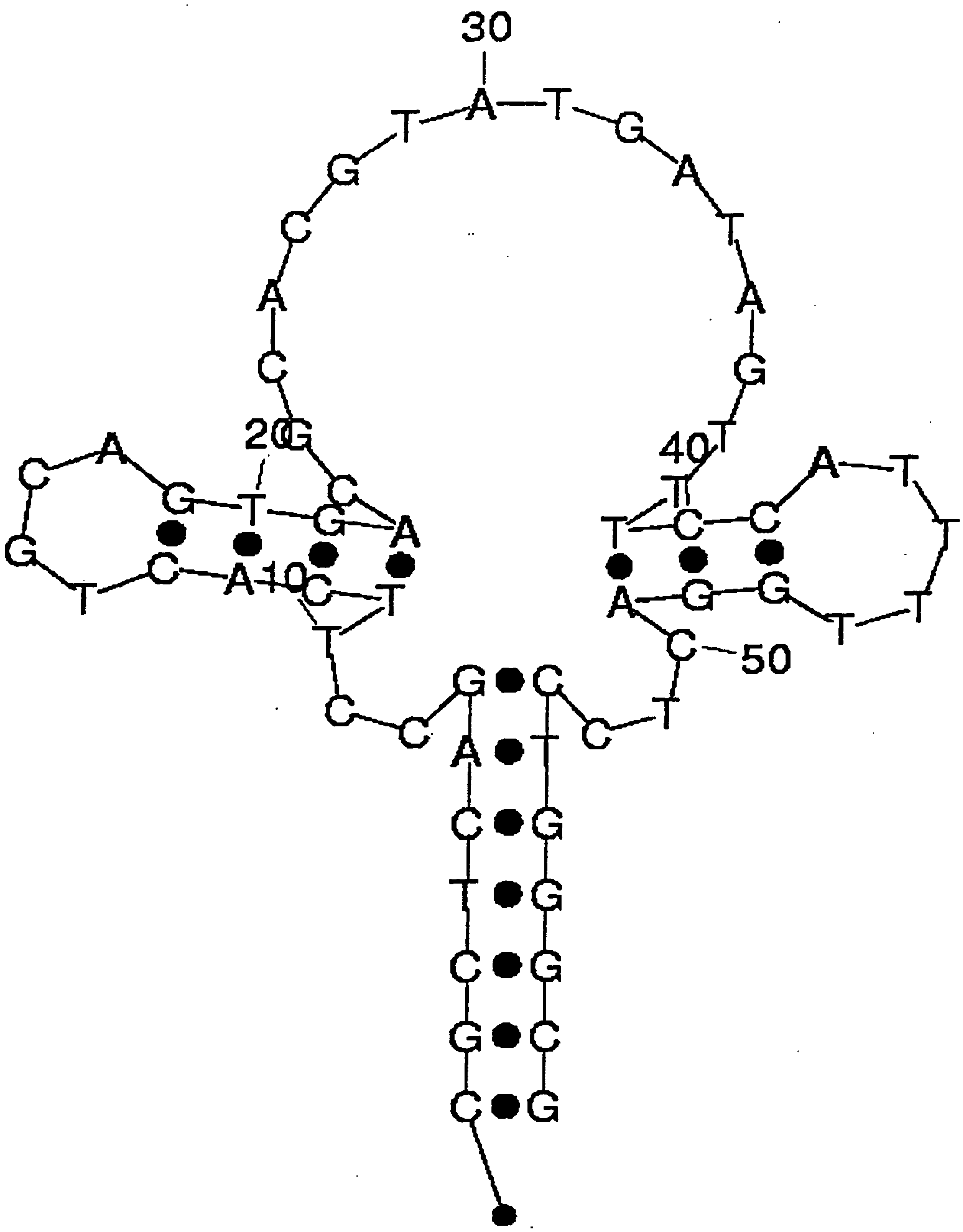


Fig. 10

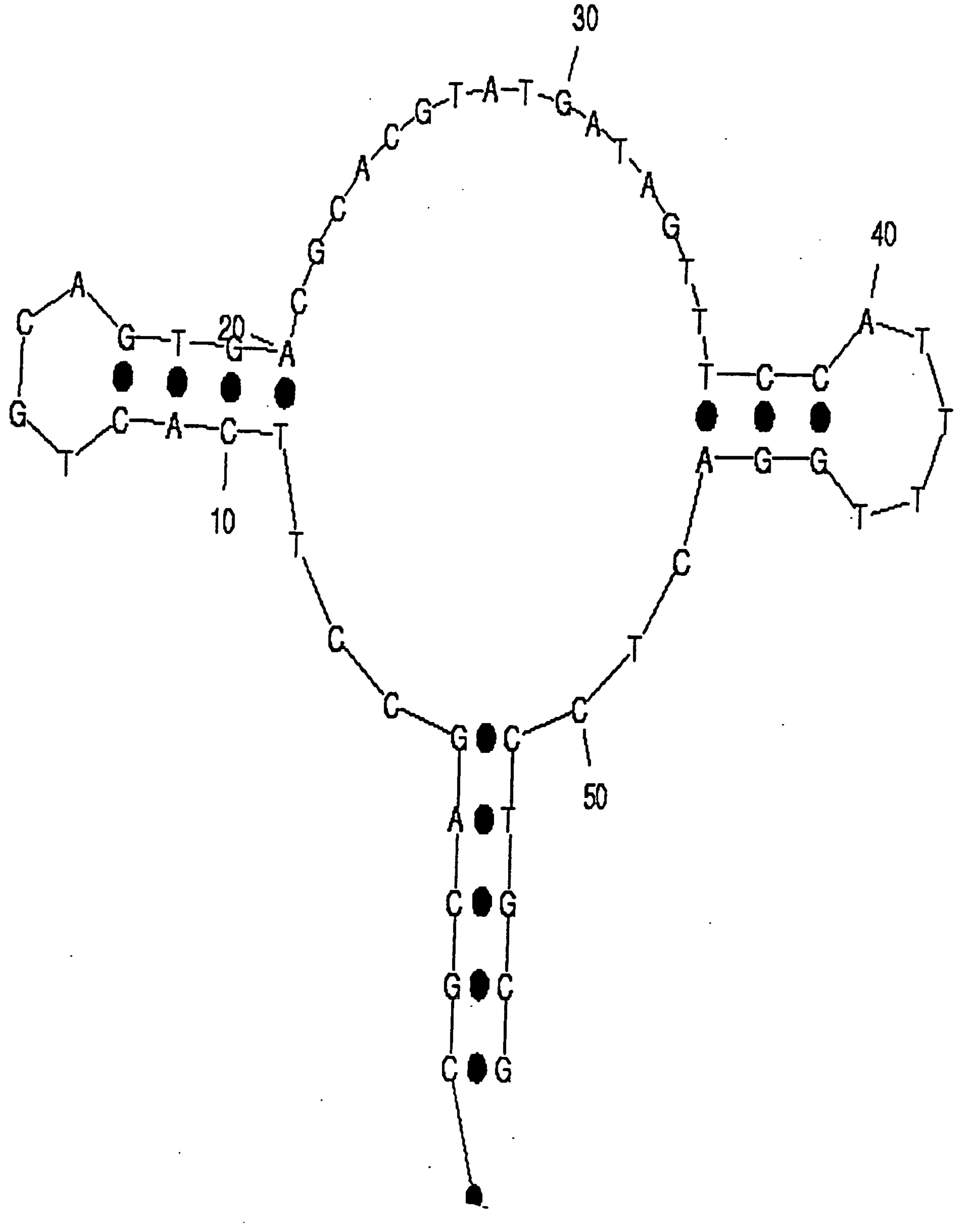


**Fig. 11**

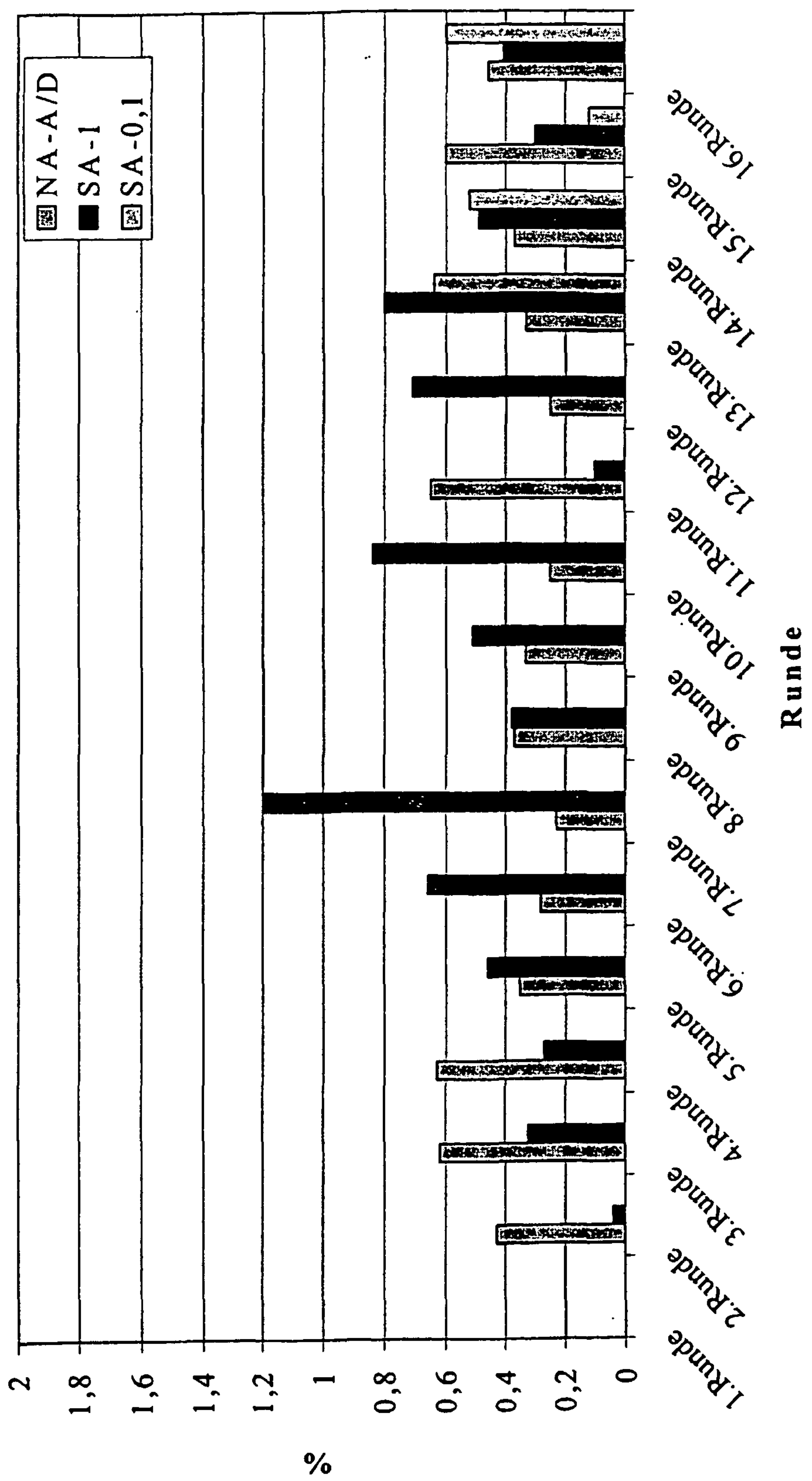
NA-A/D
SA-1
SA-0,1
2
1,8
1,6
1,4
1,2
1
0,8
0,6
0,4
0,2
0
%
1.Runde
2.Runde
3.Runde
4.Runde
5.Runde
6.Runde
7.Runde
8.Runde
9.Runde
10.Runde
11.Runde
12.Runde
13.Runde
14.Runde
15.Runde
16.Runde
Runde

Fig. 12

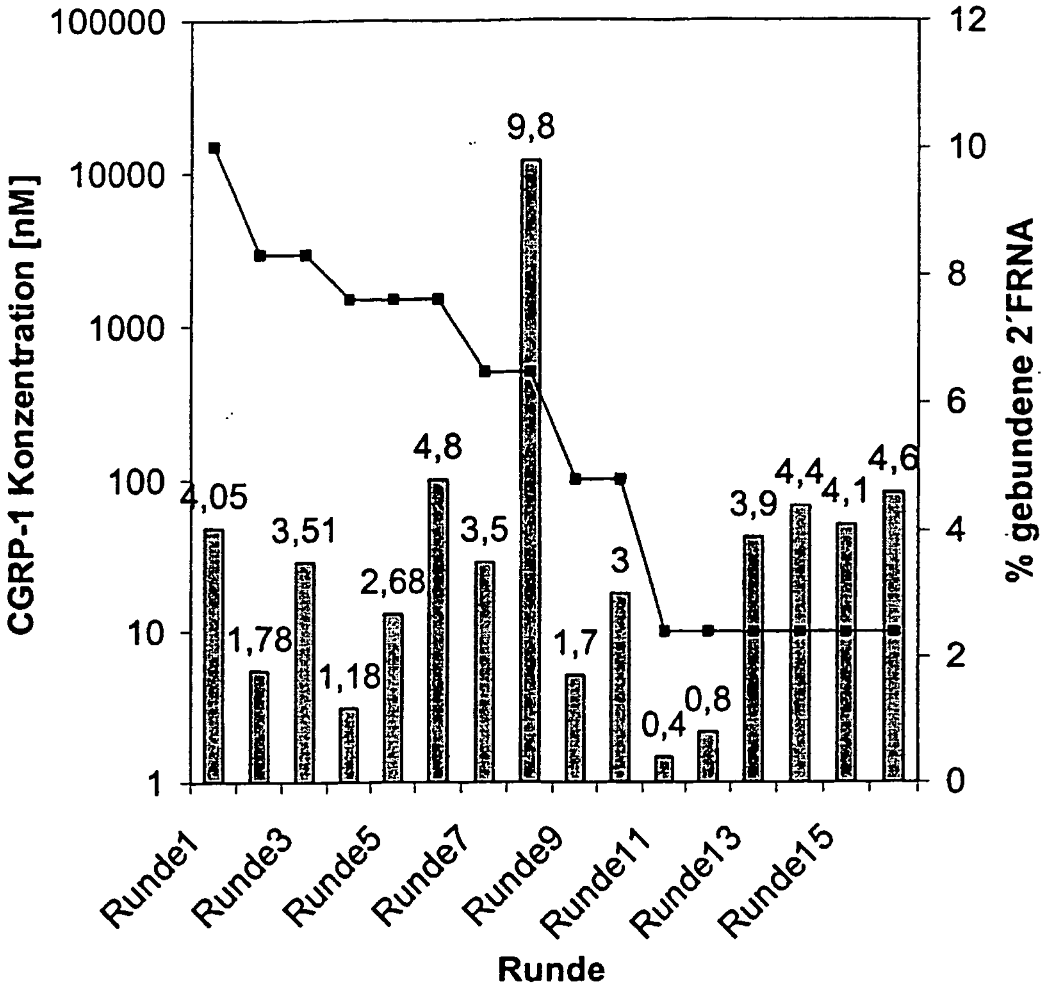
100000
10000
1000
100
10
1
12
10
8
6
4
2
0
CGRP-1 Konzentration [nM]
% gebundene 2´FRNA
4,05
1,78
3,51
1,18
2,68
4,8
3,5
9,8
1,7
3
0,4
0,8
3,9
4,4
4,1
4,6
Runde1
Runde3
Runde5
Runde7
Runde9
Runde11
Runde13
Runde15
Runde

**Fig. 13**

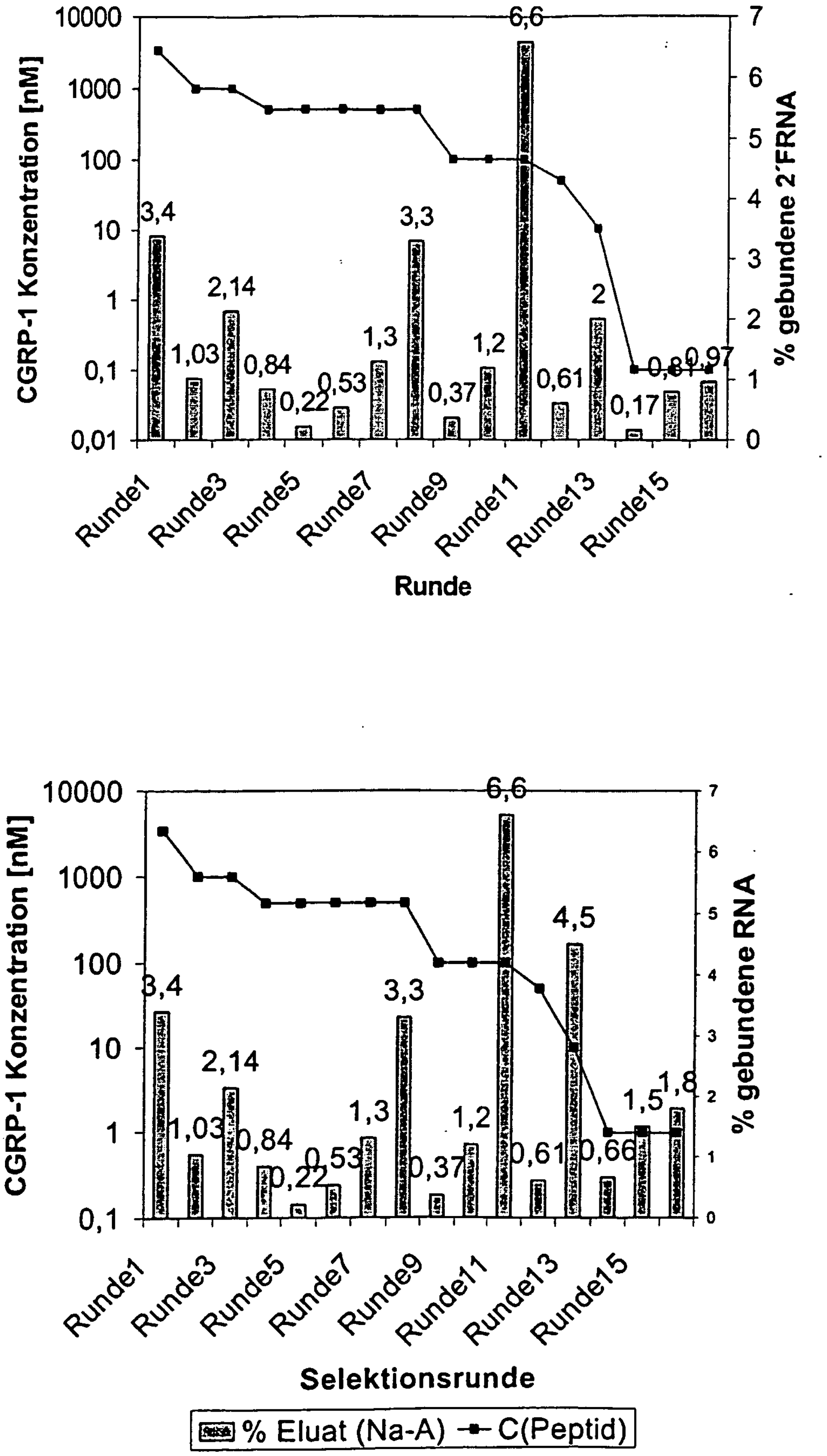

10000
1000
100
10
1
0,1
0,01
CGRP-1 Konzentration [nM]
% gebundene 2´FRNA
3,4
1,03
2,14
0,84
0,22
0,53
1,3
3,3
0,37
1,2
6,6
0,61
2
0,17
0,81
0,97
Runde1
Runde3
Runde5
Runde7
Runde9
Runde11
Runde13
Runde15
Runde
CGRP-1 Konzentration [nM]
% gebundene RNA
4,5
0,66
1,5
1,8
Selektionsrunde
% Eluat (Na-A)
C(Peptid)


Fig. 14

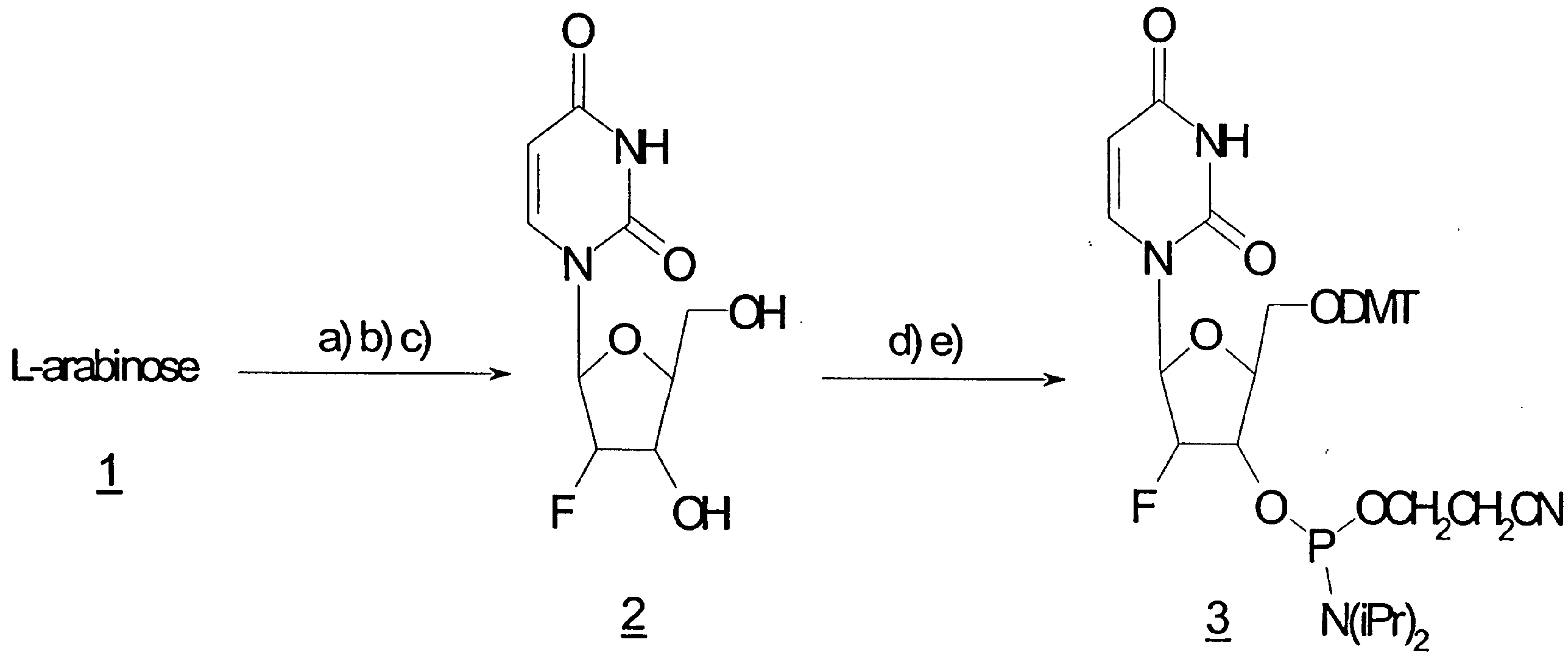


L-arabinose

1

2

3

Fig. 15

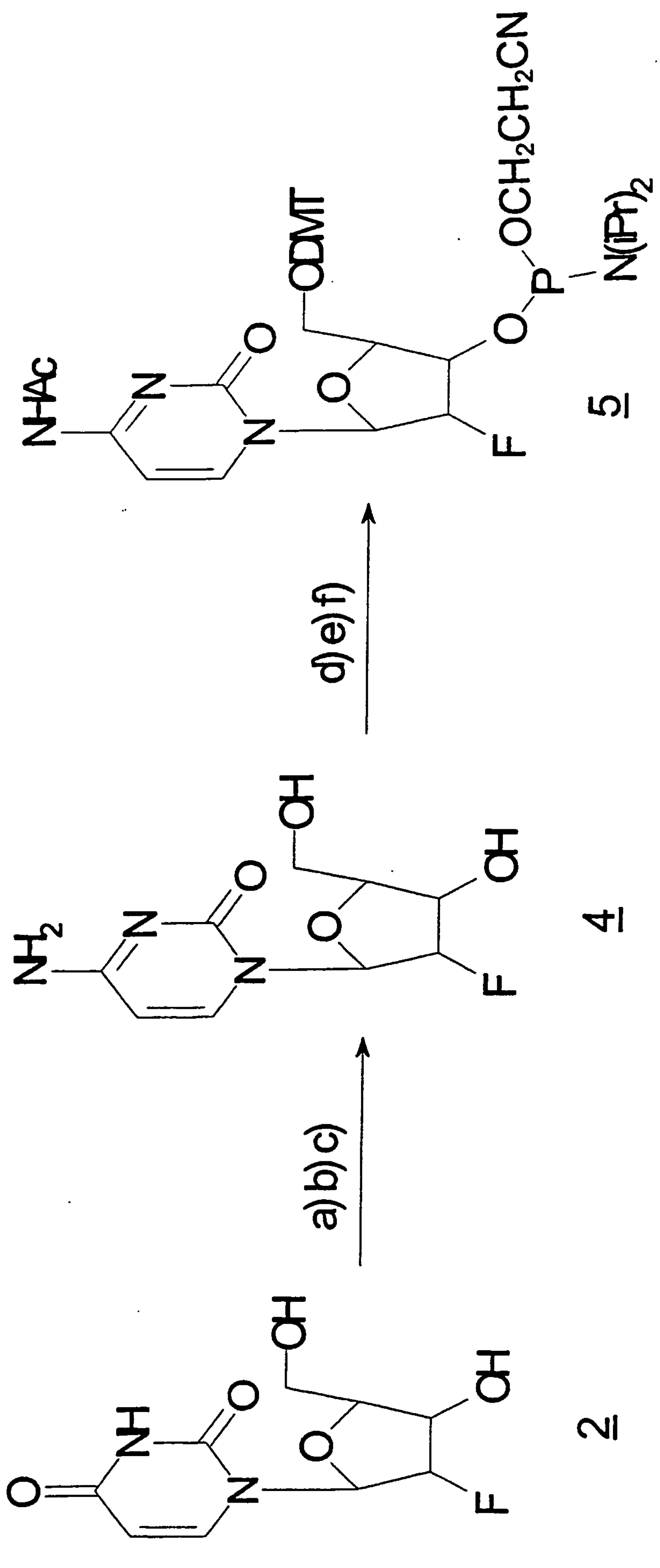


2

4

5

**Fig. 16**

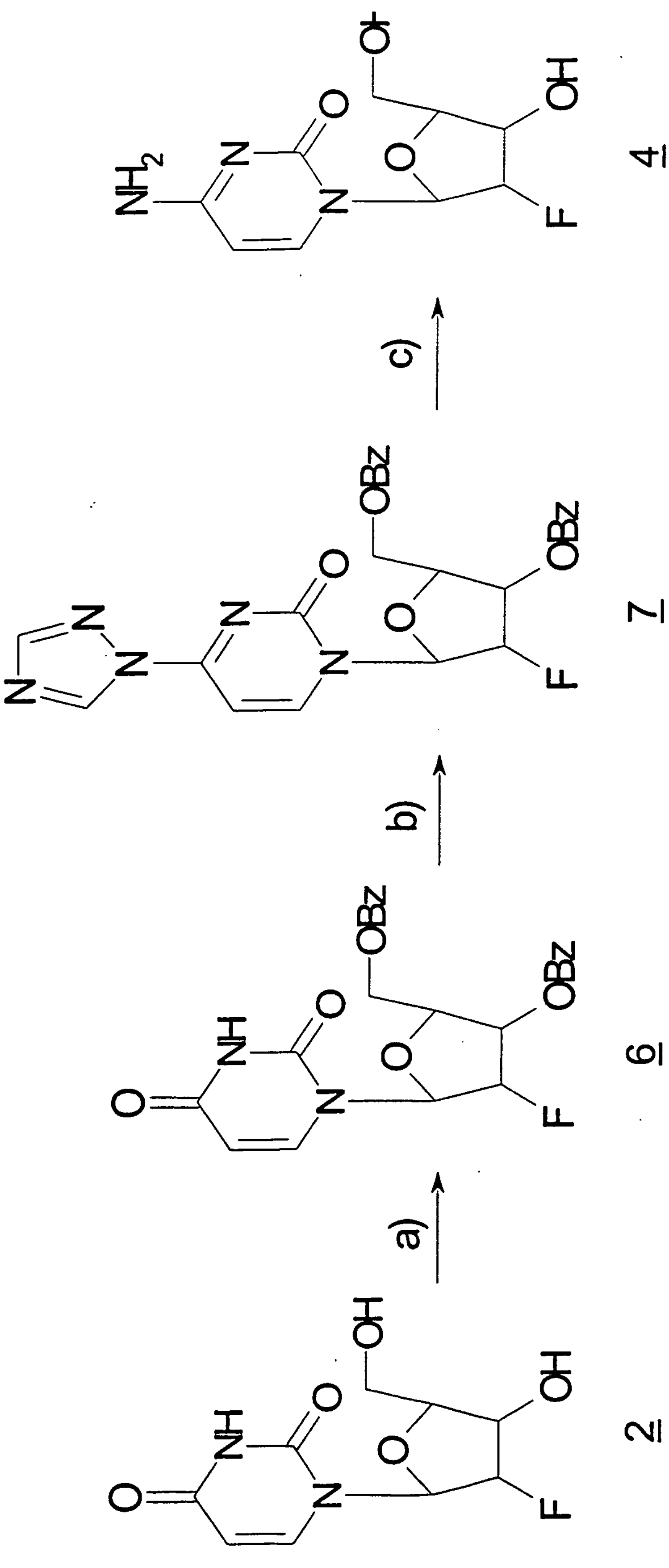


2 6 7 4

Fig. 17

## Sequenzen

```
666       GGAGCTCAGCCTTCACTGCGAAGTGACGCACGTATGATAGTTTCCATTTTGGACTCCTGGGCACCACGGTCGGATCCAC
732       GGAGCTCAGCCTTCACTGCAG--TGACGCACGTATGATAGTTTCCATTTTGGACTCCTGGGCACCACGGTCGGATCCAC
732_026   GG----CAGCCTTCACTGCAG--TGACGCACGTATGATAGTTTCCATTTTGGACTCCTG------------------CC
732_029   GG--CTCAGCCTTCACTGCAG--TGACGCACGTATGATAGTTTCCATTTTGGACTCCTGGGC----------------C
732_045   CG----CAGCCTTCACTGCAG--TGACGCACGTATGATAGTTTCCATTTTGGACTCCTG------------------CG
732_096   CG--CTCAGCCTTCACTGCAG--TGACGCACGTATGATAGTTTCCATTTTGGACTCCTGGGC----------------G
732_100   GG----CAGCCTTC-CTGCAG---GACGCACGTATGATAGTTTCC--TTTGGACTCCTG------------------CC
732_103   GG----CAGCCTTG-CTGCAG---CACGCACGTATGATAGTTTCC--TTTGGACTCCTG------------------CC
732_104   GG----CAGCCTTC-CTGCAG---GACGCACGTATGATAGTTTGC--TTTGCACTCCTG------------------CC
732_108   GG----CAGCCTTG-CTGCAG---CACGCAC-TATGATAGTTTCC--TTTGGACTCCTG------------------CC

732_070a GGCAGCCTTCACTG
732_070b CAGTGACGCACGTATGATAGTTTCCATTTTGGACTCCTGCC(OH)
732_071a GGCAGCCTTCACTGCAGTGACGCACGTATGATAGTTTCCAAG
732_071b CTTGGACTCCTGCC(OH)
```

Fig. 18

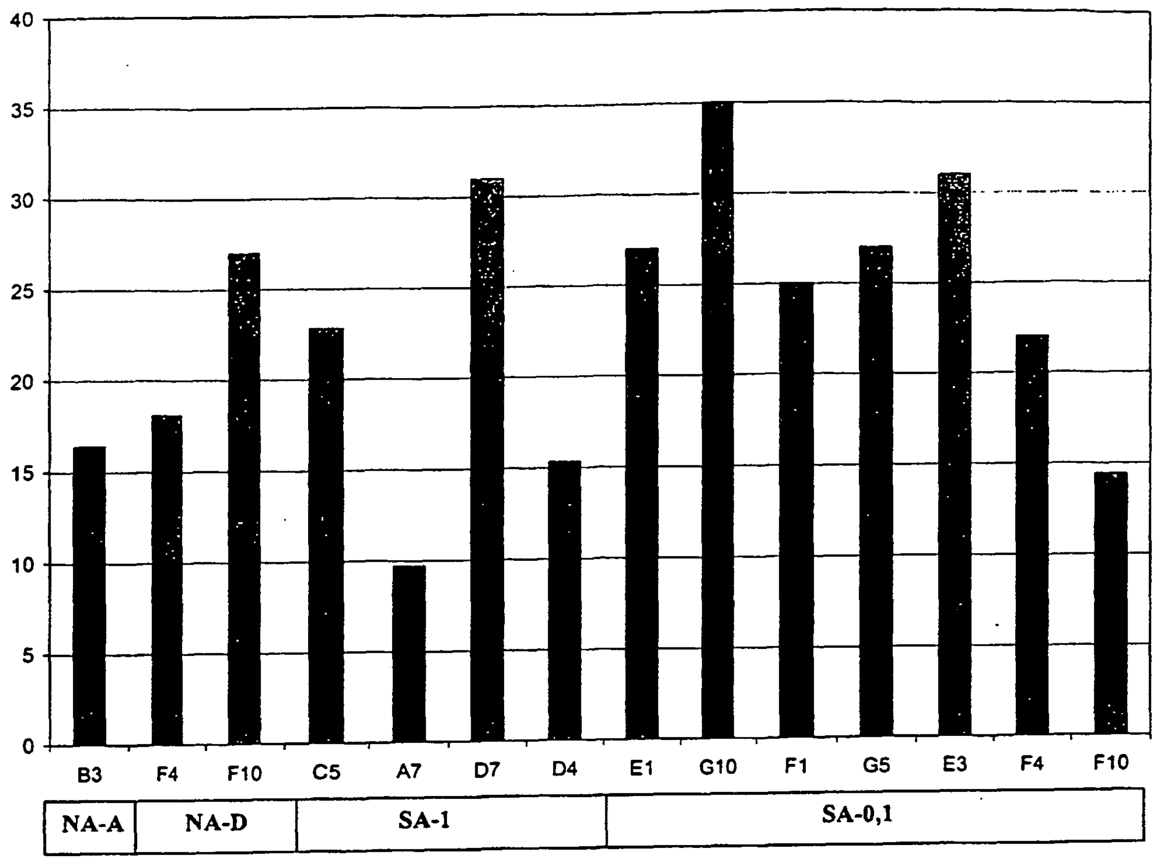
40
35
30
25
20
15
10
5
0
B3
F4
F10
C5
A7
D7
D4
E1
G10
F1
G5
E3
F4
F10
NA-A
NA-D
SA-1
SA-0,1

Fig. 19

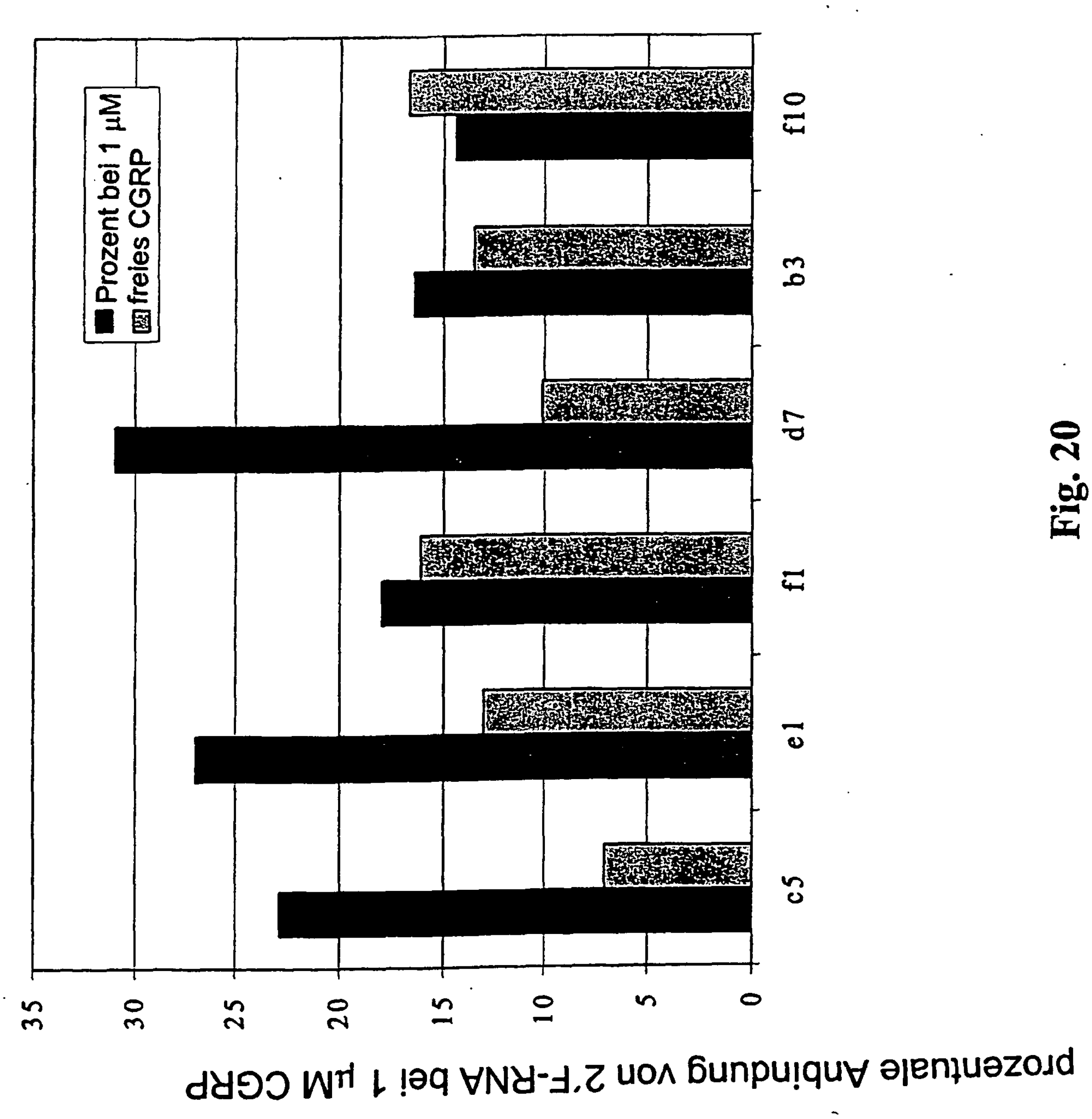
Prozent bei 1 µM
freies CGRP
35
30
25
20
15
10
5
0
c5
e1
f1
d7
b3
f10
prozentuale Anbindung von 2'F-RNA bei 1 µM CGRP

Fig. 20

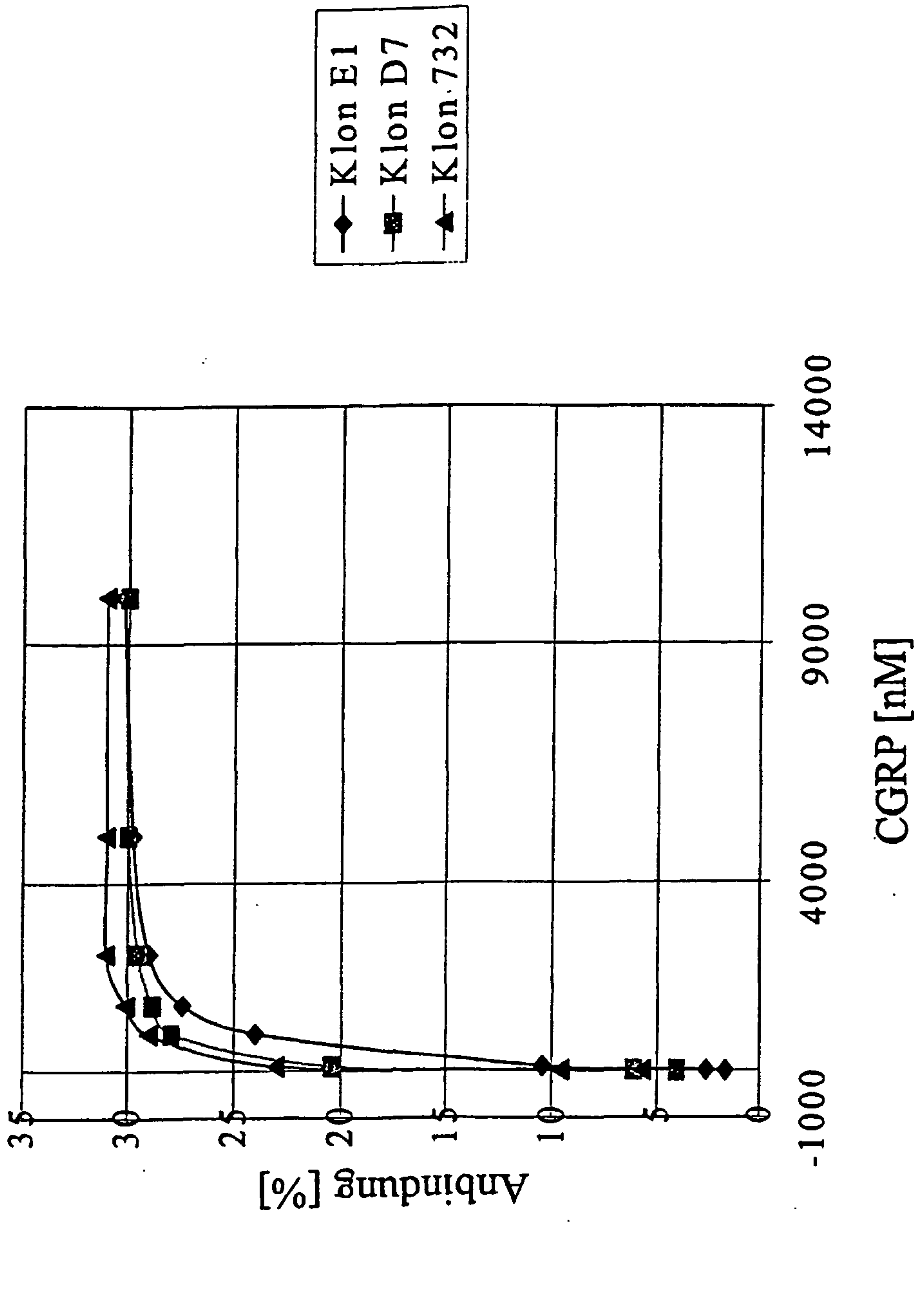
Anbindung [%]
35
30
25
20
15
10
5
0
-1000
4000
9000
14000
CGRP [nM]
Klon E1
Klon D7
Klon 732

Fig. 21

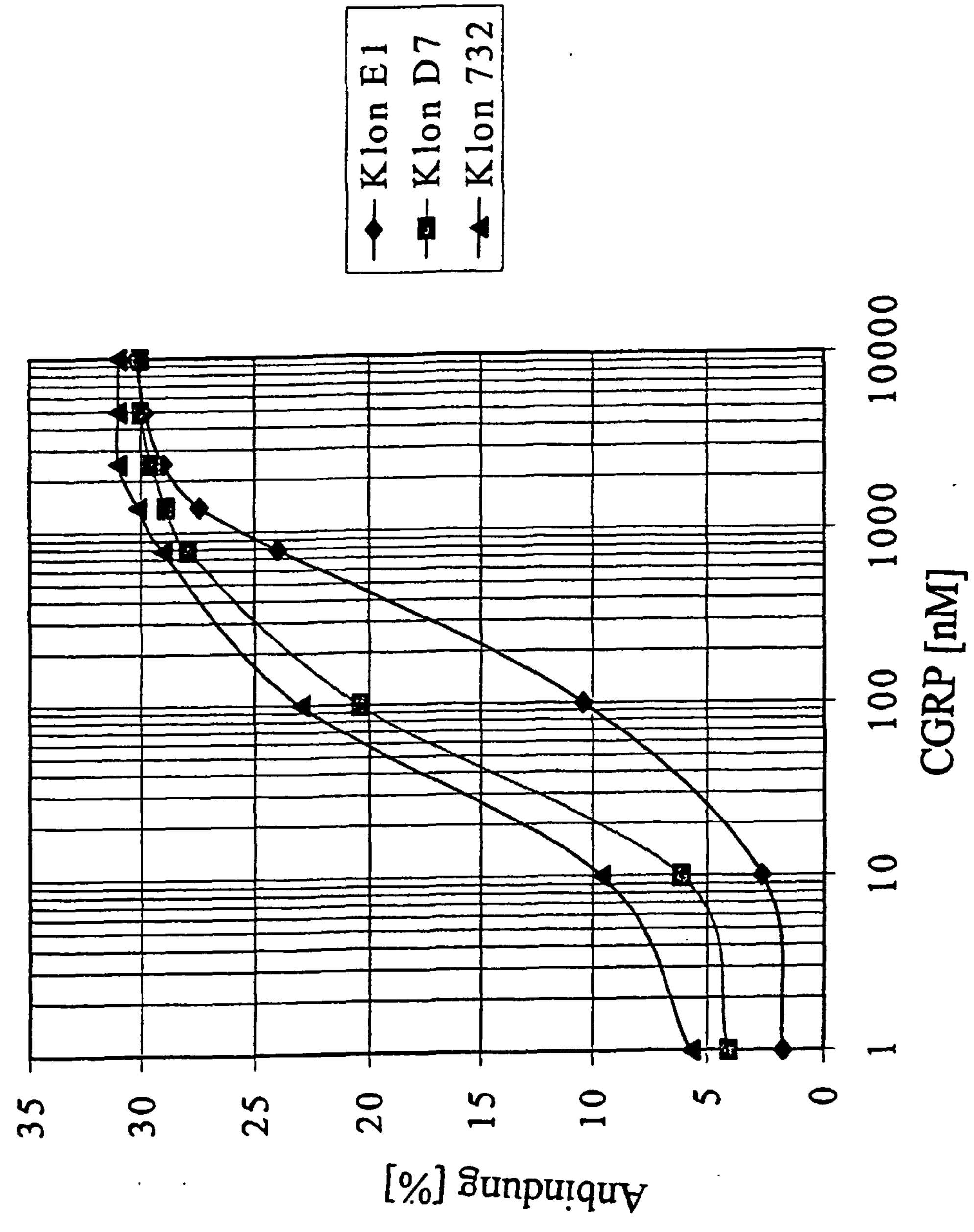
Klon E1
Klon D7
Klon 732
Anbindung [%]
0
5
10
15
20
25
30
35
1
10
100
1000
10000
CGRP [nM]

Fig. 22

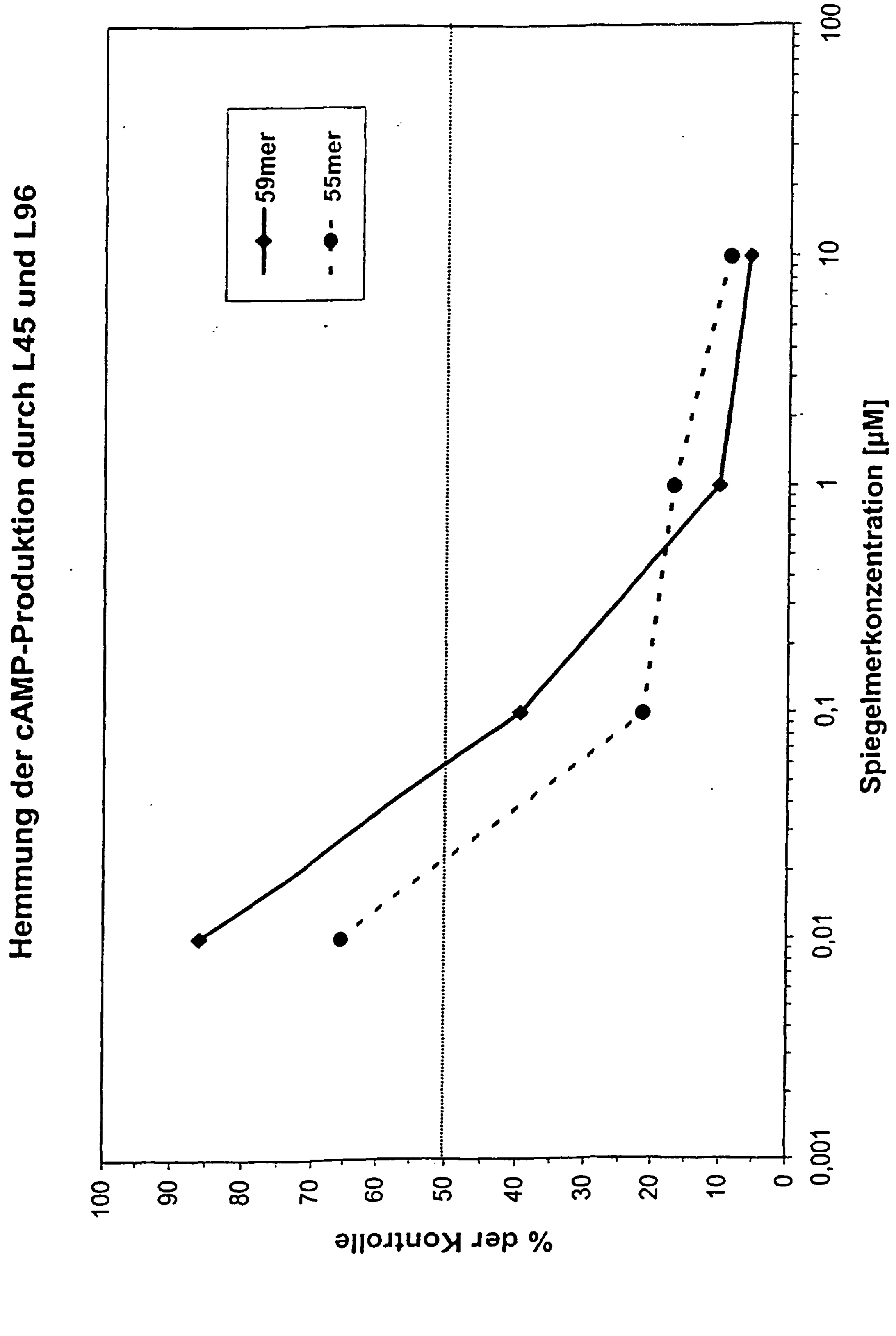
Hemmung der cAMP-Produktion durch L45 und L96
59mer
55mer
% der Kontrolle
100
90
80
70
60
50
40
30
20
10
0
0,001
0,01
0,1
1
10
100
Spiegelmerkonzentration [µM]

Fig. 23

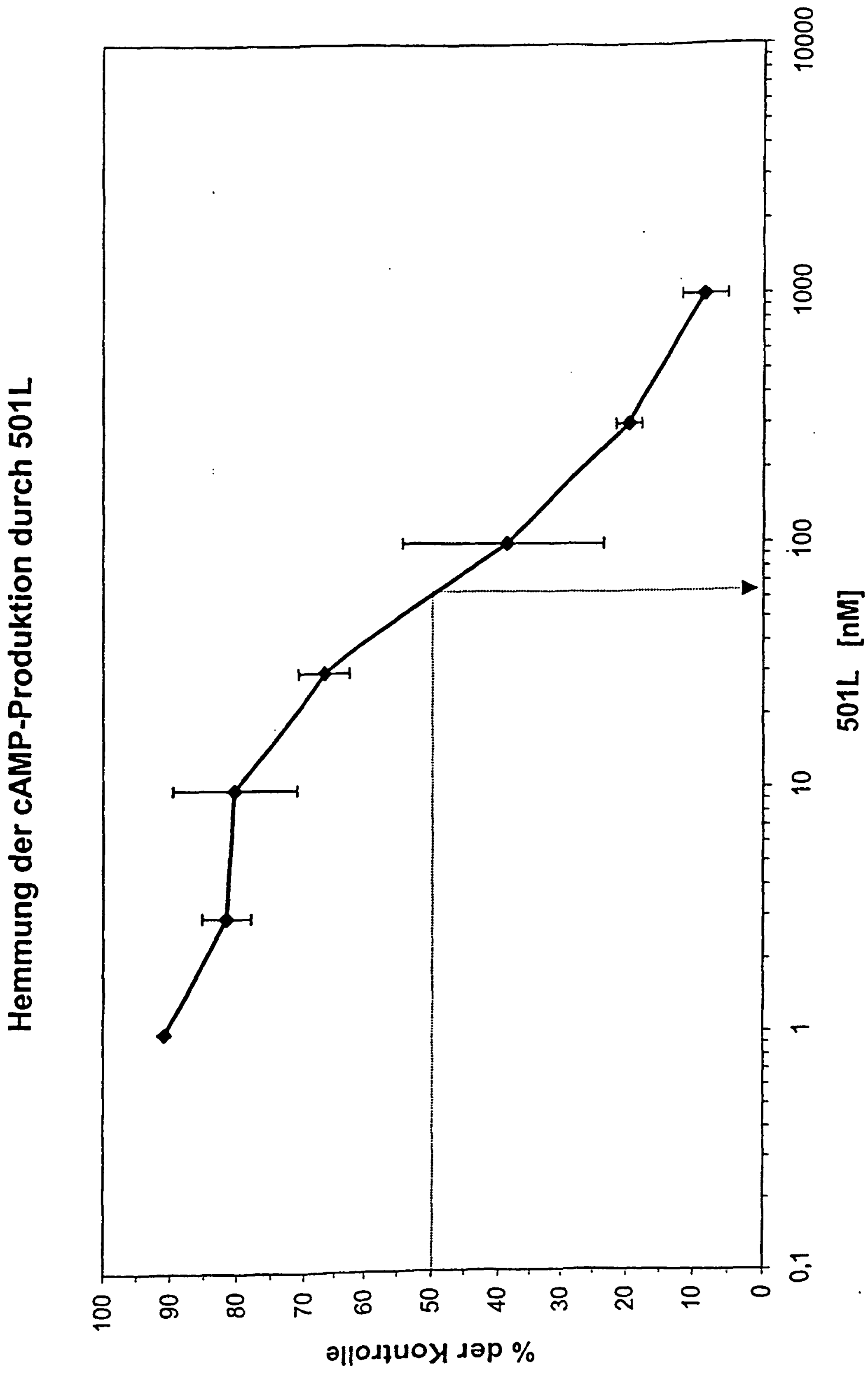
Hemmung der cAMP-Produktion durch 501L
% der Kontrolle
100
90
80
70
60
50
40
30
20
10
0
0,1
1
10
100
1000
10000
501L [nM]

Fig. 24

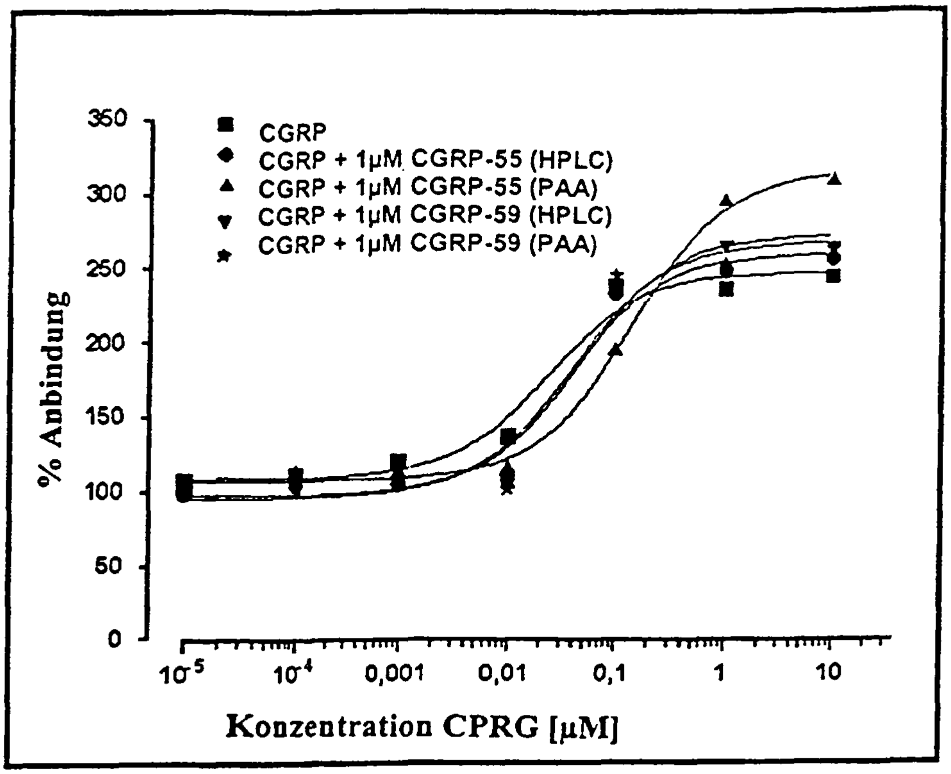
CGRP
CGRP + 1µM CGRP-55 (HPLC)
CGRP + 1µM CGRP-55 (PAA)
CGRP + 1µM CGRP-59 (HPLC)
CGRP + 1µM CGRP-59 (PAA)
% Anbindung
350
300
250
200
150
100
50
0
10⁻⁵
10⁻⁴
0,001
0,01
0,1
1
10
Konzentration CPRG [µM]

Fig. 25

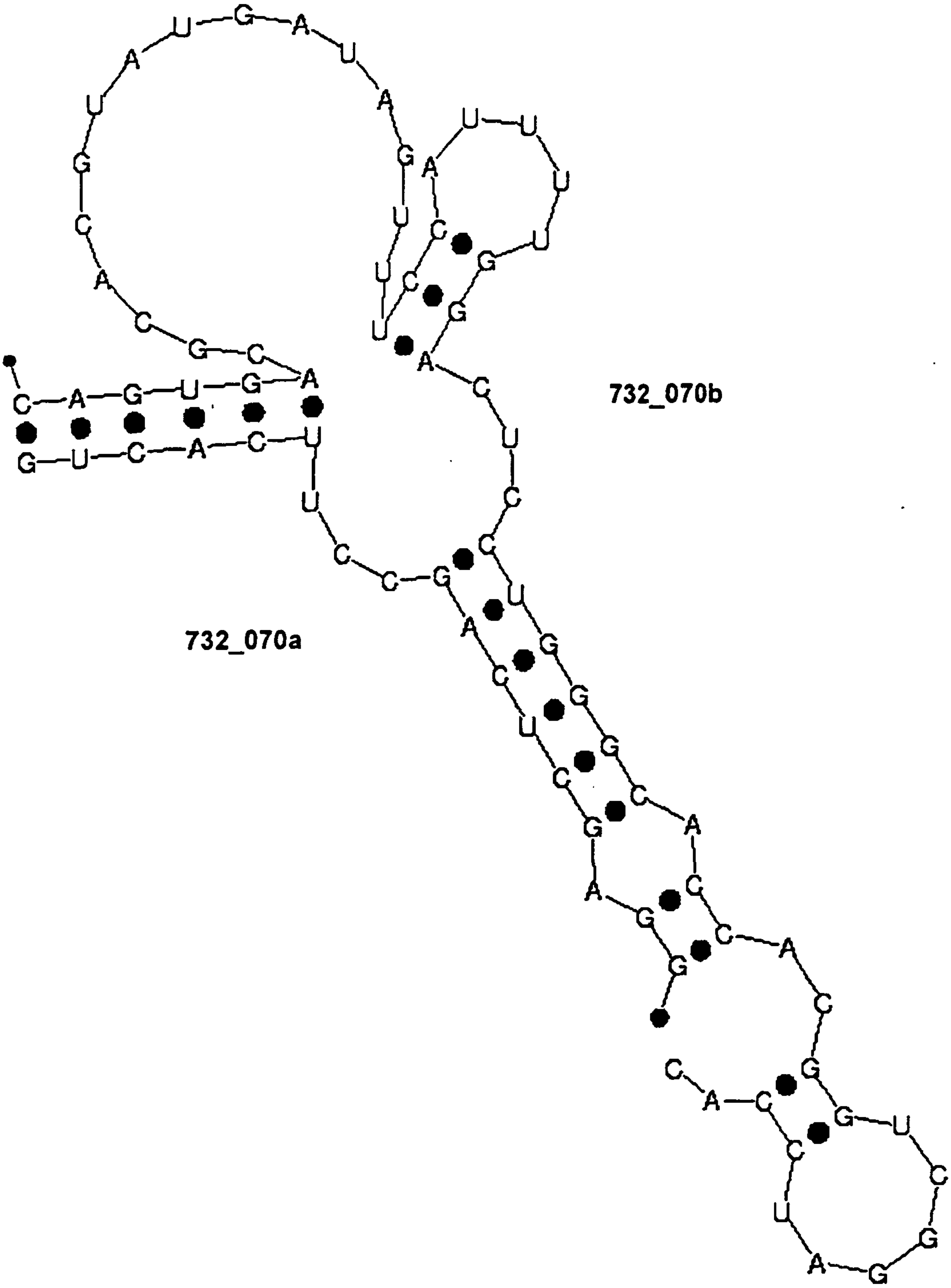


Fig. 26

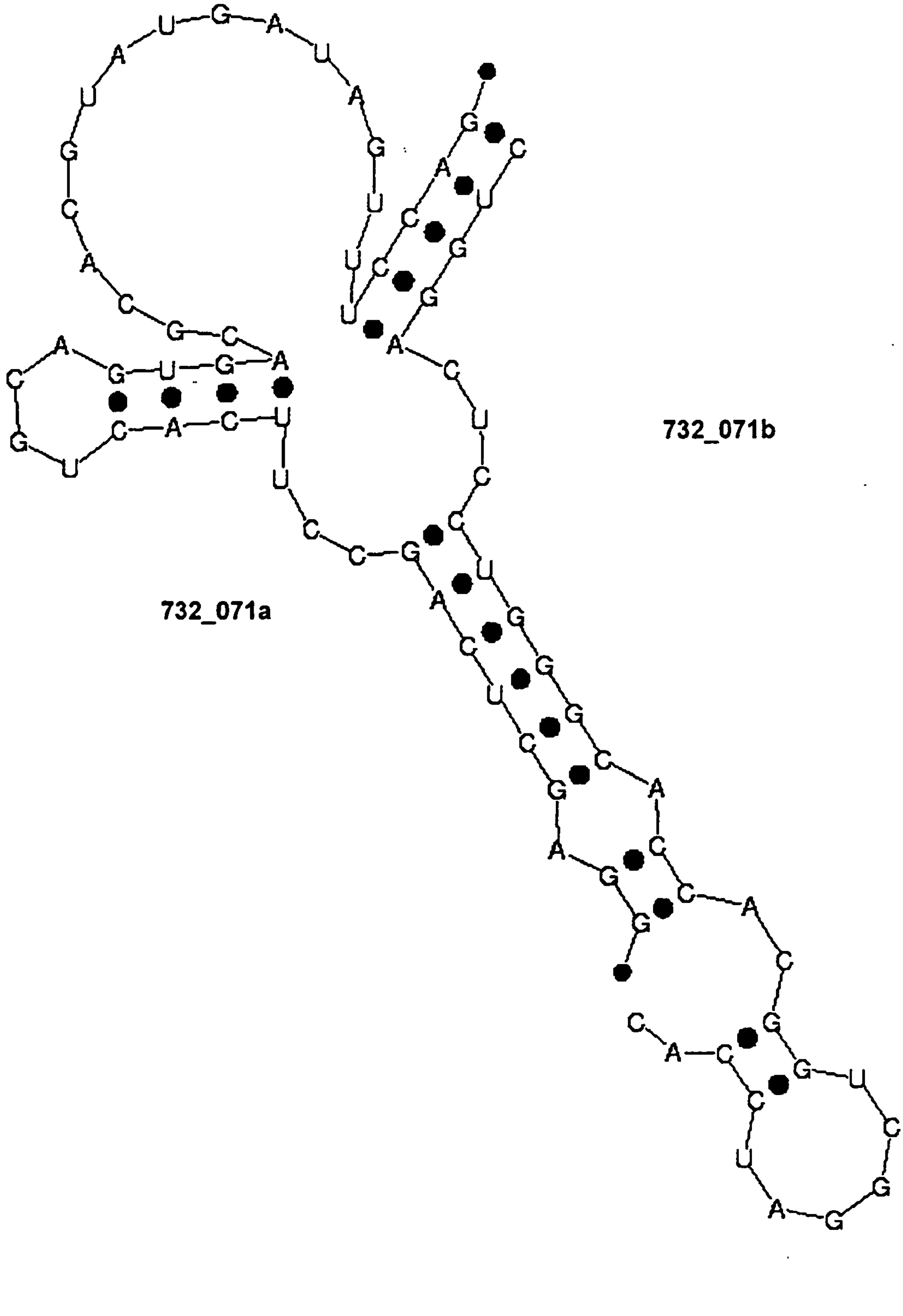


Fig. 27

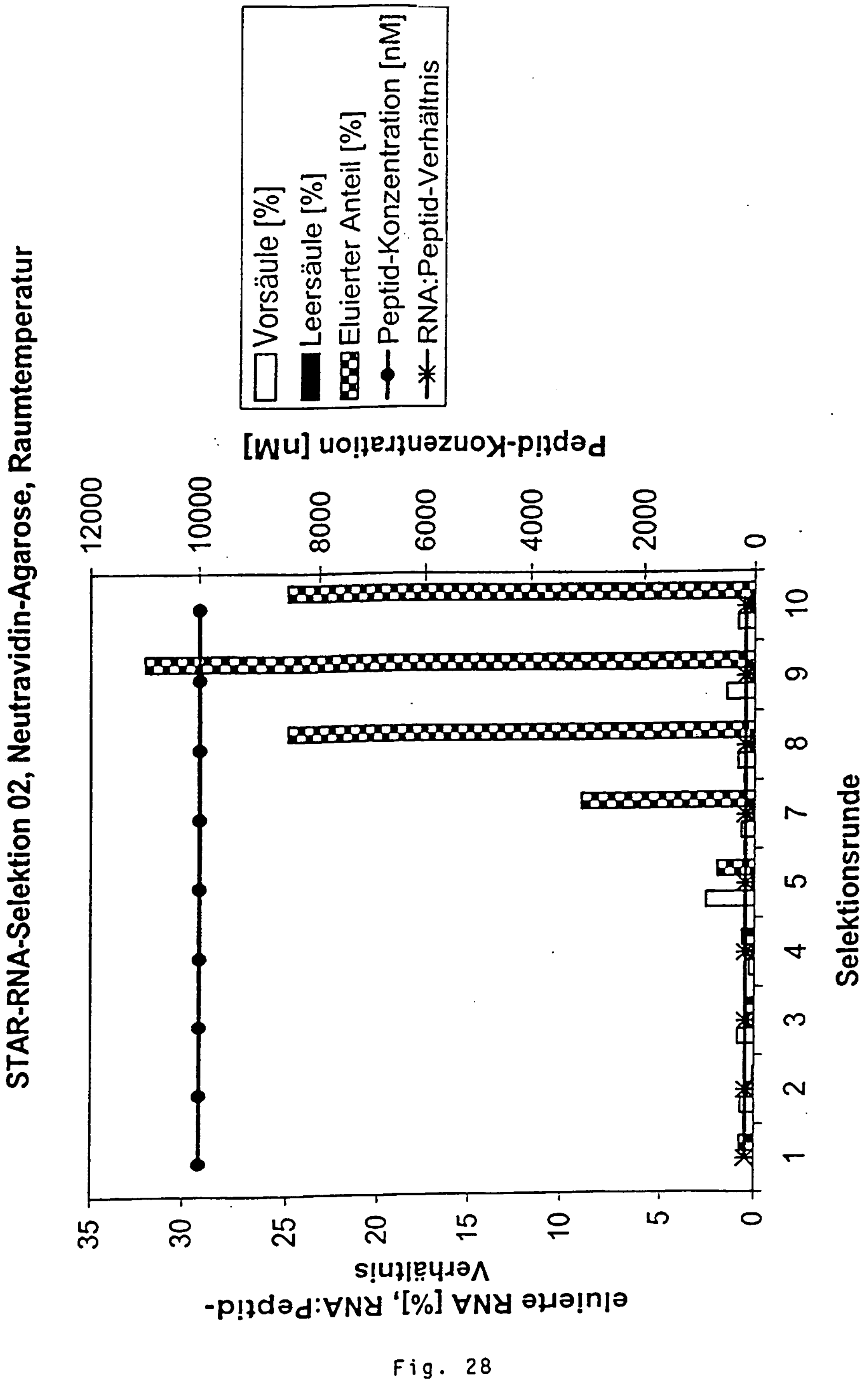
STAR-RNA-Selektion 02, Neutravidin-Agarose, Raumtemperatur
Vorsäule [%]
Leersäule [%]
Eluierter Anteil [%]
Peptid-Konzentration [nM]
RNA:Peptid-Verhältnis
Peptid-Konzentration [nM]
12000
10000
8000
6000
4000
2000
0
eluierte RNA [%], RNA:Peptid-Verhältnis
35
30
25
20
15
10
5
0
1
2
3
4
5
7
8
9
10
Selektionsrunde

Fig. 28

STAR-RNA Selektion 02, Neutravidin-Agarose, Raumtemperatur

| Selektionsrunde | Vorsäule [%] | Leersäule [%] | Eluierter Anteil [%] | RNA:Peptid-Verhältnis | Peptid-Konzentration [nM] |
|---|---|---|---|---|---|
| 1 | | | 0,8 | 0,5 | 10000 |
| 2 | 0,74 | 0,34 | 0,13 | 0,5 | 10000 |
| 3 | 0,92 | 0,33 | 0,45 | 0,5 | 10000 |
| 4 | 0,31 | 0,45 | 0,69 | 0,5 | 10000 |
| 5 | 2,59 | 0,15 | 2 | 0,5 | 10000 |
| 7 | 0,72 | 0,48 | 9 | 0,5 | 10000 |
| 8 | 0,9 | 0,27 | 25 | 0,5 | 10000 |
| 9 | 1,5 | 0,5 | 32 | 0,5 | 10000 |
| 10 | 0,9 | 0,6 | 25 | 0,5 | 10000 |

Fig. 29

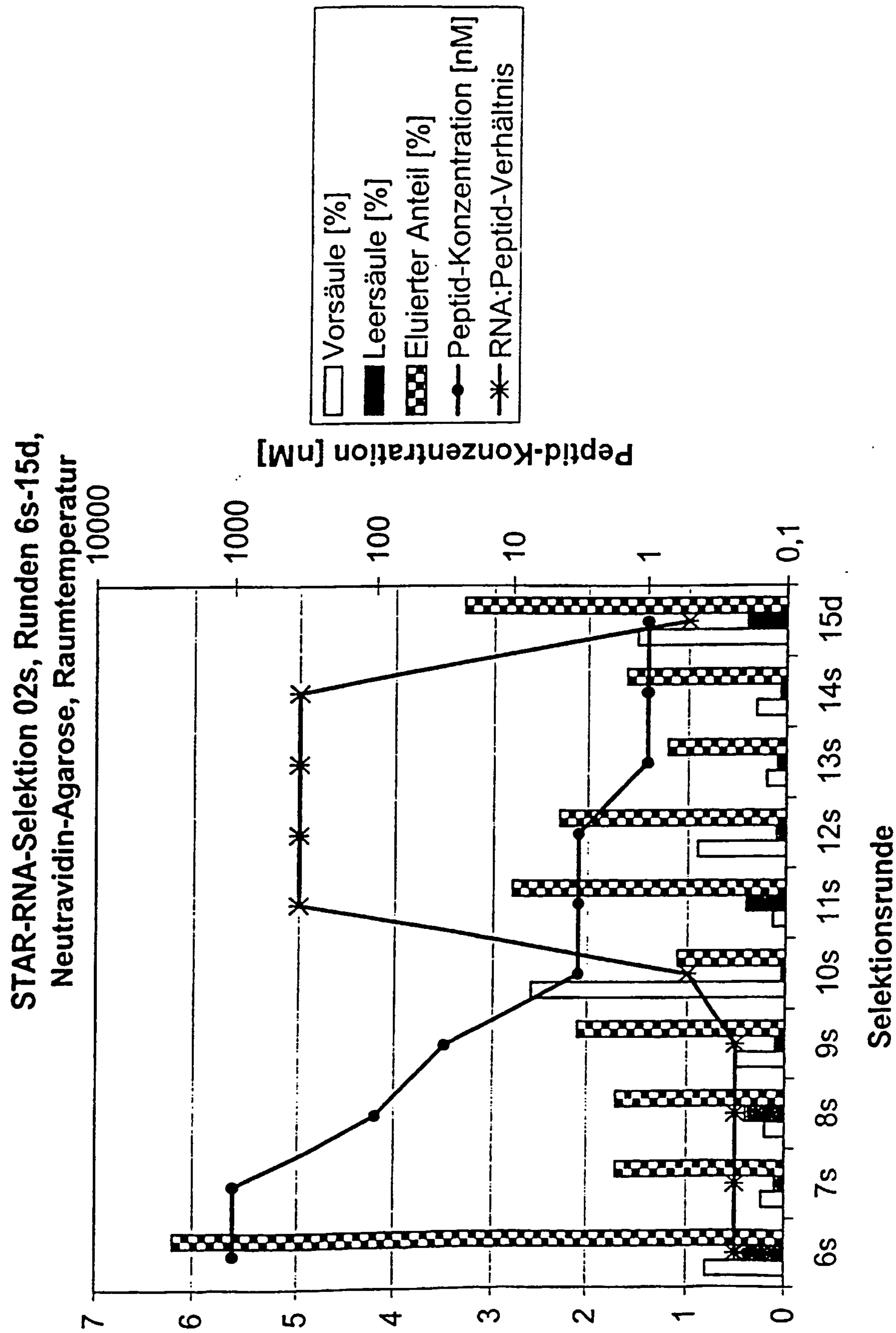
STAR-RNA-Selektion 02s, Runden 6s-15d,
Neutravidin-Agarose, Raumtemperatur
Vorsäule [%]
Leersäule [%]
Eluierter Anteil [%]
Peptid-Konzentration [nM]
RNA:Peptid-Verhältnis
Peptid-Konzentration [nM]
10000
1000
100
10
1
0,1
eluierte RNA [%], RNA:Peptid-Verhältnis
7
6
5
4
3
2
1
0
6s
7s
8s
9s
10s
11s
12s
13s
14s
15d
Selektionsrunde

Fig. 30

STAR-RNA Selektion 02s Runden 6s-15d, Neutravidin-Agarose, Raumtemperatur

| Selektionsrunde | Vorsäule [%] | Leersäule [%] | Eluierter Anteil [%] | RNA:Peptid-Verhältnis | Peptid-Konzentration [nM] |
|---|---|---|---|---|---|
| 6s | 0,8 | 0,5 | 6,2 | 0,5 | 1000 |
| 7s | 0,23 | 0,1 | 1,7 | 0,5 | 1000 |
| 8s | 0,2 | 0,4 | 1,7 | 0,5 | 100 |
| 9s | 0,5 | 0,1 | 2,1 | 0,5 | 31,6 |
| 10s | 2,6 | 0,04 | 1,1 | 1 | 3,16 |
| 11s | 0,13 | 0,4 | 2,8 | 5 | 3,16 |
| 12s | 0,9 | 0,1 | 2,3 | 5 | 3,16 |
| 13s | 0,2 | 0,09 | 1,2 | 5 | 1 |
| 14s | 0,3 | 0,06 | 1,6 | 5 | 1 |
| 15d | 1,5 | 0,4 | 3,3 | 1 | 1 |

Fig. 31

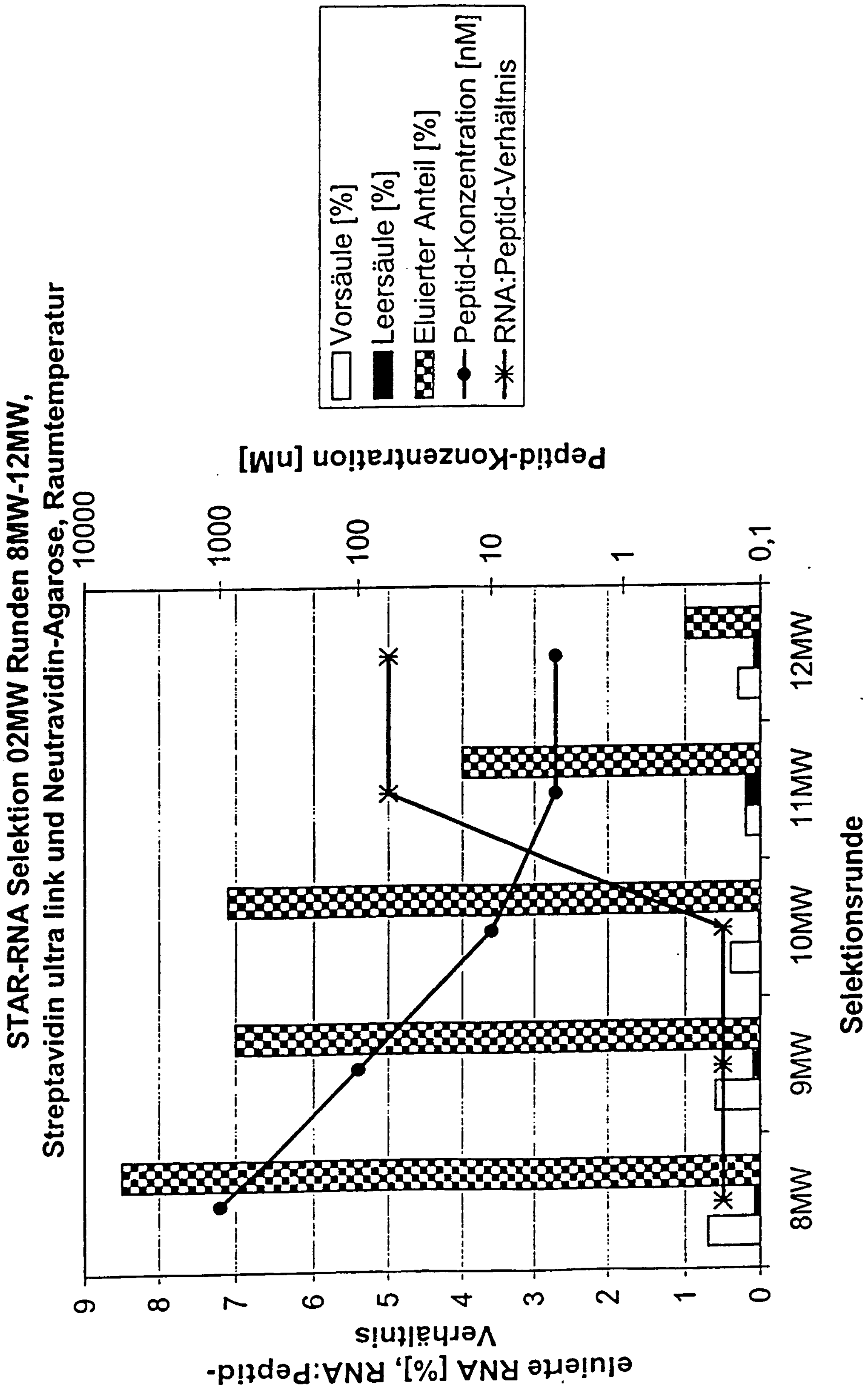
STAR-RNA Selektion 02MW Runden 8MW-12MW,
Streptavidin ultra link und Neutravidin-Agarose, Raumtemperatur
Vorsäule [%]
Leersäule [%]
Eluierter Anteil [%]
Peptid-Konzentration [nM]
RNA:Peptid-Verhältnis
eluierte RNA [%], RNA:Peptid-Verhältnis
Peptid-Konzentration [nM]
Selektionsrunde
0
1
2
3
4
5
6
7
8
9
0,1
1
10
100
1000
10000
8MW
9MW
10MW
11MW
12MW

Fig. 32

STAR-RNA Selektion 02MW Runden 8MW-12MW, Streptavidin ultra link und Neutravidin-Agarose

| Selektions runde | Vorsäule [%] | Leersäule [%] | Eluierter Anteil [%] | RNA:Peptid-Verhältnis | Peptid-Konzentration [nM] |
|---|---|---|---|---|---|
| 8MW | 0,7 | 0,08 | 8,5 | 0,5 | 1000 |
| 9MW | 0,6 | 0,1 | 7 | 0,5 | 100 |
| 10MW | 0,4 | 0,02 | 7,1 | 0,5 | 10 |
| 11MW | 0,2 | 0,2 | 4 | 5 | 3,16 |
| 12MW | 0,3 | 0,09 | 1 | 5 | 3,16 |

Fig. 33

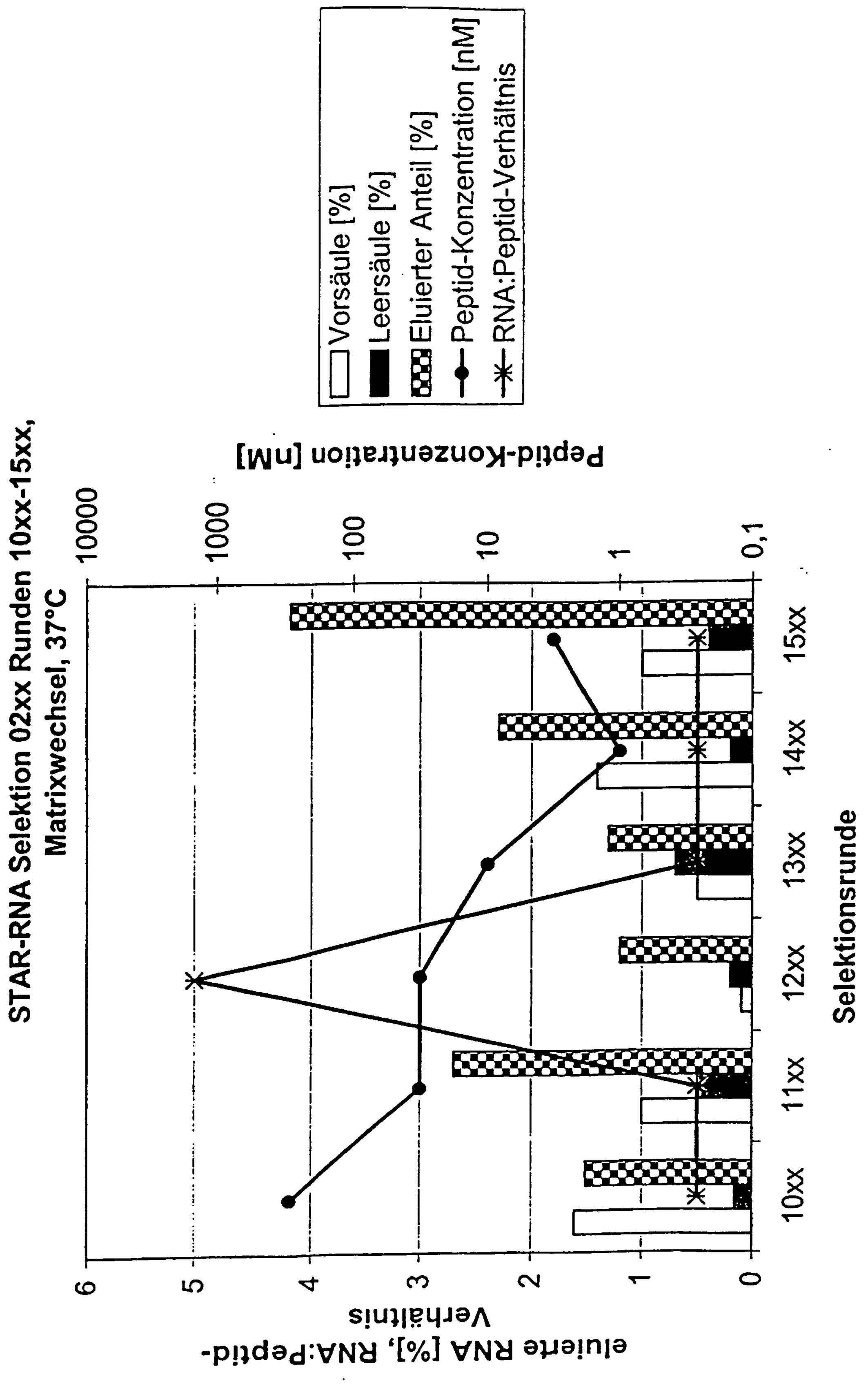
STAR-RNA Selektion 02xx Runden 10xx-15xx, Matrixwechsel, 37°C
Vorsäule [%]
Leersäule [%]
Eluierter Anteil [%]
Peptid-Konzentration [nM]
RNA:Peptid-Verhältnis
Peptid-Konzentration [nM]
10000
1000
100
10
1
0,1
eluierte RNA [%], RNA:Peptid-Verhältnis
6
5
4
3
2
1
0
10xx
11xx
12xx
13xx
14xx
15xx
Selektionsrunde

Fig. 34

STAR-RNA Selektion 02xx Runden 10xx-15xx, Matrixwechsel, 37°C

| Selektionsrunde | Vorsäule [%] | Leersäule [%] | Eluierter Anteil [%] | RNA:Peptid-Verhältnis | Peptid-Konzentration [nM] |
|---|---|---|---|---|---|
| 10xx | 1,6 | 0,16 | 1,5 | 0,5 | 316 |
| 11xx | 1 | 0,5 | 2,7 | 0,5 | 31,6 |
| 12xx | 0,1 | 0,2 | 1,2 | 5 | 31,6 |
| 13xx | 0,5 | 0,7 | 1,3 | 0,5 | 10 |
| 14xx | 1,4 | 0,2 | 2,3 | 0,5 | 1 |
| 15xx | 1 | 0,4 | 4,2 | 0,5 | 3,16 |

Fig. 35

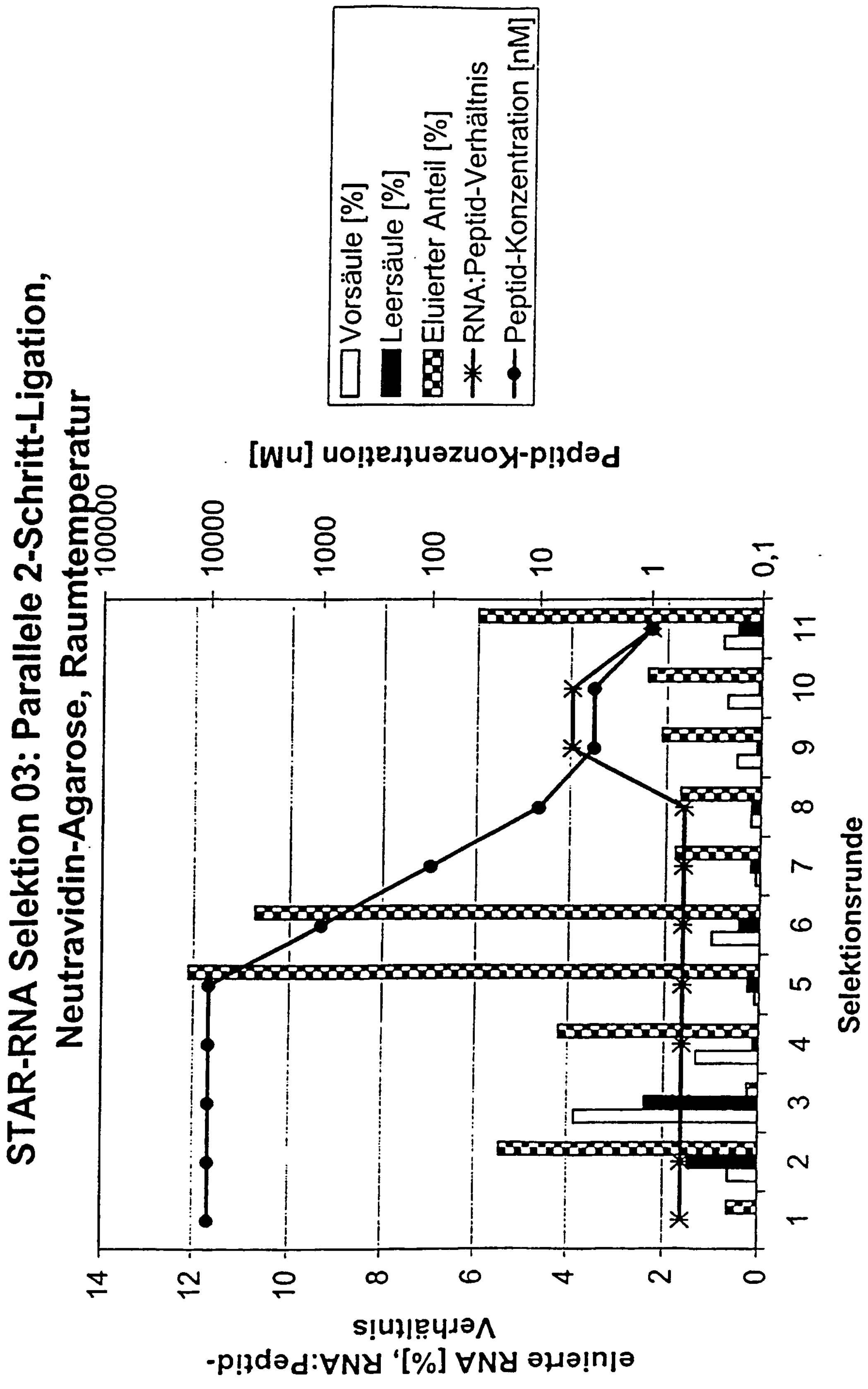
STAR-RNA Selektion 03: Parallele 2-Schritt-Ligation, Neutravidin-Agarose, Raumtemperatur
Vorsäule [%]
Leersäule [%]
Eluierter Anteil [%]
RNA:Peptid-Verhältnis
Peptid-Konzentration [nM]
Peptid-Konzentration [nM]
100000
10000
1000
100
10
1
0,1
eluierte RNA [%], RNA:Peptid-Verhältnis
14
12
10
8
6
4
2
0
1 2 3 4 5 6 7 8 9 10 11
Selektionsrunde

Fig. 36

STAR-RNA Selektion 03: Parallele 2-Schritt-Ligation, Neutravidin-Agarose, Raumtemperatur

| Selektionsrunde | Vorsäule [%] | Leersäule [%] | Eluierter Anteil [%] | RNA:Peptid-Verhältnis | Peptid-Konzentration [nM] |
|---|---|---|---|---|---|
| 1 | | | 0,63 | 0,5 | 10000 |
| 2 | 0,62 | 1,5 | 5,47 | 0,5 | 10000 |
| 3 | 3,85 | 2,4 | 0,24 | 0,5 | 10000 |
| 4 | 1,33 | 0,13 | 4,2 | 0,5 | 10000 |
| 5 | 0,1 | 0,25 | 12,1 | 0,5 | 10000 |
| 6 | 1 | 0,44 | 10,7 | 0,5 | 1000 |
| 7 | 0,1 | 0,2 | 1,8 | 0,5 | 100 |
| 8 | 0,2 | 0,2 | 1,7 | 0,5 | 10 |
| 9 | 0,5 | 0,1 | 2,1 | 5 | 3,16 |
| 10 | 0,7 | 0,07 | 2,4 | 5 | 3,16 |
| 11 | 0,8 | 0,5 | 6 | 1 | 1 |

Fig. 37

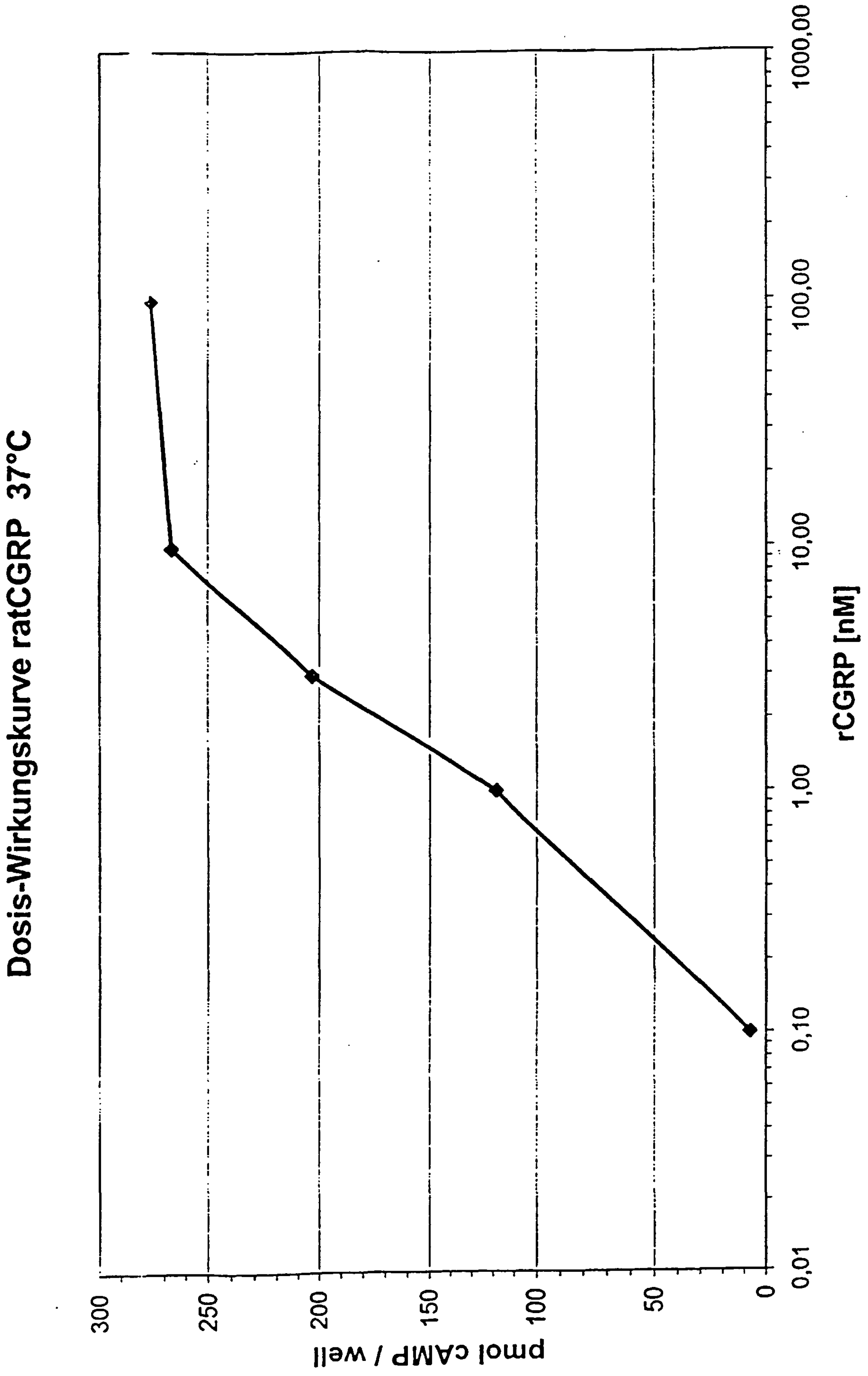
Dosis-Wirkungskurve ratCGRP 37°C
pmol cAMP / well
300
250
200
150
100
50
0
rCGRP [nM]
0,01
0,10
1,00
10,00
100,00
1000,00

Fig. 38

Spiegelmer STAR-R02-15xx-A11, 37°C

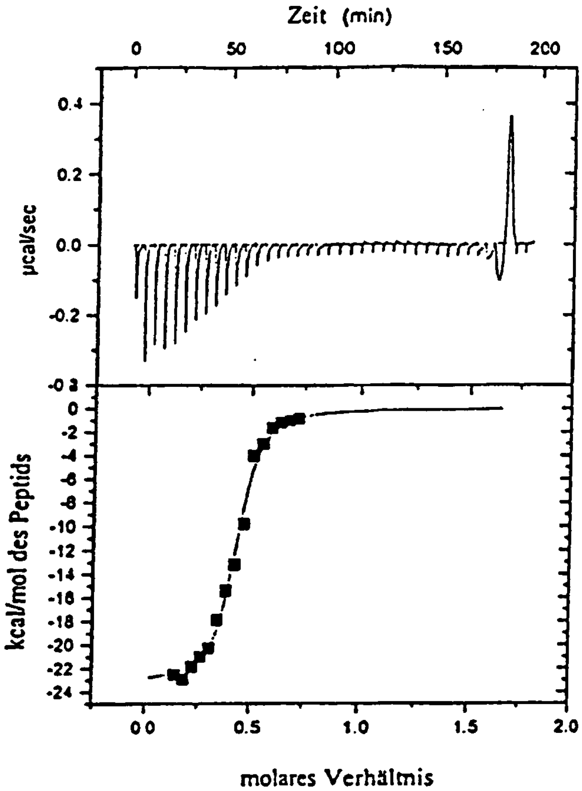


Fig. 39

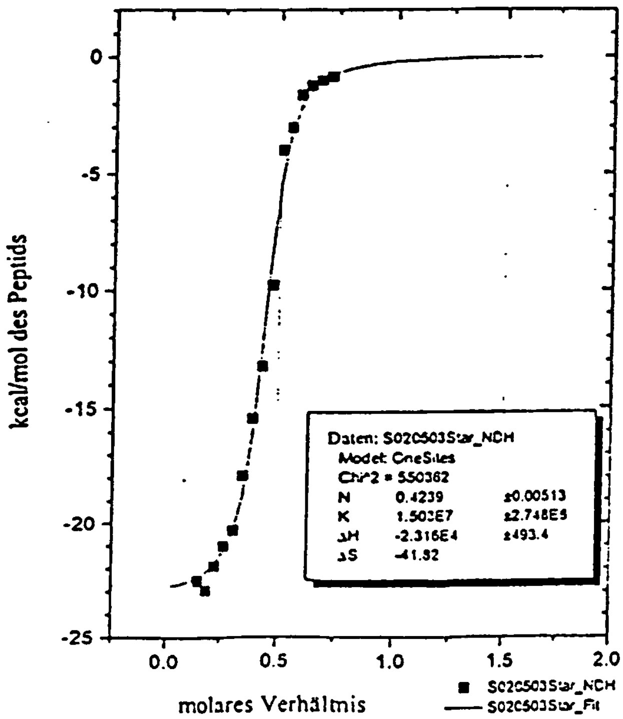


Fig. 40

STAR-R03-11-F10, 25°C

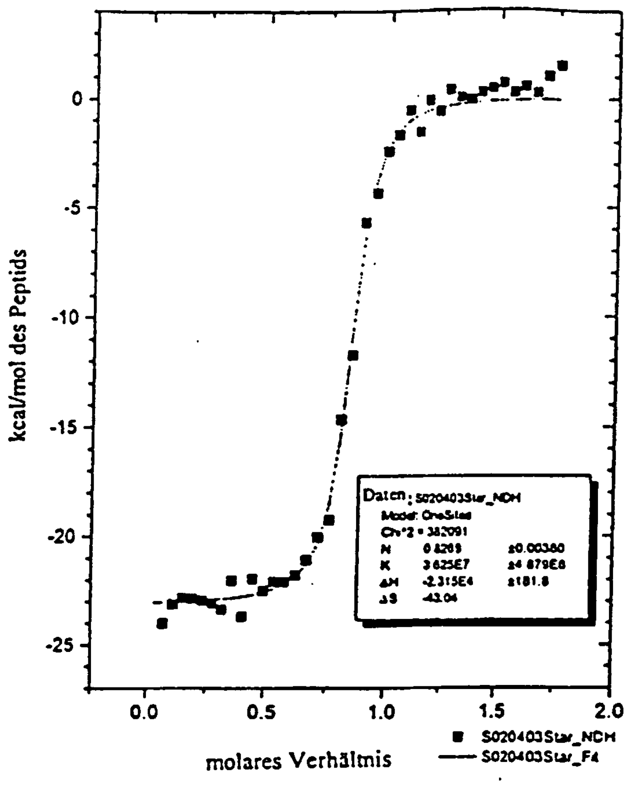


Fig. 42

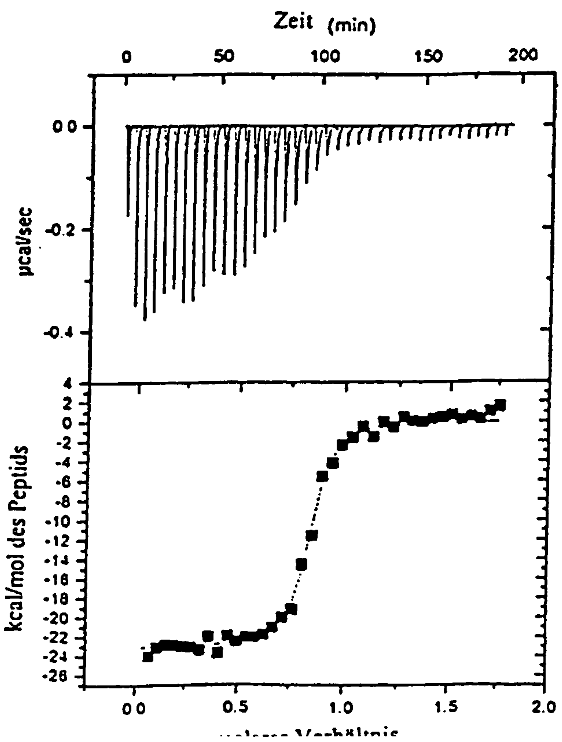


Fig. 41

| Klon # | Sequenz | SEQ ID No |
|---|---|---|
| STAR-R02-10-224 | GGACCAACAUGUGAAGAACAUACGGUGGAAGAAACGAUUGUUAGACAGG | 93 |
| STAR-R02-10-225 | GGACAAACCUGUGUAGAAGGGGGUAAUAUACAGUAGGUAGGGAGGGACAGG | 94 |
| STAR-R02-10-227 | GGACGUAAUGAUCCGGCGAUAAGUCCAGGAUGUCAGGCCGGAGAGACAGG | 95 |
| STAR-R02-10-228 | GGACGAAUGAAGUACGGCGCACCGGGGGUGUGAAGUUAGCGUGAGACAGG | 96 |
| STAR-R02-10-229 | GGACGUGUUGAUGCAGCGGGUGAACAUUCACAAACAUCCCUAGCGACAGG | 97 |
| STAR-R02-10-230 | GGACGAUAGUGGUGUGUCGUUAAUUCACCGGGAGGGUGGCGAGGGACAGG | 98 |
| STAR-R02-10-231 | GGACAGUGCUGGUGCGGCUGAUUGCAUAUAAGCAGUGAACACCCGACAGG | 99 |
| STAR-R02-10-232 | GGACCAACAUGUGAAAAACAUACGGUGGAAGAAACGAUUGUUGAGACAGG | 100 |
| STAR-R02-10-234 | GGACCAAGUAAACCCUGUGAGCCUUGGUAAAGCGGGUGGGAAGGGACAGG | 101 |
| STAR-R02-10-235 | GGACGACAUGUUCCGGGAACAUACGGUGAAAGAAACGAUUGUCGGACAGG | 102 |
| STAR-R02-10-237 | GGACUAGUGAGCGUGGGAGGGCCUCACAAAACGAAAAGUCCGGGGACAGG | 103 |
| STAR-R02-10-239 | GGACGAUUGCGUAGUGGAAGCAUACGGUGAAAGAAACGAUAUCGGACAGG | 104 |
| STAR-R02-10-240 | GGACUAUACGGUGAAAGAAACGAGGCGUUAGGAGGAAACGCUAGGACAGG | 105 |
| STAR-R02-10-243 | GGACGAACGUGCUGGUGCAGCGUGACUUAGUCGAACACCCCUGGGGACAGG | 106 |
| STAR-R02-10-244 | GGACGCCAAAAUCAGGAAGGGAGAGAAAAGGAAUGCGUACGGCGACAGG | 107 |
| STAR-R02-10-248 | GGACAAUCCUGUGGAGUAGGGUGGGGGCCUAGUAGGCCUAGUGGGACAGG | 108 |
| STAR-R02-10-249 | GGACGAUCCUGUGGAGUAGGGUGGGGGCCUAGUAGGCCUAGUGGGACAGG | 109 |
| STAR-R02-10-250 | GGACUUUAGCUGCGUAGUAGGGAAAAGAAGGGUGGGGCAGCUCCGACAGG | 110 |
| STAR-R02-10-251 | GGACUUGAUGACGCGAGUUAACGUCGCUGUCUCUCAAUCAAGGAGACAGG | 111 |
| STAR-R02-10-252 | GGACCAGUGGUGACGCGGCUGGGGCGCCACAGCGCCGGACGUCGACAGG | 112 |
| STAR-R02-10-254 | GGACAGAGGAACGUAGUAGGGUAAGGUAGGAUGAGGGGUUCCUCGACAGG | 113 |
| STAR-R02-10-255 | GGACUAUUUAAGGGCCGGUAGAGAAAUAAACCACCCUCUCUGGAGACAGG | 114 |
| STAR-R02-10-257 | GGACUGAUGGCGCGGGAUUACGCCGCUGGCGCUAGGAACAAGGCGACAGG | 115 |
| STAR-R02-10-258 | GGACGAUCCUGUGGAGUAGGGUGGGGGCCCAGUGGGCCUAGUGGGACAGG | 116 |
| STAR-R02-10-259 | GGACGUGUUGAUGCAGCGGGUGGACAUUCACAAACAUCCCUGGCGACAGG | 117 |
| STAR-R02-10-261 | GGACCCUGCUCGAGAGGACGAGCAUACGGUGAAAGAAACGAUGGGACAGG | 118 |
| STAR-R02-10-263 | GGACAUACGAUGAGAGAAGCGAUUCCCGCUGCUGACGGGAUGUCGACAGG | 119 |
| STAR-R02-10-264 | GGACGAUCCUGUGGAGUAGGGUGGGGGUCUAGUAGACCUAGUGGGACAGG | 120 |
| STAR-R02-10-266 | GGACAGUGAUGGCGCAGCUGUAACCAAGAGGUUAGAACGCUGGAGACAGG | 121 |
| STAR-R02-10-267 | GGACUGAUUCUCAGAGAAUACGAUGAAAGAAGCGAUUCAGUCUGGACAGG | 122 |

Fig. 43

| Klon # | Sequenz | SEQ ID No |
|---|---|---|
| STAR-R02-10-268 | GGACACAACUCGAAGGAGAUGGUAGGUAGGACAGCGGGGGUUGUGACAGG | 123 |
| STAR-R02-10-270 | GGACCAACAUGUGAAAAGCAUACGGUGAAAGAAACGAUUGUUGAGACAGG | 124 |
| STAR-R02-10-271 | GGACGUGAUGGCGCAGCGACUGACACAAAGGAUCAGGAACGCCCCGACAGG | 125 |
| STAR-R02-10-272 | GGACGAACUGGUAGGGUGGCUGCCCUAUACGAUGAAAGAAGCGAGACAGG | 126 |
| STAR-R02-10-274 | GGACUUGAUGACGCGAGUUAACGUCGCUGUCUCUCAAUCAAGGGGACAGG | 127 |
| STAR-R02-10-275 | GGACGACUGAUGGCGCGGUCGUGUAAUAAAACAACGCCGCUGGCGACAGG | 128 |
| STAR-R02-10-276 | GGACACAACUCGAAGGAGAUGGUAGGUAGGACAGCGGGAGUUGUGACAGG | 129 |
| STAR-R02-10-278 | GGACGAUUGCGUAGUGGGAGCAUACGGUGAAAGAAACGAUAUCGGACAGG | 130 |
| STAR-R02-10-280 | GGACAUAUGGUGAAAAGAAACAAUACUCCGUUAGUGAGGAGUUGGACAGG | 131 |
| STAR-R02-10-283 | GGACGUGUUGAUGCAGCGAGUGAACAUUCACGAACAUCCCUGGCGACAGG | 132 |
| STAR-R02-10-286 | GGACGUGUUGACGCAGCGUUACGGCUAAGGACCGUAGAACGUUAGACAGG | 133 |
| STAR-R02-10-287 | GGACACUGUCAGCGCGGUGAUGAGAAAACGCCCCUGUAACAGAUGACAGG | 134 |
| STAR-R02-10-288 | GGACAACGAAGUAAACCUUAGGCGACCCGCAUGAAGGCGGGGUGGACAGG | 135 |
| STAR-R02-10-289 | GGACAUACGGUGAAAGAAACGAUUCGGAACUUCGAAUCCGAUGGACAGG | 136 |
| STAR-R02-10-291 | GGACGAAUGAAGUACGGCGCACCGGGAGCGUGAAGUUAGCGUGAGACAGG | 137 |
| STAR-R02-10-292 | GGACCAACAUGUAAAGAGCAUACGGUGGAAGAAACGAUUGUUGAGACAGG | 138 |
| STAR-R02-10-293 | GGACGACAUGUUCCGAGAACAUACGGUGAAAUAAACGAUUGUCAGACAGG | 139 |
| STAR-R02-10-294 | GGACCAACAUGUAAGAGCAUACGGUGGAAGAAACGAUUGUUAGACAGG | 140 |
| STAR-R02-10-295 | GGACGACCCAUCAAGGAUGAAGAGCGUACGCUUGCGCAGGGGUCGACAGG | 141 |
| STAR-R02-10-296 | GGACCGUGUUAGUGCUGCGGAUAUAAAAACACUGCUGUCUAUGGGACAGG | 142 |
| STAR-R02-10-297 | GGACGAGGUGCUGGCGCUGCCUAUGCCCGAUUGGGUAAAACGCCGACAGG | 143 |
| STAR-R02-10-298 | GGACCAAGUAAACCCUGUAAGCCUUGGUAAAGCGGGUGGGAAGGGACAGG | 144 |
| STAR-R02-10-300 | GGACAACCCGUCAAGGAUGAAGAGCGUACGCUUGCGCAGGGUUCGACAGG | 145 |
| STAR-R02-10-303 | GGACGAGAUGGGCGUAAUAAGAUGGGGAAAAUAGCCGAAAGCCCGACAGG | 146 |
| STAR-R02-10-304 | GGACUCUCUGAUGGCGCGGAGAGAAUAUUACGCCACUGUCGUGAGACAGG | 147 |
| STAR-R02-10-307 | GGACGAACGAGCAUGGACGCGGGUGAGGAGAGGGCGCUAGUUCGGACAGG | 148 |
| STAR-R02-10-310 | GGACGACGAGGAAUUGGUGGGGGAUGGGGUGAGGAUCCUCGCGGGACAGG | 149 |
| STAR-R02-10-311 | GGACGAUUGCAUAGUGAGAGCAUACGGUGAAAGAAACGAUAUCGGACAGG | 150 |
| STAR-R02-10-314 | GGACGUGAUGGUGCAGCGUAGUCGGUUAAGACAAACACCCCUGGGACAGG | 151 |
| STAR-R02-10-315 | GGACAUACGAUGAGAGAAGCGAUUCCCGCUGCUAACGGAUGUCGACAGG | 152 |

Fig. 44

| Klon # | Sequenz | SEQ ID No |
|---|---|---|
| STAR-R02-10-318 | GGACAUACGAUGAAAGAAGCGAUUCCCGCUGCUGACGGGAUGUCGACAGG | 153 |
| STAR-R02-12MW-A1 | GGACGACAUGUUCCCGGAACAUACGGUGAAAGAAACGAUUAUCGGACAGG | 154 |
| STAR-R02-12MW-A4 | GGACGACAUGCUCCAGGAGCAUACGGUGAAAGAAACGAUUGUCGGACAGG | 155 |
| STAR-R02-12MW-A5 | GGACGACAUGUUCCAGGAACAUACGGUGAAAGAAACGAUUGUCGGACAGG | 156 |
| STAR-R02-12MW-B5 | GGACGAUAUGUUCCAAGAACAUACGGUGAAAGAAACGAUUGUCGGACAGG | 157 |
| STAR-R02-12MW-D2 | GGACAUACGAUGAAAGAAGCGAUUCCCGCUGCUAGCGGGAUGUCGACAGG | 158 |
| STAR-R02-12MW-E1 | GGACUACAAGCCAACAAAUCCUUGCCCACUGAGGAUCUGCUGUCGACAGG | 159 |
| STAR-R02-12MW-E3 | GGACUCAUACGGUGAAAGAAACGAUUCGUCUUGUGACGAUGAGGACAGG | 160 |
| STAR-R02-12MW-E4 | GGACGACAUGUUCGGGGAACAUACGGUGAAAGAAACGAUUGUCGGACAGG | 161 |
| STAR-R02-12MW-F3 | GGACAUACGGUGAAAGAAACGAUACGCAAUUUGUGUUGUCCCGACAGG | 162 |
| STAR-R02-12MW-F4 | GGACGAUUGCAUACUAAAAGCAUACGGUGAAAGAAACGAUAUCGGACAGG | 163 |
| STAR-R02-12MW-F6 | GGACUCAUACGGUGAAAGAAACGAUUCGUCUUAACGACGAUGAGGACAGG | 164 |
| STAR-R02-12MW-G1 | GGACUAAGUGAGCCAAGUCAGCGGGAUGUCCAUAACUUGUCGACAGG | 165 |
| STAR-R02-12MW-H1 | GGACAUACGGUGAAAGAAACGAUACAUAAUUUAUGUUCCUGUCCAAGACAGG | 166 |
| STAR-R02-12MW-H2 | GGACGAUUGCACAAUGANAGCAUACGGUGAAAGAAACGAUAUCGGACAGG | 167 |
| STAR-R02-15d-A1 | GGACGACAUGUUCUAUGAACAUACGGUGAAAGAAACGAUUGUCGGACAGG | 168 |
| STAR-R02-15d-A10 | GGACGAUUGCAUAGUAAGAGCAUACGGUGAAAGAAACGAUAUCGGACAGG | 169 |
| STAR-R02-15d-A12 | GGACGACAUGUUCCAGGAACAUACGGUGAAAAAAACGAUUGUCGGACAGG | 170 |
| STAR-R02-15d-A2 | GGACGACAUGUUCUAGGAACAUACGGUGAAAGAAACGAUUGUUGGACAGG | 171 |
| STAR-R02-15d-A9 | GGACAUACGAUGAAAGAAGCGAUUCCCGCUACUAGCGGGAUGUCGACAGG | 172 |
| STAR-R02-15d-B11 | GGACCAACAUGCAAAGAGCAUACGGUGAAAGAAACGAUUGUUGAGACAGG | 173 |
| STAR-R02-15d-B3 | GGACAUAUGGUGAAAGAAACAAUUCGGAACUUCGAUUCCGAUGGACAGG | 174 |
| STAR-R02-15d-C10 | GGACGAUUGCAUAAUAAAAGCAUACGGUGAAAGAAACGAUAUCGGACAGG | 175 |
| STAR-R02-15d-C9 | GGACGACAUGUUCUAAGAACAUACGGUGAAAGAAACGAUUGUCGGACAGG | 176 |
| STAR-R02-15d-D10 | GGACGACAUGUUCCAAGGAACAUACGGUGAAAGAAACGAUUGUCGGACAGG | 177 |
| STAR-R02-15d-D7 | GGACCAACAUGUGAAGAGCAUACGGUGAAAGAAACGAUUGUUGAGACAGG | 178 |
| STAR-R02-15d-D9 | GGACGAUUGCAUAGUGGGUGCAUACGGUGAAAGAAACGAUAUCGGACAGG | 179 |
| STAR-R02-15d-E11 | GGACGAUUGCAUAGUAAAAGCAUACGGUGAAAGAAACGAUAUCGGACAGG | 180 |
| STAR-R02-15d-E12 | GGACGACAUGUUCAAAGAACAUACGGUGAAAGAAACGAUUGUCGGACAGG | 181 |
| STAR-R02-15d-E3 | GGACCAUACGAUGAAAGAAGCGAUACUGCACCAGAAGCAGUUGGGACAGG | 182 |

Fig. 45

| Klon # | Sequenz | SEQ ID No |
|---|---|---|
| STAR-R02-15d-F8 | GGACGAUUGCAUUAAGAGCAUACGGUGAAAGAAACGAUAUCGGACAGG | 183 |
| STAR-R02-15d-F9 | GGACGACAUGUUCCGAGAACAUACGGUGAAAGAAACGAUUGUCGGACAGG | 184 |
| STAR-R02-15d-G1 | GGACGAUUGCAUAGUAGAAGCAUACGGUGAAAGAAACGAUAUCGGACAGG | 185 |
| STAR-R02-15d-G12 | GGACUCAUACGGUGAAAGAAACGAUUCGUCUUGACGACGAUGAGGACAGG | 186 |
| STAR-R02-15d-G2 | GGACAUACGAUGAAAGAAGCGAUUCCCGUUACUAGCGGGAUGUCGACAGG | 187 |
| STAR-R02-15d-H1 | GGACGACAUGUUCCCAGAACAUACGGUGAAAGAAACGAUUGUCGGACAGG | 188 |
| STAR-R02-15d-H7 | GGACAUACGGUGAAAGAAACGAUUCGGACUUGAGUCCGAUGGACAGG | 189 |
| STAR-R02-15d-H9 | GGACGAUUGCAUAAUAAGAGCAUACGGUGAAAGAAACGAUAUCGGACAGG | 190 |
| STAR-R02-15xx-A11 | GGACUGAUGGCGCGGUCUCAAAAAACGCCGAUAGGGUGAGGGGACAGG | 191 |
| STAR-R02-15xx-A7 | GGACUGAUGGCGCGGUCUUAAAAAACGCCGAUGGGGUGAGGGGACAGG | 192 |
| STAR-R02-15xx-B10 | GGACUCAUACGGUGAAAGAAACGAUUCGUCUAGCGACGAUGAGGACAGG | 193 |
| STAR-R02-15xx-B7 | GGACUCAUACGGUGAAAGAAACGAUUCGUCUUAGCGNCGAUGAGGACAGG | 194 |
| STAR-R02-15xx-C7 | GGACUGAUGGCGCGGUNUCAAAAAACGCCGAUAGGGUGAGGGGACAGG | 195 |
| STAR-R02-15xx-C9 | GGACUGAUGGCGCGGUCUCAAAAAACGCCGCUAGGGUGAGGGGACAGG | 196 |
| STAR-R02-15xx-D8 | GGACAGACGAUGGCCGUAAAUCAUCGGGAAAGGGGAUGGAGGGACAGG | 197 |
| STAR-R02-15xx-E9 | GGACUGAUGGCGCGGUCGAUAAAUACGCCCGUGAACUGGGGAAGUGGACAGG | 198 |
| STAR-R02-15xx-F12 | GGACUGAUGGCGCGGUCCUAUUACGCCGAAAGGGAGAGGGGAGACAGG | 199 |
| STAR-R02-15xx-F7 | GGACUGAUGGCGCGGUCUUAAAAAACGCCGAUAGGGUGAGGGGACAGG | 200 |
| STAR-R02-15xx-G10 | GGACAGACGAUGGCCAUAAAUCAUCGGGAAAGGGGAUGGAGGGACAGG | 201 |
| STAR-R02-15xx-G12 | GGACUCAUACGGUGAAAGAAACGAUUCGUCUUAGGACGAUGAGGACAGG | 202 |
| STAR-R02-15xx-G7 | GGACAGACGAUGNCCGUAAAUCAUCGGGAAAGGGCAUGGAGGGUACAGG | 203 |
| STAR-R03-11-A4 | GGACGACAUGUUCGCAGAACAUACGGUGAAAGAAACGAUUGUCGGACAGG | 204 |
| STAR-R03-11-A6 | GGACAUACGGUGAAAGAAACGAUUCCCGCUACUAGCGGGAUGUCGACAGG | 205 |
| STAR-R03-11-A7 | GGACGACAUGUUCAAAAGAACAUACGGUGAAAGAAACGAUUGUCGGACAGG | 206 |
| STAR-R03-11-B10 | GGACAUACGGUGAAAGAAACGAUACAUGAUUUAUGUUGUCCCGAGACAGG | 207 |
| STAR-R03-11-B7 | GGACAUACGGUGAAAGAAACGAUUCUCGCUGCUAGCGAGAUGUCGACAGG | 208 |
| STAR-R03-11-C12 | GGACGACAUGUUCCCAGGAACAUACGGUGAAAGAAACGAUUGUCGGACAGG | 209 |
| STAR-R03-11-C6 | GGACGACAUGUUCGGAGAACAUACGGUGAAAGAAACGAUUGUCGGACAGG | 210 |
| STAR-R03-11-C7 | GGACAUACGGUGAAAGAAACGAUUCGGAUUACCAAUCCGAUGGACAGG | 211 |
| STAR-R03-11-C8 | GGACUCAUACGGUGAAAGAAACGAUUCGUCUUAGCGACGAUGAGGACAGG | 212 |

Fig. 46

| Klon # | Sequenz | SEQ ID No |
| --- | --- | --- |
| STAR-R03-11-C9 | GGACGACAUGUUCCAAGAACAUACGGUGAAAGAAACGAUUGUCGGACAGG | 213 |
| STAR-R03-11-D5 | GGACGACAUGUUCAUAGAACAUACGGUGAAAGAAACGAUUGUCAGACAGG | 214 |
| STAR-R03-11-E11 | GGACGACAUGUUCUAGGAACAUACGGUGAAAGAAACGAUUGUCGGACAGG | 215 |
| STAR-R03-11-E5 | GGACGACAUGUUCCAUGGAACAUACGGUGAAAGAAACGAUUGUCGGACAGG | 216 |
| STAR-R03-11-E6 | GGACGACAUGUUCAGGGAACAUACGGUGAAAGAAACGAUUGUCGGACAGG | 217 |
| STAR-R03-11-E7 | GGACAGACGAUGGCCUUAAAUCAUCGGGAAAGGGGAUGGAGGGACAGG | 218 |
| STAR-R03-11-E8 | GGACGACAUGUUCUAGGAACAUACGAUGAAAGAAGCGAUUGUCGGACAGG | 219 |
| STAR-R03-11-F10 | GGACAUACGGUGAAAGAAACGAUACAUAAUUUAUGUUGUCCCGAGACAGG | 220 |
| STAR-R03-11-F5 | GGACGUCCAUACGGUGAAAGAAACGAUAGGGAUAGACUCCCUUGGACAGG | 221 |
| STAR-R03-11-F6 | GGACAUACGGUGAAAGAAACGAUUCGGAACUUCGAUUCCGAUGGACAGG | 222 |
| STAR-R03-11-F7 | GGACGACAUGUUCUACGAACAUACGGUGAAAGAAACGAUUGUCGGACAGG | 223 |
| STAR-R03-11-G10 | GGACCAACGCUUAAGCAAACCCCACUUACACUUAAGCGUCGAGCGACAGG | 224 |
| STAR-R03-11-G12 | GGACAUACGGUGAAAGAAACGAUUCCCACUACUAGCGGGAUGUCGACAGG | 225 |
| STAR-R03-11-G4 | GGACGACAUGUUCAAGGAACAUACGGUGAAAGAAACGAUUGUCGGACAGG | 226 |
| STAR-R03-11-G8 | GGACGACAUGUUCAGAGAACAUACGGUGAAAGAAACGAUUGUCAGACAGG | 227 |
| STAR-R03-11-G9 | GGACAUACGAUGAAAGAAGCGAUUCCCGUUGCUAGCGGGAUGUCGACAGG | 228 |
| STAR-R03-11-H12 | GGACCAUUAUCCCCCAGGAUAUACGGUGAAAGAAACGAUAUGGGACAGG | 229 |
| STAR-R03-11-H5 | GGACGACAUGUUCAAAGAACAUACGGUGAAAGAAACGAUUGUCGGACAGG | 230 |
| STAR-R03-11-H9 | GGACGACAUGUUCAGAGAACAUACGGUGAAAGAAACGAUUGUCGGACAGG | 231 |
| STAR-R03-11-F10-45-001 | CGGGACAUACGGUGAAAGAAACGAUACAUAAUUUAUGUUGUCCCG | 232 |
| STAR-R03-11-C12-48-001 | GGCCGACAUGUUCCCAGGAACAUACGGUGAAAGAAACGAUUGUCGUCC | 233 |

Fig. 47

| | | SEQ ID No |
|---|---|---|
| **Familie 1** | | |
| 156750.seq | 5'-GGGAAUUCGAGCUCGGUACC -UUAACCCGUAU-GGGGUCACUG-UUUCGAUUU-CAUUCGCCUUCUCGAGCUGA-UUCACUUGCG- CUGCAGGCAUGAAGCUUGG-3' | 33 |
| *Mutationen der Familie 1* | | |
| 156701.seq | 5'-GGGAAUUCGAGCUCGGUACC -UUAACCCGUAU-GGGGUCACUGGUUUCGAUUU-CAUUCGCCUUAUCGAGCUGA-UUCACUUGCG- CUGCAGGCAUGAAGCUUGG-3' | 34 |
| 156698.seq | 5'-GGGAAUUCGAGCUCGGUACC -UUAACCCGUAUUGGGGUCACUG-UUUCGAUUU-CAUUCGCCUUAUCGAGCUGA-UUCACUUGCG- CUGCAGGCAUGAAGCUUGG-3' | 35 |
| 156707.seq | 5'-GGGAAUUCGAGCUCGGUACC -UUAACCCGUAU-GGGGUCACUG-UUUCGAUUUUCAUUCGCCUUAUCGAGCUGA-UUCACUUGCG- CUGCAGGCAUGAAGCUUGG-3' | 36 |
| KN-158_06b_D06_x_046 | 5'-GGGAAUUCGAGCUCGGUACC -CUAACCCGUAU-GGGGUCACUG-UUUCGAUUU-CAUUCGCCUUAUCNANCUGA-UUCACUUGCG- CUGCAGGCAUGAAGCUUGG-3' | 37 |
| KN-158_06b_D12_x_094 | 5'-GGGAAUUCGAGCUCGGUACC -CUAACCCGUAU-GGGGUCACUG-UUUCGAUUU-CAUUCGCCUUAUCGAGCUGA-UUCACUUGCG- CUGCAGGCAUGAAGCUUGG-3' | 38 |
| kn-158-6_h03_m13-fp | 5'-GGGAAUUCGAGCUCGGUACC -UUAACCNGUAU-GGGGUCACUG-UUUCGAUUU-CAUUCGCCUUAUCGAGCUGAAUUCACUUGCG- CUGCAGGCAUGAAGCUUGG-3' | 39 |
| kn-158-6_f12_m13-fp | 5'-GGGAAUUCGAGCUCGGUACC -UUAACCCGUAU-GGGGUCACUG-UUUCGAUUU-CAUUCGCCUUAUCGAGCUGA-UNCACUUGCG- CUGCAGGCAUGAAGCUUGG-3' | 40 |
| **Familie 2** | | |
| 156703.seq | 5'-GGGAAUUCGAGCUCGGUACC -UUGUUACCCACUGUUUAGUAUCUCGCCGAUACUCAUUACCGAGACACAGUCCCAUUACUGC- CUGCAGGCAUGAAGCUUGG-3' | 41 |
| **Familie 3** | | |
| 156719.seq | 5'-GGGAAUUCGAGCUCGGUACC -GCACUUUCGUUACAUACGAUAUACUGGGCUAUAGUCUAUCCUUGUGCCUACAGGUACUG- CUGCAGGCAUGAAGCUUGG-3' | 42 |
| *Mutationen der Familie 3* | | |
| 156718.seq | 5'-GGGAAUUCGAGCUCGGUACC -GCACUUUCGUUACAUACGAUAUACUGGGCUAUAGUCUAUCCUUGUGCCUACAGGU--UG- CUGCAGGCAUGAAGCUUGG-3' | 43 |
| 156736.seq | 5'-GGGAAUUCGAGCUCGGUACC -GCACUUUCGUUACAUACGAUAUACUGGGCUAUAGUCUAUCCCUGUGCCUACAGGUACUG- CUGCAGGCAUGAAGCUUGG-3' | 44 |
| KN-158_06b_B10_x_077 | 5'-GGGAAUUCGAGCUCGGUACC -GCACUUUCGUUACAUACGAUAUACUGGGCUAUUG--UAUCCUUGUGCCUACAGGUACUG- CUGCAGGCAUGAAGCUUGG-3' | 45 |
| kn-158-6_f07_m13-fp | 5'-GGGAAUUCGAGCUCGGUACC -GCACUUUCGUUGCAUACGAUAUACCGGGCUAUAGUCUAUCCUUGUGCCUACAGGUACUG- CUGCAGGCAUGAAGCUUGG-3' | 46 |
| **Familie 4** | | |
| 156695.seq | 5'-GGGAAUUCGAGCUCGGUACC -UACUGCUCCGACUAAUUGUCUAGUACAAUAUGCUUACCACAUUAUCUGUUAGUGAGCUCC- CUGCAGGCAUGAAGCUUGG-3' | 47 |
| **Familie 5** | | |
| 156673.seq | 5'-GGGAAUUCGAGCUCGGUACC -UUGUUCUGACUCUGUUUAUGCGUUUUCCGCGUCUUUACCGGACUCCUUCUUCCCCAGUGC- CUGCAGGCAUGAAGCUUGG-3' | 48 |
| *Mutationen der Familie 5* | | |
| 156706.seq | 5'-GGGAAUUCGAGCUCGGUACC -UUGUUCUGACUCUGUUUAUGCGUUUUACGCGUCUUUACCGGACUCC-UCUUCCCCAGUGC- CUGCAGGCAUGAAGCUUGG-3' | 49 |
| 156761.seq | 5'-GGGAAUUCGAGCUCGGUACC -UUGUUCUGACUCUGUUUAUGCGUU-UACGCGUCUUUACCGGACUCCUUCUUCCC-AGUGC- CUGCAGGCAUGAAGCUUGG-3' | 50 |
| **Familie 6** | | |
| 156738.seq | 5'-GGGAAUUCGAGCUCGGUACC -UAUCGUCGAACAUUCGAUCUGUUUUUACG-UAAGUAACUUUACCGUCCUCGUUUUUCCCGC- CUGCAGGCAUGAAGCUUGG-3' | 51 |
| *Mutationen der Familie 6* | | |
| 156730.seq | 5'-GGGAAUUCGAGCUCGGUACC -UAUCGUCGAACAUUCGAUCUGUUUUUACGUUAAGUAACUUUACCGUCCUCGUUUUUCCCGC- CUGCAGGCAUGAAGCUUGG-3' | 52 |
| kn-158-6_f04_m13-fp.abi | 5'-GGGAAUUCGAGCUCGGUACC -UAUCGUCGAACAUUCGAUCUGUUUUUACG-UAAGUAACUUUACCGUCCUCGUUUUU-CCCGC- CUGCAGGCAUGAAGCUUGG-3' | 53 |
| kn-158-6_f06_m13-fp.abi | 5'-GGGAAUUCGAGCUCGGUACC -UAUCGUCGAACAUUCGAUCUGUUUUUACG-UAAGUAACUGUACCGUCCUCGUUUUU-CCUCC- CUGCAGGCAUGAAGCUUGG-3' | 54 |
| kn-158-6_g02_m13-fp.abi | 5'-GGGAAUUCGAGCUCGGUACC -UAUUGUCGAACAUUCGAUCUGUUUUUACG-UAAGUAACUUUACCGUCCUCGUUUUUUCCCGC- CUGCAGGCAUGAAGCUUGG-3' | 55 |
| **Familie 7** | | |
| 156697.seq | 5'-GGGAAUUCGAGCUCGGUACC -AAACAUCACUUACAUCUGCUCUGCGUUUUUUGCAUAGUUUUUUGGUCGAGCGCUUCCUCC- CUGCAGGCAUGAAGCUUGG-3' | 56 |
| *Mutation der Familie 7* | | |
| 156712.seq | 5'-GGGAAUUCGAGCUCGGUACC -AGACAUCACUUACAUGUGCUCUGCGUUUUUUGCAUCGUUUUUUGGUCGAGCGCUUCCUCC- CUGCAGGCAUGAAGCUUGG-3' | 57 |

Fig. 48

## Familie 8

| | | | | |
|---|---|---|---|---|
| 156745.seq | 5'-GGGAAUUCGAGCUCGGUACC | -GCCGAGUCUGUCACAAGAUCUCGUCCUUAUCGUUGAUGUAUCGUACAAGUCUUUGCC- | CUGCAGGCAUGAAGCUUGG-3' | 58 |

## Familie 9

| | | | | |
|---|---|---|---|---|
| kn-158-6_e07_m13-fp.abi | 5'-GGGAAUUCGAGCUCGGUACC | -AAUGCCUGCUUUGUUUUGAGUUUUCCUU-CACACUAGGGAUGGAUAAUACAGUCCCUACC- | CUGCAGGCAUGAAGCUUGG-3' | 59 |
| *Mutationen der Familie 9* | | | | |
| kn-158-6_e05_m13-fp.abi | 5'-GGGAAUUCGAGCUCGGUACC | -AAUGCCUGCUUUGUUUUGAGUUUUUCUU-CACACUAGGGAUGGAUAAUACAGUCCCUACC- | CUGCAGGCAUGAAGCUUGG-3' | 60 |
| kn-158-6_e08_m13-fp.abi | 5'-GGGAAUUCGAGCUCGGUACC | --------CUUUGUUUUGAGUUUUCCCUGUACACUAGGGAUGGAUAAUACAGUCCCUACC- | CUGCAGGCAUGAAGCUUGG-3' | 61 |

## „ORPHAN" Sequenzen

### SA-1

| | | | | |
|---|---|---|---|---|
| 156678.seq | 5'-GGGAAUUCGAGCUCGGUACC | -UGUUACAGUGUUGUACUAGUUCUAGUGUUUGGUUGAUGUACUCCCGUAACUUAUCCUGC-- | CUGCAGGCAUGAAGCUUGG-3' | 62 |
| 156758.seq | 5'-GGGAAUUCGAGCUCGGUACC | -UAAACGACUACGACCUGAUGCUGGAAGUUAAUUUGUAACUGUGUUACAUACAGUCGCGUG- | CUGCAGGCAUGAAGCUUGG-3' | 63 |
| 156708.seq | 5'-GGGAAUUCGAGCUCGGUACC | -UUAAUGGUUGACUGAUUCUUCCUUGCCAACUGUGCUUAGCUGUUACAGUUUAUCAUGUGC- | CUGCAGGCAUGAAGCUUGG-3' | 64 |
| 156693.seq | 5'-GGGAAUUCGAGCUCGGUACC | -AAGAGAUGAUCAAGCGUCGUUCUAAUCAUGAGUUGUGUCUACCCCUAACUGCCAAUUCG-- | CUGCAGGCAUGAAGCUUGG-3' | 65 |
| 156751.seq | 5'-GGGAAUUCGAGCUCGGUACC | -UUAAACACCAGUGCCUGCUCUCAAUGAUGUUGUUUUCUUCAUGCGUGUCGUGAGAUCUUC- | CUGCAGGCAUGAAGCUUGG-3' | 66 |
| 156759.seq | 5'-GGGAAUUCGAGCUCGGUACC | -GUGAACCUGUAACUUUGUCAGCCGCACGGUAAUUUUGUUCUCGAUUAAUCCCGAGAGCC-- | CUGCAGGCAUGAAGCUUGG-3' | 67 |
| 156733.seq | 5'-GGGAAUUCGAGCUCGGUACC | -AAACGGCAGCACUCGCACUUAUUCGUGGACGAUUUUGAGGUUAAUGUACGCUUGCCCC--- | CUGCAGGCAUGAAGCUUGG-3' | 68 |
| 156710.seq | 5'-GGGAAUUCGAGCUCGGUACC | -UAUCGUUGAACAUUCGAUCUGUUUUUACGUAAGUAACUUUACCGUCCUCGUUUUUCCCGC-- | CUGCAGGCAUGAAGCUUGG-3' | 69 |

### SA-0,1

| | | | | |
|---|---|---|---|---|
| 156760.seq | 5'-GGGAAUUCGAGCUCGGUACC | -CUGCAGUUUCAGAUUUAACGAUUAGCUUCUGGAUGGGUAUUGGCACAUCUUACCCUUUGG- | CUGCAGGCAUGAAGCUUGG-3' | 70 |
| 156755.seq | 5'-GGGAAUUCGAGCUCGGUACC | -AUUUGGCUAGGUAACCGACAACUUCAAUCGCUACUUCACUUGGAGUCUUGGCUAUCCAUC- | CUGCAGGCAUGAAGCUUGG-3' | 71 |
| 156714.seq | 5'-GGGAAUUCGAGCUCGGUACC | -UUUCUGCGACUGUUGCCGCAAUCUUCAACCUUGCGUGAUUAAGCCACGUAUCGUCUUG--- | CUGCAGGCAUGAAGCUUGG-3' | 72 |
| 156690.seq | 5'-GGGAAUUCGAGCUCGGUACC | -UCAUCCUCAUCAAACAGUAGCGUUAUCAUUGUGUAGUAACCCAAAGCGCUGCCAUUCC--- | CUGCAGGCAUGAAGCUUGG-3' | 73 |
| 156681.seq | 5'-GGGAAUUCGAGCUCGGUACC | -GGAUGGAACACGCAGUUUGUCUUAGUUCGUUGAUGAAACCUUUUCAACGCUUCGCC----- | CUGCAGGCAUGAAGCUUGG-3' | 74 |
| 156739.seq | 5'-GGGAAUUCGAGCUCGGUACC | -UAUACUGCUCACUUUGAAUUCAAUCACCGUUCGCGCUGCUAACUCACUAUUUCCCUUGGC- | CUGCAGGCAUGAAGCUUGG-3' | 75 |
| 156682.seq | 5'-GGGAAUUCGAGCUCGGUACC | -AGGUGAUUGCUUUCUUAACUUUGUUAGACUUCGACCAUCAGUGUCGCCCUCAC------- | CUGCAGGCAUGAAGCUUGG-3' | 76 |

### NA-A

| | | | | |
|---|---|---|---|---|
| kn-158-6_d04_m13-fp.abi | 5'-GGGAAUUCGAGCUCGGUACC | -AAUGCCUGCUUUGUUUUGAGUUUUCCUUCACACUAGGGAUGGAUAAUACAGUCCCUACC-- | CUGCAGGCAUGAAGCUUGG-3' | 77 |
| kn-158-6_a01_m13-fp.abi | 5'-GGGAAUUCGAGCUCGGUACC | -UACAAUCGUUACCUGCUCGCUUUAAUCUUGGAUCCUAUCCGAUUUAGAUCUUCCCACUAC- | CUGCAGGCAUGAAGCUUGG-3' | 78 |
| kn-158-6_c01_m13-fp.abi | 5'-GGGAAUUCGAGCUCGGUACC | -AUGCAGGUGCCCCCUUUGGUUUUUUCCAAAGGCACAUCAGAAAGUUCUCGAAUUUCCGUA- | CUGCAGGCAUGAAGCUUGG-3' | 79 |
| kn-158-6_c05_m13-fp.abi | 5'-GGGAAUUCGAGCUCGGUACC | -ACCUGCAAUUCCAUNCCGUUCUCCCAUAUGUCUUAGGCUUNUGGUGUUCUCGUCUUGGUC- | CUGCAGGCAUGAAGCUUGG-3' | 80 |
| kn-158-6_a12_m13-fp.abi | 5'-GGGAAUUCGAGCUCGGUACC | -UUUACGUAACAUUGUACCCCUUCAGCCGCUGGCUGUCGCACAUAUGAUAGUUCUCGGAUU- | CUGCAGGCAUGAAGCUUGG-3' | 81 |
| kn-158-6_a06_m13-fp.abi | 5'-GGGAAUUCGAGCUCGGUACC | -ACAGGACGCUGUUGAUCCUCGGUAAAUCGGUAUGUAAGUAUUCGAAGUAUGUGUGCCCC-- | CUGCAGGCAUGAAGCUUGG-3' | 82 |
| kn-158-6_c10_m13-fp.abi | 5'-GGGAAUUCGAGCUCGGUACC | -UACUGCUCGACUAAUUGUCUAGUACAAUAUGCUUACCACAUUAUCUGUUAGUGAGCUCC-- | CUGCAGGCAUGAAGCUUGG-3' | 83 |
| kn-158-6_b05_m13-fp.abi | 5'-GGGAAUUCGAGCUCGGUACC | -AGACAUCACUUACAUGUGCUCUGCGUUUUUUGCAUCGUUUUUUGGUCGAGCGCUUCCUCC- | CUGCAGGCAUGAAGCUUGG-3' | 84 |
| kn-158-6_c09_m13-fp.abi | 5'-GGGAAUUCGAGCUCGGUACC | -UAUCGUCGAACAUUCGAUCUGUUUUUACGUAAGUAACUUUACCGUCCUCGUUUUUCCCGC- | CUGCAGGCAUGAAGCUUGG-3' | 85 |
| kn-158-6_b09_m13-fp.abi | 5'-GGGAAUUCGAGCUCGGUACC | -UCUUGCACGCAUUAACUGAUUGCUCUUUCAUGACUUACAACUCCCUUUGCUAGCCCC---- | CUGCAGGCAUGAAGCUUGG-3' | 86 |
| kn-158-6_a02_m13-fp.abi | 5'-GGGAAUUCGAGCUCGGUACC | -GCUUAAGACAUAUUCUUCCUACCCUCUAUUGGGUCUUUACGGGCUUACUUUCCUAGGGCG- | CUGCAGGCAUGAAGCUUGG-3' | 87 |

### NA-D

| | | | | |
|---|---|---|---|---|
| kn-158-6_g10_m13-fp.abi | 5'-GGGAAUUCGAGCUCGGUACC | -AGACAUCACUUACAUGUGCUCUGCGUUUUUUGCAUCGUUUUUUGGUCGAGCGCUUCCUCC- | CUGCAGGCAUGAAGCUUGG-3' | 88 |
| kn-158-6_f11_m13-fp.abi | 5'-GGGAAUUCGAGCUCGGUACC | -UUGUUCUGACACUGUUUAUGCGUUUUACGCGUCUUUACCGGGCUCCUUCUUCCCAGUGC-- | CUGCAGGCAUGAAGCUUGG-3' | 89 |
| kn-158-6_h02_m13-fp.abi | 5'-GGGAAUUCGAGCUCGGUACC | -AAUGUACCAUAGUGUUACAUUGGGCUUGGAAUACAGUCUCGACUGAAUUCGGUUCCCCGG- | CUGCAGGCAUGAAGCUUGG-3' | 90 |
| kn-158-6_h07_m13-fp.abi | 5'-GGGAAUUCGAGCUCGGUACC | -UGCGAGCAUAGACCCACAGUCUUUUGCGAUUCGUUUUCAAACGUGUUCGUGCGUAUUCC-- | CUGCAGGCAUGAAGCUUGG-3' | 91 |
| kn-158-6_h05_m13-fp.abi | 5'-GGGAAUUCGAGCUCGGUACC | -AAAUGGUAUUGGCCCGAGACUGUGCUUUCGCACAGUUAAUCAAUAGUUUGCGAUUGUUCC- | CUGCAGGCAUGAAGCUUGG-3' | 92 |

Fig. 49

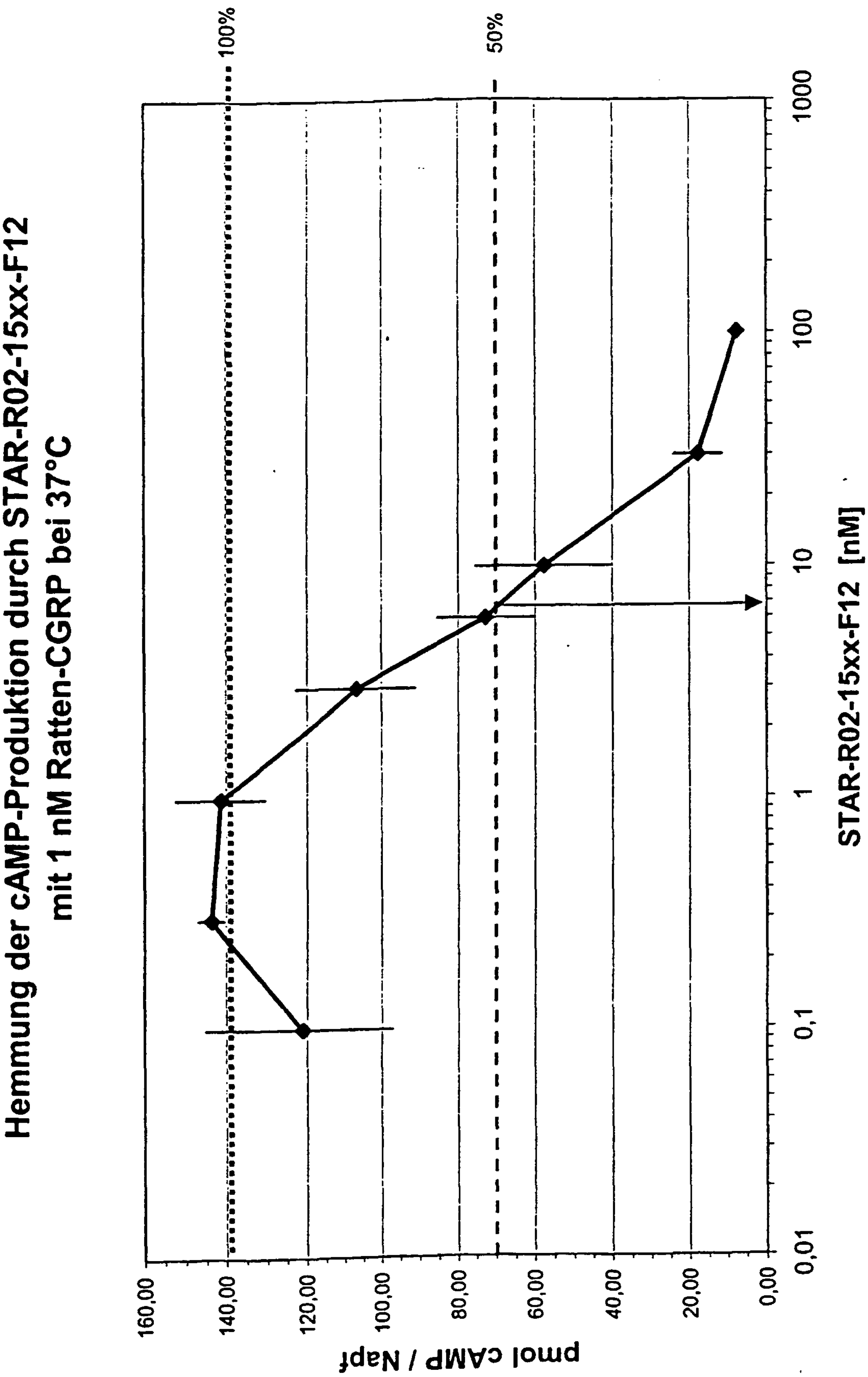

Hemmung der cAMP-Produktion durch STAR-R02-15xx-F12
mit 1 nM Ratten-CGRP bei 37°C
100%
50%
pmol cAMP / Napf
160,00
140,00
120,00
100,00
80,00
60,00
40,00
20,00
0,00
0,01
0,1
1
10
100
1000
STAR-R02-15xx-F12 [nM]


Fig. 50

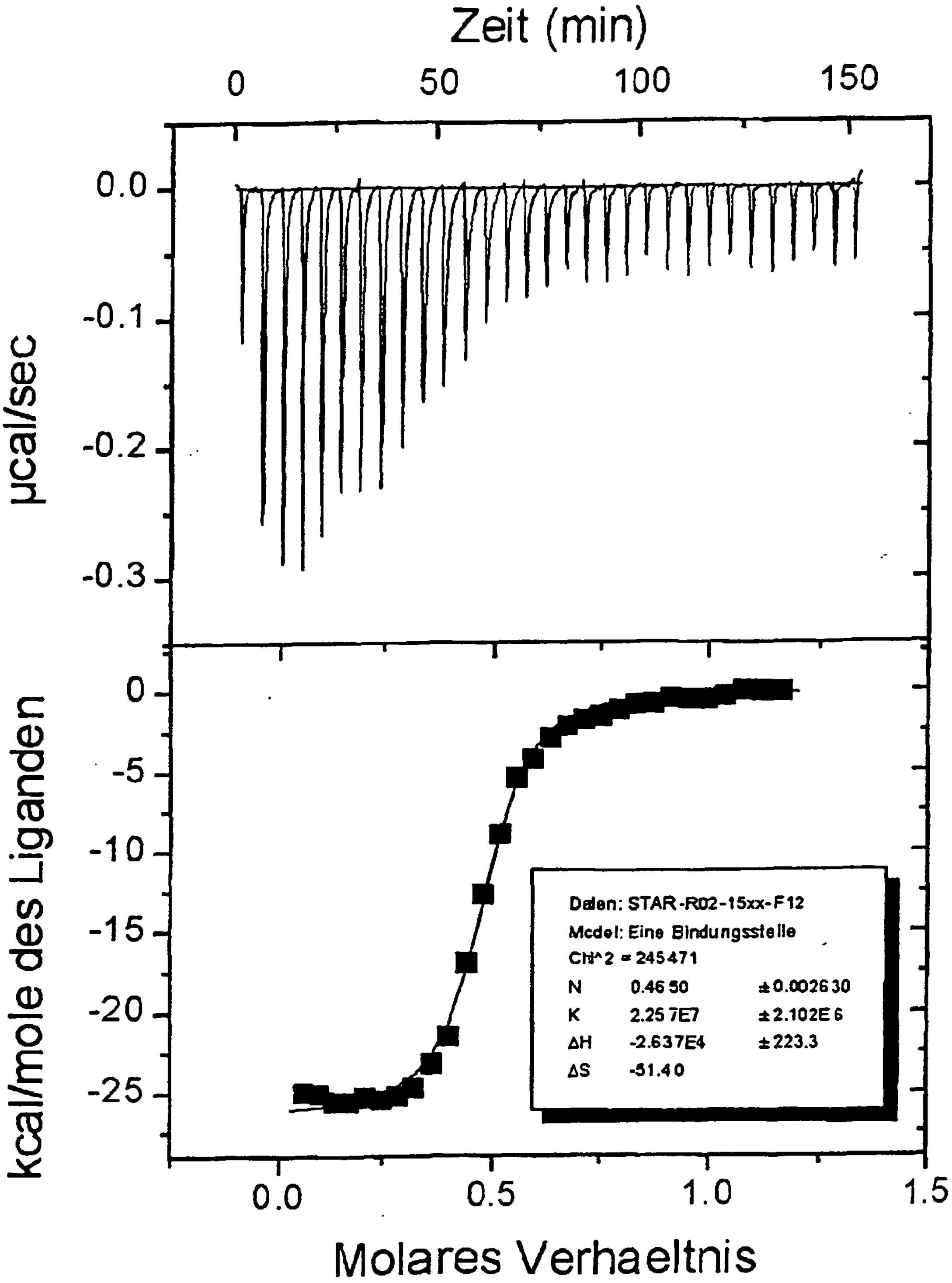
Zeit (min)
0
50
100
150
0.0
-0.1
-0.2
-0.3
µcal/sec
0
-5
-10
-15
-20
-25
kcal/mole des Liganden
0.0
0.5
1.0
1.5
Molares Verhaeltnis
Dalen: STAR-R02-15xx-F12
Mcdel: Eine Bindungsstelle
Chi^2 = 245471
N 0.4650 ±0.002630
K 2.257E7 ±2.102E6
ΔH -2.637E4 ±223.3
ΔS -51.40

Fig. 51

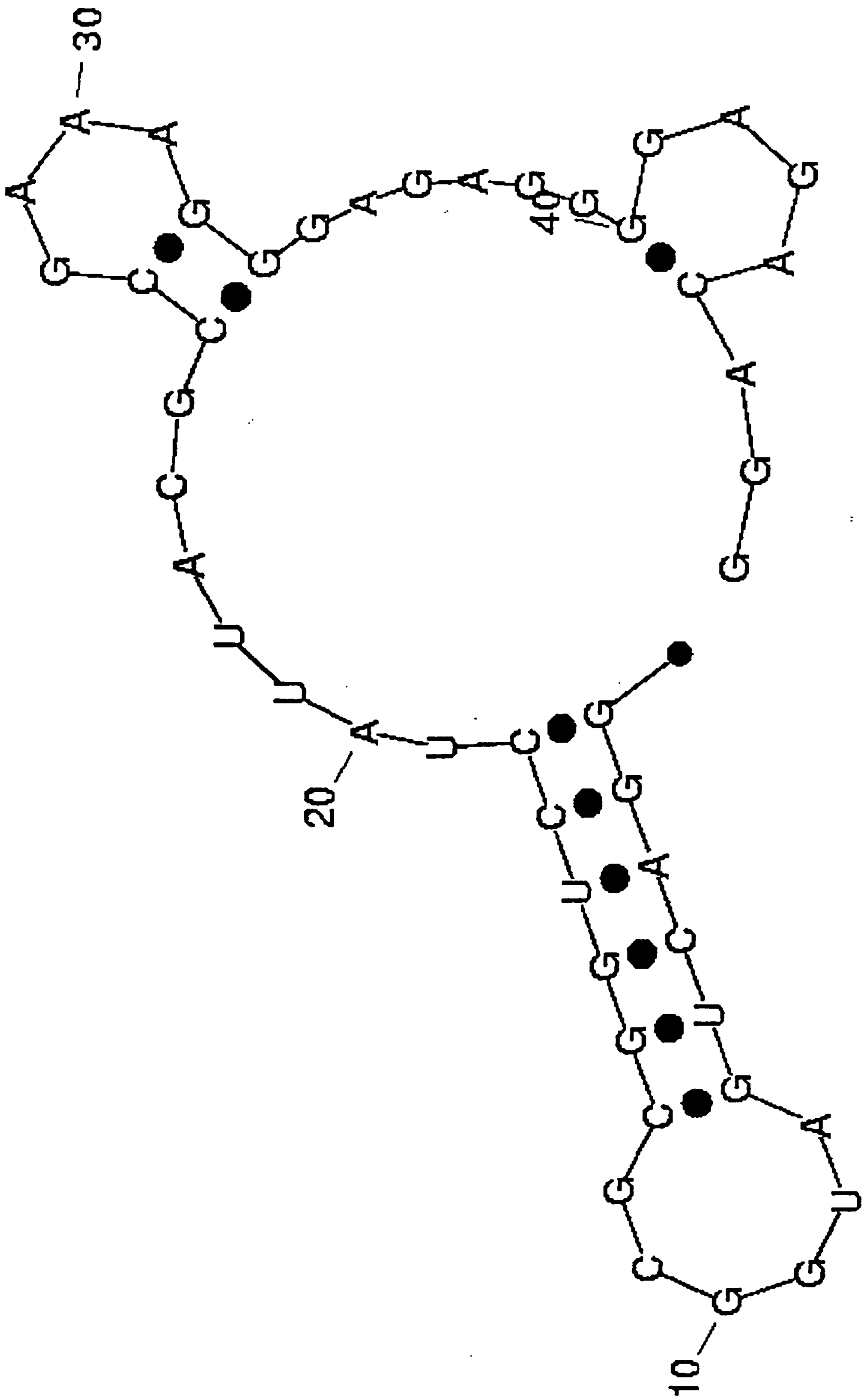


Fig. 52

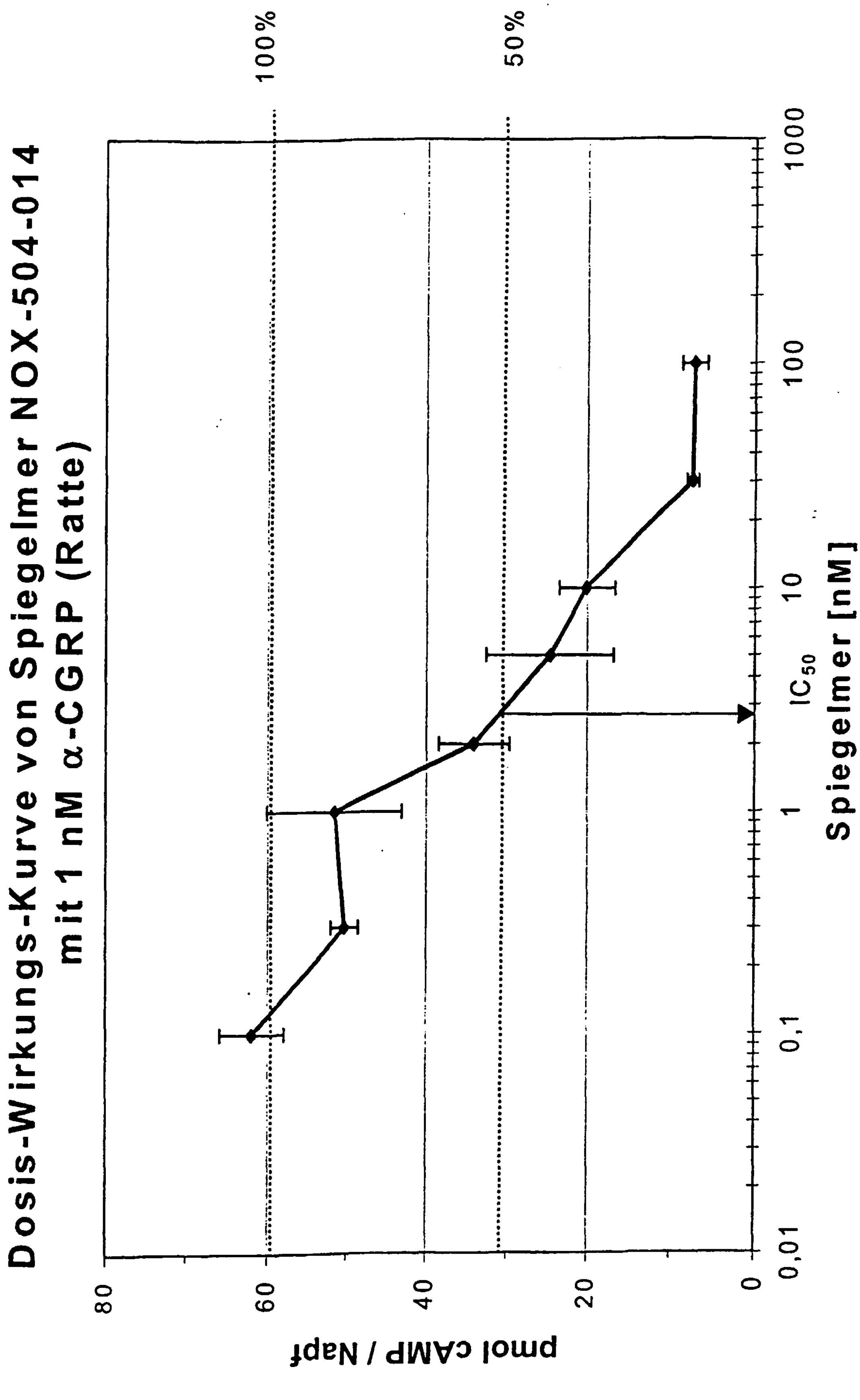
Dosis-Wirkungs-Kurve von Spiegelmer NOX-504-014
mit 1 nM α-CGRP (Ratte)
100%
50%
pmol cAMP / Napf
80
60
40
20
0
0,01
0,1
1
10
100
1000
$IC_{50}$
Spiegelmer [nM]

Fig. 53

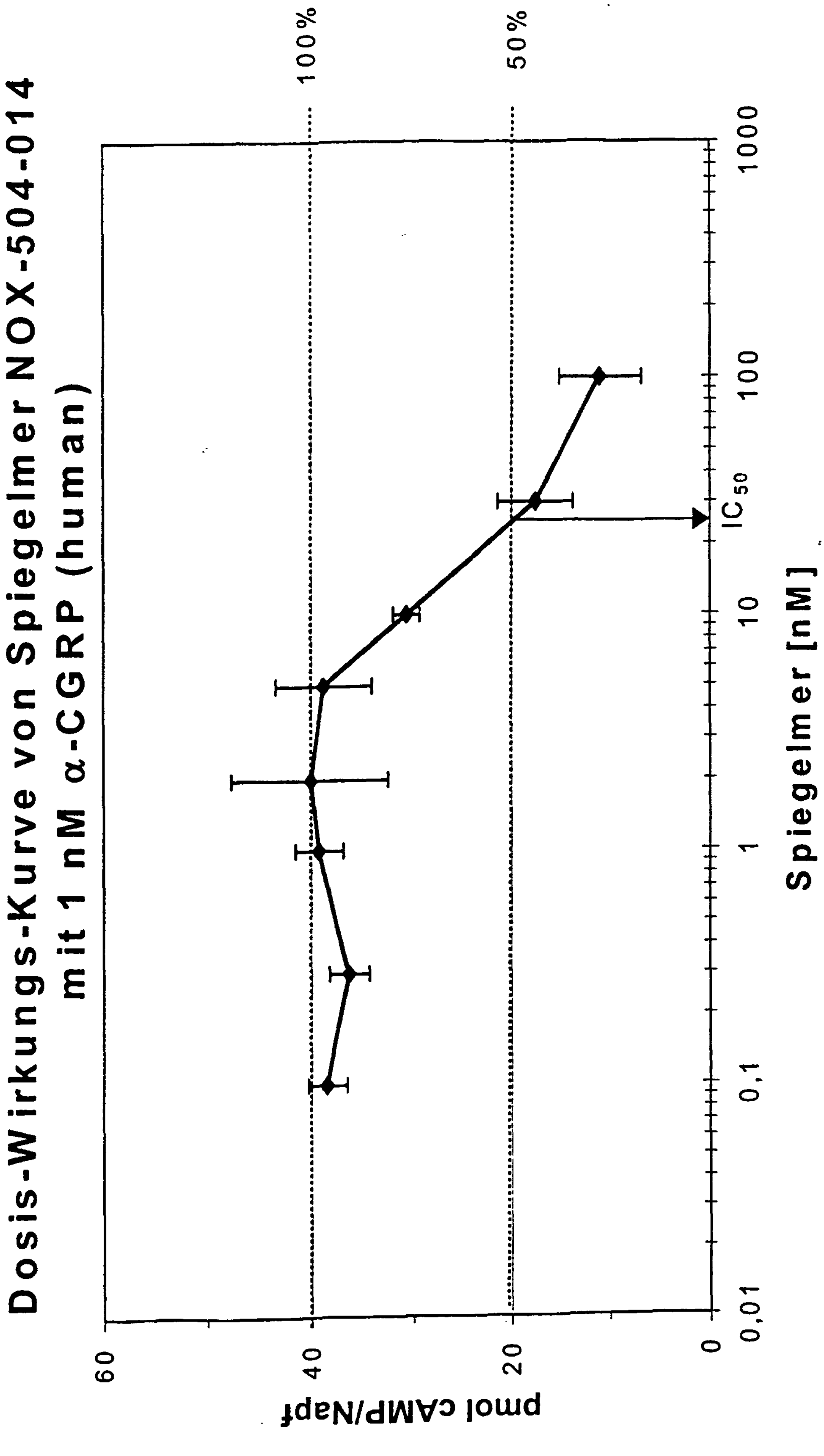
Dosis-Wirkungs-Kurve von Spiegelmer NOX-504-014
mit 1 nM α-CGRP (human)
100%
50%
pmol cAMP/Napf
60
40
20
0
0,01
0,1
1
10
100
1000
IC 50
Spiegelmer [nM]

Fig. 54

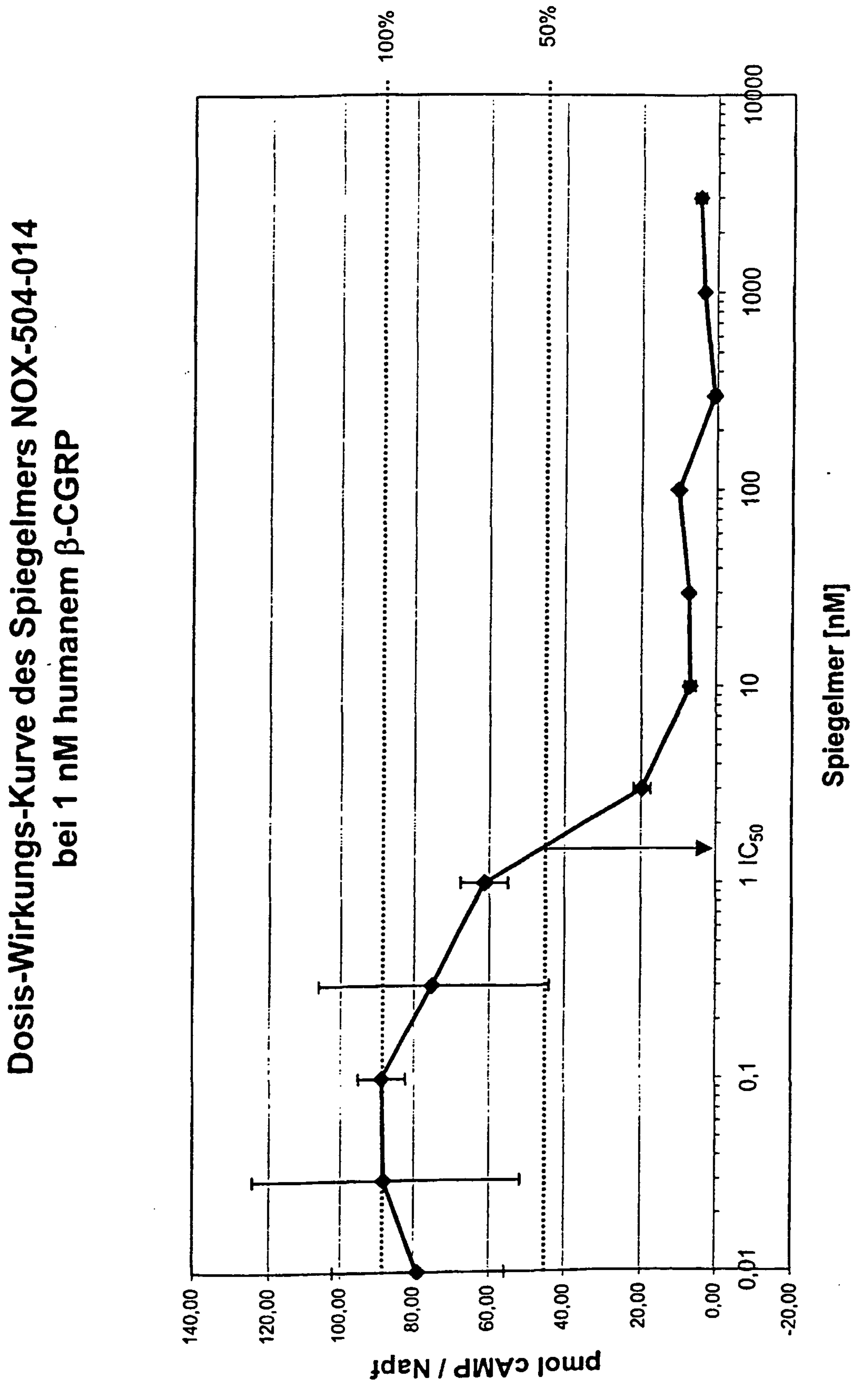
Dosis-Wirkungs-Kurve des Spiegelmers NOX-504-014
bei 1 nM humanem β-CGRP
100%
50%
140,00
120,00
100,00
80,00
60,00
40,00
20,00
0,00
-20,00
pmol cAMP / Napf
0,01
0,1
1
IC50
10
100
1000
10000
Spiegelmer [nM]

Fig. 55

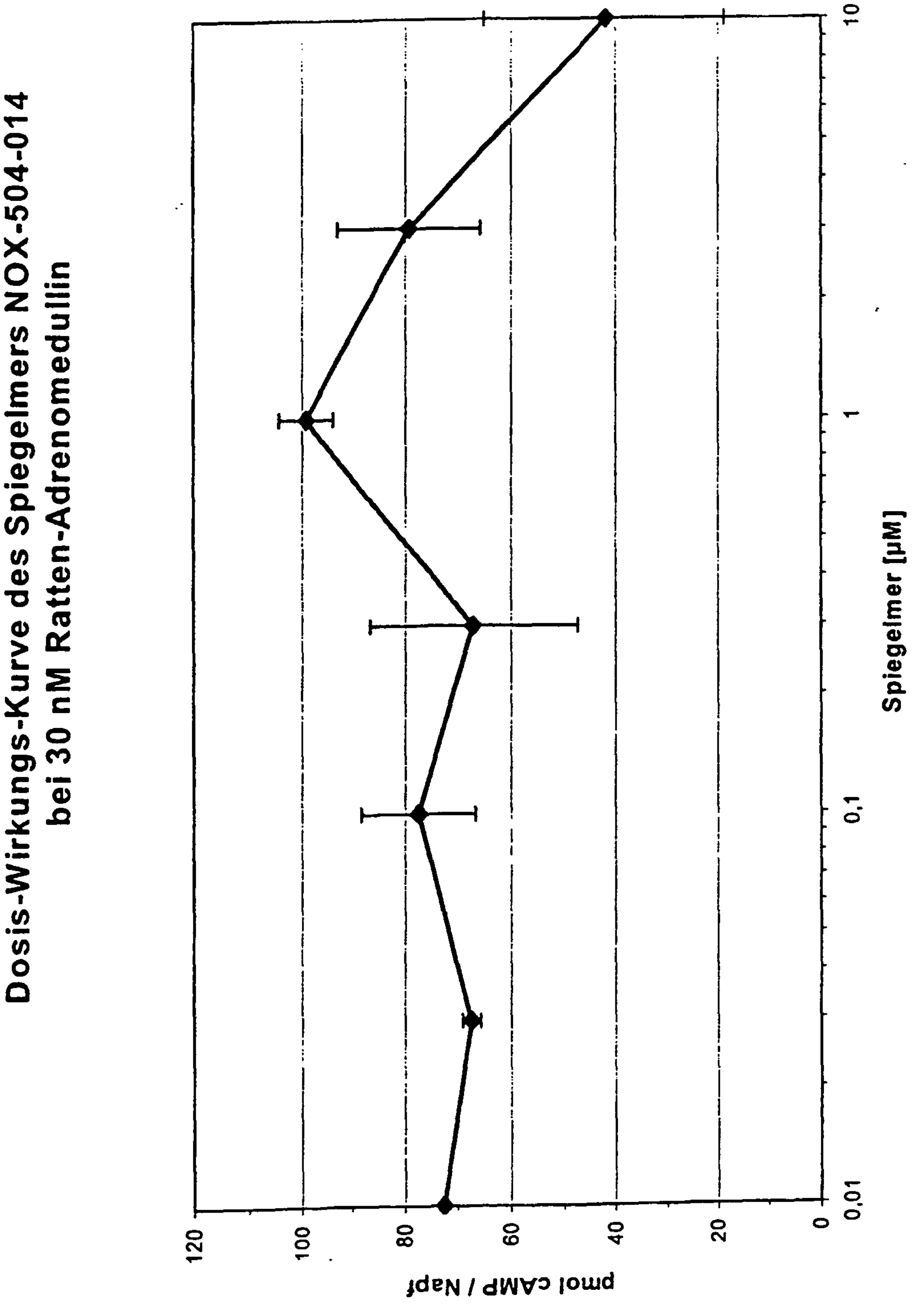

Dosis-Wirkungs-Kurve des Spiegelmers NOX-504-014
bei 30 nM Ratten-Adrenomedullin
pmol cAMP / Napf
120
100
80
60
40
20
0
0,01
0,1
1
10
Spiegelmer [µM]


Fig. 56

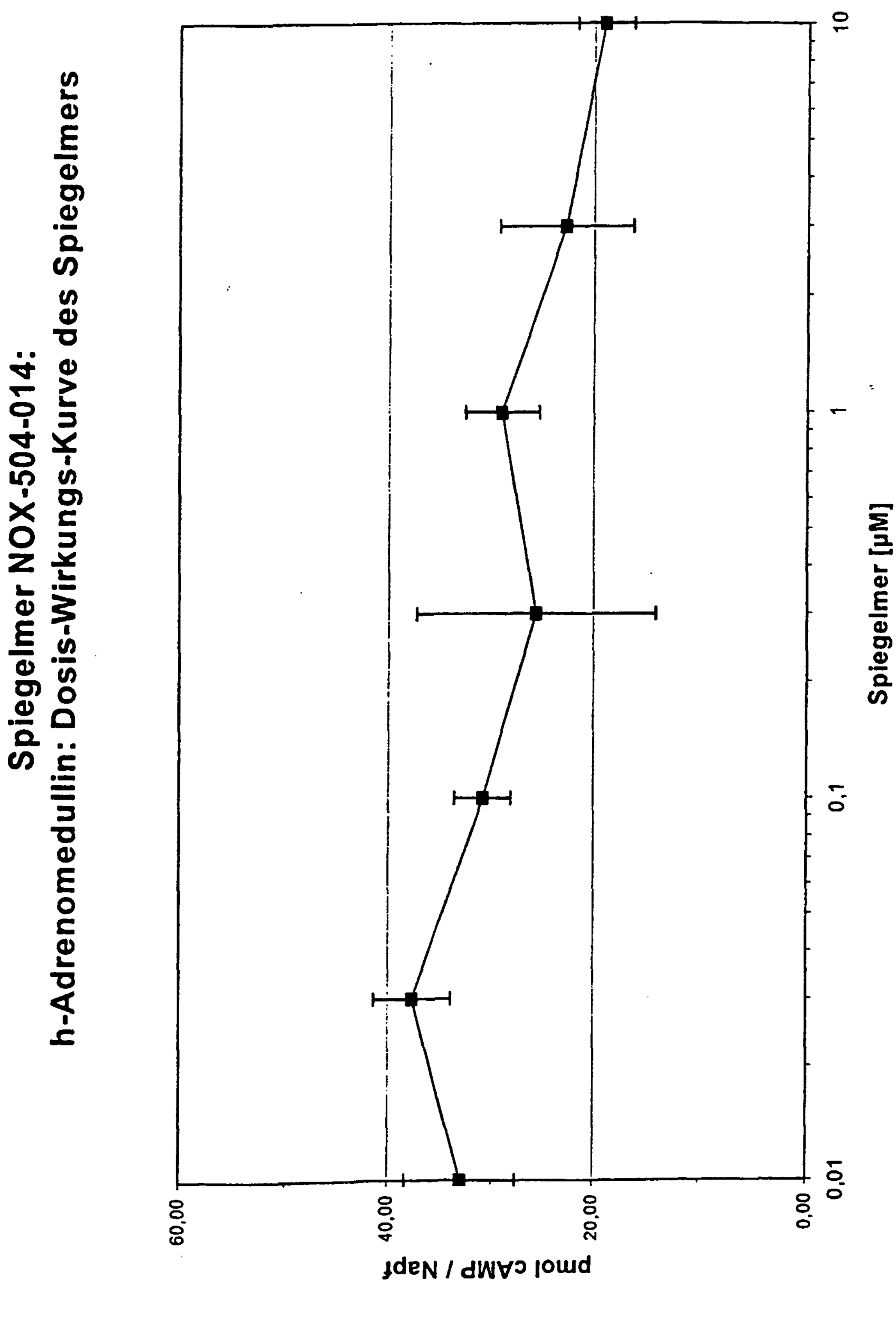
Spiegelmer NOX-504-014:
h-Adrenomedullin: Dosis-Wirkungs-Kurve des Spiegelmers
pmol cAMP / Napf
60,00
40,00
20,00
0,00
0,01
0,1
1
10
Spiegelmer [µM]

Fig. 57

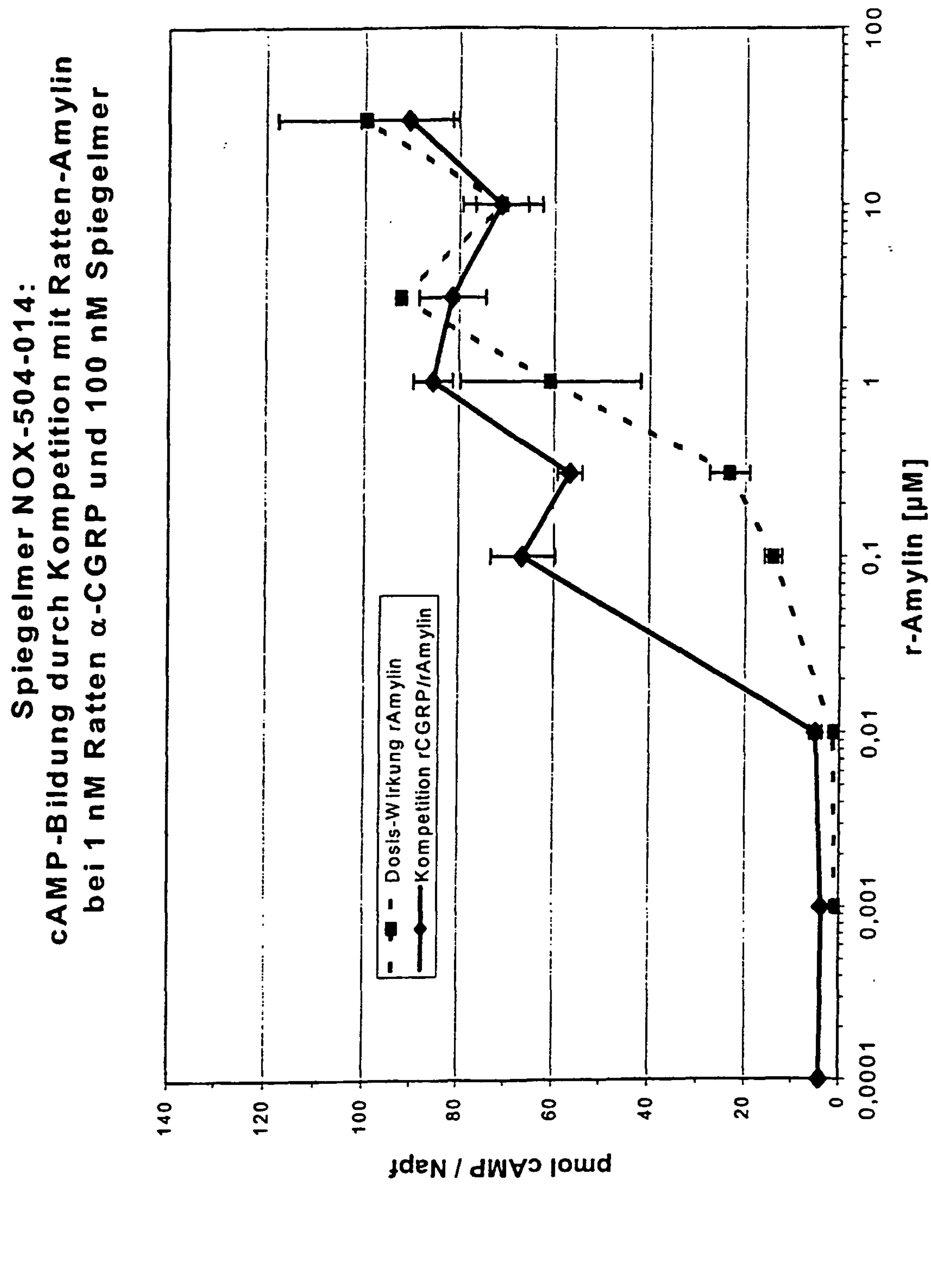
Spiegelmer NOX-504-014:
cAMP-Bildung durch Kompetition mit Ratten-Amylin
bei 1 nM Ratten α-CGRP und 100 nM Spiegelmer
Dosis-Wirkung rAmylin
Kompetition rCGRP/rAmylin
pmol cAMP / Napf
r-Amylin [µM]
0
20
40
60
80
100
120
140
0,0001
0,001
0,01
0,1
1
10
100

Fig. 58

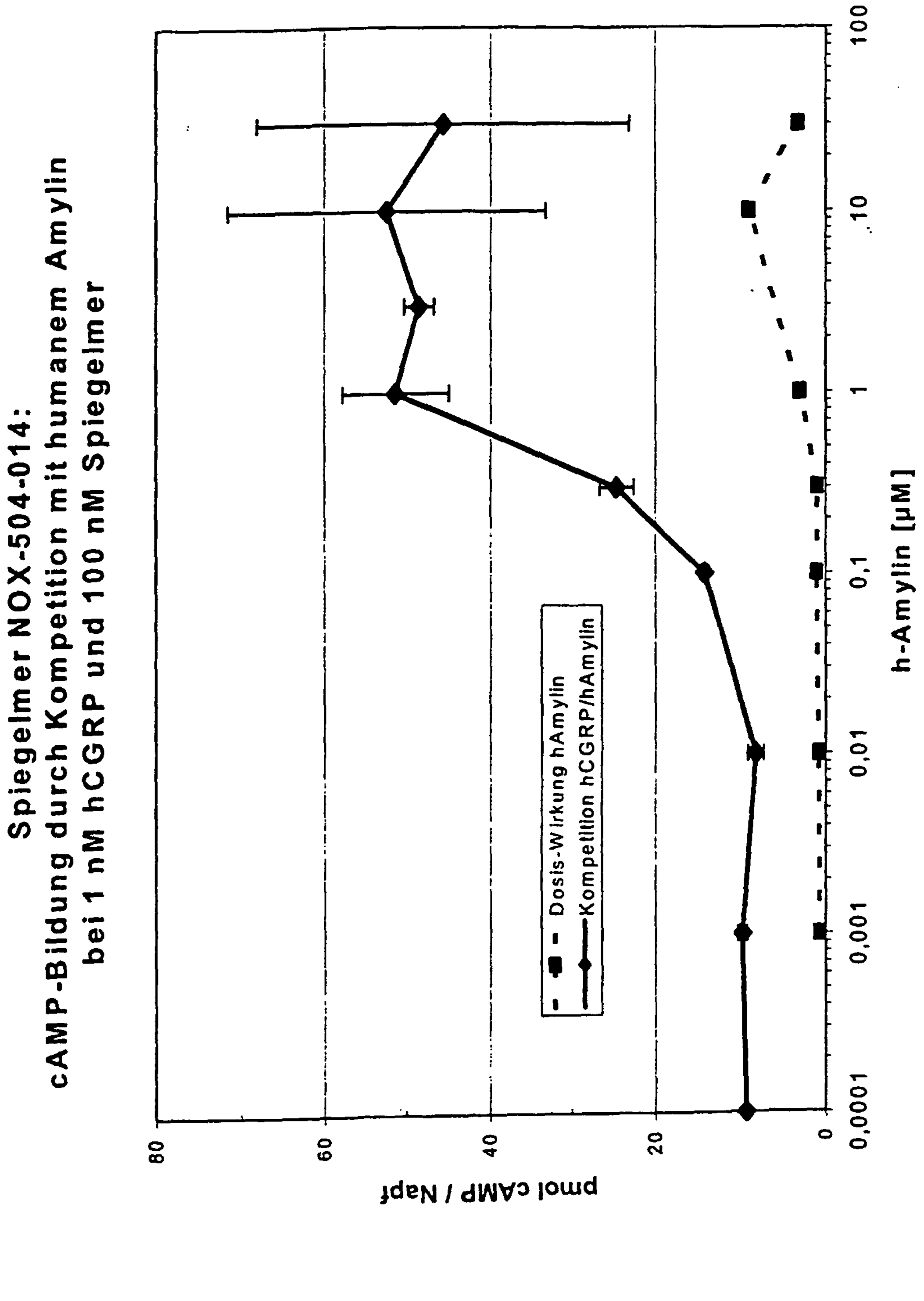
Spiegelmer NOX-504-014:
cAMP-Bildung durch Kompetition mit humanem Amylin
bei 1 nM hCGRP und 100 nM Spiegelmer
Dosis-Wirkung hAmylin
Kompetition hCGRP/hAmylin
pmol cAMP / Napf
0
20
40
60
80
0,0001
0,001
0,01
0,1
1
10
100
h-Amylin [µM]

Fig. 59

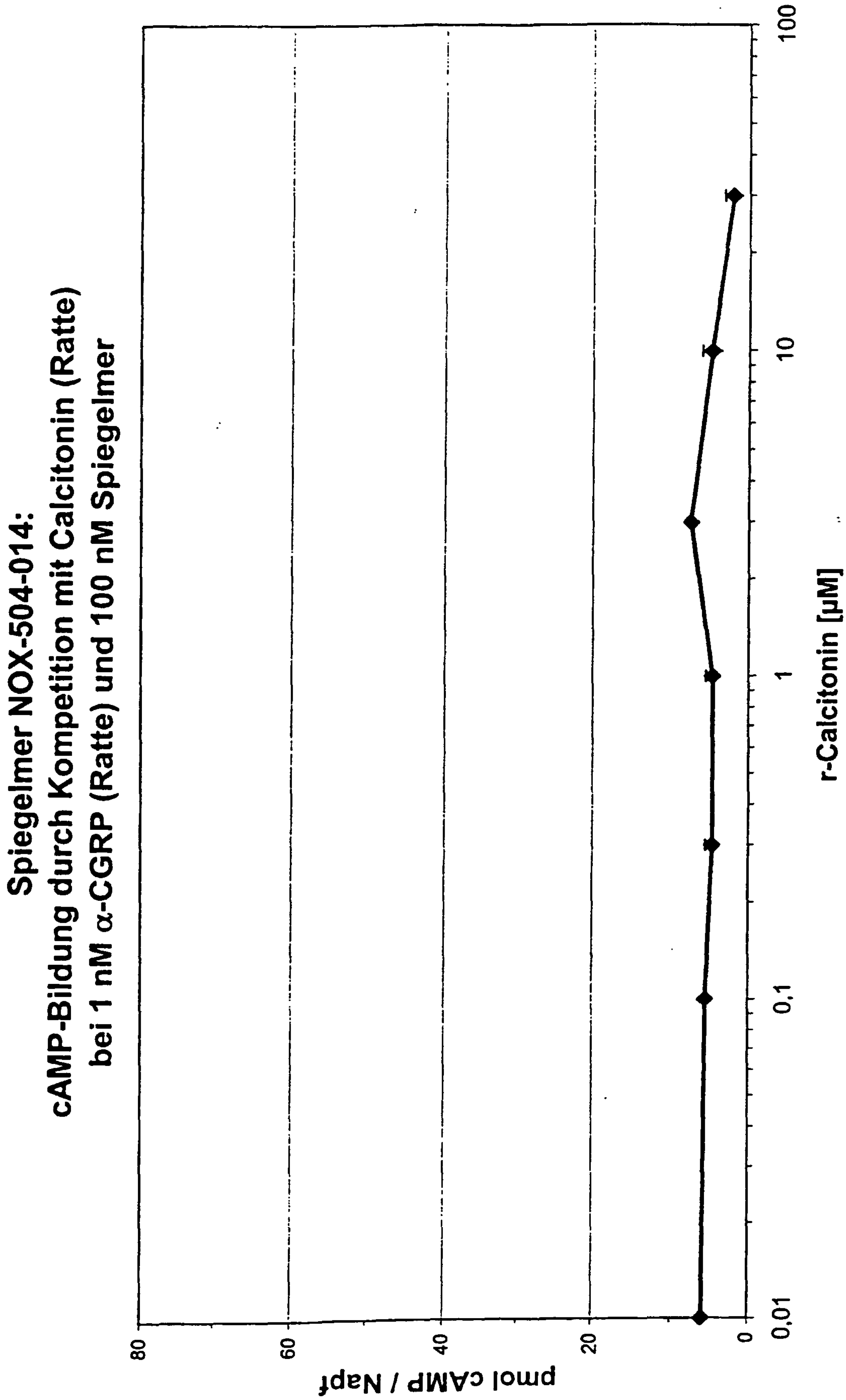
Spiegelmer NOX-504-014:
cAMP-Bildung durch Kompetition mit Calcitonin (Ratte)
bei 1 nM α-CGRP (Ratte) und 100 nM Spiegelmer
pmol cAMP / Napf
80
60
40
20
0
0,01
0,1
1
10
100
r-Calcitonin [µM]

Fig. 60

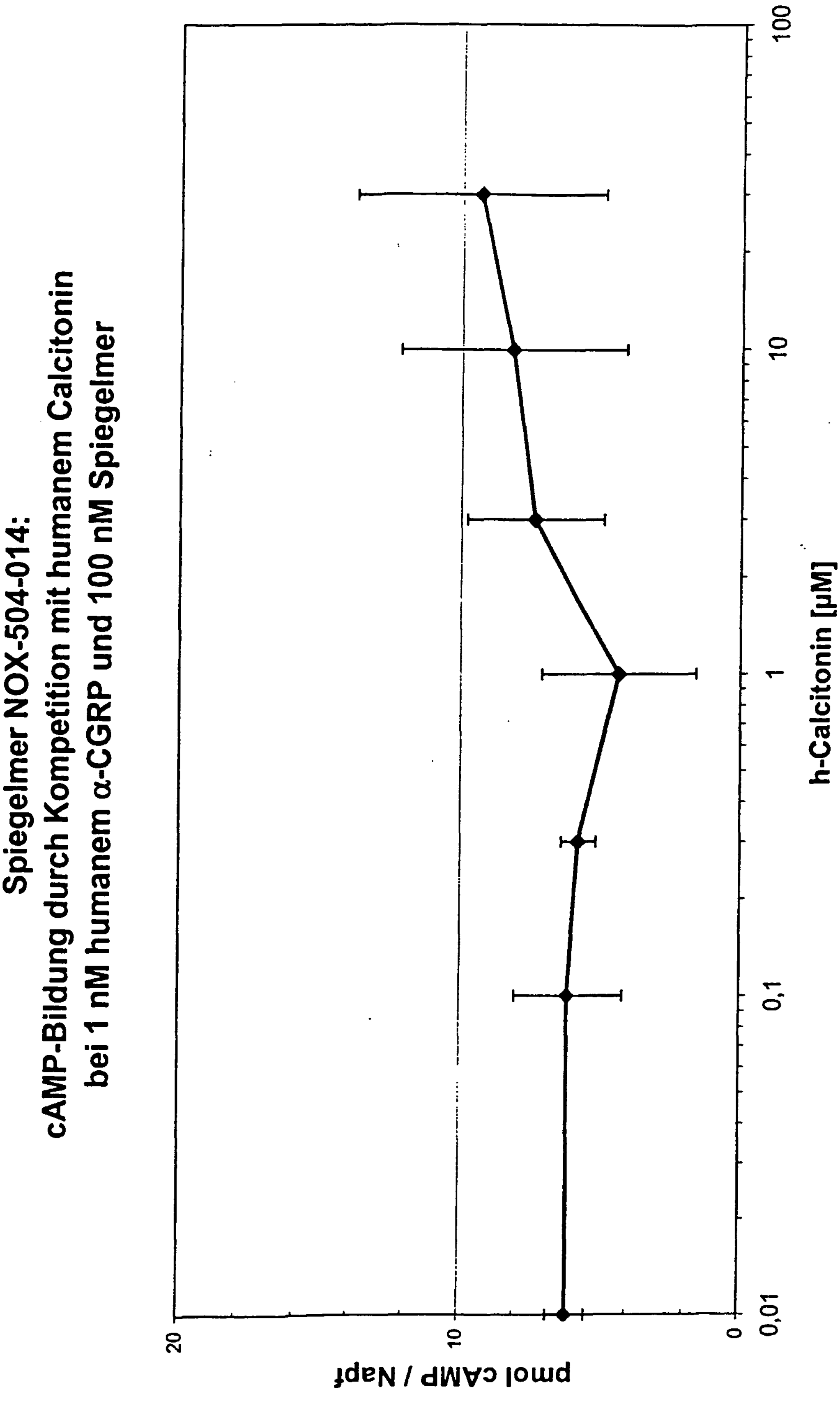
Spiegelmer NOX-504-014:
cAMP-Bildung durch Kompetition mit humanem Calcitonin
bei 1 nM humanem α-CGRP und 100 nM Spiegelmer
pmol cAMP / Napf
20
10
0
0,01
0,1
1
10
100
h-Calcitonin [µM]

Fig. 61

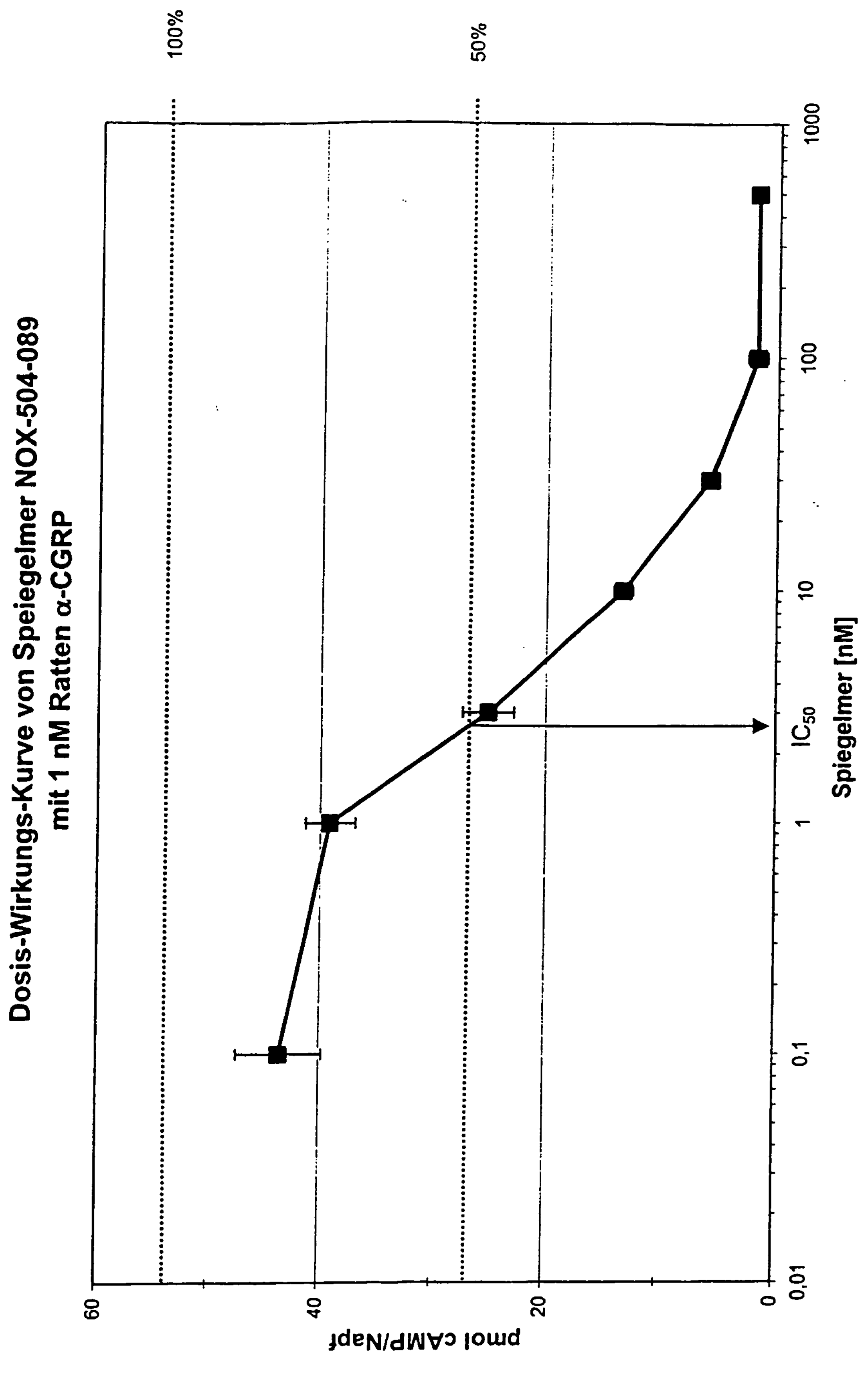
Dosis-Wirkungs-Kurve von Speiegelmer NOX-504-089
mit 1 nM Ratten α-CGRP
100%
50%
pmol cAMP/Napf
60
40
20
0
0,01
0,1
1
10
100
1000
$IC_{50}$
Spiegelmer [nM]

Fig. 62

NOX-504

GGACUGAUGGCGCGGUCCUAUUACGCCGA-AAGGGAGAGGGG-AGACAGG

NOX-504-ad3

GGACUGAUGGCGCGGUCCUAUUACGCCGA-AAGGGAGAGGGG-AGCACGG

Grt2-STAR-504-5-B0.1-C10

GGACUGAUGGCGCGGUCCUAUUACGCCGAGAAGGGAGUGGGGGAGCACGG

Fig. 63

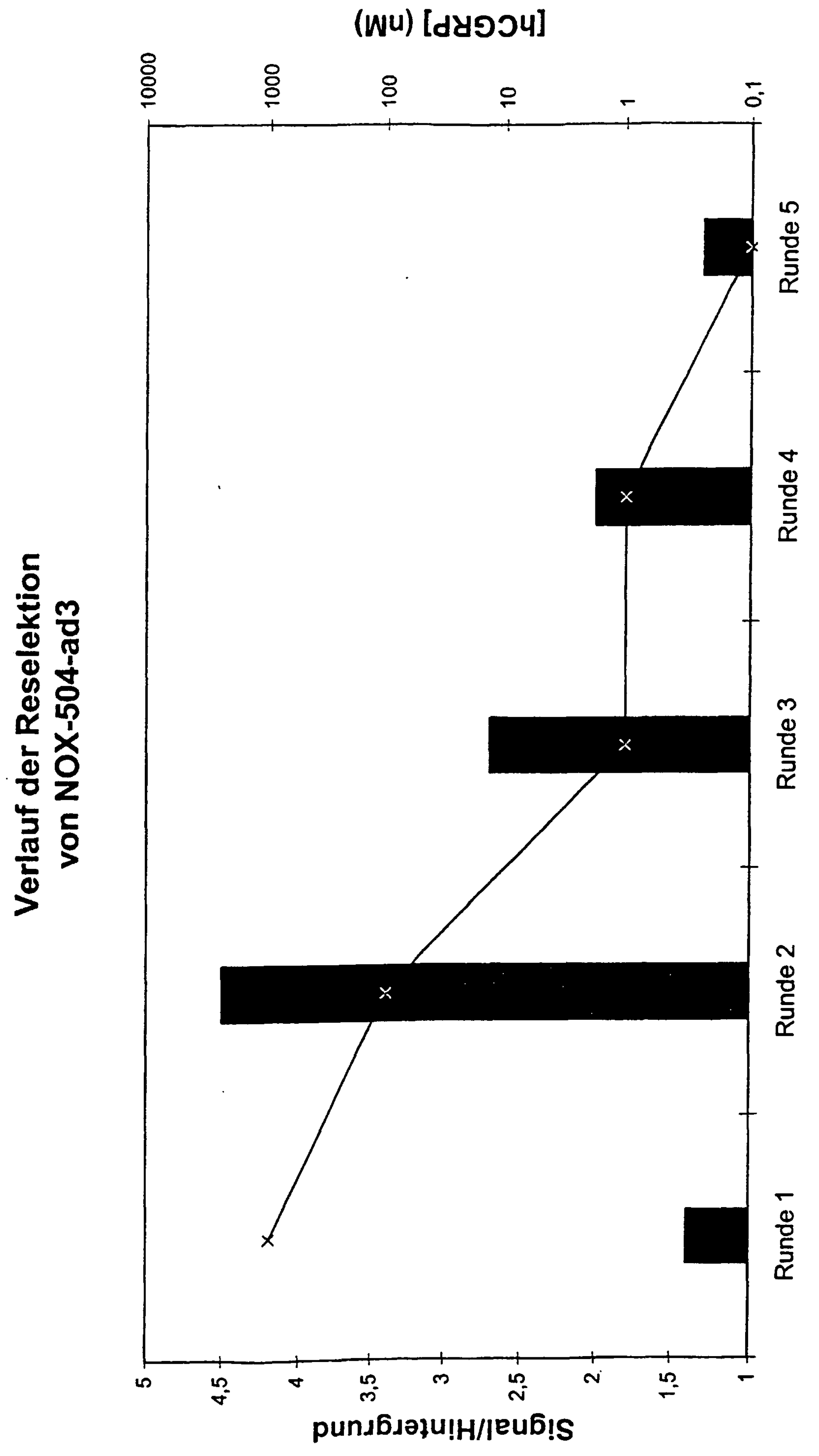
Verlauf der Reselektion
von NOX-504-ad3
[hCGRP] (nM)
10000
1000
100
10
1
0,1
Signal/Hintergrund
5
4,5
4
3,5
3
2,5
2
1,5
1
Runde 1
Runde 2
Runde 3
Runde 4
Runde 5

Fig. 64

| | |
|---|---|
| L097 | GGACUGAUGGCGCGGUCCUAUUACGCCGAGAAGGAGUGGGGGA |
| L102 | GGACUGAUGGCGCGGUCCUAUUACGCCGAGAAGGAGAGGGG-A |
| L103 | GGACUGAUGGCGCGGUCCUAUUACGCCGA-AAGGAGAGGGGGA |
| L104 | GGACUGAUGGCGCGGUCCUAUUACGCCGA-AAGGAGUGGGG-A |
| L105 | GGACUGAUGGCGCGGUCCUAUUACGCCGA-AAGGAGUGGGGGA |
| L106 | GGACUGAUGGCGCGGUCCUAUUACGCCGAGAAGGAGUGGGG-A |
| L107 | GGACUGAUGGCGCGGUCCUAUUACGCCGAGAAGGAGAGGGGGA |
| L108 | GGACUGAUGGCGCGGUCCUAUUACGCCGAGUAGGAGUGGGGGA |
| L109 | GGACUGAUGGCGCGGUCCUAUUACGCCGAGAAGGUGUGGGGGA |

Fig. 65

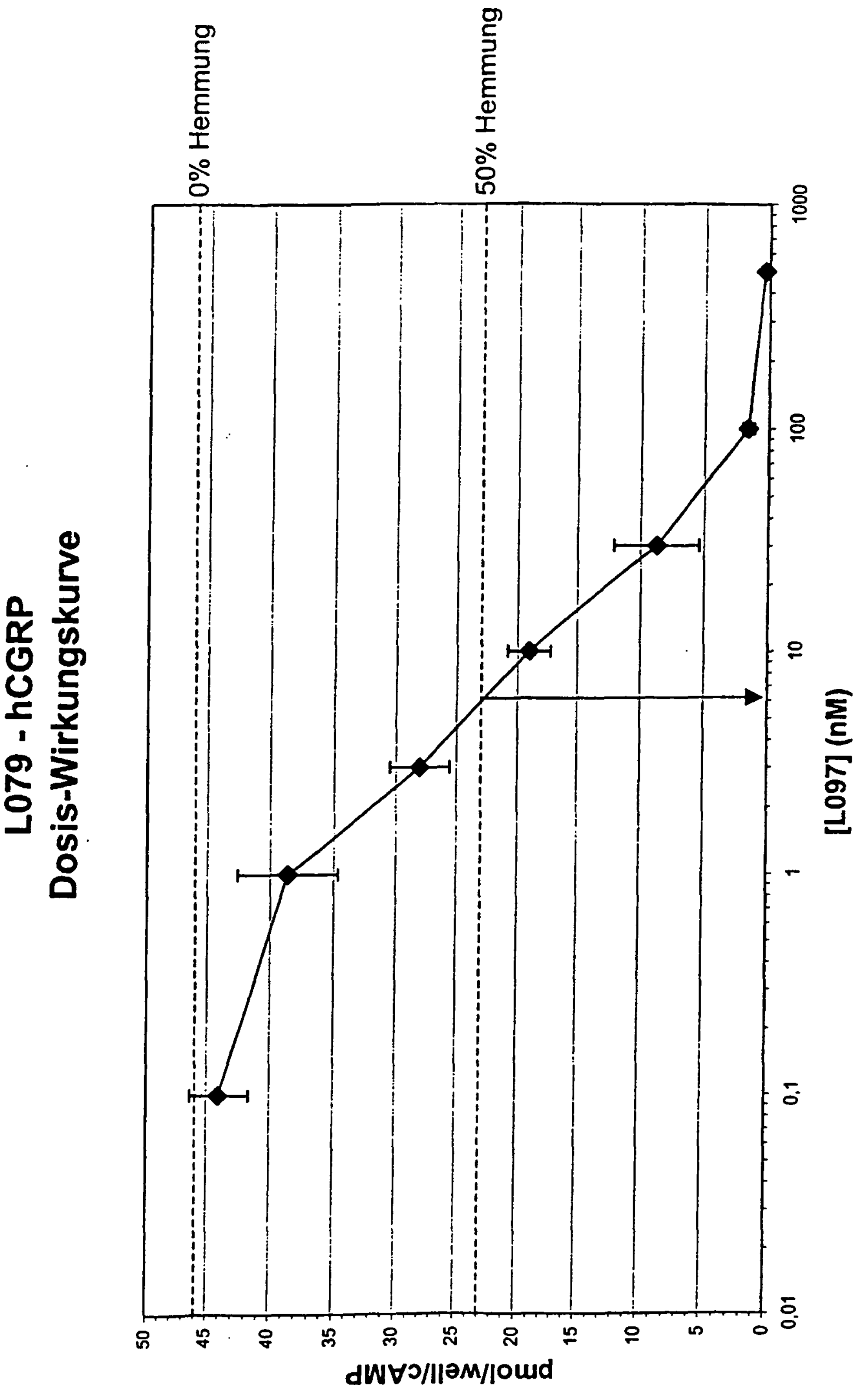
L079 - hCGRP
Dosis-Wirkungskurve
0% Hemmung
50% Hemmung
pmol/well/cAMP
50
45
40
35
30
25
20
15
10
5
0
0,01
0,1
1
10
100
1000
[L097] (nM)

Fig. 66A

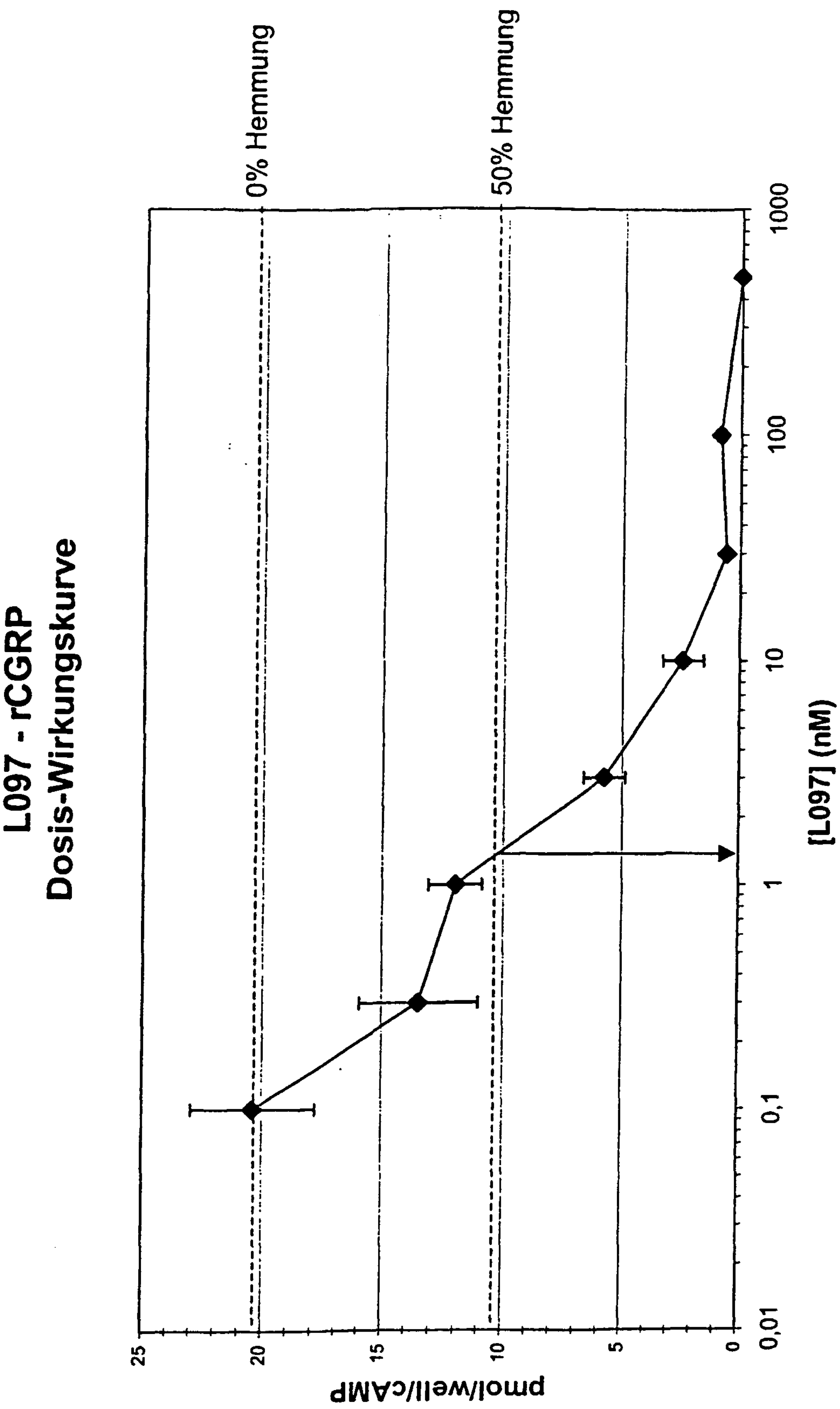
L097 - rCGRP
Dosis-Wirkungskurve
0% Hemmung
50% Hemmung
pmol/well/cAMP
25
20
15
10
5
0
0,01
0,1
1
10
100
1000
[L097] (nM)

Fig. 66B

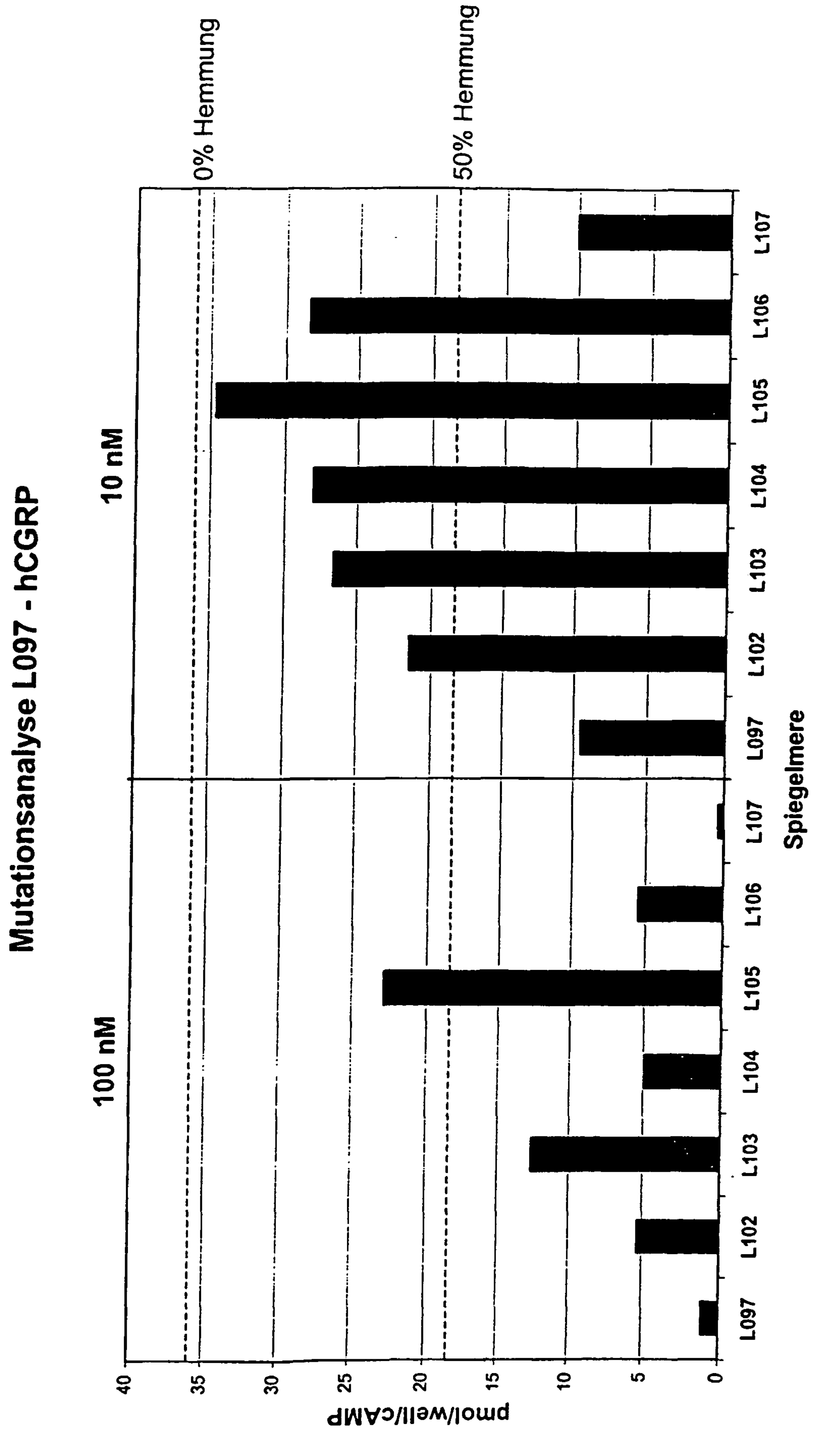
Mutationsanalyse L097 - hCGRP
100 nM
10 nM
0% Hemmung
50% Hemmung
pmol/well/cAMP
40
35
30
25
20
15
10
5
0
L097
L102
L103
L104
L105
L106
L107
L097
L102
L103
L104
L105
L106
L107
Spiegelmere

Fig. 67

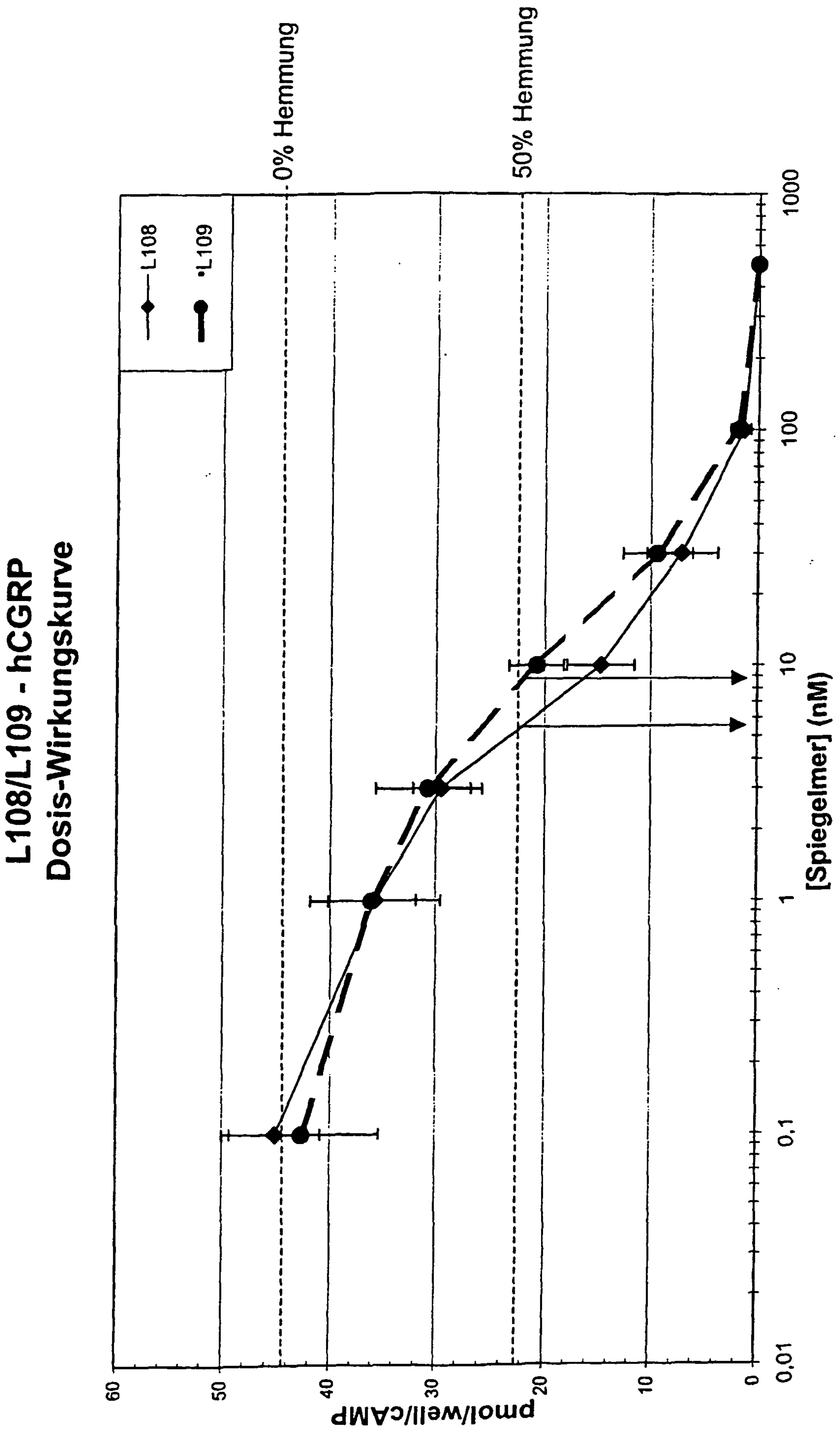
L108/L109 - hCGRP
Dosis-Wirkungskurve
0% Hemmung
50% Hemmung
L108
L109
60
50
40
30
20
10
0
pmol/well/cAMP
0,01
0,1
1
10
100
1000
[Spiegelmer] (nM)

Fig. 68

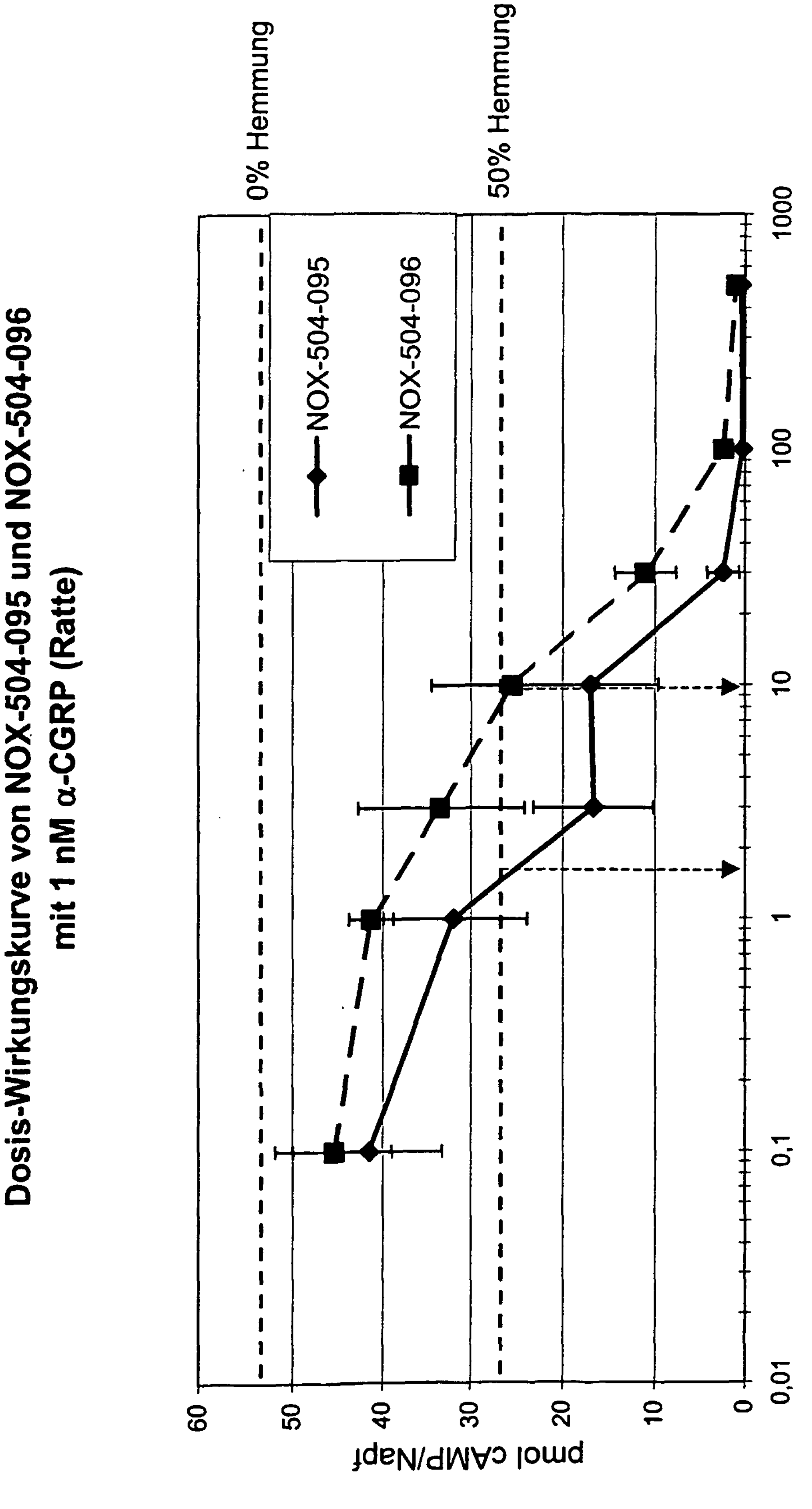
Dosis-Wirkungskurve von NOX-504-095 und NOX-504-096
mit 1 nM α-CGRP (Ratte)
0% Hemmung
50% Hemmung
NOX-504-095
NOX-504-096
pmol cAMP/Napf
60
50
40
30
20
10
0
0,01
0,1
1
10
100
1000
[Spiegelmer] (nM)

Figur 69

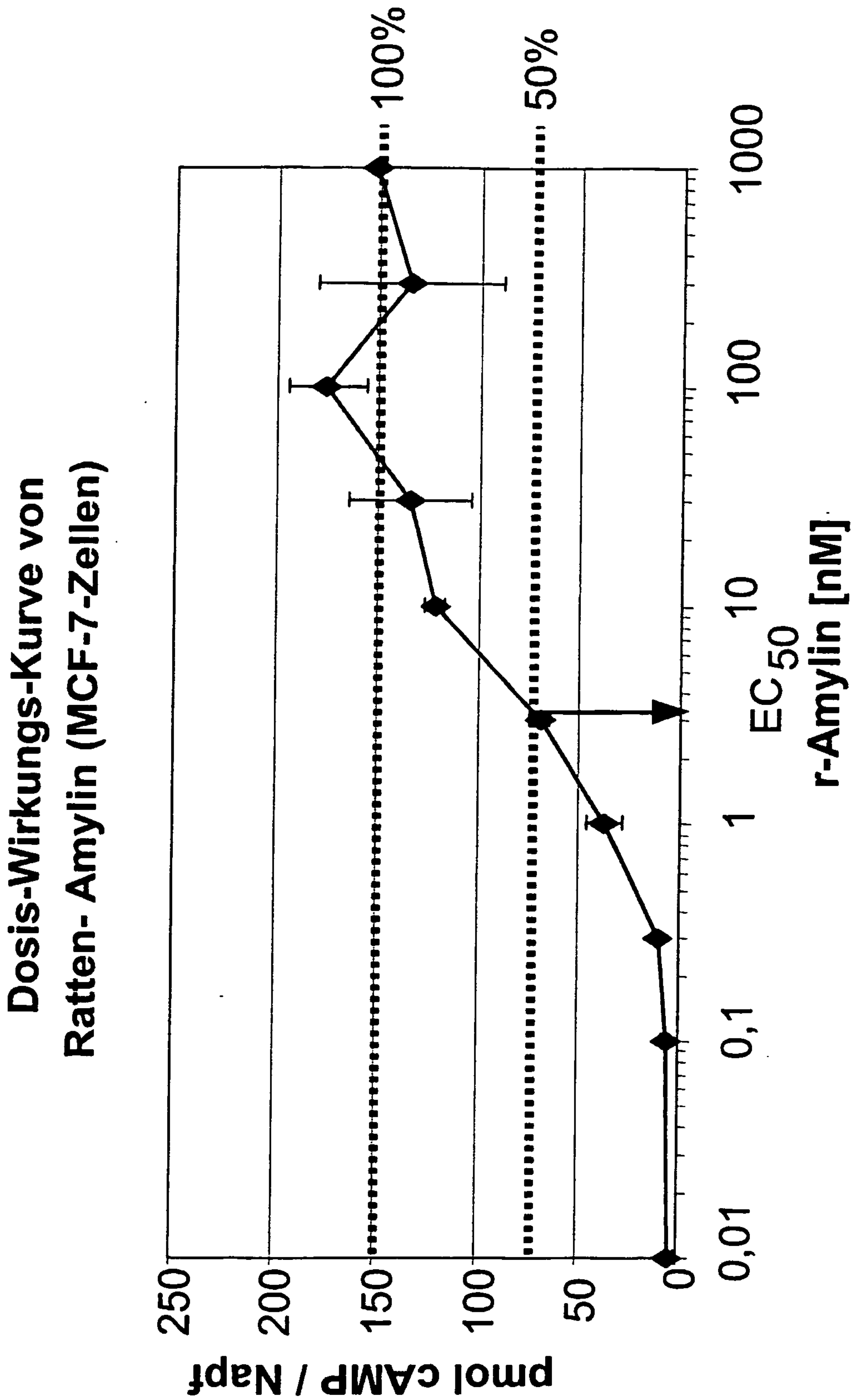
Dosis-Wirkungs-Kurve von
Ratten- Amylin (MCF-7-Zellen)
100%
50%
pmol cAMP / Napf
250
200
150
100
50
0
0,01
0,1
1
10
100
1000
$EC_{50}$
r-Amylin [nM]

Fig. 70

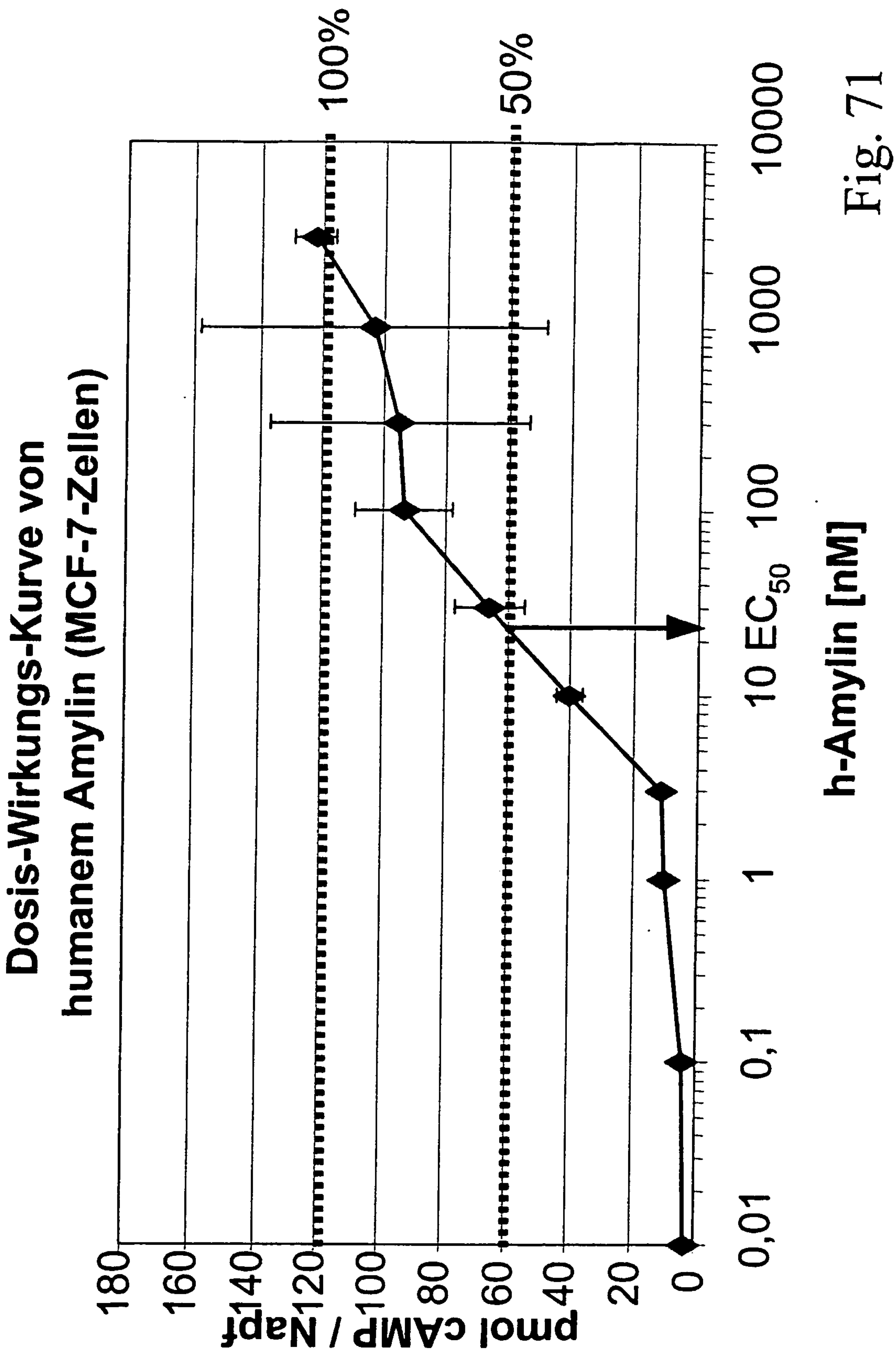

Dosis-Wirkungs-Kurve von
humanem Amylin (MCF-7-Zellen)
pmol cAMP / Napf
180
160
140
120
100
80
60
40
20
0
100%
50%
0,01
0,1
1
10 $EC_{50}$
100
1000
10000
h-Amylin [nM]


Fig. 71

Fig. 72

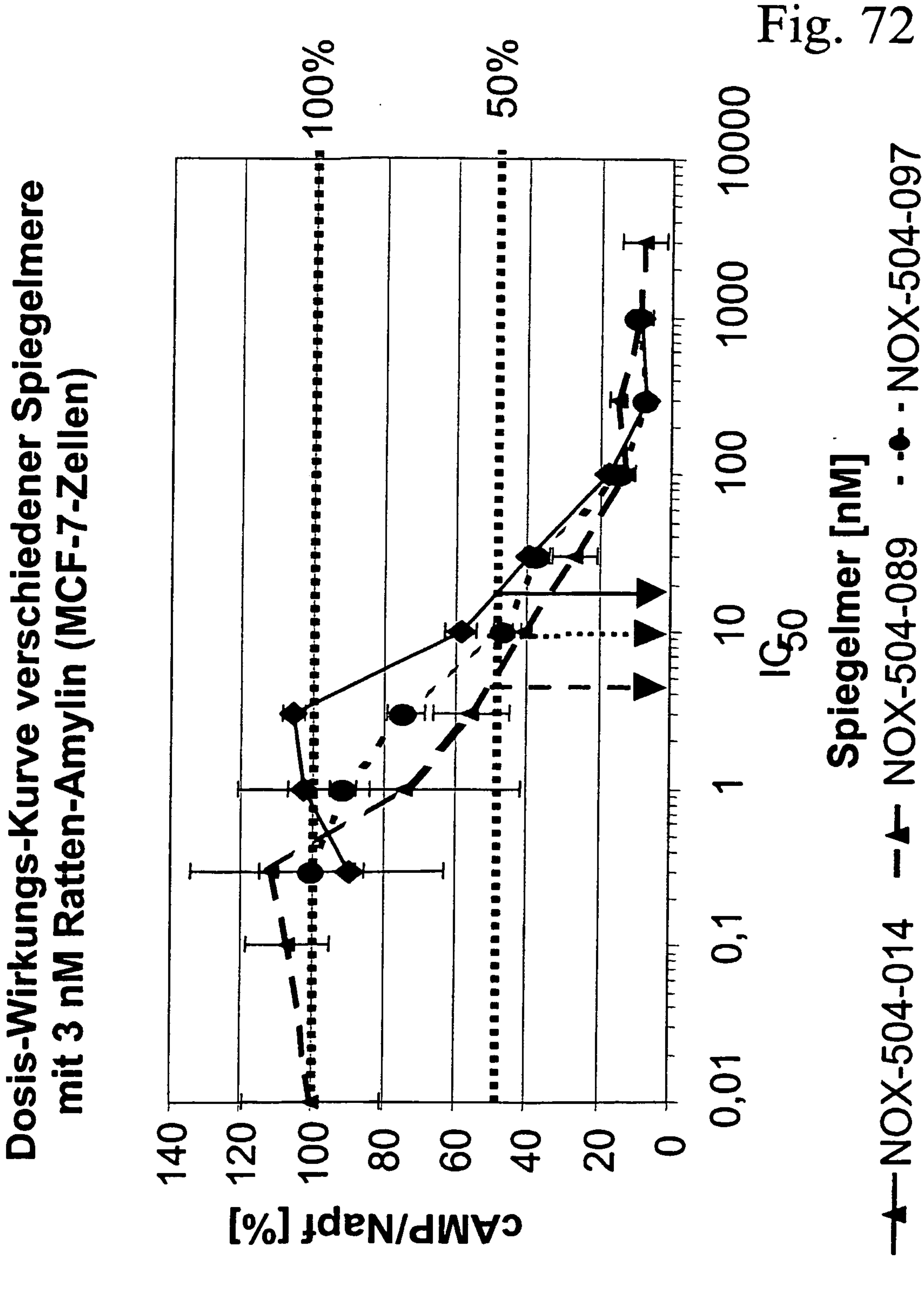
Dosis-Wirkungs-Kurve verschiedener Spiegelmere
mit 3 nM Ratten-Amylin (MCF-7-Zellen)
cAMP/Napf [%]
140
120
100
80
60
40
20
0
0,01
0,1
1
10
100
1000
10000
100%
50%
IC50
Spiegelmer [nM]
NOX-504-014
NOX-504-089
NOX-504-097

Fig. 73

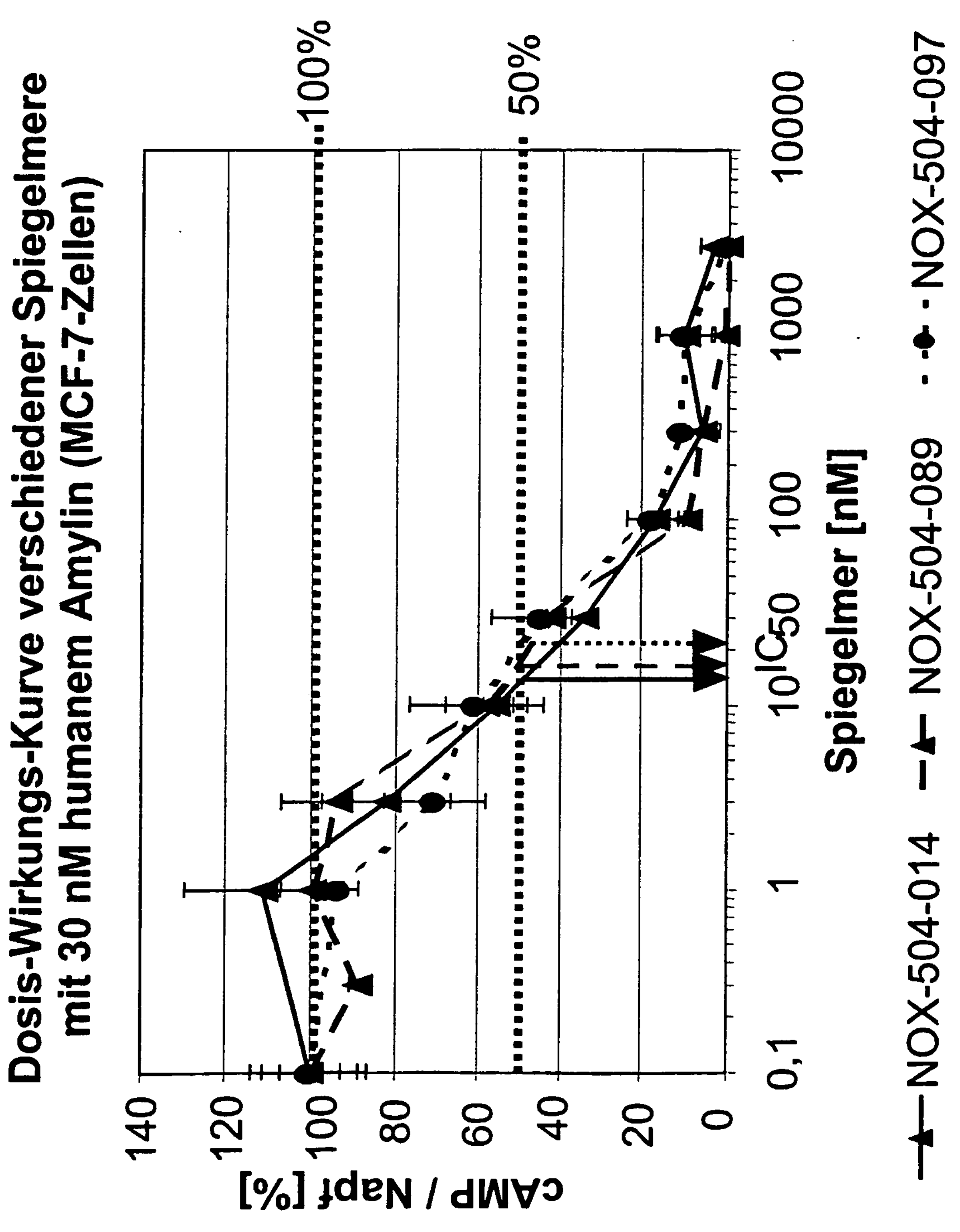
Dosis-Wirkungs-Kurve verschiedener Spiegelmere
mit 30 nM humanem Amylin (MCF-7-Zellen)
cAMP / Napf [%]
140
120
100
80
60
40
20
0
100%
50%
IC50
0,1
1
10
100
1000
10000
Spiegelmer [nM]
NOX-504-014
NOX-504-089
NOX-504-097

ITC von Spiegelmer NOX-504-L097 und hCGRP bei 37°C

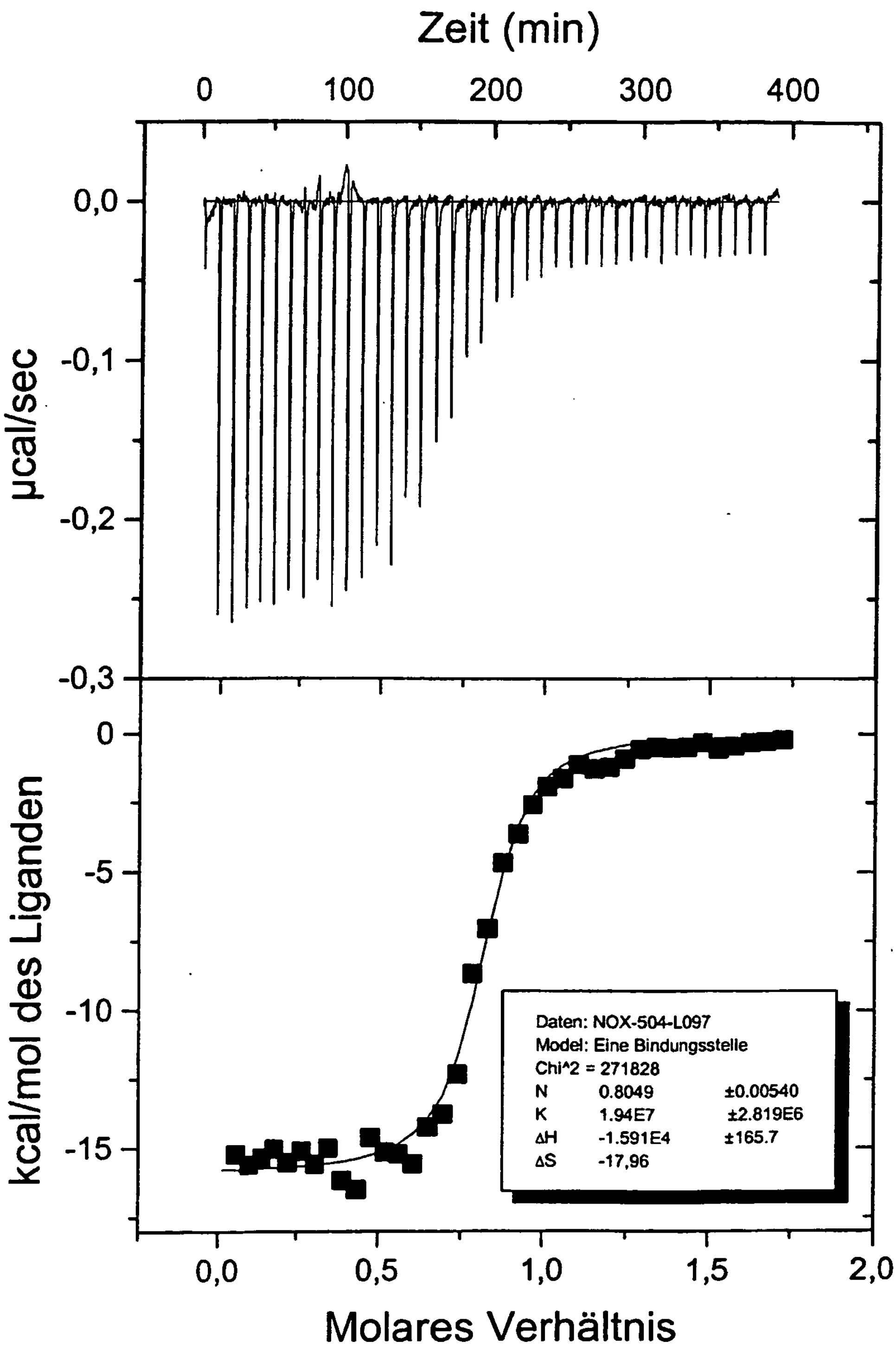


Fig. 74

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9709046 A
- DE 19911039
- WO 0132648 A
- WO 0132649 A
- WO 9809630 A
- WO 9856779 A


### In der Beschreibung aufgeführte Nicht-Patentliteratur


- **WILLIS, T.** *Cerebri anatome*
- **RAY, B.S. et al.** *Arch. Surg.,* 1940, vol. 41, 813-856
- **DOODS, H.** *Current opinion in investigational Drugs,* 2001, vol. 2 (9), 1261-1268
- **HARGREAVES, R.J. et al.** *Can. J. Neurol. Sci.,* 1999, vol. 26, 512-519
- **KUROSAWA, M. et al.** *Br. J. Pharmacol.,* 1995, vol. 114, 1397-1402
- **GOADSBY, P. et al.** *Ann. Neurol.,* 1993, vol. 33, 48-56
- **EDVINSSON, L. et al.** *Cephalalgia,* 1994, vol. 14, 320-327
- **TEPPER, S.J. et al.** *CNS Drugs,* 1999, vol. 12, 403-417
- **BROWN, E.G. et al.** *Eur. Neurol.,* vol. 31, 339-344
- **OTTERVANGER, J.P. et al.** *Lancet,* 1993, vol. 341, 861-862
- **KELLY, K.M.** *Neurology,* 1995, vol. 45, 1211-1213
- **DE HOON, J.N.J.M. et al.** *Clin. Pharmacol. Ther.,* 2000, vol. 68, 418-426
- **MOSKOWITZ, M.A et al.** *Brain Metab. Rev.,* 1993, vol. 5, 159-177
- **HUMPHREY, P.P.A et al.** *Trends Pharmacol. Sci.,* 1991, vol. 12, 444-446
- **EDVINSSON, L.** *Pharmacology & Toxicology,* 2001, vol. 89, 65-73
- **AMARA et al.** *Nature,* 1982, vol. 298, 240-244
- **AMARA, G.S. et al.** *Nature,* 1982, vol. 298, 240-244
- **BREEZE, A.L. et al.** *Biochemistry,* 1991, vol. 30, 575-582
- **DOODS, H. et al.** *Br. J. Pharmacol.,* 2000, vol. 129, 420-423
- **HAKALA J.M.L. ; VIHINEN M.** *Protein Engineering,* 1994, vol. 7 (9), 1069-1075
- **WIESENFELD-HALLIN, Z. et al.** *Neurosci. Lett.,* 1984, vol. 52, 199-203
- **SAXEN, M.A. et al.** *Life Sciences,* 1994, vol. 55, 1665-1674
- **FRIESE, N. et al.** *Regulatory Peptides,* 1997, vol. 70, 1-7
- **PLOURDE, V. et al.** *Am. J. Physiol,* 1997, vol. 36, G191-G196
- **LOFGREN, O. et al.** *Neuropeptides,* 1997, vol. 31, 601-607
- **BENNETT, A.D. et al.** *Pain,* 2000, vol. 86, 163-175
- **KURAISHI, Y. et al.** *Neurosci. Lett.,* 1988, vol. 92, 325-329
- **KAWAMURA, M. et al.** *Brain Res.,* 1989, vol. 497, 199-203
- **SATOH, M. et al.** *Pain,* 1992, vol. 49, 273-278
- **SALMON, A.-M. et al.** *Neuroreport,* 1999, vol. 10, 849-954
- **SALMON, A.-M. et al.** *Nature,* 2001, vol. 56, 357-358
- **ZHANG, L. et al.** *Pain,* 2001, vol. 89, 265-273
- **G. CHRISTOPOULOS et al.** *Mol Pharmacol.,* Juli 1999, vol. 56 (1), 235-42
- **N. TILAKARATNE.** *J Pharmacol Exp Ther,* Juli 2000, vol. 294 (1), 61-72
- **HAYDEN, M. R.** Islet amyloid, metabolic syndrome, and the natural progressive history of type 2 diabetes mellitus. *Jop,* 2002, vol. 3 (5), 126-38
- **COOPER, M. E. ; P. G. MCNALLY et al.** Amylin stimulates plasma renin concentration in humans. *Hypertension,* 1995, vol. 26 (3), 460-4
- **CARLSSON, P. O.** The renin-angiotensin system in the endocrine pancreas. *Jop,* 2001, vol. 2 (1), 26-32
- **WANG, F. ; R. L. HULL et al.** Islet amyloid develops diffusely throughout the pancreas before becoming severe and replacing endocrine cells. *Diabetes,* 2001, vol. 50 (11), 2514-20
- **JAIKARAN, E. T. ; A. CLARK.** Islet amyloid and type 2 diabetes: from molecular misfolding to islet pathophysiology. *Biochim Biophys Acta,* 2001, vol. 1537 (3), 179-203

- **SCROCCHI, L. A. ; Y. CHEN et al.** Design of peptide-based inhibitors of human islet amyloid polypeptide fibrillogenesis. *J Mol Biol,* 2002, vol. 318 (3), 697-706
- **KAPURNIOTU, A. ; A. SCHMAUDER et al.** Structure-based design and study of non-amyloidogenic, double N- methylated IAPP amyloid core sequences as inhibitors of IAPP amyloid formation and cytotoxicity. *J Mol Biol,* 2002, vol. 315 (3), 339-50
- **DE KONING, E. J. ; K. A. FLEMING et al.** High prevalence of pancreatic islet amyloid in patients with endstage renal failure on dialysis treatment. *J Pathol,* 1995, vol. 175 (2), 253-8
- **CHRISTOPOULOS G ; PERRY KJ ; MORFIS M ; TILAKARATNE N ; GAO Y ; FRASER NJ ; MAIN MJ ; FOORD SM ; SEXTON PM.** Multiple amylin receptors arise from receptor activity-modifying protein interaction with the calcitonin receptor gene product. *Mol Pharmacol.,* Juli 1999, vol. 56 (1), 235-42
- **TILAKARATNE N ; CHRISTOPOULOS G ; ZUMPE ET ; FOORD SM ; SEXTON PM.** *J Pharmacol Exp Ther,* Juli 2000, vol. 294 (1), 61-72
- **KUSSER, W.** *J Biotechnol,* 2000, vol. 74, 27-38
- **AURUP, H. et al.** *Nucleic Acids Res,* 1994, vol. 22, 20-4
- **CUMMINS, L.L. et al.** *Nucleic Acids Res,* 1995, vol. 23, 2019-24
- **EATON, B.E. et al.** *Chem Biol,* 1995, vol. 2, 633-8
- **GREEN, L.S. et al.** *Chem Biol,* 1995, vol. 2, 683-95
- **KAWASAKI, A.M. et al.** *J Med Chem,* 1993, vol. 36, 831-41
- **LESNIK, E.A. et al.** *Biochemistry,* 1993, vol. 32, 7832-8
- **MILLER, L.E. et al.** *J Physiol,* 1993, vol. 469, 213-43
- **JÖNSSON, U. et al.** *Biotechniques,* 1991, vol. 11 (5), 620
- **HAQ, I. ; LADBURG, J.** *J. Mol. Recognit.,* 2000, vol. 13 (4), 188
- **BURGSTALLER ; FAMULOK.** *Angew. Chem. Int. Ed.,* 1994, vol. 33, 1084
- **I.L. HOFACKER et al.** *Monatsh. Chem,* 1994, vol. 125, 167-188
- **EHRESMANN, C. et al.** *Nucleic Acids Res,* 1987, vol. 15 (22), 9109-28
- **I.L. HOFACKER et al.** *Monatsh. Chem.,* 1994, vol. 125, 167-188
- **CODINGTON, J. F. ; DOERR, I. L. ; FOX, J. J.** *J. Org. Chem.,* 1964, vol. 29, 558
- **SUNG W. L.** *J. Org. Chem.,* 1982, vol. 47, 3623
- **REESE C.B. ; UBASAWA A.** *Tetrahedron Lett.,* 1980, 2265
- **SUNG W. L.** *J. Chem. Soc. B,* 1981, 1089
- **KRUG A. ; SCHMIDT S. ; LEKSCHAS J. ; LEMKE K. ; CECH D.** *Journal f. prakt. Chemie,* 1989, vol. 331 (5), 835
- **AHMEND et al.** *J. Immunol. Methods,* 1994, vol. 170, 211-224
- **PAGE et al.** *Int. J. Oncology,* 1993, vol. 3, 473-476

SEQUENCE

<110> Grünenthal GmbH NOXXON Pharma AG

<120> CGRP bindende Nukleinsäuren

<130> N 10035 PCT

<160> 263

<170> PatentIn version 3.1

<210> 1
<211> 79
<212> RNA
<213> Artificial Sequence

<220>
<223> CGRP-binding nucleic acid

<220>
<221> misc_feature
<223> laboratory code: clone 666

<220>
<221> misc_feature
<223> all pyrimidines 2'-fluoro modified

<400> 1

```
ggagcucagc cuucacugcg aagugacgca cguaugauag uuuccauuuu ggacuccugg      60

gcaccacggu cggauccac                                                   79
```

<210> 2
<211> 79
<212> RNA
<213> Artificial Sequence

<220>
<223> CGRP-binding nucleic acid

<220>
<221> misc_feature
<223> laboratory code: clone 711

<220>
<221> misc_feature
<223> all pyrimidines 2'-fluoro modified

<400> 2

```
ggagcucagc cuucacugcg aagugaugca cguaugauag uuuccauuuu ggacuccugg      60

gcaccacggu cggauccac                                                   79
```

<210> 3
<211> 77
<212> RNA
<213> Artificial Sequence

<220>

<223> CGRP-binding nucleic acid

<220>
<221> misc_feature
<223> laboratory code: clone 732

<220>
<221> misc_feature
<223> all pyrimidines 2'-fluoro modified

<400> 3

ggagcucagc cuucacugca gugacgcacg uaugauaguu uccauuuugg acuccugggc 60

accacggucg gauccac 77

<210> 4
<211> 79
<212> RNA
<213> Artificial Sequence

<220>
<223> CGRP-binding nucleic acid

<220>
<221> misc_feature
<223> laboratory code: clone 669

<220>
<221> misc_feature
<223> all pyrimidines 2'-fluor modified

<400> 4

ggagcucagc cuucacugca aagugacgca cguaugauag uuuccauuuu ggacuccugg 60

gcaccacggu cggauccac 79

<210> 5
<211> 79
<212> RNA
<213> Artificial Sequence

<220>
<223> CGRP-binding nucleic acid

<220>
<221> misc_feature
<223> laboratory code: clone 670

<220>
<221> misc_feature
<223> all pyrimidines 2'-fluor modified

<400> 5

ggagcucagc cuucacugcg aagugacgca cguauuauag uuuccauuuu ggacuccugg 60

gcaccacggu cggauccac 79

<210> 6
<211> 79
<212> RNA
<213> Artificial Sequence

<220>
<223> CGRP-binding nucleic acid

<220>
<221> misc_feature
<223> laboratory code: clone 781

<220>
<221> misc_feature
<223> all pyrimidines 2'-fluor modified

<400> 6

ggagcucagc cuucacugcu aagugacgca cguaugauag uuuccauuuu ggacuccugg 60

gcaccacggu cggauccac 79

<210> 7
<211> 79
<212> RNA
<213> Artificial Sequence

<220>
<223> CGRP-binding nucleic acid

<220>
<221> misc_feature
<223> laboratory code: clone 836

<220>
<221> misc_feature
<223> all pyrimidines 2'-fluoro modified

<400> 7

ggagcucagc cuucacugcg aagugacgca cguaugauag uuuccguuuu ggacuccugg 60

gcaccacggu cggauccac 79

<210> 8
<211> 79
<212> RNA
<213> Artificial Sequence

<220>
<223> CGRP-binding nucleic acid

<220>
<221> misc_feature
<223> laboratory code: clone 748

<220>
<221> misc_feature
<223> all pyrimidines 2'-fluoro modified

<400> 8

ggagcucagc cuucacugcg aagugacgua cguaugauag uuuccauuuu ggacuccugg 60

gcaccacggu cggauccac 79

<210> 9
<211> 79
<212> RNA
<213> Artificial Sequence

<220>
<223> CGRP-binding nucleic acid

<220>
<221> misc_feature
<223> laboratory code: sequence 666

<220>
<221> misc_feature
<223> all pyrimidines 2'-fluor modified

<400> 9

ggagcucagc cuucacugcg aagugacgca cguaugauag uuuccauuuu ggacuccugg 60

gcaccacggu cggauccac 79

<210> 10
<211> 77
<212> RNA
<213> Artificial Sequence

<220>
<223> CGRP-binding nucleic acid

<220>
<221> misc_feature
<223> laboratory code: sequence 732

<220>
<221> misc_feature
<223> all pyrimidines 2'-fluoro modified

<400> 10

ggagcucagc cuucacugca gugacgcacg uaugauaguu uccauuuugg acuccugggc 60

accacggucg gauccac 77

<210> 11
<211> 55
<212> RNA
<213> Artificial Sequence

<220>
<223> CGRP-binding nucleic acid

<220>
<221> misc_feature
<223> laboratory code: sequence 732_026

<220>
<221> misc_feature
<223> all pyrimidines 2'-fluoro modified

<400> 11
ggcagccuuc acugcaguga cgcacguaug auaguuucca uuuuggacuc cugcc 55

<210> 12
<211> 59
<212> RNA
<213> Artificial Sequence

<220>
<223> CGRP-binding nucleic acid

<220>
<221> misc_feature
<223> laboratory code: sequence 732_029

<220>
<221> misc_feature
<223> all pyrimidines 2'-fluoro modified

<400> 12
ggcucagccu ucacugcagu gacgcacgua ugauaguuuc cauuuuggac uccugggcc 59

<210> 13
<211> 55
<212> RNA
<213> Artificial Sequence

<220>
<223> CGRP-binding nucleic acid

<220>
<221> misc_feature
<223> laboratory code: sequence 732_045

<221> misc_feature
<223> all pyrimidines 2'-fluoro modified

<400> 13
cgcagccuuc acugcaguga cgcacguaug auaguuucca uuuuggacuc cugcg 55

<210> 14
<211> 59
<212> RNA
<213> Artificial Sequence

<220>
<223> CGRP-binding nucleic acid

<220>
<221> misc_feature
<223> laboratory code: sequence 732_096

<220>
<221> misc_feature
<223> all pyrimidines 2'-fluoro modified

<400> 14

<210> 15
<211> 51
<212> RNA
<213> Artificial Sequence

<220>
<223> CGRP-binding nucleic acid

<220>
<221> misc_feature
<223> laboratory code: sequence 732_100

<220>
<221> misc_feature
<223> all pyrimidines 2'-fluoro modified

<400> 15
ggcagccuuc cugcaggacg cacguaugau aguuuccuuu ggacuccugc c 51

<210> 16
<211> 51
<212> RNA
<213> Artificial Sequence

<220>
<223> CGRP-binding nucleic acid

<220>
<221> misc_feature
<223> laboratory code: sequence 732_103

<220>
<221> misc_feature
<223> all pyrimidines 2'-fluoro modified

<400> 16
ggcagccuug cugcagcacg cacguaugau aguuuccuuu ggacuccugc c 51

<210> 17
<211> 51
<212> RNA
<213> Artificial Sequence

<220>
<223> CGRP-binding nucleic acid

<220>
<221> misc_feature
<223> laboratory code: sequence 732_104

<220>
<221> misc_feature
<223> all pyrimidines 2'-fluoro modified

<400> 17
ggcagccuuc cugcaggacg cacguaugau aguuugcuuu gcacuccugc c 51

<210> 18
<211> 50
<212> RNA
<213> Artificial Sequence

<220>
<223> CGRP-binding nucleic acid

<220>
<221> misc_feature
<223> laboratory code: sequence 732_108

<220>
<221> misc_feature
<223> all pyrimidines 2'-fluoro modified

<400> 18
ggcagccuug cugcagcacg cacuaugaua guuuccuuug gacuccugcc 50

<210> 19
<211> 14
<212> RNA
<213> Artificial Sequence

<220>
<223> CGRP-binding nucleic acid

<220>
<221> misc_feature
<223> laboratory code: sequence 732_070a

<220>
<221> misc_feature
<223> all pyrimidines 2'-fluoro modified

<400> 19
ggcagccuuc acug 14

<210> 20

<211> 41
<212> RNA
<213> Artificial Sequence

<220>
<223> CGRP-binding nucleic acid

<220>
<221> misc_feature
<223> laboratory code: sequence 732_070b

<220>
<221> misc_feature
<223> all pyrimidines except for 3'-terminal C 2'-fluoro modified

<400> 20
cagugacgca cguaugauag uuuccauuuu ggacuccugc c 41

<210> 21
<211> 42
<212> RNA
<213> Artificial Sequence

<220>
<223> CGRP-binding nucleic acid

<220>
<221> misc_feature
<223> laboratory code: sequence 732_071a

<220>
<221> misc_feature
<223> all pyrimidines 2'-fluoro modified

<400> 21
ggcagccuuc acugcaguga cgcacguaug auaguuucca ag 42

<210> 22
<211> 14
<212> RNA
<213> Artificial Sequence

<220>
<223> CGRP-binding nucleic acid

<220>
<221> misc_feature
<223> laboratory code: sequence 732_071b

<220>
<221> misc_feature
<223> all pyrimidines except for 3'-terminal C 2'-fluoro modified

<400> 22
cuuggacucc ugcc 14

<210> 23
<211> 99

<212> RNA
<213> Artificial Sequence

<220>
<223> CGRP-binding nucleic acid

<220>
<221> misc_feature
<223> laboratory code: family #1

<220>
<221> misc_feature
<223> all pyrimidines 2'-fluoro modified

<400> 23

gggaauucga gcucgguacc uuaacccgua uggggucacu guuucgauuu cauucgccuu 60

aucgagccga uucacuugcg cugcaggcau gaagcuugg 99

<210> 24
<211> 99
<212> RNA
<213> Artificial Sequence

<220>
<223> CGRP-binding nucleic acid

<220>
<221> misc_feature
<223> laboratory code: family #2

<220>
<221> misc_feature
<223> all pyrimidines 2'-fluoro modified

<400> 24

gggaauucga gcucgguacc uuguuaccca cuguuuagua ucucgcgaua cucauuaccg 60

agacacaguc ccauuacugc cugcaggcau gaagcuugg 99

<210> 25
<211> 98
<212> RNA
<213> Artificial Sequence

<220>
<223> CGRP-binding nucleic acid

<220>
<221> misc_feature
<223> laboratory code: family #3

<220>
<221> misc_feature

<223> all pyrimidines 2'-fluoro modified

<400> 25

gggaauucga gcucgguacc gcacuuucgu uacauacgau auacugggcu auagucuauc 60

cuugugccua cagguacugc ugcaggcaug aagcuugg 98

<210> 26
<211> 98
<212> RNA
<213> Artificial Sequence

<220>
<223> CGRP-binding nucleic acid

<220>
<221> misc_feature
<223> laboratory code: family #4

<220>
<221> misc_feature
<223> all pyrimidines 2'-fluoro modified

<400> 26

gggaauucga gcucgguacc uacugcucga cuaauugucu aguacaauau gcuuaccaca 60

uuaucuguua gugagcuccc ugcaggcaug aagcuugg 98

<210> 27
<211> 99
<212> RNA
<213> Artificial Sequence

<220>
<223> CGRP-binding nucleic acid

<220>
<221> misc_feature
<223> laboratory code: family #5

<220>
<221> misc_feature
<223> all pyrimidines 2'-fluoro modified

<400> 27

gggaauucga gcucgguacc uuguucugac ucuguuuaug cguuuuccgc gucuuuaccg 60

gacuccuucu uccccagugc cugcaggcau gaagcuugg 99

<210> 28
<211> 99
<212> RNA

<213> Artificial Sequence

<220>
<223> CGRP-binding nucleic acid

<220>
<221> misc_feature
<223> laboratory code: family #6

<220>
<221> misc_feature
<223> all pyrimidines 2'-fluoro modified

<400> 28

gggaauucga gcucgguacc uaucgucgaa cauucgaucu guuuuuacgu aaguaacuuu 60

accguccucg uuuuucccgc cugcaggcau gaagcuugg 99

<210> 29
<211> 99
<212> RNA
<213> Artificial Sequence

<220>
<223> CGRP-binding nucleic acid

<220>
<221> misc_feature
<223> laboratory code: family #7

<220>
<221> misc_feature
<223> all pyrimidines 2'-fluoro modified

<400> 29

gggaauucga gcucgguacc aaacaucacu uacaugugcu cugcguuuuu ugcauaguuu 60

uuuggucgag cgcuuccucc cugcaggcau gaagcuugg 99

<210> 30
<211> 96
<212> RNA
<213> Artificial Sequence

<220>
<223> CGRP-binding nucleic acid

<220>
<221> misc_feature
<223> laboratory code: family #8

<220>
<221> misc_feature
<223> all pyrimidines 2'-fluoro modified

<400> 30

gggaauucga gcucgguacc gcggagucug ucacaagauc ucguccuuau cguugaugua 60

ucguacaagu cuuugcccug caggcaugaa gcuugg 96

<210> 31
<211> 97
<212> RNA
<213> Artificial Sequence

<220>
<223> CGRP-binding nucleic acid

<220>
<221> misc_feature
<223> laboratory code: family #9

<220>
<221> misc_feature
<223> all pyrimidines 2'-fluoro modified

<220>
<221> misc_feature
<222> (31).. (31)

<220>
<221> misc_feature
<222> (51).. (51)
<223> any nucleotide

<400> 31

gggaauucga gcucgguacc uaauacgacu nacuauaggg aauucgagcu ngguaccuua 60

acccguaugg gguuacugcu gcaggcauga agcuugg 97

<210> 32
<211> 98
<212> RNA
<213> Artificial Sequence

<220>
<223> CGRP-binding nucleic acid

<220>
<221> misc_feature
<223> laboratory code: familiy #10

<220>
<221> misc_feature
<223> all pyrimidines 2'-fluoro modified

<400> 32

gggaauucga gcucgguacc aaugccugcu uuguuuugag uuuuccuuca cacuagggau 60

ggauaauaca guccuuaccc ugcaggcaug aagcuugg 98

<210> 33
<211> 99
<212> RNA
<213> Artificial Sequence

<220>
<223> CGRP-binding nucleic acid

<220>
<221> misc_feature
<223> laboratory code: 156750.seq

<220>
<221> misc_feature
<223> all pyrimidines 2'-fluoro modified

<400> 33

gggaauucga gcucgguacc uuaacccgua uggggucacu guuucgauuu cauucgccuu 60

cucgagcuga uucacuugcg cugcaggcau gaagcuugg 99

<210> 34
<211> 100
<212> RNA
<213> Artificial Sequence

<220>
<223> CGRP-binding nucleic acid

<220>
<221> misc_feature
<223> laboratory code: 156701.seq

<220>
<221> misc_feature
<223> all pyrimidines 2'-fluoro modified

<400> 34

gggaauucga gcucgguacc uuaacccgua uggggucacu gguuucgauu ucauucgccu 60

uaucgagcug auucacuugc gcugcaggca ugaagcuugg 100

<210> 35
<211> 100
<212> RNA
<213> Artificial Sequence

<220>
<223> CGRP-binding nucleic acid

<220>
<221> misc_feature
<223> laboratory code: 156698.seq

<220>
<221> misc_feature
<223> all pyrimidines 2'-fluoro modified

<400> 35

gggaauucga gcucgguacc uuaacccgua uuggggucac uguuucgauu ucauucgccu 60

uaucgagcug auucacuugc gcugcaggca ugaagcuugg 100

<210> 36
<211> 100
<212> RNA
<213> Artificial Sequence

<220>
<223> CGRP-binding nucleic acid

<220>
<221> misc_feature
<223> laboratory code: 156707.seq

<220>
<221> misc_feature
<223> all pyrimidines 2'-fluoro modified

<400> 36

gggaauucga gcucgguacc uuaacccgua uggggucacu guuucgauuu ucauucgccu 60

uaucgagcug auucacuugc gcugcaggca ugaagcuugg 100

<210> 37
<211> 99
<212> RNA
<213> Artificial Sequence

<220>
<223> CGRP-binding nucleic acid

<220>
<221> misc_feature
<223> laboratory code: KN-158_06b_D06_x_046

<220>
<221> misc_feature
<223> all pyrimidines 2'-fluoro modified

<220>
<221> misc_feature
<222> (64)..(64)
<223> any nucleotide

<220>
<221> misc_feature
<222> (66)..(66)
<223> any nucleotide

<400> 37

gggaauucga gcucgguacc cuaacccgua uggggucacu guuucgauuu cauucgccuu 60

aucnancuga uucacuugcg cugcaggcau gaagcuugg 99

<210> 38
<211> 99
<212> RNA
<213> Artificial Sequence

<220>
<223> CGRP-binding nucleic acid

<220>
<221> misc_feature
<223> laboratory code: KN-158_06b_D12_x_094

<220>
<221> misc_feature
<223> all pyrimidines 2'-fluoro modified

<400> 38

gggaauucga gcucgguacc cuaacccgua uggggucacu guuucgauuu cauucgccuu 60

aucgagcuga uucacuugcg cugcaggcau gaagcuugg 99

<210> 39
<211> 100
<212> RNA
<213> Artificial Sequence

<220>
<223> CGRP-binding nucleic acid

<220>
<221> misc_feature
<223> laboratory code: kn-158-6_h03_m13-fp

<220>
<221> misc_feature
<223> all pyrimidines 2'-fluoro modified

<220>
<221> misc_feature
<222> (27)..(27)
<223> any nucleotide

<400> 39

gggaauucga gcucgguacc uuaaccngua uggggucacu guuucgauuu cauucgccuu 60

aucgagcuga auucacuugc gcugcaggca ugaagcuugg 100

<210> 40
<211> 99
<212> RNA
<213> Artificial Sequence

<220>
<223> CGRP-binding nucleic acid

<220>
<221> misc_feature
<223> laboratory code: kn-158-6_f12_m13-fp

<220>
<221> misc_feature
<223> all pyrimidines 2'-fluoro modified

<220>
<221> misc_feature
<222> (72)..(72)
<223> any nucleotide

<400> 40

gggaauucga gcucgguacc uuaacccgua uggggucacu guuucgauuu cauucgccuu 60

aucgagcuga uncacuugcg cugcaggcau gaagcuugg 99

<210> 41
<211> 99
<212> RNA
<213> Artificial Sequence

<220>
<223> CGRP-binding nucleic acid

<220>
<221> misc_feature
<223> laboratory code. 156703.seq

<220>
<221> misc_feature
<223> all pyrimidines 2'-fluoro modified

<400> 41

gggaauucga gcucgguacc uuguuaccca cuguuuagua ucucgcgaua cucauuaccg 60

agacacaguc ccauuacugc cugcaggcau gaagcuugg 99

<210> 42
<211> 98

<212> RNA
<213> Artificial Sequence

<220>
<223> CGRP-binding nucleic acid

<220>
<221> misc_feature
<223> laboratory code: 156719.seq

<220>
<221> misc_feature
<223> all pyrimidines 2'-fluoro modified

<400> 42

gggaauucga gcucgguacc gcacuuucgu uacauacgau auacugggcu auagucuauc 60

cuugugccua cagguacugc ugcaggcaug aagcuugg 98

<210> 43
<211> 96
<212> RNA
<213> Artificial Sequence

<220>
<223> CGRP-binding nucleic acid

<220>
<221> misc_feature
<223> laboratory code: 156718.seq

<220>
<221> misc_feature
<223> all pyrimidines 2'-fluoro modified

<400> 43

gggaauucga gcucgguacc gcacuuucgu uacauacgau auacugggcu auagucuauc 60

cuugugccua cagguugcug caggcaugaa gcuugg 96

<210> 44
<211> 98
<212> RNA
<213> Artificial Sequence

<220>
<223> CGRP-binding nucleic acid

<220>
<221> misc_feature
<223> laboratory code: 156736.seq

<220>
<221> misc_feature

<223> all pyrimidines 2'-fluoro modified

<400> 44

gggaauucga gcucgguacc gcacuuucgu uacauacgau auacugggcu auagucuauc 60

ccugugccua cagguacugc ugcaggcaug aagcuugg 98

<210> 45
<211> 96
<212> RNA
<213> Artificial Sequence

<220>
<223> CGRP-binding nucleic acid

<220>
<221> misc_feature
<223> laboratory code: KN-158_06b_B10_x_077

<220>
<221> misc_feature
<223> all pyrimidines 2'-fluoro modified

<400> 45

gggaauucga gcucgguacc gcacuuucgu uacauacgau auacugggcu auuguauccu 60

ugugccuaca gguacugcug caggcaugaa gcuugg 96

<210> 46
<211> 98
<212> RNA
<213> Artificial Sequence

<220>
<223> CGRP-binding nucleic acid

<220>
<221> misc_feature
<223> laboratory code: kn-158-6_f07_m13-fp

<220>
<221> misc_feature
<223> all pyrimidines 2'-fluoro modified

<400> 46

gggaauucga gcucgguacc gcacuuucgu ugcauacgau auaccgggcu auagucuauc 60

cuugugccua cagguacugc ugcaggcaug aagcuugg 98

<210> 47
<211> 98
<212> RNA

<213> Artificial Sequence

<220>
<223> CGRP-binding nucleic acid

<220>
<221> misc_feature
<223> laboratory code: 156695.seq

<220>
<221> misc_feature
<223> all pyrimidines 2'-fluoro modified

<400> 47

gggaauucga gcucgguacc uacugcucga cuaauugucu aguacaauau gcuuaccaca 60

uuaucuguua gugagcuccc ugcaggcaug aagcuugg 98

<210> 48
<211> 99
<212> RNA
<213> Artificial Sequence

<220>
<223> CGRP-binding nucleic acid

<220>
<221> misc_feature
<223> laboratory code: 156673.seq

<220>
<221> misc_feature
<223> all pyrimidines 2'-fluoro modified

<400> 48

gggaauucga gcucgguacc uuguucugac ucuguuuaug cguuuuccgc gucuuuaccg 60

gacuccuucu uccccagugc cugcaggcau gaagcuugg 99

<210> 49
<211> 98
<212> RNA
<213> Artificial Sequence

<220>
<223> CGRP-binding nucleic acid

<220>
<221> misc_feature
<223> laboratory code: 156706.seq

<220>
<221> misc_feature
<223> all pyrimidines 2'-fluoro modified

<400> 49

gggaauucga gcucgguacc uuguucugac ucuguuuaug cguuuuacgc gucuuuaccg 60

gacuccucuu ccccagugcc ugcaggcaug aagcuugg 98

<210> 50
<211> 97
<212> RNA
<213> Artificial Sequence

<220>
<223> CGRP-binding nucleic acid

<220>
<221> misc_feature
<223> laboratory code: 156761.seq

<220>
<221> misc_feature
<223> all pyrimidines 2'-fluoro modified

<400> 50

gggaauucga gcucgguacc uuguucugac ucuguuuaug cguuuacgcg ucuuuaccgg 60

acuccuucuu cccagugccu gcaggcauga agcuugg 97

<210> 51
<211> 99
<212> RNA
<213> Artificial Sequence

<220>
<223> CGRP-binding nucleic acid

<220>
<221> misc_feature
<223> laboratory code: 156738.seq

<220>
<221> misc_feature
<223> all pyrimidines 2'-fluoro modified

<400> 51

gggaauucga gcucgguacc uaucgucgaa cauucgaucu guuuuuacgu aaguaacuuu 60

accguccucg uuuuucccgc cugcaggcau gaagcuugg 99

<210> 52
<211> 100
<212> RNA
<213> Artificial Sequence

<220>
<223> CGRP-binding nucleic acid

<220>
<221> misc_feature
<223> laboratory code: 156730.seq

<220>
<221> misc_feature
<223> all pyrimidines 2'-fluoro modified

<400> 52

gggaauucga gcucgguacc uaucgucgaa cauucgaucu guuuuuacgu uaaguaacuu 60

uaccguccuc guuuuucccg ccugcaggca ugaagcuugg 100

<210> 53
<211> 99
<212> RNA
<213> Artificial Sequence

<220>
<223> CGRP-binding nucleic acid

<220>
<221> misc_feature
<223> laboratory code: kn-158-6_f04_m13-fp.abi

<220>
<221> misc_feature
<223> all pyrimidines 2'-fluoro modified

<400> 53

gggaauucga gcucgguacc uaucgucgaa cauucgaucu guuuuuacgu aaguaacuuu 60

accguccucg uuuuucccgc cugcaggcau gaagcuugg 99

<210> 54
<211> 99
<212> RNA
<213> Artificial Sequence

<220>
<223> CGRP-binding nucleic acid

<220>
<221> misc_feature
<223> laboratory code: kn-158-6_f06_m13-fp.abi

<220>
<221> misc_feature
<223> all pyrimidines 2'-fluoro modified

<400> 54

gggaauucga gcucgguacc uaucgucgaa cauucgaucu guuuuuacgu aaguaacugu 60

accguccucg uuuuuccucc cugcaggcau gaagcuugg 99

<210> 55
<211> 100
<212> RNA
<213> Artificial Sequence

<220>
<223> CGRP-binding nucleic acid

<220>
<221> misc_feature
<223> laboratory code: kn-158-6_g02-m13-fp.abi

<220>
<221> misc_feature
<223> all pyrimidines 2'-fluoro modified

<400> 55

gggaauucga gcucgguacc uauugucgaa cauucgaucu guuuuuacgu aaguaacuuu 60

accguccucg uuuuuucccg ccugcaggca ugaagcuugg 100

<210> 56
<211> 99
<212> RNA
<213> Artificial Sequence

<220>
<223> CGRP-binding nucleic acid

<220>
<221> misc_feature
<223> laboratory code: 156697.seq

<220>
<221> misc_feature
<223> all pyrimidines 2'-fluoro modified

<400> 56

gggaauucga gcucgguacc aaacaucacu uacaugugcu cugcguuuuu ugcauaguuu 60

uuuggucgag cgcuuccucc cugcaggcau gaagcuugg 99

<210> 57
<211> 99
<212> RNA
<213> Artificial Sequence

<220>
<223> CGRP-binding nucleic acid

<220>
<221> misc_feature
<223> laboratory code: 156712.seq

<220>
<221> misc_feature
<223> all pyrimidines 2'-fluoro modified

<400> 57

gggaauucga gcucgguacc agacaucacu uacaugugcu cugcguuuuu ugcaucguuu 60

uuuggucgag cgcuuccucc cugcaggcau gaagcuugg 99

<210> 58
<211> 96
<212> RNA
<213> Artificial Sequence

<220>
<223> CGRP-binding nucleic acid

<220>
<221> misc_feature
<223> laboratory code: 156745.seq

<220>
<221> misc_feature
<223> all pyrimidines 2'-fluoro modified

<400> 58

gggaauucga gcucgguacc gcggagucug ucacaagauc ucguccuuau cguugaugua 60

ucguacaagu cuuugcccug caggcaugaa gcuugg 96

<210> 59
<211> 98
<212> RNA
<213> Artificial Sequence

<220>
<223> CGRP-binding nucleic acid

<220>
<221> misc_feature
<223> laboratory code: kn-158-6_e07_m13-fp.abi

<220>
<221> misc_feature
<223> all pyrimidines 2'-fluoro modified

<400> 59

gggaauucga gcucgguacc aaugccugcu uuguuuugag uuuuccuuca cacuagggau 60

ggauaauaca gucccuaccc ugcaggcaug aagcuugg 98

<210> 60
<211> 98
<212> RNA
<213> Artificial Sequence

<220>
<223> CGRP-binding nucleic acid

<220>
<221> misc_feature
<223> laboratory code: kn-158-6_e05_m13-fp.abi

<220>
<221> misc_feature
<223> all pyrimidines 2'-fluoro modified

<400> 60

gggaauucga gcucgguacc aaugccugcu uuguuuugag uuuuucuuca cacuagggau 60

ggauaauaca gucccuaccc ugcaggcaug aagcuugg 98

<210> 61
<211> 91
<212> RNA
<213> Artificial Sequence

<220>
<223> CGRP-binding nucleic acid

<220>
<221> misc_feature
<223> laboratory code: kn-158-6_e08_m13-fp.abi

<220>
<221> misc_feature
<223> all pyrimidines 2'-fluoro modified

<400> 61

gggaauucga gcucgguacc cuuuguuuug aguuuucccu guacacuagg gauggauaau 60

acagucccua cccugcaggc augaagcuug g 91

<210> 62
<211> 98
<212> RNA
<213> Artificial Sequence

<220>
<223> CGRP-binding nucleic acid

<220>
<221> misc_feature
<223> laboratory code: 156678.seq

<220>
<221> misc_feature
<223> all pyrimidines 2'-fluoro modified

<400> 62

gggaauucga gcucgguacc uguuacagug uuguacuagu ucuaguguuu gguugaugua 60

cucccguaac uuauccugcc ugcaggcaug aagcuugg 98

<210> 63
<211> 99
<212> RNA
<213> Artificial Sequence

<220>
<223> CGRP-binding nucleic acid

<220>
<221> misc_feature
<223> laboratory code: 156758.seq

<220>
<221> misc_feature
<223> all pyrimidines 2'-fluoro modified

<400> 63

gggaauucga gcucgguacc uaaacgacua cgaccugaug cuggaaguua auuuguaacu 60

guguuacaua cagucgcgug cugcaggcau gaagcuugg 99

<210> 64
<211> 99
<212> RNA
<213> Artificial Sequence

<220>
<223> CGRP-binding nucleic acid

<220>
<221> misc_feature
<223> laboratory code: 156708.seq

<220>
<221> misc_feature
<223> all pyrimidines 2'-fluoro modified

<400> 64

gggaauucga gcucgguacc uuaaugguug acugauucuu ccuugccaac ugugcuuagc 60

uguuacaguu uaucaugugc cugcaggcau gaagcuugg 99

<210> 65
<211> 98
<212> RNA
<213> Artificial Sequence

<220>
<223> CGRP-binding nucleic acid

<220>
<221> misc_feature
<223> laboratory code: 156693.seq

<220>
<221> misc_feature
<223> all pyrimidines 2'-fluoro modified

<400> 65

gggaauucga gcucgguacc aagagaugau caagcgucgu ucuaaucaug aguugugucu 60

accccuaacu gccaauucgc ugcaggcaug aagcuugg 98

<210> 66
<211> 99
<212> RNA
<213> Artificial Sequence

<220>
<223> CGRP-binding nucleic acid

<220>
<221> misc_feature
<223> laboratory code: 156751.seq

<220>
<221> misc_feature
<223> all pyrimidines 2'-fluoro modified

<400> 66

gggaauucga gcucgguacc uuaaacacca gugccugcuc ucaaugaugu uguuuucuuc 60

augcgugucg ugagaucuuc cugcaggcau gaagcuugg 99

<210> 67
<211> 98
<212> RNA
<213> Artificial Sequence

<220>
<223> CGRP-binding nucleic acid

<220>
<221> misc_feature
<223> laboratory code: 156759.seq

<220>
<221> misc_feature
<223> all pyrimidines 2'-fluoro modified

<400> 67

gggaauucga gcucgguacc gugaaccugu aacuuuguca gccgcacggu aauuuuguuc 60

ucgauuaauc ccgagagccc ugcaggcaug aagcuugg 98

<210> 68
<211> 78
<212> RNA
<213> Artificial Sequence

<220>
<223> CGRP-binding nucleic acid

<220>
<221> misc_feature
<223> laboratory code: 156733.seq

<220>
<221> misc_feature
<223> all pyrimidines 2'-fluoro modified

<400> 68

gggaauucga gcucgguacc aaacggcagc acucgcacuu auucguggac gauuuugagg 60

uuaauguacg cuugcccc 78

<210> 69
<211> 99
<212> RNA
<213> Artificial Sequence

<220>
<223> CGRP-binding nucleic acid

<220>
<221> misc_feature
<223> laboratory code: 156710.seq

<220>
<221> misc_feature
<223> all pyrimidines 2'-fluoro modified

<400> 69

gggaauucga gcucgguacc uaucguugaa cauucgaucu guuuuuacgu aaguaacuuu 60

accguccucg uuuuucccgc cugcaggcau gaagcuugg 99

<210> 70
<211> 99
<212> RNA
<213> Artificial Sequence

<220>
<223> CGRP-binding nucleic acid

<220>
<221> misc_feature
<223> laboratory code: 156760.seq

<220>
<221> misc_feature
<223> all pyrimidines 2'-fluoro modified

<400> 70

gggaauucga gcucgguacc cugcaguuuc agauuuaacg auuagcuucu ggaugguau 60

uggcacaucu uacccuuugg cugcaggcau gaagcuugg 99

<210> 71
<211> 99
<212> RNA
<213> Artificial Sequence

<220>
<223> CGRP-binding nucleic acid

<220>
<221> misc_feature
<223> laboratory code: 156755.seq

<220>
<221> misc_feature
<223> all pyrimidines 2'-fluoro modified

<400> 71

gggaauucga gcucgguacc auuuggcuag guaaccgaca acuucaaucg cuacuucacu 60

uggagucuug gcuauccauc cugcaggcau gaagcuugg 99

<210> 72
<211> 97
<212> RNA
<213> Artificial Sequence

<220>
<223> CGRP-binding nucleic acid

<220>
<221> misc_feature
<223> laboratory code: 156714.seq

<220>
<221> misc_feature
<223> all pyrimidines 2'-fluoro modified

<400> 72

```
gggaauucga gcucgguacc uuucugcgac uguugccgca aucuucaacc uugcgugauu      60

aagccacgua ucgucuugcu gcaggcauga agcuugg                               97
```

<210> 73
<211> 97
<212> RNA
<213> Artificial Sequence

<220>
<223> CGRP-binding nucleic acid

<220>
<221> misc_feature
<223> laboratory code: 156690.seq

<220>
<221> misc_feature
<223> all pyrimidines 2'-fluoro modified

<400> 73

```
gggaauucga gcucgguacc ucauccucau caaacaguag cguuaucauu guguaguaac      60

ccaaagcgcu gccauucccu gcaggcauga agcuugg                               97
```

<210> 74
<211> 95
<212> RNA
<213> Artificial Sequence

<220>
<223> CGRP-binding nucleic acid

<220>
<221> misc_feature
<223> laboratory code: 156681.seq

<220>
<221> misc_feature
<223> all pyrimidines 2'-fluoro modified

<400> 74

gggaauucga gcucgguacc ggauggaaca cgcaguuugu cuuaguucgu ugaugaaacc 60

uuuucaacgc uucgcccugc aggcaugaag cuugg 95

<210> 75
<211> 99
<212> RNA
<213> Artificial Sequence

<220>
<223> CGRP-binding nucleic acid

<220>
<221> misc_feature
<223> laboratory code: 156739.seq

<220>
<221> misc_feature
<223> all pyrimidines 2'-fluoro modified

<400> 75

gggaauucga gcucgguacc uauacugcuc acuuugaauu caaucaccgu ucgcgcugcu 60

aacucacuau uucccuuggc cugcaggcau gaagcuugg 99

<210> 76
<211> 92
<212> RNA
<213> Artificial Sequence

<220>
<223> CGRP-binding nucleic acid

<220>
<221> misc_feature
<223> laboratory code: 156682.seq

<220>
<221> misc_feature
<223> all pyrimidines 2'-fluoro modified

<400> 76

gggaauucga gcucgguacc aggugauugc uuucuuaacu uuguuagacu ucgaccauca 60

gugucgcccu caccugcagg caugaagcuu gg 92

<210> 77
<211> 98
<212> RNA
<213> Artificial Sequence

<220>
<223> CGRP-binding nucleic acid

<220>
<221> misc_feature
<223> laboratory code: kn-158-6_d04_m13-fp.abi

<220>
<221> misc_feature
<223> all pyrimidines 2'-fluoro modified

<400> 77

gggaauucga gcucgguacc aaugccugcu uuguuuugag uuuuccuuca cacuagggau 60

ggauaauaca gucccuaccc ugcaggcaug aagcuugg 98

<210> 78
<211> 99
<212> RNA
<213> Artificial Sequence

<220>
<223> CGRP-binding nucleic acid

<220>
<221> misc_feature
<223> laboratory code: kn-158-6_a01_m13-fp.abi

<220>
<221> misc_feature
<223> all pyrimidines 2'-fluoro modified

<400> 78

gggaauucga gcucgguacc uacaaucguu accugcucgc uuuaaucuug gauccuaucc 60

gauuuagauc uucccacuac cugcaggcau gaagcuugg 99

<210> 79
<211> 99
<212> RNA
<213> Artificial Sequence

<220>
<223> CGRP-binding nucleic acid

<220>
<221> misc_feature
<223> laboratory code: kn-158-6_c01_m13-fp.abi

<220>
<221> misc_feature
<223> all pyrimidines 2'-fluoro modified

<400> 79

gggaauucga gcucgguacc augcaggugc ccccuuuggu uuuuuccaaa ggcacaucag 60

aaaguucucg aauuuccgua cugcaggcau gaagcuugg 99

<210> 80
<211> 99
<212> RNA
<213> Artificial Sequence

<220>
<223> CGRP-binding nucleic acid

<220>
<221> misc_feature
<223> laboratory code: kn-158-6_c05_m13-fp.abi

<220>
<221> misc_feature
<223> all pyrimidines 2'-fluoro modified

<220>
<221> misc_feature
<222> (35)..(35)
<223> any nucleotide

<220>
<221> misc_feature
<222> (61)..(61)
<223> any nucleotide

<400> 80

gggaauucga gcucgguacc accugcaauu ccaunccguu cucccauaug ucuuaggcuu 60

nugguguucu cgucuugguc cugcaggcau gaagcuugg 99

<210> 81
<211> 99
<212> RNA
<213> Artificial Sequence

<220>
<223> CGRP-binding nucleic acid

<220>
<221> misc_feature
<223> laboratory code: kn-158-6_a12_m13-fp.abi

<220>
<221> misc_feature
<223> all pyrimidines 2'-fluoro modified

<400> 81

gggaauucga gcucgguacc uuuacguaac auuguacccc uucagccgcu ggcugucgca 60

cauaugauag uucucggauu cugcaggcau gaagcuugg 99

<210> 82
<211> 98
<212> RNA
<213> Artificial Sequence

<220>
<223> CGRP-binding nucleic acid

<220>
<221> misc_feature
<223> laboratory code: kn-158-6_a06_m13-fp.abi

<220>
<221> misc_feature
<223> all pyrimidines 2'-fluoro modified

<400> 82

gggaauucga gcucgguacc acaggacgcu guugauccuc gguaaaucgg uauguaagua 60

uucgaaguau gugugccccc ugcaggcaug aagcuugg 98

<210> 83
<211> 98
<212> RNA
<213> Artificial Sequence

<220>
<223> CGRP-binding nucleic acid

<220>
<221> misc_feature
<223> laboratory code: kn-158-6_c10_m13-fp.abi

<220>
<221> misc_feature
<223> all pyrimidines 2'-fluoro modified

<400> 83

gggaauucga gcucgguacc uacugcucga cuaauugucu aguacaauau gcuuaccaca 60

uuaucuguua gugagcuccc ugcaggcaug aagcuugg 98

<210> 84
<211> 99
<212> RNA
<213> Artificial Sequence

<220>
<223> CGRP-binding nucleic acid

<220>
<223> laboratory code: kn-158-6_b05_m13-fp.abi

<220>
<221> misc_feature
<223> all pyrimidines 2’-fluoro modified

<400> 84

gggaauucga gcucgguacc agacaucacu uacaugugcu cugcguuuuu ugcaucguuu 60

uuuggucgag cgcuucccucc cugcaggcau gaagcuugg 99

<210> 85
<211> 99
<212> RNA
<213> Artificial Sequence

<220>
<223> CGRP-binding nucleic acid

<220>
<221> misc_feature
<223> laboratory code: kn-158-6_c09_m13-fp.abi

<220>
<221> misc_feature
<223> all pyrimidines 2’-fluoro modified

<400> 85

gggaauucga gcucgguacc uaucgucgaa cauucgaucu guuuuuacgu aaguaacuuu 60

accguccucg uuuuucccgc cugcaggcau gaagcuugg 99

<210> 86
<211> 96
<212> RNA
<213> Artificial Sequence

<220>
<223> CGRP-binding nucleic acid

<220>
<221> misc_feature
<223> laboratory code: kn-158-6_b09_m13-fp.abi

<220>
<221> misc_feature
<223> all pyrimidines 2’-fluoro modified

<400> 86

gggaauucga gcucgguacc ucuugcacgc auuaacugau ugcucuuuca ugacuuacaa 60

cucccuuugc uagcccccug caggcaugaa gcuugg 96

<210> 87
<211> 99
<212> RNA
<213> Artificial Sequence

<220>
<223> CGRP-binding nucleic acid

<220>
<221> misc_feature
<223> laboratory code: kn-158-6_a02_m13-fp.abi

<220>
<221> misc_feature
<223> all pyrimidines 2'-fluoro modified

<400> 87

gggaauucga gcucgguacc gcuuaagaca uauucuuccu acccucuauu gggucuuuac 60

gggcuuacuu uccuagggcg cugcaggcau gaagcuugg 99

<210> 88
<211> 99
<212> RNA
<213> Artificial Sequence

<220>
<223> CGRP-binding nucleic acid

<220>
<221> misc_feature
<223> laboratory code: kn-158-6_g10_m13-fp.abi

<220>
<221> misc_feature
<223> all pyrimidines 2'-fluoro modified

<400> 88

gggaauucga gcucgguacc agacaucacu uacaugugcu cugcguuuuu ugcaucguuu 60

uuuggucgag cgcuuccucc cugcaggcau gaagcuugg 99

<210> 89
<211> 98
<212> RNA
<213> Artificial Sequence

<220>
<223> CGRP-binding nucleic acid

<220>
<221> misc_feature
<223> laboratory code: kn-158-6_f11_m13-fp.abi

<220>
<221> misc_feature
<223> all pyrimidines 2'-fluoro modified

<400> 89

```
gggaauucga gcucgguacc uuguucugac acuguuuaug cguuuuacgc gucuuuaccg      60

ggcuccuucu ucccagugcc ugcaggcaug aagcuugg                              98
```

<210> 90
<211> 99
<212> RNA
<213> Artificial Sequence

<220>
<223> CGRP-binding nucleic acid

<220>
<221> misc_feature
<223> laboratory code: kn-158-6_h02 m13-fp.abi

<220>
<221> misc_feature
<223> all pyrimidines 2'-fluoro modified

<400> 90

```
gggaauucga gcucgguacc aauguaccau aguguuacau ugggcuugga auacagucuc      60

gacugaauuc gguuccccgg cugcaggcau gaagcuugg                             99
```

<210> 91
<211> 98
<212> RNA
<213> Artificial Sequence

<220>
<223> CGRP-binding nucleic acid

<220>
<221> misc_feature
<223> laboratory code: kn-158-6_h07_m13-fp.abi

<220>
<221> misc_feature
<223> all pyrimidines 2'-fluoro modified

<400> 91

gggaauucga gcucgguacc ugcgagcaua gacccacagu cuuuugcgau ucguuuucaa 60

acguguucgu gcguauuccc ugcaggcaug aagcuugg 98

<210> 92
<211> 99
<212> RNA
<213> Artificial Sequence

<220>
<223> CGRP-binding nucleic acid

<220>
<221> misc_feature
<223> laboratory code: kn-158-6_h05_m13-fp.abi

<220>
<221> misc_feature
<223> all pyrimidines 2'-fluoro modified

<400> 92

gggaauucga gcucgguacc aaaugguauu ggcccgagac ugugcuuucg cacaguuaau 60

caauaguuug cgauuguucc cugcaggcau gaagcuugg 99

<210> 93
<211> 49
<212> RNA
<213> Artificial Sequence

<220>
<223> CGRP-binding nucleic acid

<220>
<221> misc_feature
<223> laboratory code: STAR-R02_10-224

<400> 93
ggaccaacau gugaagaaca uacgguggaa gaaacgauug uuagacagg 49

<210> 94
<211> 51
<212> RNA
<213> Artificial Sequence

<220>
<223> CGRP-binding nucleic acid

<220>
<221> misc_feature
<223> laboratory code: STAR_R02-10-225

<400> 94
ggacaaaccu guguagaagg ggguaauaua caguagguag ggagggacag g 51

<210> 95
<211> 50
<212> RNA
<213> Artificial Sequence

<220>
<223> CGRP-binding nucleic acid

<220>
<221> misc_feature
<223> laboratory code: STAR-R02-10-227

<400> 95
ggacguaaug auccggcgau aaguccagga ugucaggccg gagagacagg 50

<210> 96
<211> 50
<212> RNA
<213> Artificial Sequence

<220>
<223> CGRP-binding nucleic acid

<220>
<221> misc_feature
<223> laboratory code: STAR-R02-10-228

<400> 96
ggacgaauga aguacggcgc accgggggug ugaaguuagc gugagacagg 50

<210> 97
<211> 50
<212> RNA
<213> Artificial Sequence

<220>
<223> CGRP-binding nucleic acid

<220>
<221> misc_feature
<223> laboratory code: STAR-R02-10-229

<400> 97
ggacguguug augcagcggg ugaacauuca caaacauccc uagcgacagg 50

<210> 98
<211> 50
<212> RNA
<213> Artificial Sequence

<220>
<223> CGRP-binding nucleic acid

<220>
<221> misc_feature
<223> laboratory code: STAR-R02-10-230

<400> 98

ggacgauagu ggugugucgu uaauucaccg ggaggguggc gagggacagg 50

<210> 99
<211> 50
<212> RNA
<213> Artificial Sequence

<220>
<223> CGRP-binding nucleic acid

<220>
<221> misc_feature
<223> laboratory code: STAR-R02-10-231

<400> 99
ggacagugcu ggugcggcug auugcauaua agcagugaac acccgacagg 50

<210> 100
<211> 50
<212> RNA
<213> Artificial Sequence

<220>
<223> CGRP-binding nucleic acid

<220>
<221> misc_feature
<223> laboratory code: STAR-R02-10-232

<400> 100
ggaccaacau gugaaaaaca uacgguggaa gaaacgauug uugagacagg 50

<210> 101
<211> 50
<212> RNA
<213> Artificial Sequence

<220>
<223> CGRP-binding nucleic acid

<220>
<221> misc_feature
<223> laboratory code: STAR-R02-10-234

<400> 101
ggaccaagua aacccuguga gccuugguaa agcggguggg aagggacagg 50

<210> 102
<211> 50
<212> RNA
<213> Artificial Sequence

<220>
<223> CGRP-binding nucleic acid

<220>
<221> misc_feature
<223> laboratory code: STAR-R02-10-235

<400> 102
ggacgacaug uuccgggaac auacggugaa agaaacgauu gucggacagg 50

<210> 103
<211> 50
<212> RNA
<213> Artificial Sequence

<220>
<223> CGRP-binding nucleic acid

<220>
<221> misc_feature
<223> laboratory code: STAR-R02-10-237

<400> 103
ggacuaguga gcgugggagg gccucacaaa acgaaaaguc cggggacagg 50

<210> 104
<211> 50
<212> RNA
<213> Artificial Sequence

<220>
<223> CGRP-binding nucleic acid

<220>
<221> misc_feature
<223> laboratory code: STAR-R02-10-239

<400> 104
ggacgauugc guaguggaag cauacgguga aagaaacgau aucggacagg 50

<210> 105
<211> 50
<212> RNA
<213> Artificial Sequence

<220>
<223> CGRP-binding nucleic acid

<220>
<221> misc_feature
<223> laboratory code: STAR-R02-10-240

<400> 105
ggacuauacg gugaaagaaa cgaggcguua ggaggaaacg cuaggacagg 50

<210> 106
<211> 51
<212> RNA
<213> Artificial Sequence

<220>
<223> CGRP-binding nucleic acid

<220>
<221> misc_feature

<223> laboratory code: STAR_R02-10-243

<400> 106
ggacgaacgu gcuggugcag cgugacuuag ucgaacaccc cuggggacag g 51

<210> 107
<211> 49
<212> RNA
<213> Artificial Sequence

<220>
<223> CGRP-binding nucleic acid

<220>
<221> misc_feature
<223> laboratory code: STAR-R02-10-244

<400> 107
ggacgccaaa aucaggaagg gagagaaaag gaaugcguac ggcgacagg 49

<210> 108
<211> 50
<212> RNA
<213> Artificial Sequence

<220>
<223> CGRP-binding nucleic acid

<220>
<221> misc_feature
<223> laboratory code: STAR-R02-10-248

<400> 108
ggacaauccu guggaguagg gugggggccu aguaggccua gugggacagg 50

<210> 109
<211> 50
<212> RNA
<213> Artificial Sequence

<220>
<223> CGRP-binding nucleic acid

<220>
<221> misc_feature
<223> laboratory code: STAR-R02-10-249

<400> 109
ggacgauccu guggaguagg gugggggccu aguaggccua gugggacagg 50

<210> 110
<211> 50
<212> RNA
<213> Artificial Sequence

<220>
<223> CGRP-binding nucleic acid

<220>
<221> misc_feature
<223> laboratory code: STAR-R02-10-250

<400> 110
ggacuuuagc ugcguaguag ggaaaagaag ggugggg cag cuccgacagg 50

<210> 111
<211> 50
<212> RNA
<213> Artificial Sequence

<220>
<223> CGRP-binding nucleic acid

<220>
<221> misc_feature
<223> laboratory code: STAR-R02-10-251

<400> 111
ggacuugaug acgcgaguua acgucgcugu cucucaauca aggagacagg 50

<210> 112
<211> 49
<212> RNA
<213> Artificial Sequence

<220>
<223> CGRP-binding nucleic acid

<220>
<221> misc_feature
<223> laboratory code: STAR-R02-10-252

<400> 112
ggaccagugg ugacgcggcu ggggcgccac agcgccggac gucgacagg 49

<210> 113
<211> 50
<212> RNA
<213> Artificial Sequence

<220>
<223> CGRP-binding nucleic acid

<220>
<221> misc_feature
<223> laboratory code: STAR-R02-10-254

<400> 113
ggacagagga acguaguagg guaagguagg augaggggguu ccucgacagg 50

<210> 114
<211> 50
<212> RNA
<213> Artificial Sequence

<220>

<223> CGRP-binding nucleic acid

<220>
<221> misc_feature
<223> laboratory code: STAR-R02-10-255

<400> 114
ggacuauuua agggccggua gagaaauaaa ccacccucuc uggagacagg 50

<210> 115
<211> 50
<212> RNA
<213> Artificial Sequence

<220>
<223> CGRP-binding nucleic acid

<220>
<221> misc_feature
<223> laboratory code: STAR-R02-10-257

<400> 115
ggacugaugg cgcgggauua cgccgcuggc gcuaggaaca aggcgacagg 50

<210> 116
<211> 50
<212> RNA
<213> Artificial Sequence

<220>
<223> CGRP-binding nucleic acid

<220>
<221> misc_feature
<223> laboratory code: STAR-R02-10-258

<400> 116
ggacgauccu guggaguagg gugggggccc agugggccua gugggacagg 50

<210> 117
<211> 50
<212> RNA
<213> Artificial Sequence

<220>
<223> CGRP-binding nucleic acid

<220>
<221> misc_feature
<223> laboratory code: STAR-R02-10-259

<400> 117
ggacguguug augcagcggg uggacauuca caaacauccc uggcgacagg 50

<210> 118
<211> 50
<212> RNA
<213> Artificial Sequence

<220>
<223> CGRP-binding nucleic acid

<220>
<221> misc_feature
<223> laboratory code: STAR-R02-10-261

<400> 118
ggacccugcu cgagaggacg agcauacggu gaaagaaacg augggacagg 50

<210> 119
<211> 50
<212> RNA
<213> Artificial Sequence

<220>
<223> CGRP-binding nucleic acid

<220>
<221> misc_feature
<223> laboratory code: STAR-R02-10-263

<400> 119
ggacauacga ugagagaagc gauucccgcu gcugacggga ugucgacagg 50

<210> 120
<211> 50
<212> RNA
<213> Artificial Sequence

<220>
<223> CGRP-binding nucleic acid

<220>
<221> misc_feature
<223> laboratory code: STAR-R02-10-264

<400> 120
ggacgauccu guggaguagg gugggggucu aguagaccua gugggacagg 50

<210> 121
<211> 50
<212> RNA
<213> Artificial Sequence

<220>
<223> CGRP-binding nucleic acid

<220>
<221> misc_feature
<223> laboratory code: STAR-R02-10-266

<400> 121
ggacagugau ggcgcagcug uaaccaagag guuagaacgc uggagacagg 50

<210> 122
<211> 50
<212> RNA

<213> Artificial Sequence

<220>
<223> CGRP-binding nucleic acid

<220>
<221> misc_feature
<223> laboratory code: STAR-R02-10-267

<400> 122
ggacugauuc ucagagaaua cgaugaaaga agcgauucag ucuggacagg 50

<210> 123
<211> 50
<212> RNA
<213> Artificial Sequence

<220>
<223> CGRP-binding nucleic acid

<220>
<221> misc_feature
<223> laboratory code: STAR-R02-10-268

<400> 123
ggacacaacu cgaaggagau gguagguagg acagcggggg uugugacagg 50

<210> 124
<211> 50
<212> RNA
<213> Artificial Sequence

<220>
<223> CGRP-binding nucleic acid

<220>
<221> misc_feature
<223> laboratory code: STAR-R02-10-270

<400> 124
ggaccaacau gugaaaagca uacggugaaa gaaacgauug uugagacagg 50

<210> 125
<211> 51
<212> RNA
<213> Artificial Sequence

<220>
<223> CGRP-binding nucleic acid

<220>
<221> misc_feature
<223> laboratory code: STAR-R02-10-271

<400> 125
ggacgugaug gcgcagcgac ugacacaaag gaucaggaac gccccgacag g 51

<211> 50

<212> RNA
<213> Artificial Sequence

<220>
<223> CGRP-binding nucleic acid

<220>
<221> misc_feature
<223> laboratory code: STAR-R02-10-272

<400> 126
ggacgaacug guaggguggc ugcccuauac gaugaaagaa gcgagacagg 50

<210> 127
<211> 50
<212> RNA
<213> Artificial Sequence

<220>
<223> CGRP-binding nucleic acid

<220>
<221> misc_feature
<223> laboratory code: STAR-R02-10-274

<400> 127
ggacuugaug acgcgaguua acgucgcugu cucucaauca aggggacagg 50

<210> 128
<211> 50
<212> RNA
<213> Artificial Sequence

<220>
<223> CGRP-binding nucleic acid

<220>
<221> misc_feature
<223> laboratory code: STAR-R02-10-275

<400> 128
ggacgacuga uggcgcgguc guguaauaaa acaacgccgc uggcgacagg 50

<210> 129
<211> 50
<212> RNA
<213> Artificial Sequence

<220>
<223> CGRP-binding nucleic acid

<220>
<221> misc_feature
<223> laboratory code: STAR-R02-10-276

<400> 129
ggacacaacu cgaaggagau gguagguagg acagcgggag uugugacagg 50

<210> 130
<211> 50
<212> RNA
<213> Artificial Sequence

<220>
<223> CGRP-binding nucleic acid

<220>
<221> misc_feature
<223> laboratory code: STAR-R02-10-278

<400> 130
ggacgauugc guagugggag cauacgguga aagaaacgau aucggacagg 50

<210> 131
<211> 50
<212> RNA
<213> Artificial Sequence

<220>
<223> CGRP-binding nucleic acid

<220>
<221> misc_feature
<223> laboratory code: STAR-R02-10-280

<400> 131
ggacauaugg ugaaaagaaa caauacuccg uuagugagga guuggacagg 50

<210> 132
<211> 50
<212> RNA
<213> Artificial Sequence

<220>
<223> CGRP-binding nucleic acid

<220>
<221> misc_feature
<223> laboratory code: STAR-R02-10-283

<400> 132
ggacguguug augcagcgag ugaacauuca cgaacauccc uggcgacagg 50

<210> 133
<211> 50
<212> RNA
<213> Artificial Sequence

<220>
<223> CGRP-binding nucleic acid

<220>
<221> misc_feature
<223> laboratory code: STAR-R02-10-286

<400> 133

ggacguguug acgcagcguu acggcuaagg accguagaac guuagacagg 50

<210> 134
<211> 50
<212> RNA
<213> Artificial Sequence

<220>
<223> CGRP-binding nucleic acid

<220>
<221> misc_feature
<223> laboratory code: STAR-R02-10-287

<400> 134
ggacacuguc agcgcgguga ugagaaaacg ccccuguaac agaugacagg 50

<210> 135
<211> 50
<212> RNA
<213> Artificial Sequence

<220>
<223> CGRP-binding nucleic acid

<220>
<221> misc_feature
<223> laboratory code: STAR-R02-10-288

<400> 135
ggacaacgaa guaaaccuua ggcgacccgc augaaggcgg gguggacagg 50

<210> 136
<211> 49
<212> RNA
<213> Artificial Sequence

<220>
<223> CGRP-binding nucleic acid

<220>
<221> misc_feature
<223> laboratory code: STAR-R02-10-289

<400> 136
ggacauacgg ugaaagaaac gauucggaac uucgaauccg auggacagg 49

<210> 137
<211> 50
<212> RNA
<213> Artificial Sequence

<220>
<223> CGRP-binding nucleic acid

<220>
<221> misc_feature
<223> laboratory code: STAR-R02-10-291

<400> 137
ggacgaauga aguacggcgc accgggagcg ugaaguuagc gugagacagg 50

<210> 138
<211> 50
<212> RNA
<213> Artificial Sequence

<220>
<223> CGRP-binding nucleic acid

<220>
<221> misc_feature
<223> laboratory code: STAR-R02-10-292

<400> 138
ggaccaacau guaaagagca uacgguggaa gaaacgauug uugagacagg 50

<210> 139
<211> 50
<212> RNA
<213> Artificial Sequence

<220>
<223> CGRP-binding nucleic acid

<220>
<221> misc_feature
<223> laboratory code: STAR-R02-10-293

<400> 139
ggacgacaug uuccgagaac auacggugaa auaaacgauu gucagacagg 50

<210> 140
<211> 48
<212> RNA
<213> Artificial Sequence

<220>
<223> CGRP-binding nucleic acid

<220>
<221> misc_feature
<223> laboratory code: STAR-R02-10-294

<400> 140
ggaccaacau guaagagcau acgguggaag aaacgauugu uagacagg 48

<210> 141
<211> 50
<212> RNA
<213> Artificial Sequence

<220>
<223> CGRP-binding nucleic acid

<220>
<221> misc_feature

<223> laboratory code: STAR-R02-10-295

<400> 141
ggacgaccca ucaaggauga agagcguacg cuugcgcagg ggucgacagg 50

<210> 142
<211> 50
<212> RNA
<213> Artificial Sequence

<220>
<223> CGRP-binding nucleic acid

<220>
<221> misc_feature
<223> laboratory code: STAR-R02-10-296

<400> 142
ggaccguguu agugcugcgg auauaaaaac acugcugucu augggacagg 50

<210> 143
<211> 50
<212> RNA
<213> Artificial Sequence

<220>
<223> CGRP-binding nucleic acid

<220>
<221> misc_feature
<223> laboratory code: STAR-R02-10-297

<400> 143
ggacgaggug cuggcgcugc cuaugcccga uuggguaaaa cgccgacagg 50

<210> 144
<211> 50
<212> RNA
<213> Artificial Sequence

<220>
<223> CGRP-binding nucleic acid

<220>
<221> misc_feature
<223> laboratory code: STAR-R02-10-298

<400> 144
ggaccaagua aacccuguaa gccuugguaa agcggguggg aagggacagg 50

<210> 145
<211> 50
<212> RNA
<213> Artificial Sequence

<220>
<223> CGRP-binding nucleic acid

<220>
<221> misc_feature
<223> laboratory code: STAR-R02-10-300

<400> 145
ggacaacccg ucaaggauga agagcguacg cuugcgcagg guucgacagg 50

<210> 146
<211> 50
<212> RNA
<213> Artificial Sequence

<220>
<223> CGRP-binding nucleic acid

<220>
<221> misc_feature
<223> laboratory code: STAR-R02-10-303

<400> 146
ggacgagaug ggcguaauaa gauggggaaa auagccgaaa gcccgacagg 50

<210> 147
<211> 50
<212> RNA
<213> Artificial Sequence

<220>
<223> CGRP-binding nucleic acid

<220>
<221> misc_feature
<223> laboratory code: STAR-R02-10-304

<400> 147
ggacucucug auggcgcgga gagaauauua cgccacuguc gugagacagg 50

<210> 148
<211> 50
<212> RNA
<213> Artificial Sequence

<220>
<223> CGRP-binding nucleic acid

<220>
<221> misc_feature
<223> laboratory code: STAR-R02-10-307

<400> 148
ggacgaacga gcauggacgc gggugaggag agggcgcuag uucggacagg 50

<210> 149
<211> 50
<212> RNA
<213> Artificial Sequence

<220>

<223> CGRP-binding nucleic acid

<220>
<221> misc_feature
<223> laboratory code: STAR-R02-10-310

<400> 149
ggacgacgag gaauuggugg gggaugggguu gaggauccuc gcgggacagg 50

<210> 150
<211> 50
<212> RNA
<213> Artificial Sequence

<220>
<223> CGRP-binding nucleic acid

<220>
<221> misc_feature
<223> laboratory code: STAR-R02-10-311

<400> 150
ggacgauugc auagugagag cauacgguga aagaaacgau aucggacagg 50

<210> 151
<211> 50
<212> RNA
<213> Artificial Sequence

<220>
<223> CGRP-binding nucleic acid

<220>
<221> misc_feature
<223> laboratory code: STAR-R02-10-314

<400> 151
ggacgugaug gugcagcgua gucgguuaag acaaacaccc cugggacagg 50

<210> 152
<211> 50
<212> RNA
<213> Artificial Sequence

<220>
<223> CGRP-binding nucleic acid

<220>
<221> misc_feature
<223> laboratory code: STAR-R02-10-315

<400> 152
ggacauacga ugagagaagc gauucccgcu gcuaacggga ugucgacagg 50

<210> 153
<211> 50
<212> RNA
<213> Artificial Sequence

<220>
<223> CGRP-binding nucleic acid

<220>
<221> misc_feature
<223> laboratory code: STAR-R02-10-318

<400> 153
ggacauacga ugaaagaagc gauucccgcu gcugacggga ugucgacagg 50

<210> 154
<211> 50
<212> RNA
<213> Artificial Sequence

<220>
<223> CGRP-binding nucleic acid

<220>
<221> misc_feature
<223> laboratory code: STAR-R02-12MW-A1

<400> 154
ggacgacaug uucccggaac auacggugaa agaaacgauu aucggacagg 50

<210> 155
<211> 50
<212> RNA
<213> Artificial Sequence

<220>
<223> CGRP-binding nucleic acid

<220>
<221> misc_feature
<223> laboratory code: STAR-R02-12MW-A4

<400> 155
ggacgacaug cuccaggagc auacggugaa agaaacgauu gucggacagg 50

<210> 156
<211> 50
<212> RNA
<213> Artificial Sequence

<220>
<223> CGRP-binding nucleic acid

<220>
<221> misc_feature
<223> laboratory code: STAR-R02-12MW-A5

<400> 156
ggacgacaug uuccaggaac auacggugaa agaaacgauu gucggacagg 50

<210> 157
<211> 50
<212> RNA

<213> Artificial Sequence

<220>
<223> CGRP-binding nucleic acid

<220>
<221> misc_feature
<223> laboratory code: STAR-R02-12MW-B5

<400> 157
ggacgauaug uuccaagaac auacggugaa agaaacgauu gucggacagg 50

<210> 158
<211> 50
<212> RNA
<213> Artificial Sequence

<220>
<223> CGRP-binding nucleic acid

<220>
<221> misc_feature
<223> laboratory code: STAR-R02-12MW-D2

<400> 158
ggacauacga ugaaagaagc gauucccgcu gcuagcggga ugucgacagg 50

<210> 159
<211> 50
<212> RNA
<213> Artificial Sequence

<220>
<223> CGRP-binding nucleic acid

<220>
<221> misc_feature
<223> laboratory code: STAR-R02-12MW-E1

<400> 159
ggacuacaag ccaacaaauc cuugcccacu gaggaucugc ugucgacagg 50

<210> 160
<211> 49
<212> RNA
<213> Artificial Sequence

<220>
<223> CGRP-binding nucleic acid

<220>
<221> misc_feature
<223> laboratory code: STAR-R02-12MW-E3

<400> 160
ggacucauac ggugaaagaa acgauucguc uugugacgau gaggacagg 49

<210> 161

<211> 50
<212> RNA
<213> Artificial Sequence

<220>
<223> CGRP-binding nucleic acid

<220>
<221> misc_feature
<223> laboratory code: STAR-R02-12MW-E4

<400> 161
ggacgacaug uucggggaac auacggugaa agaaacgauu gucggacagg 50

<210> 162
<211> 48
<212> RNA
<213> Artificial Sequence

<220>
<223> CGRP-binding nucleic acid

<220>
<221> misc_feature
<223> laboratory code: STAR-R02-12MW-F3

<400> 162
ggacauacgg ugaaagaaac gauacgcaau uuguguuguc ccgacagg 48

<210> 163
<211> 50
<212> RNA
<213> Artificial Sequence

<220>
<223> CGRP-binding nucleic acid

<220>
<221> misc_feature
<223> laboratory code: STAR-R02-12MW-F4

<400> 163
ggacgauugc auacuaaaag cauacgguga aagaaacgau aucggacagg 50

<210> 164
<211> 50
<212> RNA
<213> Artificial Sequence

<220>
<223> CGRP-binding nucleic acid

<220>
<221> misc_feature
<223> laboratory code: STAR-R02-12MW-F6

<400> 164
ggacucauac ggugaaagaa acgauucguc uuaacgacga ugaggacagg 50

<210> 165
<211> 47
<212> RNA
<213> Artificial Sequence

<220>
<223> CGRP-binding nucleic acid

<220>
<221> misc_feature
<223> laboratory code: STAR-R02-12MW-G1

<400> 165
ggacuaagug agccaaguca gcgggauguc cauaacuugu cgacagg 47

<210> 166
<211> 52
<212> RNA
<213> Artificial Sequence

<220>
<223> CGRP-binding nucleic acid

<220>
<221> misc_feature
<223> laboratory code: STAR-R02-12MW-H1

<400> 166
ggacauacgg ugaaagaaac gauacauaau uuauguuccu guccaagaca gg 52

<210> 167
<211> 50
<212> RNA
<213> Artificial Sequence

<220>
<223> CGRP-binding nucleic acid

<220>
<221> misc_feature
<223> laboratory code: STAR-R02-12MW-H2

<220>
<221> misc_feature
<222> (18)..(18)
<223> any nucleotide

<400> 167
ggacgauugc acaauganag cauacgguga aagaaacgau aucggacagg 50

<210> 168
<211> 50
<212> RNA
<213> Artificial Sequence

<220>
<223> CGRP-binding nucleic acid

<220>
<221> misc_feature
<223> laboratory code: STAR-R02-15d-A1

<400> 168
ggacgacaug uucuaugaac auacggugaa agaaacgauu gucggacagg 50

<210> 169
<211> 50
<212 > RNA
<213> Artificial Sequence

<220>
<223> CGRP-binding nucleic acid

<220>
<221> misc_feature
<223> laboratory code: STAR-15d-A10

<400> 169
ggacgauugc auaguaagag cauacgguga aagaaacgau aucggacagg 50

<210> 170
<211> 50
<212> RNA
<213> Artificial Sequence

<220>
<223> CGRP-binding nucleic acid

<220>
<221> misc_feature
<223> laboratory code: STAR-R02-15d-A12

<400> 170
ggacgacaug uuccaggaac auacggugaa aaaaacgauu gucggacagg 50

<210> 171
<211> 50
<212> RNA
<213> Artificial Sequence

<220>
<223> CGRP-binding nucleic acid

<220>
<221> misc_feature
<223> laboratory code: STAR-R02-15d-A2

<400> 171
ggacgacaug uucuaggaac auacggugaa agaaacgauu guuggacagg 50

<210> 172
<211> 50
<212> RNA
<213> Artificial Sequence

<220>

<223> CGRP-binding nucleic acid

<220>
<221> misc_feature
<223> laboratory code: STAR-R02-15d-A9

<400> 172
ggacauacga ugaaagaagc gauucccgcu acuagcggga ugucgacagg 50

<210> 173
<211> 50
<212> RNA
<213> Artificial Sequence

<220>
<223> CGRP-binding nucleic acid

<220>
<221> misc_feature
<223> laboratory code: STAR-R02-15d-B11

<400> 173
ggaccaacau gcaaagagca uacggugaaa gaaacgauug uugagacagg 50

<210> 174
<211> 49
<212> RNA
<213> Artificial Sequence

<220>
<223> CGRP-binding nucleic acid

<220>
<221> misc_feature
<223> laboratory code: STAR-R02-15d-B3

<400> 174
ggacauaugg ugaaagaaac aauucggaac uucgauuccg auggacagg 49

<210> 175
<211> 50
<212> RNA
<213> Artificial Sequence

<220>
<223> CGRP-binding nucleic acid

<220>
<221> misc_feature
<223> laboratory code: STAR-R02-15d-C10

<400> 175
ggacgauugc auaauaaaag cauacgguga aagaaacgau aucggacagg 50

<210> 176
<211> 50
<212> RNA
<213> Artificial Sequence

<220>
<223> CGRP-binding nucleic acid

<220>
<221> misc_feature
<223> laboratory code: STAR-R02-15d-C9

<400> 176
ggacgacaug uucuaagaac auacggugaa agaaacgauu gucggacagg 50

<210> 177
<211> 51
<212> RNA
<213> Artificial Sequence

<220>
<223> CGRP-binding nucleic acid

<220>
<221> misc_feature
<223> laboratory code: STAR-R02-15d-D10

<400> 177
ggacgacaug uuccaaggaa cauacgguga aagaaacgau ugucggacag g 51

<210> 178
<211> 50
<212> RNA
<213> Artificial Sequence

<220>
<223> CGRP-binding nucleic acid

<220>
<221> misc_feature
<223> laboratory code: STAR-R02-15d-D7

<400> 178
ggaccaacau gugaagagca uacggugaaa gaaacgauug uugagacagg 50

<210> 179
<211> 50
<212> RNA
<213> Artificial Sequence

<220>
<223> CGRP-binding nucleic acid

<220>
<221> misc_feature
<223> laboratory code: STAR-R02-15d-D9

<400> 179
ggacgauugc auagugggug cauacgguga aagaaacgau aucggacagg 50

<210> 180
<211> 50
<212> RNA

<213> Artificial Sequence

<220>
<223> CGRP-binding nucleic acid

<220>
<221> misc_feature
<223> laboratory code: STAR-R02-15d-E11

<400> 180
ggacgauugc auaguaaaag cauacgguga aagaaacgau aucggacagg 50

<210> 181
<211> 50
<212> RNA
<213> Artificial Sequence

<220>
<223> CGRP-binding nucleic acid

<220>
<221> misc_feature
<223> laboratory code: STAR-R02-15d-E12

<400> 181
ggacgacaug uucaaagaac auacggugaa agaaacgauu gucggacagg 50

<210> 182
<211> 50
<212> RNA
<213> Artificial Sequence

<220>
<223> CGRP-binding nucleic acid

<220>
<221> misc_feature
<223> laboratory code: STAR-R02-15d-E3

<400> 182
ggaccauacg augaaagaag cgauacugca ccagaagcag uugggacagg 50

<210> 183
<211> 48
<212> RNA
<213> Artificial Sequence

<220>
<223> CGRP-binding nucleic acid

<220>
<221> misc_feature
<223> laboratory code: STAR-R02-15d-F8

<400> 183
ggacgauugc auuaagagca uacggugaaa gaaacgauau cggacagg 48

<210> 184

<211> 50
<212> RNA
<213> Artificial Sequence

<220>
<223> CGRP-binding nucleic acid

<220>
<221> misc_feature
<223> laboratory code: STAR-R02-15d-F9

<400> 184
ggacgacaug uuccgagaac auacggugaa agaaacgauu gucggacagg 50

<210> 185
<211> 50
<213> Artificial Sequence

<220>
<223> CGRP-binding nucleic acid

<220>
<221> misc_feature
<223> laboratory code: STAR-R02-15d-G1

<400> 185
ggacgauugc auaguagaag cauacgguga aagaaacgau aucggacagg 50

<210> 186
<211> 50
<212> RNA
<213> Artificial Sequence

<220>
<223> CGRP-binding nucleic acid

<220>
<221> misc_feature
<223> laboratory code: STAR-R02-15d-G12

<400> 186
ggacucauac ggugaaagaa acgauucguc uugacgacga ugaggacagg 50

<210> 187
<211> 50
<212> RNA
<213> Artificial Sequence

<220>
<223> CGRP-binding nucleic acid

<220>
<221> misc_feature
<223> laboratory code: STAR-R02-15d-G2

<400> 187
ggacauacga ugaaagaagc gauucccguu acuagcggga ugucgacagg 50

<210> 188
<211> 50
<212> RNA
<213> Artificial Sequence

<220>
<223> CGRP-binding nucleic acid

<220>
<221> misc_feature
<223> laboratory code: STAR-R02-15d-H1

<400> 188
ggacgacaug uucccagaac auacggugaa agaaacgauu gucggacagg 50

<210> 189
<211> 47
<212> RNA
<213> Artificial Sequence

<220>
<223> CGRP-binding nucleic acid

<220>
<221> misc_feature
<223> laboratory code: STAR-R02-15d-H7

<400> 189
ggacauacgg ugaaagaaac gauucggacu ugaguccgau ggacagg 47

<210> 190
<211> 50
<212> RNA
<213> Artificial Sequence

<220>
<223> CGRP-binding nucleic acid

<220>
<221> misc_feature
<223> laboratory code: STAR-R02-15d-H9

<400> 190
ggacgauugc auaauaagag cauacgguga aagaaacgau aucggacagg 50

<210> 191
<211> 48
<212> RNA
<213> Artificial Sequence

<220>
<223> CGRP-binding and/or amylin-binding nucleic acid

<220>
<221> misc_feature
<223> laboratory code: STAR-R02-15xx-A11

<400> 191

ggacugaugg cgcggucuca aaaaacgccg auagggugag gggacagg 48

<210> 192
<211> 48
<212> RNA
<213> Artificial Sequence

<220>
<223> CGRP-binding nucleic acid

<220>
<221> misc_feature
<223> laboratory code: STAR-R02-15xx-A7

<400> 192
ggacugaugg cgcggucuua aaaaacgccg auggggugag gggacagg 48

<210> 193
<211> 49
<212> RNA
<213> Artificial Sequence

<220>
<223> CGRP-binding nucleic acid

<220>
<221> misc_feature
<223> laboratory code: STAR-R02-15xx-B10

<400> 193
ggacucauac ggugaaagaa acgauucguc uagcgacgau gaggacagg 49

<210> 194
<211> 50
<212> RNA
<213> Artificial Sequence

<220>
<223> CGRP-binding nucleic acid

<220>
<221> misc_feature
<223> laboratory code: STAR-R02-15xx-B7

<220>
<221> misc_feature
<222> (37)..(37)
<223> any nucleotide

<400> 194
ggacucauac ggugaaagaa acgauucguc uuagcgncga ugaggacagg 50

<210> 195
<211> 48
<212> RNA
<213> Artificial Sequence

<220>

<223> CGRP-binding nucleic acid

<220>
<221> misc_feature
<223> laboratory code: STAR-R02-15xx-C7

<220>
<221> misc_feature
<222> (17)..(17)
<223> any nucleotide

<400> 195
ggacugaugg cgcggunuca aaaaacgccg auagggugag gggacagg 48

<210> 196
<211> 48
<212> RNA
<213> Artificial Sequence

<220>
<223> CGRP-binding and/or amylin-binding nucleic acid

<220>
<221> misc_feature
<223> laboratory code: STAR-R02-15xx-C9

<400> 196
ggacugaugg cgcggucuca aaaaacgccg cuagggugag gggacagg 48

<210> 197
<211> 48
<212> RNA
<213> Artificial Sequence

<220>
<223> CGRP-binding nucleic acid

<220>
<221> misc_feature
<223> laboratory code: STAR-R02-15xx-D8

<400> 197
ggacagacga uggccguaaa ucaucgggaa aggggaugga gggacagg 48

<210> 198
<211> 52
<212> RNA
<213> Artificial Sequence

<220>
<223> CGRP-binding nucleic acid

<220>
<221> misc_feature
<223> laboratory code: STAR-R02-15xx-E9

<400> 198
ggacugaugg cgcggucgau aaauacgccc gugaacuggg gaaguggaca gg 52

<210> 199
<211> 48
<212> RNA
<213> Artificial Sequence

<220>
<223> CGRP-binding nucleic acid

<220>
<221> misc_feature
<223> laboratory code: STAR-R02-15xx-F12

<400> 199
ggacugaugg cgcgguccua uuacgccgaa agggagaggg gagacagg 48

<210> 200
<211> 48
<212> RNA
<213> Artificial Sequence

<220>
<223> CGRP-binding nucleic acid

<220>
<221> misc_feature
<223> laboratory code: STAR-R02-15xx-F7

<400> 200
ggacugaugg cgcggucuua aaaaacgccg auagggugag gggacagg 48

<210> 201
<211> 48
<212> RNA
<213> Artificial Sequence

<220>
<223> CGRP-binding nucleic acid

<220>
<221> misc_feature
<223> laboratory code: STAR-R02-15xx-G10

<400> 201
ggacagacga uggccauaaa ucaucgggaa aggggaugga gggacagg 48

<210> 202
<211> 49
<212> RNA
<213> Artificial Sequence

<220>
<223> CGRP-binding nucleic acid

<220>
<221> misc_feature
<223> laboratory code: STAR-R02-15xx-G12

<400> 202

ggacucauac ggugaaagaa acgauucguc uuaggacgau gaggacagg 49

<210> 203
<211> 49
<212> RNA
<213> Artificial Sequence

<220>
<223> CGRP-binding nucleic acid

<220>
<221> misc_feature
<223> laboratory code: STAR-R02-15xx-G7

<220>
<221> misc_feature
<222> (13)..(13)
<223> any nucleotide

<400> 203
ggacagacga ugnccguaaa ucaucgggaa agggcaugga ggguacagg 49

<210> 204
<211> 50
<212> RNA
<213> Artificial Sequence

<220>
<223> CGRP-binding nucleic acid

<220>
<221> misc_feature
<223> laboratory code: STAR-R03-11-A4

<400> 204
ggacgacaug uucgcagaac auacggugaa agaaacgauu gucggacagg 50

<210> 205
<211> 50
<212> RNA
<213> Artificial Sequence

<220>
<223> CGRP-binding nucleic acid

<220>
<221> misc_feature
<223> laboratory code: STAR-R03-11-A6

<400> 205
ggacauacgg ugaaagaaac gauucccgcu acuagcggga ugucgacagg 50

<210> 206
<211> 51
<212> RNA
<213> Artificial Sequence

<220>

<223> CGRP-binding nucleic acid

<220>
<221> misc_feature
<223> laboratory code: STAR-R03-11-A7

<400> 206
ggacgacaug uucaaaagaa cauacgguga aagaaacgau ugucggacag g 51

<210> 207
<211> 50
<212> RNA
<213> Artificial Sequence

<220>
<223> CGRP-binding nucleic acid

<220>
<221> misc_feature
<223> laboratory code: STAR-R03-11-B10

<400> 207
ggacauacgg ugaaagaaac gauacaugau uuauguuguc ccgagacagg 50

<210> 208
<211> 50
<212> RNA
<213> Artificial Sequence

<220>
<223> CGRP-binding nucleic acid

<220>
<221> misc_feature
<223> laboratory code: STAR-R03-11-B7

<400> 208
ggacauacgg ugaaagaaac gauucucgcu gcuagcgaga ugucgacagg 50

<210> 209
<211> 51
<212> RNA
<213> Artificial Sequence

<220>
<223> CGRP-binding nucleic acid

<220>
<221> misc_feature
<223> laboratory code: STAR-R03-11-C12

<400> 209
ggacgacaug uucccaggaa cauacgguga aagaaacgau ugucggacag g 51

<210> 210
<211> 50
<212> RNA
<213> Artificial Sequence

<220>
<223> CGRP-binding nucleic acid

<220>
<221> misc_feature
<223> laboratory code: STAR-R03-11-C6

<400> 210
ggacgacaug uucggagaac auacggugaa agaaacgauu gucggacagg 50

<210> 211
<211> 48
<212> RNA
<213> Artificial Sequence

<220>
<223> CGRP-binding nucleic acid

<220>
<221> misc_feature
<223> laboratory code: STAR-R03-11-C7

<400> 211
ggacauacgg ugaaagaaac gauucggauu accaauccga uggacagg 48

<210> 212
<211> 50
<212> RNA
<213> Artificial Sequence

<220>
<223> CGRP-binding nucleic acid

<220>
<221> misc_feature
<223> laboratory code: STAR-R03-11-C8

<400> 212
ggacucauac ggugaaagaa acgauucguc uuagcgacga ugaggacagg 50

<210> 213
<211> 50
<212> RNA
<213> Artificial Sequence

<220>
<223> CGRP-binding nucleic acid

<220>
<221> misc_feature
<223> laboratory code: STAR-R03-11-C9

<400> 213
ggacgacaug uuccaagaac auacggugaa agaaacgauu gucggacagg 50

<210> 214
<211> 50
<212> RNA

<213> Artificial Sequence

<220>
<223> CGRP-binding nucleic acid

<220>
<221> misc_feature
<223> laboratory code: STAR-R03-11-D5

<400> 214
ggacgacaug uucauagaac auacggugaa agaaacgauu gucagacagg 50

<210> 215
<211> 50
<212> RNA
<213> Artificial Sequence

<220>
<223> CGRP-binding nucleic acid

<220>
<221> misc_feature
<223> laboratory code: STAR-R03-11-E11

<400> 215
ggacgacaug uucuaggaac auacggugaa agaaacgauu gucggacagg 50

<210> 216
<211> 51
<212> RNA
<213> Artificial Sequence

<220>
<223> CGRP-binding nucleic acid

<220>
<221> misc_feature
<223> laboratory code: STAR-R03-11-E5

<400> 216
ggacgacaug uuccauggaa cauacgguga aagaaacgau ugucggacag g 51

<210> 217
<211> 50
<212> RNA
<213> Artificial Sequence

<220>
<223> CGRP-binding nucleic acid

<220>
<221> misc_feature
<223> laboratory code: STAR-R03-11-E6

<400> 217
ggacgacaug uucagggaac auacggugaa agaaacgauu gucggacagg 50

<210> 218

<211> 48
<212> RNA
<213> Artificial Sequence

<220>
<223> CGRP-binding nucleic acid

<220>
<221> misc_feature
<223> laboratory code: STAR-R03-11-E7

<400> 218
ggacagacga uggccuuaaa ucaucgggaa aggggaugga gggacagg 48

<210> 219
<211> 50
<212> RNA
<213> Artificial Sequence

<220>
<223> CGRP-binding nucleic acid

<220>
<221> misc_feature
<223> laboratory code: STAR-R03-11-E8

<400> 219
ggacgacaug uucuaggaac auacgaugaa agaagcgauu gucggacagg 50

<210> 220
<211> 50
<212> RNA
<213> Artificial Sequence

<220>
<223> CGRP-binding nucleic acid

<220>
<221> misc_feature
<223> laboratory code: STAR-R03-11-F10

<400> 220
ggacauacgg ugaaagaaac gauacauaau uuauguuguc ccgagacagg 50

<210> 221
<211> 50
<212> RNA
<213> Artificial Sequence

<220>
<223> CGRP-binding nucleic acid

<220>
<221> misc_feature
<223> laboratory code: STAR-R03-11-F5

<400> 221
ggacguccau acggugaaag aaacgauagg gauagacucc cuuggacagg 50

<210> 222
<211> 49
<212> RNA
<213> Artificial Sequence

<220>
<223> CGRP-binding nucleic acid

<220>
<221> misc_feature
<223> laboratory code: STAR-R03-11-F6

<400> 222
ggacauacgg ugaaagaaac gauucggaac uucgauuccg auggacagg 49

<210> 223
<211> 50
<212> RNA
<213> Artificial Sequence

<220>
<223> CGRP-binding nucleic acid

<220>
<221> misc_feature
<223> laboratory code: STAR-R03-11-F7

<400> 223
ggacgacaug uucuacgaac auacggugaa agaaacgauu gucggacagg 50

<210> 224
<211> 50
<212> RNA
<213> Artificial Sequence

<220>
<223> CGRP-binding nucleic acid

<220>
<221> misc_feature
<223> laboratory code: STAR-R03-11-G10

<400> 224
ggaccaacgc uuaagcaaac cccacuuaca cuuaagcguc gagcgacagg 50

<210> 225
<211> 50
<212> RNA
<213> Artificial Sequence

<220>
<223> CGRP-binding nucleic acid

<220>
<221> misc_feature
<223> laboratory code: STAR-R03-11-G12

<400> 225

ggacauacgg ugaaagaaac gauucccacu acuagcggga ugucgacagg 50

<210> 226
<211> 50
<212> RNA
<213> Artificial Sequence

<220>
<223> CGRP-binding nucleic acid

<220>
<221> misc_feature
<223> laboratory code: STAR-R03-11-G4

<400> 226
ggacgacaug uucaaggaac auacggugaa agaaacgauu gucggacagg 50

<210> 227
<211> 50
<212> RNA
<213> Artificial Sequence

<220>
<223> CGRP-binding nucleic acid

<220>
<221> misc_feature
<223> laboratory code: STAR-R03-11-G8

<400> 227
ggacgacaug uucagagaac auacggugaa agaaacgauu gucagacagg 50

<210> 228
<211> 50
<212> RNA
<213> Artificial Sequence

<220>
<223> CGRP-binding nucleic acid

<220>
<221> misc_feature
<223> laboratory code: STAR-R03-11-G9

<400> 228
ggacauacga ugaaagaagc gauucccguu gcuagcggga ugucgacagg 50

<210> 229
<211> 49
<212> RNA
<213> Artificial Sequence

<220>
<223> CGRP-binding nucleic acid

<220>
<221> misc_feature
<223> laboratory code: STAR-R03-11-H12

<400> 229
ggaccauuau cccccaggau auacggugaa agaaacgaua ugggacagg 49

<210> 230
<211> 50
<212> RNA
<213> Artificial Sequence

<220>
<223> CGRP-binding nucleic acid

<220>
<221> misc_feature
<223> laboratory code: STAR-R03-11-H5

<400> 230
ggacgacaug uucaaagaac auacggugaa agaaacgauu gucggacagg 50

<210> 231
<211> 50
<212> RNA
<213> Artificial Sequence

<220>
<223> CGRP-binding nucleic acid

<220>
<221> misc_feature
<223> laboratory code: STAR-R03-11-H9

<400> 231
ggacgacaug uucagagaac auacggugaa agaaacgauu gucggacagg 50

<210> 232
<211> 45
<212> RNA
<213> Artificial Sequence

<220>
<223> CGRP-binding nucleic acid

<220>
<221> misc_feature
<223> laboratory code: STAR-R03-11-F10-45-001

<400> 232
cgggacauac ggugaaagaa acgauacaua auuuauguug ucccg 45

<210> 233
<211> 48
<212> RNA
<213> Artificial Sequence

<220>
<223> CGRP-binding nucleic acid

<220>
<221> misc_feature

<223> laboratory code: STAR-R03-11-C12-48-001

<400> 233
ggccgacaug uucccaggaa cauacgguga aagaaacgau ugucgucc 48

<210> 234
<211> 79
<212> RNA
<213> Artificial Sequence

<220>
<223> CGRP-binding nucleic acid

<220>
<221> misc_feature
<223> laboratory code: G 12

<400> 234

ggagcucagc cuucacugca ucgaggcgau ccaaguugua ggaaugggu ggcuuggagg 60

gcaccacggu cggauccag 79

<210> 235
<211> 78
<212> RNA
<213> Artificial Sequence

<220>
<223> CGRP-binding nucleic acid

<220>
<221> misc_feature
<223> laboratory code: G 12a

<400> 235

ggagcucagc cuucacugca ucgaggcauc caaguuauag gaauagggug gcuugaaggg 60

caccacgguc ggauccag 78

<210> 236
<211> 78
<212> RNA
<213> Artificial Sequence

<220>
<223> CGRP-binding nucleic acid

<220>
<221> misc_feature
<223> laboratory code: G 12b

<400> 236

ggagcucagc cuucacugca ucgaggcauc caaguuauag gaauagggug gcuugaaggg 60

caccacgguc ggauccag 78

<210> 237
<211> 78
<212> RNA
<213> Artificial Sequence

<220>
<223> CGRP-binding nucleic acid

<220>
<221> misc_feature
<223> laboratory code: G 12c

<400> 237

ggagcucagc cuucacugca ucgaggcgau ccaaguugag gaaugggggug gcuuggaggg 60

caccacgguc ggauccag 78

<210> 238
<211> 81
<212> RNA
<213> Artificial Sequence

<220>
<223> CGRP-binding nucleic acid

<220>
<221> misc_feature
<223> laboratory code: G 12d

<400> 238

ggagcucagc cuucacugca ucgaaggcga uccaaguugu aagaaugggg guggcuugga 60

gggcaccacg gucggaucca g 81

<210> 239
<211> 79
<212> RNA
<213> Artificial Sequence

<220>
<223> CGRP-binding nucleic acid

<220>
<221> misc_feature
<223> laboratory code: G 12e

<400> 239

ggagcucagc cuucacugca ucgaggcgau ccaaguugua ggaauuggau gacuuggagg 60

gcaccacggu cggauccag 79

<210> 240
<211> 80
<212> RNA
<213> Artificial Sequence

<220>
<223> CGRP-binding nucleic acid

<220>
<221> misc_feature
<223> laboratory code: G 12f

<400> 240

ggagcucagc cuucacugca ucgagggcga uccaaguugu aggaaugggg uggcuuggag 60

ggcaccacgg ucggauccag 80

<210> 241
<211> 79
<212> RNA
<213> Artificial Sequence

<220>
<223> CGRP-binding nucleic acid

<220>
<221> misc_feature
<223> laboratory code: G 12g

<400> 241

ggagcucagc cuucacugca ucgaagcgau ccaaguugua ggaaugggguagcuuggagg 60

gcaccacggu cggauccag 79

<210> 242
<211> 78
<212> RNA
<213> Artificial Sequence

<220>
<223> CGRP-binding nucleic acid

<220>
<221> misc_feature
<223> laboratory code: H 08a

<400> 242

ggagcucagc cuucacugcg ggugggaggg uggaugguga agaacgagcg cugaccgcgg 60

caccacgguc ggauccag 78

<210> 243
<211> 78
<212> RNA

<220>
<223> CGRP-binding nucleic acid

<220>
<221> misc_feature
<223> laboratory code: H 08b

<400> 243

ggagcucagc cuucacugcg ggugggaggg uggauggugg agaacgagca cugaccucgg 60

caccacgguc ggauccag 78

<210> 244
<211> 79
<212> RNA
<213> Artificial Sequence

<220>
<223> CGRP-binding nucleic acid

<220>
<221> misc_feature
<223> laboratory code: H 03a

<400> 244

ggagcucagc cuucacugcc auugaggaua ugccgcgggu ccgcauggag ugggguguugg 60

gcaccacggu cggauccag 79

<210> 245
<211> 42
<212> RNA
<213> Artificial Sequence

<220>
<223> CGRP-binding and/or amylin-binding nucleic acid

<220>
<221> misc_feature
<223> laboratory code: STAR-R02-15xx-014 (= NOX-504-014)

<400> 245
ggacugaugg cgcgguccua uuacgccgaa agggagaggg ga 42

<210> 246

<211> 42
<212> RNA
<213> Artificial Sequence

<220>
<223> CGRP-binding and/or amylin-binding nucleic acid

<220>
<221> misc_feature
<223> laboratory code: NOX-504-089

<400> 246
ggacugaugg cgcgguccua uuacgccgau agggugaggg ga 42

<210> 247
<211> 73
<212> DNA
<213> Artificial Sequence

<220>
<223> CGRP-binding and/or amylin-binding nucleic acid

<220>
<221> misc_feature
<223> laboratory code: NOX-504-ad3-18%-Bibliothek

<220>
<221> misc_feature
<223> laboratory code: NOX-504-ad3-18%-Bibliothek (reverser ssDNA-Stran g)

<220>
<221> misc_feature
<222> (1)..(2)
<223> O-methylated nucleotide

<220>
<221> misc_feature
<222> (7)..(44)
<223> other nucleotides at a mutation rate of 0.18

<400> 247

**ccgtgctccc ctctcccttt cggcgtaata ggaccgcgcc atcagtccta tagtgagtcg 60**

**tattagtagt cgc 73**

<210> 248
<211> 24
<212> DNA
<213> Artificial Sequence

<220>
<223> primer

<220>
<221> misc_feature
<223> laboratory code: STAR-1 Reverse Primer Ribo U (=STAR-1 Reverse M atrix)

<220>
<221> misc_feature
<223> DNA with ribonucleotides at positions 12 and 18

<400> 248
gcgagttcag cucagcgucc gtgc 24

<210> 249
<211> 24
<212> DNA
<213> Artificial Sequence

<220>
<223> primer

<220>
<221> misc_feature
<223> laboratory code: STAR-1 N+1 Reverse Matrix Ribo I

<220>
<221> misc_feature
<223> DNA with ribonucleotides at positions 12 and 18

<220>
<221> misc_feature
<222> (18).. (18)
<223> inosin

<400> 249
gcgagttcag cucagcgncc gtgc 24

<210> 250
<211> 48
<212> RNA
<213> Artificial Sequence

<220>
<223> CGRP-binding and/or amylin-binding nucleic acid

<220>
<221> misc_feature
<223> laboratory code: NOX-504

<400> 250
ggacugaugg cgcgguccua uuacgccgaa agggagaggg gagacagg 48

<210> 251
<211> 48
<212> RNA
<213> Artificial Sequence

<220>
<223> CGRP-binding and/or amylin-binding nucleic acid

<220>
<221> misc_feature
<223> laboratory code: NOX-504-ad3

<400> 251
ggacugaugg cgcgguccua uuacgccgaa agggagaggg gagcacgg 48

<210> 252
<211> 50
<212> RNA
<213> Artificial Sequence

<220>
<223> CGRP-binding and/or amylin-binding nucleic acid

<400> 252
ggacugaugg cgcgguccua uuacgccgag aagggagugg gggagcacgg 50

<210> 253
<211> 44
<212> RNA
<213> Artificial Sequence

<220>
<223> CGRP-binding and/or amylin-binding nucleic acid

<220>
<221> misc_feature
<223> laboratory code: L097

<400> 253
ggacugaugg cgcgguccua uuacgccgag aagggagugg ggga 44

<210> 254
<211> 43
<212> RNA
<213> Artificial Sequence

<220>
<223> CGRP-binding and/or amylin-binding nucleic acid

<220>
<221> misc_feature
<223> laboratory code: L102

<400> 254
ggacugaugg cgcgguccua uuacgccgag aagggagagg gga 43

<210> 255
<211> 43
<212> RNA
<213> Artificial Sequence

<220>
<223> CGRP-binding and/or amylin-binding nucleic acid

<220>
<221> misc_feature
<223> laboratory code: L103

<400> 255
ggacugaugg cgcgguccua uuacgccgaa agggagaggg gga 43

<210> 256
<211> 42
<212> RNA
<213> Artificial Sequence

<220>

<220>
<221> misc_feature
<223> laboratory code: L104

<400> 256
ggacugaugg cgcgguccua uuacgccgaa agggaguggg ga 42

<210> 257
<211> 43
<212> RNA
<213> Artificial Sequence

<220>
<223> CGRP-binding and/or amylin-binding nucleic acid

<220>
<221> misc_feature
<223> laboratory code: L105

<400> 257
ggacugaugg cgcgguccua uuacgccgaa agggaguggg gga 43

<210> 258
<211> 43
<212> RNA
<213> Artificial Sequence

<220>
<223> CGRP-binding and/or amylin-binding nucleic acid

<220>
<221> misc_feature
<223> laboratory code: L106

<400> 258
ggacugaugg cgcgguccua uuacgccgag aagggagugg gga 43

<210> 259
<211> 44
<212> RNA
<213> Artificial Sequence

<220>
<223> CGRP-binding and/or amylin-binding nucleic acid

<220>
<221> misc_feature
<223> laboratory code: L107

<400> 259
ggacugaugg cgcgguccua uuacgccgag aagggagagg ggga 44

<210> 260
<211> 44
<212> RNA
<213> Artificial Sequence

<220>
<223> CGRP-binding and/or amylin-binding nucleic acid

<220>
<221> misc_feature
<223> laboratory code: L108

<400> 260
ggacugaugg cgcgguccua uuacgccgag uagggagugg ggga 44

<210> 261
<211> 44
<212> RNA
<213> Artificial Sequence

<220>
<223> CGRP-binding and/or amylin-binding nucleic acid

<220>
<221> misc_feature
<223> laboratory code: L109

<400> 261
ggacugaugg cgcgguccua uuacgccgag aagggugugg ggga 44

<210> 262
<211> 42
<212> RNA
<213> Artificial Sequence

<220>
<223> CGRP-binding and/or amylin-binding nucleic acid

<220>
<221> misc_feature
<223> laboratory code: NOX-504-095

<400> 262
ggacugaugg cgcgguccua uuacgccgag agggaguggg ga 42

<210> 263
<211> 42
<212> RNA
<213> Artificial Sequence

<220>
<223> CGRP-binding and/or amylin-binding nucleic acid

<220>
<221> misc_feature
<223> laboratory code: NOX-504-096

<400> 263

ggacugaugg cgcgguccua uuacgccgau agggugug gg ga 42